(19) Europäisches Patentamt / European Patent Office / Office européen des brevets

(11) EP 3 960 876 A1

(12) EUROPEAN PATENT APPLICATION

(43) Date of publication: **02.03.2022 Bulletin 2022/09**

(21) Application number: **20193628.3**

(22) Date of filing: **31.08.2020**

(51) International Patent Classification (IPC): ***C12Q 1/6886*** *(2018.01)*

(52) Cooperative Patent Classification (CPC): **C12Q 1/6886;** C12Q 2600/106; C12Q 2600/118; C12Q 2600/158

(84) Designated Contracting States: **AL AT BE BG CH CY CZ DE DK EE ES FI FR GB GR HR HU IE IS IT LI LT LU LV MC MK MT NL NO PL PT RO RS SE SI SK SM TR**
Designated Extension States: **BA ME**
Designated Validation States: **KH MA MD TN**

(71) Applicant: **Koninklijke Philips N.V. 5656 AG Eindhoven (NL)**

(72) Inventors:
- **HOFFMANN, Ralf Dieter 5656 AE Eindhoven (NL)**
- **STOFFELS, Monique 5656 AE Eindhoven (NL)**

(74) Representative: **Philips Intellectual Property & Standards High Tech Campus 52 5656 AG Eindhoven (NL)**

(54) **PREDICTION OF A RESPONSE OF A PROSTATE CANCER SUBJECT TO RADIOTHERAPY BASED ON PDE4D7 CORRELATED GENES**

(57) The invention relates to a method of predicting a response of a prostate cancer subject to radiotherapy, comprising determining or receiving the result of a determination of a gene expression profile for each of one or more PDE4D7 correlated genes selected from the group consisting of: ABCC5, CUX2, KIAA1549, PDE4D, RAP1GAP2, SLC39A11, TDRD1, and VWA2, said gene expression profile(s) being determined in a biological sample obtained from the subject, and determining the prediction of the radiotherapy response based on the gene expression profile(s) for the one or more PDE4D7 correlated genes.

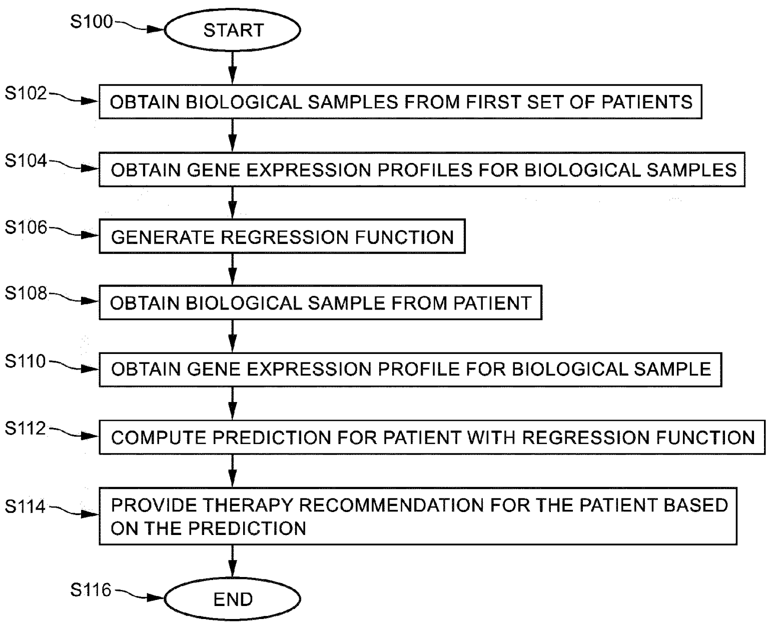


FIG. 1

EP 3 960 876 A1

SEQUENCE LISTING

<110> Koninklijke Philips N.V.

<120> Prediction of a response of a prostate cancer subject to radiotherapy based on PDE4D7 correlated genes

<130> 2020PF00491

<160> 42

<170> PatentIn version 3.5

<210> 1
<211> 2009
<212> DNA
<213> Homo sapiens

<400> 1
gggaatgctt tgtgcagcgc gcttgcgcgg tgtggcggcc gatgccgcta taaaggcttg 60

ttttgctgca gggctcatgc tcgggagcgt ggttgagcgg ctggcgcggt tgtcctggag 120

caggggcgca ggaattctga tgtgaaacta acagtctgtg agccctggaa cctccactca 180

gagaagatga aggatatcga cataggaaaa gagtatatca tccccagtcc tgggtataga 240

agtgtgaggg agagaaccag cacttctggg acgcacagag accgtgaaga ttccaagttc 300

aggagaactc gaccgttgga atgccaagat gccttggaaa cagcagcccg agccgagggc 360

ctctctcttg atgcctccat gcattctcag ctcagaatcc tggatgagga gcatcccaag 420

ggaaagtacc atcatggctt gagtgctctg aagcccatcc ggactacttc caaacaccag 480

cacccagtgg acaatgctgg gctttttcc tgtatgactt tttcgtggct ttcttctctg 540

gcccgtgtgg cccacaagaa gggggagctc tcaatggaag acgtgtggtc tctgtccaag 600

cacgagtctt ctgacgtgaa ctgcagaaga ctagagagac tgtggcaaga agagctgaat 660

gaagttgggc cagacgctgc ttccctgcga agggttgtgt ggatcttctg ccgcaccagg 720

ctcatcctgt ccatcgtgtg cctgatgatc acgcagctgg ctggcttcag tggaccaaat 780

tttcaggatg gctgtattct gcggtcagaa tgagagagtc aagctgggca gaatctctcg 840

ccaagagttc agccttcctt tggagactgc tccatcagtg ccgaggtgtg tgggaacagg 900

cttcactgca ccgccatctt actgagttgc ttcacgtgag gaaaaggggg ctttggccct 960

gtgactcagt tccacatttt ggattgcata ctggaaaaga agccaatctt cttgctagta 1020

aaccagcaac ccggctgtat acagtggtga cccaagcaat ggatataaac ctaaaaatct 1080

gagggagggg agaggtggaa tacagtagtt cttggaatct gaagtctcct atttgatcag 1140

gttatttcct gggacttggc aaaaatctga ttggtgggga tctcctagga cctagtggac 1200

atctggtatt aatttaatct caggaaaaac aagaaattaa cccagagaga gtctgggttt 1260

tggaattcag cgtagctacc tccagaccgt ggtgtctggc ctccattttt gtctgtcatt 1320

```
cagctctgac ttacagctgc agtcaccttt gctataaggc acctgggtag aagggtggat    1380
gggcttcaca tcaatttttt tcttccttta gggtggggga ttggtttggc tttcttttgt    1440
tgtggttttt tgttttattt ttgtcaagat tgatttttag atgcaaggac ttgaaaagac    1500
ccagaaggat gccaccagtt tttccttgag gcctaggatt ttttattctg tcccgagcag    1560
aggtaattcc tcacaactta gtgcaccagt agcaccagcc attttgagca gagtacctct    1620
ttggggagct tttcgttttg ttttgttttt aattctcttt ccttagcagc aaggtctttt    1680
ttcctagaga atctactccg ttgcagaatc attgcaacct caggagccct cactgattga    1740
gtgctgtcag cctgatatac tactttggac tctggaaaca gatatgggtt ctattctcta    1800
tttctactgt gtgtcgttaa acaaccgtcg gagaccagat gacctgttag atggctagtc    1860
ctgtataact cgactctgta tgtttcaatg tatgttactg caatgcttca cctgctgtac    1920
agtgtttgtg agatgctctt tgaagatggt acttttatat tttttcattt tcaataaaag    1980
tacattcctt cccacaaaaa aaaaaaaaa                                      2009
```

```
<210>  2
<211>  5872
<212>  DNA
<213>  Homo sapiens

<400>  2
gggaatgctt tgtgcagcgc gcttgcgcgg tgtggcggcc gatgccgcta taaaggcttg      60
ttttgctgca gggctcatgc tcgggagcgt ggttgagcgg ctggcgcggt tgtcctggag     120
caggggcgca ggaattctga tgtgaaacta acagtctgtg agccctggaa cctccactca     180
gagaagatga aggatatcga cataggaaaa gagtatatca tccccagtcc tgggtataga     240
agtgtgaggg agagaaccag cacttctggg acgcacagag accgtgaaga ttccaagttc     300
aggagaactc gaccgttgga atgccaagat gccttggaaa cagcagcccg agccgagggc     360
ctctctcttg atgcctccat gcattctcag ctcagaatcc tggatgagga gcatcccaag     420
ggaaagtacc atcatggctt gagtgctctg aagcccatcc ggactacttc caaacaccag     480
cacccagtgg acaatgctgg gctttttttcc tgtatgactt tttcgtggct ttcttctctg     540
gcccgtgtgg cccacaagaa gggggagctc tcaatggaag acgtgtggtc tctgtccaag     600
cacgagtctt ctgacgtgaa ctgcagaaga ctagagagac tgtggcaaga agagctgaat     660
gaagttgggc cagacgctgc ttccctgcga agggttgtgt ggatcttctg ccgcaccagg     720
ctcatcctgt ccatcgtgtg cctgatgatc acgcagctgg ctggcttcag tggaccagcc     780
ttcatggtga aacacctctt ggagtatacc caggcaacag agtctaacct gcagtacagc     840
ttgttgttag tgctgggcct cctcctgacg gaaatcgtgc ggtcttggtc gcttgcactg     900
acttgggcat tgaattaccg aaccggtgtc cgcttgcggg gggccatcct aaccatggca     960
```

tttaagaaga tccttaagtt aaagaacatt aaagagaaat ccctgggtga gctcatcaac 1020
atttgctcca acgatgggca gagaatgttt gaggcagcag ccgttggcag cctgctggct 1080
ggaggacccg ttgttgccat cttaggcatg atttataatg taattattct gggaccaaca 1140
ggcttcctgg gatcagctgt ttttatcctc ttttacccag caatgatgtt tgcatcacgg 1200
ctcacagcat atttcaggag aaaatgcgtg gccgccacgg atgaacgtgt ccagaagatg 1260
aatgaagttc ttacttacat taaatttatc aaaatgtatg cctgggtcaa agcattttct 1320
cagagtgttc aaaaaatccg cgaggaggag cgtcggatat tggaaaaagc tgggtacttc 1380
cagagcatca ctgtgggtgt ggctcccatt gtggtggtga ttgccagcgt ggtgaccttc 1440
tctgttcata tgaccctggg cttcgatctg acagcagcac aggctttcac agtggtgaca 1500
gtcttcaatt ccatgacttt tgctttgaaa gtaacaccgt tttcagtaaa gtccctctca 1560
gaagcctcag tggctgttga cagatttaag agtttgtttc taatggaaga ggttcacatg 1620
ataaagaaca aaccagccag tcctcacatc aagatagaga tgaaaaatgc caccttggca 1680
tgggactcct cccactccag tatccagaac tcgcccaagc tgacccccaa aatgaaaaaa 1740
gacaagaggg cttccagggg caagaaagag aaggtgaggc agctgcagcg cactgagcat 1800
caggcggtgc tggcagagca gaaaggccac ctcctcctgg acagtgacga gcggcccagt 1860
cccgaagagg aagaaggcaa gcacatccac ctgggccacc tgcgcttaca gaggacactg 1920
cacagcatcg atctggagat ccaagagggt aaactggttg gaatctgtgg cagtgtggga 1980
agtggaaaaa cctctctcat ttcagccatt ttaggccaga tgacgcttct agagggcagc 2040
attgcaatca gtggaacctt cgcttatgtg gcccagcagg cctggatcct caatgctact 2100
ctgagagaca acatcctgtt tgggaaggaa tatgatgaag aaagatacaa ctctgtgctg 2160
aacagctgct gcctgaggcc tgacctggcc attcttccca gcagcgacct gacggagatt 2220
ggagagcgag gagccaacct gagcggtggg cagcgccaga ggatcagcct tgcccgggcc 2280
ttgtatagtg acaggagcat ctacatcctg gacgaccccc tcagtgcctt agatgcccat 2340
gtgggcaacc acatcttcaa tagtgctatc cggaaacatc tcaagtccaa gacagttctg 2400
tttgttaccc accagttaca gtacctggtt gactgtgatg aagtgatctt catgaaagag 2460
ggctgtatta cggaaagagg cacccatgag gaactgatga atttaaatgg tgactatgct 2520
accattttta ataacctgtt gctgggagag acaccgccag ttgagatcaa ttcaaaaaag 2580
gaaaccagtg gttcacagaa gaagtcacaa gacaagggtc ctaaaacagg atcagtaaag 2640
aaggaaaaag cagtaaagcc agaggaaggg cagcttgtgc agctggaaga gaaagggcag 2700
ggttcagtgc cctggtcagt atatggtgtc tacatccagg ctgctggggg ccccttggca 2760
ttcctggtta ttatggccct tttcatgctg aatgtaggca gcaccgcctt cagcacctgg 2820
tggttgagtt actggatcaa gcaaggaagc gggaacacca ctgtgactcg agggaacgag 2880

acctcggtga gtgacagcat gaaggacaat cctcatatgc agtactatgc cagcatctac 2940
gccctctcca tggcagtcat gctgatcctg aaagccattc gaggagttgt ctttgtcaag 3000
ggcacgctgc gagcttcctc ccggctgcat gacgagcttt tccgaaggat ccttcgaagc 3060
cctatgaagt tttttgacac gacccccaca gggaggattc tcaacaggtt ttccaaagac 3120
atggatgaag ttgacgtgcg gctgccgttc caggccgaga tgttcatcca gaacgttatc 3180
ctggtgttct tctgtgtggg aatgatcgca ggagtcttcc cgtggttcct tgtggcagtg 3240
gggccccttg tcatcctctt ttcagtcctg cacattgtct ccagggtcct gattcgggag 3300
ctgaagcgtc tggacaatat cacgcagtca cctttcctct cccacatcac gtccagcata 3360
cagggccttg ccaccatcca cgcctacaat aaagggcagg agtttctgca cagataccag 3420
gagctgctgg atgacaacca agctcctttt tttttgttta cgtgtgcgat gcggtggctg 3480
gctgtgcggc tggacctcat cagcatcgcc ctcatcacca ccacggggct gatgatcgtt 3540
cttatgcacg ggcagattcc cccagcctat gcgggtctcg ccatctctta tgctgtccag 3600
ttaacggggc tgttccagtt tacggtcaga ctggcatctg agacagaagc tcgattcacc 3660
tcggtggaga ggatcaatca ctacattaag actctgtcct tggaagcacc tgccagaatt 3720
aagaacaagg ctccctcccc tgactggccc caggagggag aggtgacctt tgagaacgca 3780
gagatgaggt accgagaaaa cctccctctc gtcctaaaga aagtatcctt cacgatcaaa 3840
cctaaagaga agattggcat tgtggggcgg acaggatcag ggaagtcctc gctggggatg 3900
gccctcttcc gtctggtgga gttatctgga ggctgcatca agattgatgg agtgagaatc 3960
agtgatattg gccttgccga cctccgaagc aaactctcta tcattcctca agagccggtg 4020
ctgttcagtg gcactgtcag atcaaatttg gaccccttca accagtacac tgaagaccag 4080
atttgggatg ccctggagag gacacacatg aaagaatgta ttgctcagct acctctgaaa 4140
cttgaatctg aagtgatgga gaatggggat aacttctcag tgggggaacg gcagctcttg 4200
tgcatagcta gagccctgct ccgccactgt aagattctga ttttagatga agccacagct 4260
gccatggaca cagagacaga cttattgatt caagagacca tccgagaagc atttgcagac 4320
tgtaccatgc tgaccattgc ccatcgcctg cacacggttc taggctccga taggattatg 4380
gtgctggccc agggacaggt ggtggagttt gacaccccat cggtccttct gtccaacgac 4440
agttcccgat tctatgccat gtttgctgct gcagagaaca aggtcgctgt caagggctga 4500
ctcctccctg ttgacgaagt ctcttttctt tagagcattg ccattccctg cctggggcgg 4560
gcccctcatc gcgtcctcct accgaaacct tgcctttctc gattttatct ttcgcacagc 4620
agttccggat tggcttgtgt gtttcacttt tagggagagt catattttga ttattgtatt 4680
tattccatat tcatgtaaac aaaatttagt tttgttctt aattgcactc taaaaggttc 4740

agggaaccgt tattataatt gtatcagagg cctataatga agctttatac gtgtagctat 4800

atctatatat aattctgtac atagcctata tttacagtga aaatgtaagc tgtttatttt 4860

atattaaaat aagcactgtg ctaataacag tgcatattcc tttctatcat ttttgtacag 4920

tttgctgtac tagagatctg gttttgctat tagactgtag gaagagtagc atttcattct 4980

tctctagctg gtggtttcac ggtgccaggt tttctgggtg tccaaaggaa gacgtgtggc 5040

aatagtgggc cctccgacag ccccctctgc cgcctcccca cggccgctcc aggggtggct 5100

ggagacgggt gggcggctgg agaccatgca gagcgccgtg agttctcagg gctcctgcct 5160

tctgtcctgg tgtcacttac tgtttctgtc aggagagcag cggggcgaag cccaggcccc 5220

ttttcactcc ctccatcaag aatggggatc acagagacat tcctccgagc cggggagttt 5280

ctttcctgcc ttcttctttt tgctgttgtt tctaaacaag aatcagtcta tccacagaga 5340

gtcccactgc ctcaggttcc tatggctggc cactgcacag agctctccag ctccaagacc 5400

tgttggttcc aagccctgga gccaactgct gctttttgag gtggcacttt ttcatttgcc 5460

tattcccaca cctccacagt tcagtggcag ggctcaggat ttcgtgggtc tgttttcctt 5520

tctcaccgca gtcgtcgcac agtctctctc tctctctccc ctcaaagtct gcaactttaa 5580

gcagctcttg ctaatcagtg tctcacactg gcgtagaagt ttttgtactg taaagagacc 5640

tacctcaggt tgctggttgc tgtgtggttt ggtgtgttcc cgcaaacccc ctttgtgctg 5700

tggggctggt agctcaggtg ggcgtggtca ctgctgtcat caattgaatg gtcagcgttg 5760

catgtcgtga ccaactagac attctgtcgc cttagcatgt ttgctgaaca ccttgtggaa 5820

gcaaaaatct gaaaatgtga ataaaattat tttggatttt gtaaaaaaaa aa 5872

<210> 3
<211> 208
<212> PRT
<213> Homo sapiens

<400> 3

Met Lys Asp Ile Asp Ile Gly Lys Glu Tyr Ile Ile Pro Ser Pro Gly
1 5 10 15

Tyr Arg Ser Val Arg Glu Arg Thr Ser Thr Ser Gly Thr His Arg Asp
20 25 30

Arg Glu Asp Ser Lys Phe Arg Arg Thr Arg Pro Leu Glu Cys Gln Asp
35 40 45

Ala Leu Glu Thr Ala Ala Arg Ala Glu Gly Leu Ser Leu Asp Ala Ser
50 55 60

Met His Ser Gln Leu Arg Ile Leu Asp Glu Glu His Pro Lys Gly Lys

65 70 75 80

Tyr His His Gly Leu Ser Ala Leu Lys Pro Ile Arg Thr Thr Ser Lys
85 90 95

His Gln His Pro Val Asp Asn Ala Gly Leu Phe Ser Cys Met Thr Phe
100 105 110

Ser Trp Leu Ser Ser Leu Ala Arg Val Ala His Lys Lys Gly Glu Leu
115 120 125

Ser Met Glu Asp Val Trp Ser Leu Ser Lys His Glu Ser Ser Asp Val
130 135 140

Asn Cys Arg Arg Leu Glu Arg Leu Trp Gln Glu Glu Leu Asn Glu Val
145 150 155 160

Gly Pro Asp Ala Ala Ser Leu Arg Arg Val Val Trp Ile Phe Cys Arg
165 170 175

Thr Arg Leu Ile Leu Ser Ile Val Cys Leu Met Ile Thr Gln Leu Ala
180 185 190

Gly Phe Ser Gly Pro Asn Phe Gln Asp Gly Cys Ile Leu Arg Ser Glu
195 200 205

<210> 4
<211> 1437
<212> PRT
<213> Homo sapiens

<400> 4

Met Lys Asp Ile Asp Ile Gly Lys Glu Tyr Ile Ile Pro Ser Pro Gly
1 5 10 15

Tyr Arg Ser Val Arg Glu Arg Thr Ser Thr Ser Gly Thr His Arg Asp
20 25 30

Arg Glu Asp Ser Lys Phe Arg Arg Thr Arg Pro Leu Glu Cys Gln Asp
35 40 45

Ala Leu Glu Thr Ala Ala Arg Ala Glu Gly Leu Ser Leu Asp Ala Ser
50 55 60

Met His Ser Gln Leu Arg Ile Leu Asp Glu Glu His Pro Lys Gly Lys
65 70 75 80

Tyr His His Gly Leu Ser Ala Leu Lys Pro Ile Arg Thr Thr Ser Lys

85 90 95

His Gln His Pro Val Asp Asn Ala Gly Leu Phe Ser Cys Met Thr Phe
100 105 110

Ser Trp Leu Ser Ser Leu Ala Arg Val Ala His Lys Lys Gly Glu Leu
115 120 125

Ser Met Glu Asp Val Trp Ser Leu Ser Lys His Glu Ser Ser Asp Val
130 135 140

Asn Cys Arg Arg Leu Glu Arg Leu Trp Gln Glu Glu Leu Asn Glu Val
145 150 155 160

Gly Pro Asp Ala Ala Ser Leu Arg Arg Val Val Trp Ile Phe Cys Arg
165 170 175

Thr Arg Leu Ile Leu Ser Ile Val Cys Leu Met Ile Thr Gln Leu Ala
180 185 190

Gly Phe Ser Gly Pro Ala Phe Met Val Lys His Leu Leu Glu Tyr Thr
195 200 205

Gln Ala Thr Glu Ser Asn Leu Gln Tyr Ser Leu Leu Leu Val Leu Gly
210 215 220

Leu Leu Leu Thr Glu Ile Val Arg Ser Trp Ser Leu Ala Leu Thr Trp
225 230 235 240

Ala Leu Asn Tyr Arg Thr Gly Val Arg Leu Arg Gly Ala Ile Leu Thr
245 250 255

Met Ala Phe Lys Lys Ile Leu Lys Leu Lys Asn Ile Lys Glu Lys Ser
260 265 270

Leu Gly Glu Leu Ile Asn Ile Cys Ser Asn Asp Gly Gln Arg Met Phe
275 280 285

Glu Ala Ala Ala Val Gly Ser Leu Leu Ala Gly Gly Pro Val Val Ala
290 295 300

Ile Leu Gly Met Ile Tyr Asn Val Ile Ile Leu Gly Pro Thr Gly Phe
305 310 315 320

Leu Gly Ser Ala Val Phe Ile Leu Phe Tyr Pro Ala Met Met Phe Ala
325 330 335

Ser Arg Leu Thr Ala Tyr Phe Arg Arg Lys Cys Val Ala Ala Thr Asp
340 345 350

Glu Arg Val Gln Lys Met Asn Glu Val Leu Thr Tyr Ile Lys Phe Ile
355 360 365

Lys Met Tyr Ala Trp Val Lys Ala Phe Ser Gln Ser Val Gln Lys Ile
370 375 380

Arg Glu Glu Glu Arg Arg Ile Leu Glu Lys Ala Gly Tyr Phe Gln Ser
385 390 395 400

Ile Thr Val Gly Val Ala Pro Ile Val Val Val Ile Ala Ser Val Val
405 410 415

Thr Phe Ser Val His Met Thr Leu Gly Phe Asp Leu Thr Ala Ala Gln
420 425 430

Ala Phe Thr Val Val Thr Val Phe Asn Ser Met Thr Phe Ala Leu Lys
435 440 445

Val Thr Pro Phe Ser Val Lys Ser Leu Ser Glu Ala Ser Val Ala Val
450 455 460

Asp Arg Phe Lys Ser Leu Phe Leu Met Glu Glu Val His Met Ile Lys
465 470 475 480

Asn Lys Pro Ala Ser Pro His Ile Lys Ile Glu Met Lys Asn Ala Thr
485 490 495

Leu Ala Trp Asp Ser Ser His Ser Ser Ile Gln Asn Ser Pro Lys Leu
500 505 510

Thr Pro Lys Met Lys Lys Asp Lys Arg Ala Ser Arg Gly Lys Lys Glu
515 520 525

Lys Val Arg Gln Leu Gln Arg Thr Glu His Gln Ala Val Leu Ala Glu
530 535 540

Gln Lys Gly His Leu Leu Leu Asp Ser Asp Glu Arg Pro Ser Pro Glu
545 550 555 560

Glu Glu Glu Gly Lys His Ile His Leu Gly His Leu Arg Leu Gln Arg
565 570 575

Thr Leu His Ser Ile Asp Leu Glu Ile Gln Glu Gly Lys Leu Val Gly
580 585 590

Ile Cys Gly Ser Val Gly Ser Gly Lys Thr Ser Leu Ile Ser Ala Ile
595 600 605

Leu Gly Gln Met Thr Leu Leu Glu Gly Ser Ile Ala Ile Ser Gly Thr
610 615 620

Phe Ala Tyr Val Ala Gln Gln Ala Trp Ile Leu Asn Ala Thr Leu Arg
625 630 635 640

Asp Asn Ile Leu Phe Gly Lys Glu Tyr Asp Glu Glu Arg Tyr Asn Ser
645 650 655

Val Leu Asn Ser Cys Cys Leu Arg Pro Asp Leu Ala Ile Leu Pro Ser
660 665 670

Ser Asp Leu Thr Glu Ile Gly Glu Arg Gly Ala Asn Leu Ser Gly Gly
675 680 685

Gln Arg Gln Arg Ile Ser Leu Ala Arg Ala Leu Tyr Ser Asp Arg Ser
690 695 700

Ile Tyr Ile Leu Asp Asp Pro Leu Ser Ala Leu Asp Ala His Val Gly
705 710 715 720

Asn His Ile Phe Asn Ser Ala Ile Arg Lys His Leu Lys Ser Lys Thr
725 730 735

Val Leu Phe Val Thr His Gln Leu Gln Tyr Leu Val Asp Cys Asp Glu
740 745 750

Val Ile Phe Met Lys Glu Gly Cys Ile Thr Glu Arg Gly Thr His Glu
755 760 765

Glu Leu Met Asn Leu Asn Gly Asp Tyr Ala Thr Ile Phe Asn Asn Leu
770 775 780

Leu Leu Gly Glu Thr Pro Pro Val Glu Ile Asn Ser Lys Lys Glu Thr
785 790 795 800

Ser Gly Ser Gln Lys Lys Ser Gln Asp Lys Gly Pro Lys Thr Gly Ser
805 810 815

Val Lys Lys Glu Lys Ala Val Lys Pro Glu Glu Gly Gln Leu Val Gln
820 825 830

Leu Glu Glu Lys Gly Gln Gly Ser Val Pro Trp Ser Val Tyr Gly Val
835 840 845

Tyr Ile Gln Ala Ala Gly Gly Pro Leu Ala Phe Leu Val Ile Met Ala
850 855 860

Leu Phe Met Leu Asn Val Gly Ser Thr Ala Phe Ser Thr Trp Trp Leu
865 870 875 880

Ser Tyr Trp Ile Lys Gln Gly Ser Gly Asn Thr Thr Val Thr Arg Gly
885 890 895

Asn Glu Thr Ser Val Ser Asp Ser Met Lys Asp Asn Pro His Met Gln
900 905 910

Tyr Tyr Ala Ser Ile Tyr Ala Leu Ser Met Ala Val Met Leu Ile Leu
915 920 925

Lys Ala Ile Arg Gly Val Val Phe Val Lys Gly Thr Leu Arg Ala Ser
930 935 940

Ser Arg Leu His Asp Glu Leu Phe Arg Arg Ile Leu Arg Ser Pro Met
945 950 955 960

Lys Phe Phe Asp Thr Thr Pro Thr Gly Arg Ile Leu Asn Arg Phe Ser
965 970 975

Lys Asp Met Asp Glu Val Asp Val Arg Leu Pro Phe Gln Ala Glu Met
980 985 990

Phe Ile Gln Asn Val Ile Leu Val Phe Phe Cys Val Gly Met Ile Ala
995 1000 1005

Gly Val Phe Pro Trp Phe Leu Val Ala Val Gly Pro Leu Val Ile
1010 1015 1020

Leu Phe Ser Val Leu His Ile Val Ser Arg Val Leu Ile Arg Glu
1025 1030 1035

Leu Lys Arg Leu Asp Asn Ile Thr Gln Ser Pro Phe Leu Ser His
1040 1045 1050

Ile Thr Ser Ser Ile Gln Gly Leu Ala Thr Ile His Ala Tyr Asn
1055 1060 1065

Lys Gly Gln Glu Phe Leu His Arg Tyr Gln Glu Leu Leu Asp Asp
1070 1075 1080

Asn Gln Ala Pro Phe Phe Leu Phe Thr Cys Ala Met Arg Trp Leu

1085 1090 1095

Ala Val Arg Leu Asp Leu Ile Ser Ile Ala Leu Ile Thr Thr Thr
1100 1105 1110

Gly Leu Met Ile Val Leu Met His Gly Gln Ile Pro Pro Ala Tyr
1115 1120 1125

Ala Gly Leu Ala Ile Ser Tyr Ala Val Gln Leu Thr Gly Leu Phe
1130 1135 1140

Gln Phe Thr Val Arg Leu Ala Ser Glu Thr Glu Ala Arg Phe Thr
1145 1150 1155

Ser Val Glu Arg Ile Asn His Tyr Ile Lys Thr Leu Ser Leu Glu
1160 1165 1170

Ala Pro Ala Arg Ile Lys Asn Lys Ala Pro Ser Pro Asp Trp Pro
1175 1180 1185

Gln Glu Gly Glu Val Thr Phe Glu Asn Ala Glu Met Arg Tyr Arg
1190 1195 1200

Glu Asn Leu Pro Leu Val Leu Lys Lys Val Ser Phe Thr Ile Lys
1205 1210 1215

Pro Lys Glu Lys Ile Gly Ile Val Gly Arg Thr Gly Ser Gly Lys
1220 1225 1230

Ser Ser Leu Gly Met Ala Leu Phe Arg Leu Val Glu Leu Ser Gly
1235 1240 1245

Gly Cys Ile Lys Ile Asp Gly Val Arg Ile Ser Asp Ile Gly Leu
1250 1255 1260

Ala Asp Leu Arg Ser Lys Leu Ser Ile Ile Pro Gln Glu Pro Val
1265 1270 1275

Leu Phe Ser Gly Thr Val Arg Ser Asn Leu Asp Pro Phe Asn Gln
1280 1285 1290

Tyr Thr Glu Asp Gln Ile Trp Asp Ala Leu Glu Arg Thr His Met
1295 1300 1305

Lys Glu Cys Ile Ala Gln Leu Pro Leu Lys Leu Glu Ser Glu Val
1310 1315 1320

```
Met Glu Asn Gly Asp Asn Phe Ser Val Gly Glu Arg Gln Leu Leu
    1325                1330                1335

Cys Ile Ala Arg Ala Leu Leu Arg His Cys Lys Ile Leu Ile Leu
    1340                1345                1350

Asp Glu Ala Thr Ala Ala Met Asp Thr Glu Thr Asp Leu Leu Ile
    1355                1360                1365

Gln Glu Thr Ile Arg Glu Ala Phe Ala Asp Cys Thr Met Leu Thr
    1370                1375                1380

Ile Ala His Arg Leu His Thr Val Leu Gly Ser Asp Arg Ile Met
    1385                1390                1395

Val Leu Ala Gln Gly Gln Val Val Glu Phe Asp Thr Pro Ser Val
    1400                1405                1410

Leu Leu Ser Asn Asp Ser Ser Arg Phe Tyr Ala Met Phe Ala Ala
    1415                1420                1425

Ala Glu Asn Lys Val Ala Val Lys Gly
    1430                1435

<210>  5
<211>  6855
<212>  DNA
<213>  Homo sapiens

<400>  5
ggccggaggg cgcccgaggg gccccgggcc gcggcgctca gggcccgggc ggccggcggc     60

ggccccgggg ctgggggag tccagcccgg atattgagtg cagccattga gaaaagccaa     120

actcttgtgt gtgcgcgtct cgatagcccc caagatggcc gccaatgtgg gatcgatgtt    180

tcaatattgg aagcgatttg atctacggcg actccagaag gagcttaatt ccgtcgcttc    240

tgagctgtct gcacggcagg aggagagtga acattctcat aaacatttaa ttgaactccg    300

ccgggaattt aagaaaaatg tacctgagga aatcagagag atggtggctc ctgtattaaa    360

aagcttccaa gccgaggtgg tggcccttag taagagaagt caggaggcgg aggctgcttt    420

tctgagtgtt tacaagcaat taattgaagc accagacccc gtgcctgtgt ttgaggcggc    480

acgcagccta gacgacagac tgcagccccc cagctttgac cccagtgggc agccccggcg    540

agacctccac acttcgtgga agaggaaccc cgagctcctc agccccaaag agcagagaga    600

ggggacgtcg cctgccgggc ccacgctgac cgagggaagc cgcctcccag gcattcccgg    660

gaaagccctc ctgacagaaa ccttgctgca gagaaatgag gcggaaaaac aaaagggcct    720

tcaagaagta cagatcactt tggcggccag actgggggag gcagaggaga aaatcaaagt    780
```

```
cctacattca gcgctaaagg ctacgcaggc agagctgcta gagctgcggc ggaagtacga      840
cgaggaggca gcatccaagg cagatgaagt cggcctgatc atgaccaacc tggagaaagc      900
taatcagcga gctgaggctg cccagcggga ggtggaaagt ctccgggaac agctggcctc      960
tgtcaacagc tccatccgcc tggcttgctg ctctccccag gggcccagtg gggataaggt     1020
gaacttcact ctgtgctcgg gccctcggct ggaggccgcg ctggcctcca aggacaggga     1080
gatcctgcgg ctgctgaagg acgtgcagca cctccagagc tcactgcagg agctggagga     1140
ggcatccgcc aaccagatcg ccgacctgga gcggcagctc acggccaagt ccgaggccat     1200
agaaaagctg gaagagaagc tccaggccca gtctgactat gaggaaatta aaacggagct     1260
gagcatcctg aaagccatga agctggcctc cagcacctgc agcctccccc agggcatggc     1320
caagcctgaa gactcactgc ttattgcaaa ggaggccttc ttccccacgc agaaattcct     1380
tctggagaag cccagcctcc tggccagccc tgaggaagac ccatcagagg acgattccat     1440
caaggattca ctgggcacgg agcagtccta cccctcccct cagcagctcc cacctccacc     1500
agggccagaa gacccccctgt ctcccagccc cgggcagccc ctgctgggcc ccagcttggg     1560
gcctgacggc actcggactt tctcgctgtc cccttcccc agcctggcat caggggagag     1620
actgatgatg cccccagccg ccttcaaggg agaggcgggc ggcctgctgg tgttcccccc     1680
agccttctat ggcgccaagc cccccacagc ccctgccacc ccggcccctg gccctgagcc     1740
actgggcggt cctgagcccg cggatggtgg tgggggcgga gcggcggggc ccggggcaga     1800
ggaggagcag ctggacacgg cagagatcgc cttccaggtg aaggagcagc tgctgaaaca     1860
caacatcggg cagcgggtgt ttgggcatta cgtgctgggg ctgtcgcagg gctcggtcag     1920
cgagatccta gcccggccca agccctggcg caagctcacg gtgaagggca aggagccctt     1980
catcaagatg aagcagttcc tgtcggatga gcagaatgta ctggcgctca ggaccatcca     2040
agtgcggcag cgaggcagca tcaccccgag aatccgcacg cctgagacag gctcagacga     2100
cgccatcaag agcattctag agcaggccaa gaaggagatc gagtcgcaga agggcggcga     2160
gcccaagacc tcggtggccc cgctgagcat cgccaacggc acgacccccg ccagcacctc     2220
ggaggacgcc atcaagagca tcctggagca ggcacgccgt gagatgcagg cgcaacagca     2280
ggcgctgctg gagatggagg tggcgcccag ggccgctcg gtgcccccct cgcccccgga     2340
gcggccatca ctggccaccg cgagccagaa cggggccccg gccttggtga agcaggagga     2400
gggcagcggg ggccccgcgc aggcgccgct cccggtcctg tcccccgccg ccttcgtgca     2460
gagcatcatc cgcaaggtca agtccgagat cggcgacgcc ggctacttcg accaccactg     2520
ggcctccgac cgcggcctgc tcagccgccc ctacgcctcc gtgtcgccct cgctgtcctc     2580
ctcctcctcc tctggctact ctggccagcc caacggccgc gcctggcccc gcggggacga     2640
```

ggcccctgtg ccccccgagg acgaggcggc ggcaggggcg gaggacgaac cccccaggac 2700

gggcgagctc aaggctgagg gcgcgacggc cgaggcgggc gcgcggctgc cctactaccc 2760

ggcctacgtg ccgcgcaccc tgaagcccac cgtgccgccg ctgacccccg agcagtacga 2820

gctgtacatg taccgtgagg tagacacgct ggagctcacc cgccaggtca aggagaagct 2880

ggccaagaac ggcatctgcc agaggatctt cggggagaag gtgctgggcc tgtcacaggg 2940

cagcgtgagc gacatgctgt cccggccgaa gccatggagc aagctgacgc agaaggggcg 3000

ggagcccttc atccgcatgc agctgtggct ctctgaccag ctcggccagg cagtgggcca 3060

gcagcctggt gcctcccagg ccagtcccac agaaccaagg tcctcaccat ccccaccccc 3120

cagccccaca gagcctgaga agagctccca ggagccgttg agcctgtccc tggagagcag 3180

caaggagaac cagcagccag agggccgctc cagctcctcg ttgagcggga agatgtactc 3240

aggcagccag gccccagggg gcatccagga gatcgtggcc atgtcccccg agctggacac 3300

gtactccatc accaagaggg tgaaggaggt cctcacagac aacaatctag ggcagcggct 3360

gtttggggaa agcatcctgg gtctgacaca gggctccgtg tctgacctgc tgtcccggcc 3420

caaaccctgg cacaagctga gcctgaaggg gcgggagcct tttgtccgca tgcagctgtg 3480

gctcaatgac ccccataacg tggagaagct gagggatatg aagaagctgg agaagaaagc 3540

ctacctgaaa cgtcgctatg gcctcatcag caccggctca gacagtgagt ccccggccac 3600

ccgctcagag tgccccagcc cctgcctgca gccccaggac ctgagcctcc tgcagatcaa 3660

gaagccccgg gtggtgctgg cacccgagga gaaggaggca ctgcggaagg cctatcagct 3720

ggaaccctac ccctcgcagc agaccatcga gctcctctcc ttccagctca acctcaagac 3780

caacaccgtc atcaactggt tccacaacta caggtcccgg atgcgccggg agatgttggt 3840

ggaggggacc caggatgagc cagaccttga tccaagcggg ggtcctggaa tcctaccgcc 3900

aggccactcc cacccagacc ccacccccgca gagccctgac tctgagactg aggaccagaa 3960

gccaaccgtg aaggaactgg agcttcagga gggccctgag gagaacagca cacccctgac 4020

cacccaggac aaggcccaag tgaggatcaa gcaggaacag atggaggagg atgctgagga 4080

agaggcaggc agccagcccc aggactcagg ggagctggac aaaggccaag gtccccccaa 4140

agaggagcat cccgaccctc cgggtaatga tggactccca aaagtggctc ccgggcccct 4200

ccttccaggt ggatccaccc cagactgtcc ctcacttcat ccccaacagg agagtgaggc 4260

cggggagcga cttcacccgg accctttaag ttttaagtca gcctcagagt cctcacgctg 4320

cagcctggag gtgtcactga actcgccctc ggccgcctcc tcaccaggcc tcatgatgtc 4380

tgtgtcacct gtcccctcct cctcagctcc catctcccca tccccacctg gcgccccccc 4440

tgccaaagtg ccgagtgcca gccccactgc tgacatggct ggagccttgc accccagtgc 4500

caaggtgaac cccaacttgc agcggcggca tgagaagatg gccaatctga acaacatcat 4560

ttaccgagta gagcgggctg ccaatcggga ggaggccctg gagtgggagt tctgaaggca 4620
gggtgagggg gcaagggaca taccctggta actaccttcc ttctcgcact tactctcctc 4680
aacaggatgg ggtaagggag ggaggaactc aaccatcaaa atgtggacag caatgttatg 4740
ccgtttacgt tttttgttgt aatcctagtt ctatgaagct gtgtgagcag gtgggtcaaa 4800
tgccattgcc tccacttttc tgcacccccc tgctcctctt caccctgacc cctctgcagg 4860
aggcagaagc aaaatggcac cacatattca cctgaaaact ccaaactctt ttagaaaaat 4920
aaataaatat ttatagacct cttttagata ttttaataaa ggatcctttg gaatttatcc 4980
cagctgatgc tgttttgata ttacagagag ttataaaatc aggatgctgt cacaactgtt 5040
gcgaagtata cactgaagtt gtgtcgtttt tgccactaga tgagattaaa agaagacaat 5100
tattcaaagc catcacaaaa cactataaga ctgaccaaaa tttagataac ctttgaacca 5160
cgattttttt ccacatctgt ctgtgagaca cagcgcaatg ctactgccct tccagaaact 5220
gtgctaaaaa gagaaagtcc aaaagactct aaacaaaaac ctcgacgccg ttgaggatgt 5280
gtttcattct ggtggtctgt tttgcaagct tgataacaga atgtccgtgc cattgtaaat 5340
gttgtagaga tgtgggccgt ggcccaaccg tcctatatga gatgtagcat ggtacagaac 5400
aaactgctta cacaggtctc actagttaga aacctgtggg ccatggaggt cagacatcca 5460
tcttgtccat ctataggcaa gaagtgtttc cagatccttt ggaaaggtgg gcatggggca 5520
ggtgcttgga gagtggcgtt tgagccagag cgacccccatt tcccgtgtga accataggca 5580
caacccagga agtttcccca cttgtaggag tgtgggtatt ccagagcaag actgtggcca 5640
ccatcttccc ctcttggtgt tttccgaaag tgacagtgtt ggtcatccca tgaccactga 5700
agcttagtaa ccagcgccaa aaagtagatt catcaaacta gagaccccag ctccccttct 5760
cgccatcttc tttctcaagt tgaccgtggt gctgtttctg gaaggcatct gcaactccaa 5820
gtccatgcag aactctggaa ggccaagttc atcgcagcat gttcaccata tcccagcctc 5880
caaatctatc ctcctacctt ccaacgcatg acctgttggg gagcagagac ttaaccccca 5940
actcagagga acccttcctc cagcgtcttt ggcatggttt ctagggtgag agttcccaat 6000
ttggatagaa cggccaccat attggttact gaatctctct cccttgtttt tattacgttt 6060
cctttttcaa actgtccatg ggaaggctga attgagtgac tccccagaat gaagatgaga 6120
aggtgaatat aatcaatgcc aatgtaatgc cagcgggtga gatggccgat ggaggtttca 6180
aagatgtagc tagcattttg aaaccatatg ggcaaaaccc ggcaaccaga aggggacaga 6240
taaggaccgt tccagaaatc ccaactctca cacccagccc aggctgcagt ctccacacca 6300
aacagtcaac aaaacacaaa ccctgaagga aaacctttc catacaccca ggctatgcat 6360
tgaagagttt tccactgtat acatttttat ccagatgaag gtatttttat attttgacaa 6420

taggaaacag tgaccatttt cagagtaatc aaatctggaa caaatgaaac atcttttagc 6480

caccaccacc ctgttgcaat taagacaacc gtgggggaac acaccacttt ttactgttga 6540

aaccaacaca acgttgaaat ccaggcttat acgcagactc cgattcctag agaactaaat 6600

ttggctttag tgtgacggga tttgattaag cacttagtat agtcttttga acacggaaat 6660

cctgttgtac ttaaagctag cggacccgtg aacaactttg tcaggttcac gtcctataac 6720

ggttaaaaaa cacacacaca catacacaaa ccgtttctat gagagattga tgaactttgt 6780

ttaaaatttt aaaaaaagga acacgttctg taaacgagtc gctaaataca gaattgtata 6840

ataaaaaaaa aaaaa 6855

<210> 6
<211> 1486
<212> PRT
<213> Homo sapiens

<400> 6

Met Ala Ala Asn Val Gly Ser Met Phe Gln Tyr Trp Lys Arg Phe Asp
1 5 10 15

Leu Arg Arg Leu Gln Lys Glu Leu Asn Ser Val Ala Ser Glu Leu Ser
20 25 30

Ala Arg Gln Glu Glu Ser Glu His Ser His Lys His Leu Ile Glu Leu
35 40 45

Arg Arg Glu Phe Lys Lys Asn Val Pro Glu Glu Ile Arg Glu Met Val
50 55 60

Ala Pro Val Leu Lys Ser Phe Gln Ala Glu Val Val Ala Leu Ser Lys
65 70 75 80

Arg Ser Gln Glu Ala Glu Ala Ala Phe Leu Ser Val Tyr Lys Gln Leu
85 90 95

Ile Glu Ala Pro Asp Pro Val Pro Val Phe Glu Ala Ala Arg Ser Leu
100 105 110

Asp Asp Arg Leu Gln Pro Pro Ser Phe Asp Pro Ser Gly Gln Pro Arg
115 120 125

Arg Asp Leu His Thr Ser Trp Lys Arg Asn Pro Glu Leu Leu Ser Pro
130 135 140

Lys Glu Gln Arg Glu Gly Thr Ser Pro Ala Gly Pro Thr Leu Thr Glu
145 150 155 160

Gly Ser Arg Leu Pro Gly Ile Pro Gly Lys Ala Leu Leu Thr Glu Thr
165 170 175

Leu Leu Gln Arg Asn Glu Ala Glu Lys Gln Lys Gly Leu Gln Glu Val
180 185 190

Gln Ile Thr Leu Ala Ala Arg Leu Gly Glu Ala Glu Glu Lys Ile Lys
195 200 205

Val Leu His Ser Ala Leu Lys Ala Thr Gln Ala Glu Leu Leu Glu Leu
210 215 220

Arg Arg Lys Tyr Asp Glu Glu Ala Ala Ser Lys Ala Asp Glu Val Gly
225 230 235 240

Leu Ile Met Thr Asn Leu Glu Lys Ala Asn Gln Arg Ala Glu Ala Ala
245 250 255

Gln Arg Glu Val Glu Ser Leu Arg Glu Gln Leu Ala Ser Val Asn Ser
260 265 270

Ser Ile Arg Leu Ala Cys Cys Ser Pro Gln Gly Pro Ser Gly Asp Lys
275 280 285

Val Asn Phe Thr Leu Cys Ser Gly Pro Arg Leu Glu Ala Ala Leu Ala
290 295 300

Ser Lys Asp Arg Glu Ile Leu Arg Leu Leu Lys Asp Val Gln His Leu
305 310 315 320

Gln Ser Ser Leu Gln Glu Leu Glu Glu Ala Ser Ala Asn Gln Ile Ala
325 330 335

Asp Leu Glu Arg Gln Leu Thr Ala Lys Ser Glu Ala Ile Glu Lys Leu
340 345 350

Glu Glu Lys Leu Gln Ala Gln Ser Asp Tyr Glu Glu Ile Lys Thr Glu
355 360 365

Leu Ser Ile Leu Lys Ala Met Lys Leu Ala Ser Ser Thr Cys Ser Leu
370 375 380

Pro Gln Gly Met Ala Lys Pro Glu Asp Ser Leu Leu Ile Ala Lys Glu
385 390 395 400

Ala Phe Phe Pro Thr Gln Lys Phe Leu Leu Glu Lys Pro Ser Leu Leu
405 410 415

Ala Ser Pro Glu Glu Asp Pro Ser Glu Asp Asp Ser Ile Lys Asp Ser
420 425 430

Leu Gly Thr Glu Gln Ser Tyr Pro Ser Pro Gln Gln Leu Pro Pro Pro
435 440 445

Pro Gly Pro Glu Asp Pro Leu Ser Pro Ser Pro Gly Gln Pro Leu Leu
450 455 460

Gly Pro Ser Leu Gly Pro Asp Gly Thr Arg Thr Phe Ser Leu Ser Pro
465 470 475 480

Phe Pro Ser Leu Ala Ser Gly Glu Arg Leu Met Met Pro Pro Ala Ala
485 490 495

Phe Lys Gly Glu Ala Gly Gly Leu Leu Val Phe Pro Pro Ala Phe Tyr
500 505 510

Gly Ala Lys Pro Pro Thr Ala Pro Ala Thr Pro Ala Pro Gly Pro Glu
515 520 525

Pro Leu Gly Gly Pro Glu Pro Ala Asp Gly Gly Gly Gly Gly Ala Ala
530 535 540

Gly Pro Gly Ala Glu Glu Glu Gln Leu Asp Thr Ala Glu Ile Ala Phe
545 550 555 560

Gln Val Lys Glu Gln Leu Leu Lys His Asn Ile Gly Gln Arg Val Phe
565 570 575

Gly His Tyr Val Leu Gly Leu Ser Gln Gly Ser Val Ser Glu Ile Leu
580 585 590

Ala Arg Pro Lys Pro Trp Arg Lys Leu Thr Val Lys Gly Lys Glu Pro
595 600 605

Phe Ile Lys Met Lys Gln Phe Leu Ser Asp Glu Gln Asn Val Leu Ala
610 615 620

Leu Arg Thr Ile Gln Val Arg Gln Arg Gly Ser Ile Thr Pro Arg Ile
625 630 635 640

Arg Thr Pro Glu Thr Gly Ser Asp Asp Ala Ile Lys Ser Ile Leu Glu
645 650 655

Gln Ala Lys Lys Glu Ile Glu Ser Gln Lys Gly Gly Glu Pro Lys Thr

```
            660                 665                 670

Ser Val Ala Pro Leu Ser Ile Ala Asn Gly Thr Thr Pro Ala Ser Thr
        675                 680                 685

Ser Glu Asp Ala Ile Lys Ser Ile Leu Glu Gln Ala Arg Arg Glu Met
    690                 695                 700

Gln Ala Gln Gln Gln Ala Leu Leu Glu Met Glu Val Ala Pro Arg Gly
705                 710                 715                 720

Arg Ser Val Pro Pro Ser Pro Pro Glu Arg Pro Ser Leu Ala Thr Ala
                725                 730                 735

Ser Gln Asn Gly Ala Pro Ala Leu Val Lys Gln Glu Glu Gly Ser Gly
            740                 745                 750

Gly Pro Ala Gln Ala Pro Leu Pro Val Leu Ser Pro Ala Ala Phe Val
        755                 760                 765

Gln Ser Ile Ile Arg Lys Val Lys Ser Glu Ile Gly Asp Ala Gly Tyr
    770                 775                 780

Phe Asp His His Trp Ala Ser Asp Arg Gly Leu Leu Ser Arg Pro Tyr
785                 790                 795                 800

Ala Ser Val Ser Pro Ser Leu Ser Ser Ser Ser Ser Ser Gly Tyr Ser
                805                 810                 815

Gly Gln Pro Asn Gly Arg Ala Trp Pro Arg Gly Asp Glu Ala Pro Val
            820                 825                 830

Pro Pro Glu Asp Glu Ala Ala Ala Gly Ala Glu Asp Glu Pro Pro Arg
        835                 840                 845

Thr Gly Glu Leu Lys Ala Glu Gly Ala Thr Ala Glu Ala Gly Ala Arg
    850                 855                 860

Leu Pro Tyr Tyr Pro Ala Tyr Val Pro Arg Thr Leu Lys Pro Thr Val
865                 870                 875                 880

Pro Pro Leu Thr Pro Glu Gln Tyr Glu Leu Tyr Met Tyr Arg Glu Val
                885                 890                 895

Asp Thr Leu Glu Leu Thr Arg Gln Val Lys Glu Lys Leu Ala Lys Asn
            900                 905                 910
```

```
Gly Ile Cys Gln Arg Ile Phe Gly Glu Lys Val Leu Gly Leu Ser Gln
        915                 920                 925

Gly Ser Val Ser Asp Met Leu Ser Arg Pro Lys Pro Trp Ser Lys Leu
    930                 935                 940

Thr Gln Lys Gly Arg Glu Pro Phe Ile Arg Met Gln Leu Trp Leu Ser
945                 950                 955                 960

Asp Gln Leu Gly Gln Ala Val Gly Gln Gln Pro Gly Ala Ser Gln Ala
                965                 970                 975

Ser Pro Thr Glu Pro Arg Ser Ser Pro Ser Pro Pro Pro Ser Pro Thr
            980                 985                 990

Glu Pro Glu Lys Ser Ser Gln Glu  Pro Leu Ser Leu Ser  Leu Glu Ser
        995                 1000                 1005

Ser Lys  Glu Asn Gln Gln Pro  Glu Gly Arg Ser Ser  Ser Ser Leu
    1010                 1015                 1020

Ser Gly  Lys Met Tyr Ser Gly  Ser Gln Ala Pro Gly  Gly Ile Gln
    1025                 1030                 1035

Glu Ile  Val Ala Met Ser Pro  Glu Leu Asp Thr Tyr  Ser Ile Thr
    1040                 1045                 1050

Lys Arg  Val Lys Glu Val Leu  Thr Asp Asn Asn Leu  Gly Gln Arg
    1055                 1060                 1065

Leu Phe  Gly Glu Ser Ile Leu  Gly Leu Thr Gln Gly  Ser Val Ser
    1070                 1075                 1080

Asp Leu  Leu Ser Arg Pro Lys  Pro Trp His Lys Leu  Ser Leu Lys
    1085                 1090                 1095

Gly Arg  Glu Pro Phe Val Arg  Met Gln Leu Trp Leu  Asn Asp Pro
    1100                 1105                 1110

His Asn  Val Glu Lys Leu Arg  Asp Met Lys Lys Leu  Glu Lys Lys
    1115                 1120                 1125

Ala Tyr  Leu Lys Arg Arg Tyr  Gly Leu Ile Ser Thr  Gly Ser Asp
    1130                 1135                 1140

Ser Glu  Ser Pro Ala Thr Arg  Ser Glu Cys Pro Ser  Pro Cys Leu
    1145                 1150                 1155
```

Gln Pro Gln Asp Leu Ser Leu Leu Gln Ile Lys Lys Pro Arg Val
1160 1165 1170

Val Leu Ala Pro Glu Glu Lys Glu Ala Leu Arg Lys Ala Tyr Gln
1175 1180 1185

Leu Glu Pro Tyr Pro Ser Gln Gln Thr Ile Glu Leu Leu Ser Phe
1190 1195 1200

Gln Leu Asn Leu Lys Thr Asn Thr Val Ile Asn Trp Phe His Asn
1205 1210 1215

Tyr Arg Ser Arg Met Arg Arg Glu Met Leu Val Glu Gly Thr Gln
1220 1225 1230

Asp Glu Pro Asp Leu Asp Pro Ser Gly Gly Pro Gly Ile Leu Pro
1235 1240 1245

Pro Gly His Ser His Pro Asp Pro Thr Pro Gln Ser Pro Asp Ser
1250 1255 1260

Glu Thr Glu Asp Gln Lys Pro Thr Val Lys Glu Leu Glu Leu Gln
1265 1270 1275

Glu Gly Pro Glu Glu Asn Ser Thr Pro Leu Thr Thr Gln Asp Lys
1280 1285 1290

Ala Gln Val Arg Ile Lys Gln Glu Gln Met Glu Glu Asp Ala Glu
1295 1300 1305

Glu Glu Ala Gly Ser Gln Pro Gln Asp Ser Gly Glu Leu Asp Lys
1310 1315 1320

Gly Gln Gly Pro Pro Lys Glu Glu His Pro Asp Pro Pro Gly Asn
1325 1330 1335

Asp Gly Leu Pro Lys Val Ala Pro Gly Pro Leu Leu Pro Gly Gly
1340 1345 1350

Ser Thr Pro Asp Cys Pro Ser Leu His Pro Gln Gln Glu Ser Glu
1355 1360 1365

Ala Gly Glu Arg Leu His Pro Asp Pro Leu Ser Phe Lys Ser Ala
1370 1375 1380

Ser Glu Ser Ser Arg Cys Ser Leu Glu Val Ser Leu Asn Ser Pro
1385 1390 1395

Ser Ala Ala Ser Ser Pro Gly Leu Met Met Ser Val Ser Pro Val
1400 1405 1410

Pro Ser Ser Ser Ala Pro Ile Ser Pro Ser Pro Pro Gly Ala Pro
1415 1420 1425

Pro Ala Lys Val Pro Ser Ala Ser Pro Thr Ala Asp Met Ala Gly
1430 1435 1440

Ala Leu His Pro Ser Ala Lys Val Asn Pro Asn Leu Gln Arg Arg
1445 1450 1455

His Glu Lys Met Ala Asn Leu Asn Asn Ile Ile Tyr Arg Val Glu
1460 1465 1470

Arg Ala Ala Asn Arg Glu Glu Ala Leu Glu Trp Glu Phe
1475 1480 1485

<210> 7
<211> 12379
<212> DNA
<213> Homo sapiens

<400> 7
gacccgagag ccgctgagcc gcgaggccgg gccggggcgc cggccgggaa tgccgggggc 60

gcggcgccga cgccgaggcg cggccatgga ggggaagccc cgcgccgggg tcgcgctggc 120

cccggggccg agcggccgac ggccttccgc ccgctgcgcc cgccgccgcc gcccggggct 180

gctgcttcct ggcctctggc tgctgctgct ggcccggccg gcctcgtgcg ccccagatga 240

gctctctccg gaacagcaca acctttcttt atactccatg gagctcgtgc tgaagaaaag 300

cactgggcac agcgctgcac aagtggcctt aacagaaact gctcccggct cccagcacag 360

cagtcctctc catgtcacag ccccgccgtc tgccactact tttgatacag ccttttttaa 420

ccaaggaaaa cagaccaaaa gtacagcaga tcccagcatc tttgtggcaa cttacgtgtc 480

agtgacgagt aaagaggtgg ccgtcaatga cgatgagatg gataactttc tgccagatac 540

tcactggacc actccacgga tggtttctcc aatacagtat atcacagtca gcccaccagg 600

gctgcccagg gaagcattag aacctatgct cactccatca ttacccatgg tttctttaca 660

agatgaagaa gtgacatcgg gctggcagaa cacaacgcga caaccagcgg catatgctga 720

gtccgccagt catttccaca cctttcggtc agcttttcgc acctctgagg gcatcgttcc 780

aactcctggc aggaatttgg tgctttatcc tactgatgct tacagtcatt tatcaagcag 840

gactctgcca gagattgtgg cttccctaac agagggtgtg gaaaccaccc ttttttttaag 900

ctcccggtct ttaatgccac agccgttagg cgacggcatt actataccgt tgccctcctt 960

gggggaggtc tcacagcctc cagaggaggt ttgggccaca agtgcagaca gatacactga 1020
tgtgaccact gtgttgagtc aaagcctaga agaaaccatc tctccaagaa cataccccac 1080
tgtgactgca tcgcacgcag cccttgcatt cagcaggaca cattctccat tgctttcaac 1140
tcctcttgca tttgcgtcct ctgcttcacc aactgatgtt tcatctaacc cctttctccc 1200
tagcgactcc agcaaaacat ccgaattgca tagcaattca gccctccccg gtcctgtgga 1260
caacactcat atcctgagcc cggtgtcctc attcagacca tacacttggt gtgcggcctg 1320
cactgtgcct tcacctcagc aagttctggc cacgagcctc atggagaaag acgtgggatc 1380
aggggatggt gccgagactc tgtgcatgac cgtgctggaa gaaagcagca tctctctaat 1440
gagtagcgtc gtagcagact tctctgaatt tgaggaagat cctcaagtat ttaatacgct 1500
tttcccctcc agacctatcg tcccactttc ttctagatcc atggaaatct cagagacgag 1560
tgttggcatt tctgccgagg tggatatgag tagtgttaca accacacagg ttccccctgc 1620
ccacggccgc ctctctgtgc cggcgtcact tgatcctact gctggctcct tgtctgttgc 1680
tgaaacccaa gtgacgccat ccagcgtgac cactgcattt ttctcggtca tcaccagcat 1740
tctccttgac tcatctttct ctgtcatagc aaacaaaaac acaccgtcgc ttgccgtcag 1800
agacccgagt gtttttacgc cttatagtct ggttccttca gtggagtctt cacttttctc 1860
tgaccaagaa cgttccagtt tttctgagca taaacccaga ggtgctttgg attttgcatc 1920
cagcttttc tcaacacccc cgctggaact cagcggctcc atctcttcgc cttcggaagc 1980
acctgcgtct ctgtctctga tgccgagtga cttgtccccc ttcacatctc agtcttttc 2040
tcccttggtt gagacattta cattgtttga ctctagtgat ctgcagtcat ctcagctgtc 2100
tcttcccagt tccacaaatc ttgagttttc gcagctccag ccaagttccg agctgccttt 2160
aaacaccatc atgttgctac ctagccgttc tgaggtgtca ccatggtcaa gcttcccttc 2220
tgattctctc gagtttgttg aagcgtctac ggtttcactg acggattcag aagctcattt 2280
tacctcagct ttcattgaaa ctacctccta tcttgagtct tcactcattt cccatgaatc 2340
cgcagtcact gcactggtgc cccccggctc tgagtctttt gacattttga ctgccgggat 2400
tcaagcaaca tcaccattga ccactgtcca cacaacgccc attttaactg agtcttcttt 2460
gttctcaact ctgacacctc ctgacgacca aatcagtgct ctagacggtc acgtgtctgt 2520
cctggcctct ttctccaaag ccattcccac tggtacggtg ttgatcactg acgcgtacct 2580
gccatcagga tcctcgtttg tttctgaagc aaccccccttc cctctgccca cagagctgac 2640
cgtcgtgggc ccatcactca cacccacaga ggtgccactg aacacctcca cggaagtgag 2700
cacaaccagc accggtgctg ccactggtgg tcccctcgac tccaccctga tgggtgacgc 2760
cgcaagtcag agccccccag agagtagtgc tgctcctccc ctgccatccc tgcgtcccgt 2820

gactgccttc actctcgaag caacagtcga cacaccaaca ctggctactg ccaagccgcc 2880
atatgtttgt gatatcacag tccccgatgc ctatctgatc acaactgtgc tggccagaag 2940
agctgtgcag gagtacatca ttacagcaat caaagaagta ctgaggattc acttcaaccg 3000
tgcagtggaa ctgaaggttt acgaactatt tactgacttc acttttctgg taacatccgg 3060
tcctttcgtt tacacggcaa tatccgtcat aaatgtgctt ataaacagta agcttgtccg 3120
tgaccagact cctttaatcc tgtctgtgaa accttctttc cttgtgccag agtccaggtt 3180
ccaagttcaa acagtacttc agtttgtgcc tccgagtgtg gatactggct tctgcaactt 3240
cacccagcgc attgagaaag gcctaatgac agctctcttt gaagtgagaa aacaccacca 3300
gggaacgtat aacctcacgg tgcagatctt gaatatcacc atcagttcct caagggtgac 3360
tcctcggcgg ggcccggtga atatcatctt tgcggttaaa agcacacagg gatttttgaa 3420
tgggtcggaa gtgagcgagc tgctcagaaa cttgagtgtg gtggagttca gtttctatct 3480
gggataccca gtgctgcaga tcgcagagcc cttccagtat ccacagctca acttatctca 3540
gttgctgaag tcctcttggg tcagaacagt tctcctgggc gtcatggaga agcaactcca 3600
gaatgaagtg tttcaagccg agatggaacg caagctggcc cagctgctca gcgaggtttc 3660
caccagaagg cggatgtgga gaagggccac tgtagctgca gggaacagtg tggtgcaggt 3720
ggtaaatgtg tcgaggctgg agggagatga caatccggta cagctcatct actttgtgga 3780
ggatcaagat ggagaaagac tcagtgcagt caagtcttcg gacctgatta acaaaatgga 3840
cctccagaga gcagccatca tcttgggtta ccgaattcaa ggtgtcattg cccagcctgt 3900
cgacagggtg aagaggccgt ctccggaatc ccagagcaac aacttgtggg tcattgttgg 3960
cgtggtcatc ccagtgctgg tggtgatggt gattgttgtc atcctctact ggaaactatg 4020
ccgcacagac aagctagact ttcagcctga cactgtggcc aacattcagc agcgtcagaa 4080
gctgcagatc cctagtgtga agggcttcga ttttgctaag cagcatctgg gtcagcacaa 4140
taaagacgac atattgatta ttcatgagcc agcgccactg ccaggacctc tgaaggacca 4200
caccacgccc tcggaaaatg gagacgtgcc aagccccaag tcaaagatcc cttccaagaa 4260
tgttcgtcac agaggaagag tttctccctc agatgctgac tctacggtca gtgaagagtc 4320
cagcgagagg gacgcaggag ataagacgcc gggagccgtc aacgatggca ggtcccacag 4380
agctccgcag agcgggccac cactgcccag ttcgggaaat gagcagcact catcagcctc 4440
catcttcgag cacgtggaca ggatctcccg ccccccggag gctagccggc gggtccccag 4500
taagatccag cttatcgcca tgcagccgat cccggcacct cccgtccagc gcccctcccc 4560
agccgaccga gtggcggaaa gcaataaaat caacaaagag attcagaccg cgctgcggca 4620
caagtctgag atcgagcacc atcgcaacaa gatccgcctg cgcgccaagc gccgcgggca 4680
ctacgagttc ccggtggtag acgacctgtc ctcgggcgac actaaggagc gacaccgggt 4740

gtaccgcagg gcacagatgc agatcgacaa gatcctggac cccacggcca gcgtgccctc 4800

cgtgttcata gagcccagga agagctcacg gataaaacgt tctcccaagc ctcgccggaa 4860

acaccaggtc aacggctgtc ctgccgacgc tgagaaggac cggctcatca ccacagacag 4920

cgatggcacc tacaggaggc cccccggcgt ccacaactca gcctacatcg gatgcccatc 4980

ggatcctgac ctcccagccg atgtgcagac accatcctcg gtggaactgg ggaggtatcc 5040

agcccttccc ttcccggcct cccagtacat cccaccccag ccgtccatcg aggaggcacg 5100

ccagaccatg cactccctcc tggacgacgc ctttgccctc gtggccccca gcagccagcc 5160

tgccagcacc gcaggtgtag gccccggagt cccacccggc ctgcccgcaa acagcacccc 5220

ttcccaggaa gagaggcgag ccacccagtg gggtccttc tacagcccag cccagacggc 5280

caacaatccc tgcagtagat acgaagacta tggaatgact cccccgacgg gtccattgcc 5340

aagaccaggt tttggccccg gtttgctgca gtctacagag ctggtgcccc ctgaccctca 5400

gcagccacag gcctccgccg aagccccatt tgctgccaga gggatctact cggaggagat 5460

gccgtcggtg gcccggcctc ggcctgtcgg gggtaccaca ggctcccaga tccagcacct 5520

gacacaggtg gggattgcca gcagaattgg agctcagcca gtggaaatcc cgccaagcag 5580

aggcagccag tatgggggc caggctggcc ttcgtacggg gaggacgaag cggggcgaag 5640

agaggccgtg ccaaggactt caggcaggga gccctcagct ccttccggga acctccccca 5700

ccggggactg cagggccctg ggctgggtta ccccaccagc tccacggaag acctccagcc 5760

tggccactcc tcggcctctc tcatcaaagc aatccgcgag gagctcctcc ggctctccca 5820

gaaacagagc accgtgcaga acttccacag ctgatcggcc tcgcctcgca gatttgccaa 5880

gtatccgctt cctgtggaag caagaccaaa aggaaatcaa ctgagtgggt gtttggaaga 5940

ggaaggagca actctcgggc agcctgccca agggagggag caagttgcaa tttagaagat 6000

gccatacgtc gtgtgacagc tcatgagcct ttcactgggc tggcaattgt ctgaacactt 6060

gggttcagtt gaaatatatg tattttggcc aaaagccagc agcacttcac aaaaacaaaa 6120

cacaaaccta agctaacaaa atgactgcat tcgtctcttt tttaaaggta gagattaaac 6180

tgtatagaca gcatagggat gaaaggaacc aagcgtttct gtgggattga gactggtacg 6240

tgtacgatga acctgctgct ttgttttctg agaagaggtt tgaagacatt ttattaacag 6300

cttaattttt ctcttttact ccataggaac ttattttaat agtaacatta acaacaagaa 6360

tactaagact gtttgggaat tttaaaaagc tactagtgag aaaccaaatg ataggttgta 6420

gagcctgatg actccaaaca aagccatcac ccgcattctt cctccttctt ctggtgctac 6480

agctccaagg gcccttcacc ttcatgtctg aaatggaact ttggcttttt cagtggaaga 6540

atatgttgaa ggtttcattt tgttctagaa aaaaaaatc cctcccaaag tggggcaaaa 6600

agctttatat ttatttgatt atccaaaata cagatcaaag tttagatcta cattcttcat 6660
tgtatttgct gtttcttaat tgggcacaca caactcctgg tcatgtccca gttcagcacc 6720
gatcgctaag gccgatcctg tagaatgcgg ctttcaagag gtcctaactg atcctttctt 6780
ccactttgct gttgatttgt tcacaaatta tgtctgaatg agaaatcttc tgtaagcaga 6840
agttatttaa taattcccag caccacgaaa ttgttataca tacatcccta ccagtcacat 6900
tccctgtgtt cagagcctgg aaatgaaatt gagttcagtt cagcctctct gtaatgaatc 6960
aagaaatgta aaagagcttt gagaccccaa gggaaaggag agagttgctt ctcatttctg 7020
attttattgt gctgttactc tgtcgagtgg ctattaaaat tgtcttatgc tctttgggct 7080
aagaggggat catctcgctt aagatgtact cctgatgtaa acatttcccc cactttccaa 7140
ataatggtaa agaaaacagt ggcaagggct gttctgtttt ctggtacctg agtgaaactc 7200
agaagaaaag caggcttagc taagagattt tcactattaa cctgttttta ttagatcagc 7260
gaagacagtc atgcatcgct taatgatggg gatgcattct gagaaatgtg tcattaggct 7320
gttttgttgc tgtgccaaca tcctagattg tacttacaca acctacatgg tatagcctac 7380
tacacacctg ggctgtgtgg catggcctat tcctcttagg ctacaaacct gtacagcatg 7440
ttactgtact gaacacaatg gtaagtattt gtgtatctaa acatagctaa acatagaaaa 7500
ggtatggtaa aaatacaatt ttataatctt atgggactac catcaatatg tagaccgtca 7560
ttgaccaaaa catcctgatg tgtgcatgac tgtacttttc attaaataac agataagggg 7620
ctatagaatt tggggctctg actgatcaaa acctgtgtat ctgtgtcaag ttttaagacc 7680
cctgaaacag ctaaaacagg tttccaaagt gtaatcactg gatttcaaaa ccatgtttta 7740
gcccttcatt aatgaagcct gttgtacaga tttagagtct tacaatatga gggaagttgg 7800
ttctgggaag gtaagatgta gccagttttc atctgggcac ctctgaaaga gaaggagtgc 7860
aggagagtaa gtctctcaag gacgttcaac aaaggaccag cagcatctta tcaggcgatc 7920
ctcagaggct ccaaggagca ttggagagaa tgcccctttc tgggcttgat gtcttggttt 7980
tggtttaata ccaaatttct cttggtcttg gtttaatacc cgactcctcc caaattgaac 8040
gattatcttt gtatgtcact caaaaatacc aggtttcctg aatatgttat taagaatttc 8100
agatatccaa gcagaatttt attatggaca ccttgtaatg aattcaaatc gtgtgacagc 8160
aaacgggata aatggcccct tctcacccag ttccgctcag ggccatacag gcttgcacat 8220
agagtgtgct aaaaatggta acctcctttg agcattgcag aaagagggtt ctgtttcaaa 8280
ttgggctgac cgtaaaagca attttgatgt ctttcaaact accataaagg gattttaact 8340
gtatttttat tcttagaaac aatacacctt taaacagatg tcaagaccaa agatgatgta 8400
taataaacag ccggtggtta tggtgtggtg tgagcaggcc cccggatcac cagccctctg 8460
ctggtggtca taaagcacac acagtttgta cttgcttcct ctcgacgcct gccacaacag 8520

tttttgccagt caccaaactc agaaaaaact agctgtgtga gcagcaccgt accctagcgg 8580
tcttcagaca ttaagttcac gtcatccact gaaaggaagg gtccttgcgt tgacagcgtg 8640
tggctttgga gtccgtttat ggaagcactc tacaatgaac agttgaattt tatgtctatt 8700
ctttttttcct attttaaaag ttctagtggt aacacaaact gtaaatttga gtcagaatgt 8760
ttggagaaat gttgtttttt tttttttcag agactacttt gtcactcact gaccatgtct 8820
ggttccttct ctgaacttca ttctccccat aagccaaaca agaacagacc tcccccgcca 8880
cctcccaggc accgtagttg tgagaatcag atgtgatcat gtgtgcagat gtcctgtgag 8940
gagggagcat aaagcactgt gaaaatgtag catgctgtct atgtggacgc cctaggatga 9000
gtgatgtgaa acgctgcact actgcggtga atgggttcac acatgggtaa tgagcaaaac 9060
cgaaagctcg gtgtgactca gtttcctcac tcccattttg ctttaatttc acttccttca 9120
ccaattttcc gattgcattt attaagcatc tttctgctcc cgactttctg gtgtctctgg 9180
caccaaataa cccagcagac atgagccttg ccttctgaga ttttagaatc taagatagca 9240
cgagtgggta tagaagatgg aagataccga taaataacat ggtttaagtg tcaataaaat 9300
tcattcgaag agatcaggcg cggtggctca cgcctgtaat cccagcattt tgggaggctg 9360
aggcaggtgg atcacttgag atcaagagct taaaaccagc ccaggcaaca tggcaaaacc 9420
ccgtctctac aaaaaaatac aaaaactagc tgggtgtggt ggcgcatgcc tgtagtccca 9480
gctgaggcac gataattgct tgaacccggg gggtggaggt tgcagtgagc tgagattgca 9540
ccactgtact ccaacctggg cgacagagca agactgtgtc tcaaaaagaa aaaaatttgt 9600
tcaaaagaca taaatgaatt aggcacccag aagaagttgc atgtttggaa atgcaatatc 9660
ttggggagac gtcaacttct gaagtgactt tgaaggcagg gcagctaccc aggcaaatgg 9720
cgtggaagga agagctgaga gtgggcctgg gaaaggctgt agggaaggag tgaagctgtc 9780
actgaaatga acttgatctt gatctgcttt gatatgggga cagaatcccc gactgcacta 9840
ttgagggagt ttcagaagag aaggaagagg tgctggaagg atgcttttgc agtcatgcag 9900
caaggaaacg accagggctg tgaagatcta cagaggaagt actccagtgt gggggaggca 9960
agggaagagg acgaaaatga tgagaatgac actgaggtca ttgtttagag cagatctcaa 10020
gcagccgttt ggccacgcca tatcctttgt ggagcatagt gggttatcta tctgtctgtc 10080
tgtctatgta tctgtctatc tatctatata tatattcagc ttctttactg ctacctacac 10140
atttttctcc aaattttatt aatttcatag tgttttgatt tgggtggcag atgactttta 10200
agcagtgggt gtttgccggc cagatctttc ctggcatgcg gactgtgagg caaagcacgg 10260
gtgaaggtag gcgctaaagg ctttgggcta aagccagcac gcggttctgt gctataggag 10320
tctcccgttt cccgtggaca ggttcagcgt tccttctttc gcacaacttt tttctaagtg 10380

ttccagtgac caagccagtc attcggacac tgatttgcag tgcattggca gtaattcaca 10440

aattagttgt tatataagtc tctctcatcc ctttcacact agattctcag acatgaatga 10500

atttgtcgtt tggaaggaaa gctgggtaac gttttgggca caggggaagg aggactccgg 10560

tcttaactcc cacgctaact ttagctcaag tggagttttc accgtggtca tttctacctc 10620

cggagcaagg tgccagcgcc agtactagag cctgcttatc cacatttgcc ctggacagga 10680

gcaggaggaa gtccacttct gtaccggcag acagagcatg tgaacacaaa acacatttct 10740

atggcatagt caactgaact tcatttttac atttaatcta acatgttaac acgttctaac 10800

agggtttcta tgagcagctg ctgtaacata ctcatcaact atgatagact taacacttgt 10860

tacctaatga acaaggagga tgtgcatttc gggtttcttt tgatattcgt ggaggtgatt 10920

gtcagtgttt aaacaggact actttctcca ctatgaagat gacttggaaa tcgcaacatc 10980

atggtacatt gctaagtcca tgcttgtgtg tgtgacagta ggcttgataa tttatcttaa 11040

aacacagcag cattgaaatt taggaaaaga atattaaatg cctttggaaa catgaaacaa 11100

agttaggagc cagtaagaaa gtgacagaag caatgaatgt cttaatgcag tatagttaaa 11160

atggcttccc acagggtaga taattatcca ggatccttcc ttttccttct tcctccaggt 11220

ttttcagggg tcacttcaca tttctaaaga aagagtgaat aggctgggca tggtggctca 11280

agcctctaat ctcaacgctt tgggaggctg aggcaggagg atcgcttgag gccaggagtt 11340

tgagaccacc ttgggcaaca taacgagacc ccgtctctac aaaaaaaatt taaaaattag 11400

ctgagcatgg tggcatgtgc cagtagtccc agctactcga aaggctaaga ctggaggatc 11460

gcttgagcca atgagttgga ggctgcagtg agctataatc acgccactgc actccagcct 11520

gggctgcagg gtgaggtcct gtctctggaa aaaaaaaaaa aaaaaaaagg aataggtaaa 11580

agggacagag gtgaaatttt gagtgacttg agtctcttgc agtccctgat tacacagaac 11640

ctttctgggc tacttggagc atcacgaata gtctttcctg tacttaccag atttcaagta 11700

ttcataactt gactccctaa gtgtacaagt tgggaatagt acagggccaa gttcaagtcg 11760

catatgctgt actgttcctc ctgcaaatgt ggggaaagaa gagggagata ctagaggaac 11820

tgaggctcca cccattcatt cagttgctct aagcaccaga ggacttgttt cagaaaaggg 11880

gagtgggaac gccctcgact ttgccctcct ccggagcatc tctgggacgc agggagtctg 11940

gctagcgtta ataggaaagg ttgctcggca gagctgccct ggagtactga cttgtctctc 12000

cctcctttgt caaggtccat gtttttctgg ctcttcctgc acactcatcc ctagattatg 12060

agcgttaatg tacgaaatgt gcagagcaaa ttgaggccaa ctgtggcatc aatactgcaa 12120

cttaaacttg acaatcatgg tttgctggtc ctcaaacagg cactgaaatc tcagtatggg 12180

tatacgattt aataatcggc ccctcccttc tatttgtcgg ttttatgttt ctatccttca 12240

atagctgcac tgttttctaa tgtgctgtag agtttttgaa ataatttatg caagtatttg 12300

gtttccatgt ttacaattta tacagctttc ctagcatttt aaatggaaat gtcaataaat 12360

gctatgaatt ggtgtcaaa 12379

<210> 8
<211> 12498
<212> DNA
<213> Homo sapiens

<400> 8
ctctccggcc tccctccgcg ccggtgcgcg gcgccccgtc ccagccgcag ccgcagcggc 60

cgcccctccc ggacccgaga gccgctgagc cgcgaggccg ggccggggcg ccggccggga 120

atgccggggg cgcggcgccg acgccgaggc gcggccatgg aggggaagcc ccgcgccggg 180

gtcgcgctgg ccccggggcc gagcggccga cggccttccg cccgctgcgc ccgccgccgc 240

cgcccggggc tgctgcttcc tggcctctgg ctgctgctgc tggcccggcc ggcctcgtgc 300

gccccagatg agctctctcc ggaacagcac aacctttctt tatactccat ggagctcgtg 360

ctgaagaaaa gcactgggca cagcgctgca caagtggcct taacagaaac tgctcccggc 420

tcccagcaca gcagtcctct ccatgtcaca gccccgccgt ctgccactac ttttgataca 480

gcctttttta accaaggaaa acagaccaaa agtacagcag atcccagcat ctttgtggca 540

acttacgtgt cagtgacgag taaagaggtg gccgtcaatg acgatgagat ggataacttt 600

ctgccagata ctcactggac cactccacgg atggtttctc caatacagta tatcacagtc 660

agcccaccag ggctgcccag ggaagcatta gaacctatgc tcactccatc attacccatg 720

gtttctttac aagatgaaga agtgacatcg ggctggcaga acacaacgcg acaaccagcg 780

gcatatgctg agtccgccag tcatttccac acctttcggt cagcttttcg cacctctgag 840

ggcatcgttc caactcctgg caggaatttg gtgctttatc ctactgatgc ttacagtcat 900

ttatcaagca ggactctgcc agagattgtg gcttccctaa cagagggtgt ggaaaccacc 960

cttttttttaa gctcccggtc tttaatgcca cagccgttag gcgacggcat tactataccg 1020

ttgccctcct tgggggaggt ctcacagcct ccagaggagg tttgggccac aagtgcagac 1080

agatacactg atgtgaccac tgtgttgagt caaagcctag aagaaaccat ctctccaaga 1140

acatacccca ctgtgactgc atcgcacgca gcccttgcat tcagcaggac acattctcca 1200

ttgctttcaa ctcctcttgc atttgcgtcc tctgcttcac caactgatgt ttcatctaac 1260

ccctttctcc ctagcgactc cagcaaaaca tccgaattgc atagcaattc agccctcccc 1320

ggtcctgtgg acaacactca tatcctgagc ccggtgtcct cattcagacc atacacttgg 1380

tgtgcggcct gcactgtgcc ttcacctcag caagttctgg ccacgagcct catggagaaa 1440

gacgtgggat caggggatgg tgccgagact ctgtgcatga ccgtgctgga agaaagcagc 1500

atctctctaa tgagtagcgt cgtagcagac ttctctgaat ttgaggaaga tcctcaagta 1560

tttaatacgc ttttcccctc cagacctatc gtcccacttt cttctagatc catggaaatc 1620
tcagagacga gtgttggcat ttctgccgag gtggatatga gtagtgttac aaccacacag 1680
gttccccctg cccacggccg cctctctgtg ccggcgtcac ttgatcctac tgctggctcc 1740
ttgtctgttg ctgaaaccca agtgacgcca tccagcgtga ccactgcatt tttctcggtc 1800
atcaccagca ttctccttga ctcatctttc tctgtcatag caaacaaaaa cacaccgtcg 1860
cttgccgtca gagacccgag tgtttttacg ccttatagtc tggttccttc agtggagtct 1920
tcacttttct ctgaccaaga acgttccagt ttttctgagc ataaacccag aggtgctttg 1980
gattttgcat ccagcttttt ctcaacaccc ccgctggaac tcagcggctc catctcttcg 2040
ccttcggaag cacctgcgtc tctgtctctg atgccgagtg acttgtcccc cttcacatct 2100
cagtcttttt ctcccttggt tgagacattt acattgtttg actctagtga tctgcagtca 2160
tctcagctgt ctcttcccag ttccacaaat cttgagtttt cgcagctcca gccaagttcc 2220
gagctgcctt taaacaccat catgttgcta cctagccgtt ctgaggtgtc accatggtca 2280
agcttccctt ctgattctct cgagtttgtt gaagcgtcta cggtttcact gacggattca 2340
gaagctcatt ttacctcagc tttcattgaa actacctcct atcttgagtc ttcactcatt 2400
tcccatgaat ccgcagtcac tgcactggtg ccccccggct ctgagtcttt tgacattttg 2460
actgccggga ttcaagcaac atcaccattg accactgtcc acacaacgcc cattttaact 2520
gagtcttctt tgttctcaac tctgacacct cctgacgacc aaatcagtgc tctagacggt 2580
cacgtgtctg tcctggcctc tttctccaaa gccattccca ctggtacggt gttgatcact 2640
gacgcgtacc tgccatcagg atcctcgttt gtttctgaag caaccccctt ccctctgccc 2700
acagagctga ccgtcgtggg cccatcactc acacccacag aggtgccact gaacacctcc 2760
acggaagtga gcacaaccag caccggtgct gccactggtg gtcccctcga ctccaccctg 2820
atgggtgacg ccgcaagtca gagcccccca gagagtagtg ctgctcctcc cctgccatcc 2880
ctgcgtcccg tgactgcctt cactctcgaa gcaacagtcg acacaccaac actggctact 2940
gccaagccgc catatgtttg tgatatcaca gtccccgatg cctatctgat cacaactgtg 3000
ctggccagaa gagctgtgca ggagtacatc attacagcaa tcaaagaagt actgaggatt 3060
cacttcaacc gtgcagtgga actgaaggtt tacgaactat ttactgactt cacttttctg 3120
gtaacatccg gtcctttcgt ttacacggca atatccgtca taaatgtgct tataaacagt 3180
aagcttgtcc gtgaccagac tcctttaatc ctgtctgtga aaccttcttt ccttgtgcca 3240
gagtccaggt tccaagttca aacagtactt cagtttgtgc ctccgagtgt ggatactggc 3300
ttctgcaact tcacccagcg cattgagaaa ggcctaatga cagctctctt tgaagtgaga 3360
aaacaccacc agggaacgta taacctcacg gtgcagatct tgaatatcac catcagttcc 3420

tcaagggtga ctcctcggcg gggcccggtg aatatcatct ttgcggttaa aagcacacag 3480
ggatttttga atgggtcgga agtgagcgag ctgctcagaa acttgagtgt ggtggagttc 3540
agtttctatc tgggataccc agtgctgcag atcgcagagc ccttccagta tccacagctc 3600
aacttatctc agttgctgaa gtcctcttgg gtcagaacag ttctcctggg cgtcatggag 3660
aagcaactcc agaatgaagt gtttcaagcc gagatggaac gcaagctggc ccagctgctc 3720
agcgaggttt ccaccagaag gcggatgtgg agaagggcca ctgtagctgc agggaacagt 3780
gtggtgcagg tggtaaatgt gtcgaggctg gagggagatg acaatccggt acagctcatc 3840
tactttgtgg aggatcaaga tggagaaaga ctcagtgcag tcaagtcttc ggacctgatt 3900
aacaaaatgg acctccagag agcagccatc atcttgggtt accgaattca aggtgtcatt 3960
gcccagcctg tcgacagggt gaagaggccg tctccggaat cccagagcaa caacttgtgg 4020
gtcattgttg gcgtggtcat cccagtgctg gtggtgatgg tgattgttgt catcctctac 4080
tggaaactat gccgcacaga caagctagac tttcagcctg acactgtggc caacattcag 4140
cagcgtcaga agctgcagat ccctagtgtg aagggcttcg attttgctaa gcagcatctg 4200
ggtcagcaca ataaagacga catattgatt attcatgagc cagcgccact gccaggacct 4260
ctgaaggacc acaccacgcc ctcggaaaat ggagacgtgc caagccccaa gtcaaagatc 4320
ccttccaaga atgttcgtca cagaggaaga gtttctccct cagatgctga ctctacggtc 4380
agtgaagagt ccagcgagag ggacgcagga gataagacgc cgggagccgt caacgatggc 4440
aggtcccaca gagctccgca gagcgggcca ccactgccca gttcgggaaa tgagcagcac 4500
tcatcagcct ccatcttcga gcacgtggac aggatctccc gccccccgga ggctagccgg 4560
cgggtcccca gtaagatcca gcttatcgcc atgcagccga tcccggcacc tcccgtccag 4620
cgcccctccc cagccgaccg agtggcggaa agcaataaaa tcaacaaaga gattcagacc 4680
gcgctgcggc acaagtctga gatcgagcac catcgcaaca agatccgcct gcgcgccaag 4740
cgccgcgggc actacgagtt cccggtggta gacgacctgt cctcgggcga cactaaggag 4800
cgacaccggg tgtaccgcag ggcacagatg cagatcgaca agatcctgga ccccacggcc 4860
agcgtgccct ccgtgttcat agagcccagg aagagctcac ggataaaacg ttctcccaag 4920
cctcgccgga aacaccaggt caacggctgt cctgccgacg ctgagaagga ccggctcatc 4980
accacagaca gcgatggcac ctacaggagg cccccggcg tccacaactc agcctacatc 5040
ggatgcccat cggatcctga cctcccagcc gatgtgcaga caccatcctc ggtggaactg 5100
gggaggtatc cagcccttcc cttcccggcc tcccagtaca tcccaccccca gccgtccatc 5160
gaggaggcac gccagaccat gcactccctc ctggacgacg cctttgccct cgtggccccc 5220
agcagccagc ctgccagcac cgcaggtgta ggccccggag tcccacccgg cctgcccgca 5280
aacagcaccc cttcccagga agagaggcga gccacccagt gggggtcctt ctacagccca 5340

gcccagacgg ccaacaatcc ctgcagtaga tacgaagact atggaatgac tcccccgacg 5400
ggtccattgc caagaccagg ttttggcccc ggtttgctgc agtctacaga gctggtgccc 5460
cctgaccctc agcagccaca ggcctccgcc gaagccccat ttgctgccag agggatctac 5520
tcggaggaga tgccgtcggt ggcccggcct cggcctgtcg gggtaccac aggctcccag 5580
atccagcacc tgacacaggt ggggattgcc agcagaattg gagctcagcc agtggaaatc 5640
ccgccaagca gaggcagcca gtatgggggg ccaggctggc cttcgtacgg ggaggacgaa 5700
gcggggcgaa gagaggccac acacatgctc ggacatcaag agtattcttc ttcaccgcta 5760
tttcaggtgc caaggacttc aggcagggag ccctcagctc cttccgggaa cctcccccac 5820
cggggactgc agggccctgg gctgggttac cccaccagct ccacggaaga cctccagcct 5880
ggccactcct cggcctctct catcaaagca atccgcgagg agctcctccg gctctcccag 5940
aaacagagca ccgtgcagaa cttccacagc tgatcggcct cgcctcgcag atttgccaag 6000
tatccgcttc ctgtggaagc aagaccaaaa ggaaatcaac tgagtgggtg tttggaagag 6060
gaaggagcaa ctctcgggca gcctgcccaa gggagggagc aagttgcaat ttagaagatg 6120
ccatacgtcg tgtgacagct catgagcctt tcactgggct ggcaattgtc tgaacacttg 6180
ggttcagttg aaatatatgt attttggcca aaagccagca gcacttcaca aaaacaaaac 6240
acaaacctaa gctaacaaaa tgactgcatt cgtctctttt ttaaaggtag agattaaact 6300
gtatagacag catagggatg aaaggaacca agcgtttctg tgggattgag actggtacgt 6360
gtacgatgaa cctgctgctt tgttttctga gaagaggttt gaagacattt tattaacagc 6420
ttaatttttc tcttttactc cataggaact tattttaata gtaacattaa caacaagaat 6480
actaagactg tttgggaatt ttaaaaagct actagtgaga aaccaaatga taggttgtag 6540
agcctgatga ctccaaacaa agccatcacc cgcattcttc ctccttcttc tggtgctaca 6600
gctccaaggg cccttcacct tcatgtctga aatggaactt tggctttttc agtggaagaa 6660
tatgttgaag gtttcatttt gttctagaaa aaaaaaatcc ctcccaaagt ggggcaaaaa 6720
gctttatatt tatttgatta tccaaaatac agatcaaagt ttagatctac attcttcatt 6780
gtatttgctg tttcttaatt gggcacacac aactcctggt catgtcccag ttcagcaccg 6840
atcgctaagg ccgatcctgt agaatgcggc tttcaagagg tcctaactga tcctttcttc 6900
cactttgctg ttgatttgtt cacaaattat gtctgaatga gaaatcttct gtaagcagaa 6960
gttatttaat aattcccagc accacgaaat tgttatacat acatccctac cagtcacatt 7020
ccctgtgttc agagcctgga aatgaaattg agttcagttc agcctctctg taatgaatca 7080
agaaatgtaa aagagctttg agaccccaag ggaaaggaga gagttgcttc tcatttctga 7140
ttttattgtg ctgttactct gtcgagtggc tattaaaatt gtcttatgct ctttgggcta 7200

agaggggatc atctcgctta agatgtactc ctgatgtaaa catttccccc actttccaaa 7260

taatggtaaa gaaaacagtg gcaagggctg ttctgttttc tggtacctga gtgaaactca 7320

gaagaaaagc aggcttagct aagagatttt cactattaac ctgtttttat tagatcagcg 7380

aagacagtca tgcatcgctt aatgatgggg atgcattctg agaaatgtgt cattaggctg 7440

ttttgttgct gtgccaacat cctagattgt acttacacaa cctacatggt atagcctact 7500

acacacctgg gctgtgtggc atggcctatt cctcttaggc tacaaacctg tacagcatgt 7560

tactgtactg aacacaatgg taagtatttg tgtatctaaa catagctaaa catagaaaag 7620

gtatggtaaa aatacaattt tataatctta tgggactacc atcaatatgt agaccgtcat 7680

tgaccaaaac atcctgatgt gtgcatgact gtacttttca ttaaataaca gataaggggc 7740

tatagaattt ggggctctga ctgatcaaaa cctgtgtatc tgtgtcaagt tttaagaccc 7800

ctgaaacagc taaaacaggt ttccaaagtg taatcactgg atttcaaaac catgttttag 7860

cccttcatta atgaagcctg ttgtacagat ttagagtctt acaatatgag ggaagttggt 7920

tctgggaagg taagatgtag ccagttttca tctgggcacc tctgaaagag aaggagtgca 7980

ggagagtaag tctctcaagg acgttcaaca aaggaccagc agcatcttat caggcgatcc 8040

tcagaggctc caaggagcat tggagagaat gccccttctct gggcttgatg tcttggtttt 8100

ggtttaatac caaatttctc ttggtcttgg tttaataccc gactcctccc aaattgaacg 8160

attatctttg tatgtcactc aaaaatacca ggtttcctga atatgttatt aagaatttca 8220

gatatccaag cagaatttta ttatggacac cttgtaatga attcaaatcg tgtgacagca 8280

aacgggataa atggcccctt ctcacccagt tccgctcagg gccatacagg cttgcacata 8340

gagtgtgcta aaaatggtaa cctcctttga gcattgcaga aagagggttc tgtttcaaat 8400

tgggctgacc gtaaaagcaa ttttgatgtc tttcaaacta ccataaaggg attttaactg 8460

tatttttatt cttagaaaca atacaccttt aaacagatgt caagaccaaa gatgatgtat 8520

aataaacagc cggtggttat ggtgtggtgt gagcaggccc ccggatcacc agccctctgc 8580

tggtggtcat aaagcacaca cagtttgtac ttgcttcctc tcgacgcctg ccacaacagt 8640

tttgccagtc accaaactca gaaaaaacta gctgtgtgag cagcaccgta ccctagcggt 8700

cttcagacat taagttcacg tcatccactg aaaggaaggg tccttgcgtt gacagcgtgt 8760

ggctttggag tccgtttatg gaagcactct acaatgaaca gttgaatttt atgtctattc 8820

ttttttccta ttttaaaagt tctagtggta acacaaactg taaatttgag tcagaatgtt 8880

tggagaaatg ttgttttttt ttttttcaga gactactttg tcactcactg accatgtctg 8940

gttccttctc tgaacttcat tctccccata agccaaacaa gaacagacct cccccgccac 9000

ctcccaggca ccgtagttgt gagaatcaga tgtgatcatg tgtgcagatg tcctgtgagg 9060

agggagcata aagcactgtg aaaatgtagc atgctgtcta tgtggacgcc ctaggatgag 9120

tgatgtgaaa cgctgcacta ctgcggtgaa tgggttcaca catgggtaat gagcaaaacc 9180

gaaagctcgg tgtgactcag tttcctcact cccattttgc tttaatttca cttccttcac 9240

caattttccg attgcattta ttaagcatct ttctgctccc gactttctgg tgtctctggc 9300

accaaataac ccagcagaca tgagccttgc cttctgagat tttagaatct aagatagcac 9360

gagtgggtat agaagatgga agataccgat aaataacatg gtttaagtgt caataaaatt 9420

cattcgaaga gatcaggcgc ggtggctcac gcctgtaatc ccagcatttt gggaggctga 9480

ggcaggtgga tcacttgaga tcaagagctt aaaaccagcc caggcaacat ggcaaaaccc 9540

cgtctctaca aaaaaataca aaaactagct gggtgtggtg gcgcatgcct gtagtcccag 9600

ctgaggcacg ataattgctt gaacccgggg ggtggaggtt gcagtgagct gagattgcac 9660

cactgtactc caacctgggc gacagagcaa gactgtgtct caaaaagaaa aaaatttgtt 9720

caaaagacat aaatgaatta ggcacccaga agaagttgca tgtttggaaa tgcaatatct 9780

tggggagacg tcaacttctg aagtgacttt gaaggcaggg cagctaccca ggcaaatggc 9840

gtggaaggaa gagctgagag tgggcctggg aaaggctgta gggaaggagt gaagctgtca 9900

ctgaaatgaa cttgatcttg atctgctttg atatggggac agaatccccg actgcactat 9960

tgagggagtt tcagaagaga aggaagaggt gctggaagga tgcttttgca gtcatgcagc 10020

aaggaaacga ccagggctgt gaagatctac agaggaagta ctccagtgtg ggggaggcaa 10080

gggaagagga cgaaaatgat gagaatgaca ctgaggtcat tgtttagagc agatctcaag 10140

cagccgtttg gccacgccat atcctttgtg gagcatagtg ggttatctat ctgtctgtct 10200

gtctatgtat ctgtctatct atctatatat atattcagct tctttactgc tacctacaca 10260

tttttctcca aattttatta atttcatagt gttttgattt gggtggcaga tgacttttaa 10320

gcagtgggtg tttgccggcc agatctttcc tggcatgcgg actgtgaggc aaagcacggg 10380

tgaaggtagg cgctaaaggc tttgggctaa agccagcacg cggttctgtg ctataggagt 10440

ctcccgtttc ccgtggacag gttcagcgtt ccttctttcg cacaactttt ttctaagtgt 10500

tccagtgacc aagccagtca ttcggacact gatttgcagt gcattggcag taattcacaa 10560

attagttgtt atataagtct ctctcatccc tttcacacta gattctcaga catgaatgaa 10620

tttgtcgttt ggaaggaaag ctgggtaacg ttttgggcac aggggaagga ggactccggt 10680

cttaactccc acgctaactt tagctcaagt ggagttttca ccgtggtcat ttctacctcc 10740

ggagcaaggt gccagcgcca gtactagagc ctgcttatcc acatttgccc tggacaggag 10800

caggaggaag tccacttctg taccggcaga cagagcatgt gaacacaaaa cacatttcta 10860

tggcatagtc aactgaactt catttttaca tttaatctaa catgttaaca cgttctaaca 10920

gggtttctat gagcagctgc tgtaacatac tcatcaacta tgatagactt aacacttgtt 10980

acctaatgaa caaggaggat gtgcatttcg ggtttctttt gatattcgtg gaggtgattg 11040

tcagtgttta aacaggacta ctttctccac tatgaagatg acttggaaat cgcaacatca 11100

tggtacattg ctaagtccat gcttgtgtgt gtgacagtag gcttgataat ttatcttaaa 11160

acacagcagc attgaaattt aggaaaagaa tattaaatgc ctttggaaac atgaaacaaa 11220

gttaggagcc agtaagaaag tgacagaagc aatgaatgtc ttaatgcagt atagttaaaa 11280

tggcttccca cagggtagat aattatccag gatccttcct tttccttctt cctccaggtt 11340

tttcaggggt cacttcacat ttctaaagaa agagtgaata ggctgggcat ggtggctcaa 11400

gcctctaatc tcaacgcttt gggaggctga ggcaggagga tcgcttgagg ccaggagttt 11460

gagaccacct tgggcaacat aacgagaccc cgtctctaca aaaaaaattt aaaaattagc 11520

tgagcatggt ggcatgtgcc agtagtccca gctactcgaa aggctaagac tggaggatcg 11580

cttgagccaa tgagttggag gctgcagtga gctataatca cgccactgca ctccagcctg 11640

ggctgcaggg tgaggtcctg tctctggaaa aaaaaaaaaa aaaaaaagga ataggtaaaa 11700

gggacagagg tgaaattttg agtgacttga gtctcttgca gtccctgatt acacagaacc 11760

tttctgggct acttggagca tcacgaatag tctttcctgt acttaccaga tttcaagtat 11820

tcataacttg actccctaag tgtacaagtt gggaatagta cagggccaag ttcaagtcgc 11880

atatgctgta ctgttcctcc tgcaaatgtg gggaaagaag agggagatac tagaggaact 11940

gaggctccac ccattcattc agttgctcta agcaccagag gacttgtttc agaaaagggg 12000

agtgggaacg ccctcgactt tgccctcctc cggagcatct ctgggacgca gggagtctgg 12060

ctagcgttaa taggaaaggt tgctcggcag agctgccctg gagtactgac ttgtctctcc 12120

ctcctttgtc aaggtccatg tttttctggc tcttcctgca cactcatccc tagattatga 12180

gcgttaatgt acgaaatgtg cagagcaaat tgaggccaac tgtggcatca atactgcaac 12240

ttaaacttga caatcatggt ttgctggtcc tcaaacaggc actgaaatct cagtatgggt 12300

atacgattta ataatcggcc cctcccttct atttgtcggt tttatgtttc tatccttcaa 12360

tagctgcact gttttctaat gtgctgtaga gtttttgaaa taatttatgc aagtatttgg 12420

tttccatgtt tacaatttat acagctttcc tagcatttta aatggaaatg tcaataaatg 12480

ctatgaattg gtgtcaaa 12498

<210> 9
<211> 1934
<212> PRT
<213> Homo sapiens

<400> 9

Met Pro Gly Ala Arg Arg Arg Arg Arg Gly Ala Ala Met Glu Gly Lys
1 5 10 15

Pro Arg Ala Gly Val Ala Leu Ala Pro Gly Pro Ser Gly Arg Arg Pro
20 25 30

Ser Ala Arg Cys Ala Arg Arg Arg Arg Pro Gly Leu Leu Leu Pro Gly
35 40 45

Leu Trp Leu Leu Leu Leu Ala Arg Pro Ala Ser Cys Ala Pro Asp Glu
50 55 60

Leu Ser Pro Glu Gln His Asn Leu Ser Leu Tyr Ser Met Glu Leu Val
65 70 75 80

Leu Lys Lys Ser Thr Gly His Ser Ala Ala Gln Val Ala Leu Thr Glu
85 90 95

Thr Ala Pro Gly Ser Gln His Ser Ser Pro Leu His Val Thr Ala Pro
100 105 110

Pro Ser Ala Thr Thr Phe Asp Thr Ala Phe Phe Asn Gln Gly Lys Gln
115 120 125

Thr Lys Ser Thr Ala Asp Pro Ser Ile Phe Val Ala Thr Tyr Val Ser
130 135 140

Val Thr Ser Lys Glu Val Ala Val Asn Asp Asp Glu Met Asp Asn Phe
145 150 155 160

Leu Pro Asp Thr His Trp Thr Thr Pro Arg Met Val Ser Pro Ile Gln
165 170 175

Tyr Ile Thr Val Ser Pro Pro Gly Leu Pro Arg Glu Ala Leu Glu Pro
180 185 190

Met Leu Thr Pro Ser Leu Pro Met Val Ser Leu Gln Asp Glu Glu Val
195 200 205

Thr Ser Gly Trp Gln Asn Thr Thr Arg Gln Pro Ala Ala Tyr Ala Glu
210 215 220

Ser Ala Ser His Phe His Thr Phe Arg Ser Ala Phe Arg Thr Ser Glu
225 230 235 240

Gly Ile Val Pro Thr Pro Gly Arg Asn Leu Val Leu Tyr Pro Thr Asp
245 250 255

Ala Tyr Ser His Leu Ser Ser Arg Thr Leu Pro Glu Ile Val Ala Ser
260 265 270

Leu Thr Glu Gly Val Glu Thr Thr Leu Phe Leu Ser Ser Arg Ser Leu
275 280 285

Met Pro Gln Pro Leu Gly Asp Gly Ile Thr Ile Pro Leu Pro Ser Leu
290 295 300

Gly Glu Val Ser Gln Pro Pro Glu Glu Val Trp Ala Thr Ser Ala Asp
305 310 315 320

Arg Tyr Thr Asp Val Thr Thr Val Leu Ser Gln Ser Leu Glu Glu Thr
325 330 335

Ile Ser Pro Arg Thr Tyr Pro Thr Val Thr Ala Ser His Ala Ala Leu
340 345 350

Ala Phe Ser Arg Thr His Ser Pro Leu Leu Ser Thr Pro Leu Ala Phe
355 360 365

Ala Ser Ser Ala Ser Pro Thr Asp Val Ser Ser Asn Pro Phe Leu Pro
370 375 380

Ser Asp Ser Ser Lys Thr Ser Glu Leu His Ser Asn Ser Ala Leu Pro
385 390 395 400

Gly Pro Val Asp Asn Thr His Ile Leu Ser Pro Val Ser Ser Phe Arg
405 410 415

Pro Tyr Thr Trp Cys Ala Ala Cys Thr Val Pro Ser Pro Gln Gln Val
420 425 430

Leu Ala Thr Ser Leu Met Glu Lys Asp Val Gly Ser Gly Asp Gly Ala
435 440 445

Glu Thr Leu Cys Met Thr Val Leu Glu Glu Ser Ser Ile Ser Leu Met
450 455 460

Ser Ser Val Val Ala Asp Phe Ser Glu Phe Glu Glu Asp Pro Gln Val
465 470 475 480

Phe Asn Thr Leu Phe Pro Ser Arg Pro Ile Val Pro Leu Ser Ser Arg
485 490 495

Ser Met Glu Ile Ser Glu Thr Ser Val Gly Ile Ser Ala Glu Val Asp
500 505 510

Met Ser Ser Val Thr Thr Thr Gln Val Pro Pro Ala His Gly Arg Leu

515 520 525

Ser Val Pro Ala Ser Leu Asp Pro Thr Ala Gly Ser Leu Ser Val Ala
530 535 540

Glu Thr Gln Val Thr Pro Ser Ser Val Thr Thr Ala Phe Phe Ser Val
545 550 555 560

Ile Thr Ser Ile Leu Leu Asp Ser Ser Phe Ser Val Ile Ala Asn Lys
565 570 575

Asn Thr Pro Ser Leu Ala Val Arg Asp Pro Ser Val Phe Thr Pro Tyr
580 585 590

Ser Leu Val Pro Ser Val Glu Ser Ser Leu Phe Ser Asp Gln Glu Arg
595 600 605

Ser Ser Phe Ser Glu His Lys Pro Arg Gly Ala Leu Asp Phe Ala Ser
610 615 620

Ser Phe Phe Ser Thr Pro Pro Leu Glu Leu Ser Gly Ser Ile Ser Ser
625 630 635 640

Pro Ser Glu Ala Pro Ala Ser Leu Ser Leu Met Pro Ser Asp Leu Ser
645 650 655

Pro Phe Thr Ser Gln Ser Phe Ser Pro Leu Val Glu Thr Phe Thr Leu
660 665 670

Phe Asp Ser Ser Asp Leu Gln Ser Ser Gln Leu Ser Leu Pro Ser Ser
675 680 685

Thr Asn Leu Glu Phe Ser Gln Leu Gln Pro Ser Ser Glu Leu Pro Leu
690 695 700

Asn Thr Ile Met Leu Leu Pro Ser Arg Ser Glu Val Ser Pro Trp Ser
705 710 715 720

Ser Phe Pro Ser Asp Ser Leu Glu Phe Val Glu Ala Ser Thr Val Ser
725 730 735

Leu Thr Asp Ser Glu Ala His Phe Thr Ser Ala Phe Ile Glu Thr Thr
740 745 750

Ser Tyr Leu Glu Ser Ser Leu Ile Ser His Glu Ser Ala Val Thr Ala
755 760 765

```
Leu Val Pro Pro Gly Ser Glu Ser Phe Asp Ile Leu Thr Ala Gly Ile
    770                 775                 780

Gln Ala Thr Ser Pro Leu Thr Thr Val His Thr Thr Pro Ile Leu Thr
785                 790                 795                 800

Glu Ser Ser Leu Phe Ser Thr Leu Thr Pro Pro Asp Asp Gln Ile Ser
                805                 810                 815

Ala Leu Asp Gly His Val Ser Val Leu Ala Ser Phe Ser Lys Ala Ile
            820                 825                 830

Pro Thr Gly Thr Val Leu Ile Thr Asp Ala Tyr Leu Pro Ser Gly Ser
        835                 840                 845

Ser Phe Val Ser Glu Ala Thr Pro Phe Pro Leu Pro Thr Glu Leu Thr
    850                 855                 860

Val Val Gly Pro Ser Leu Thr Pro Thr Glu Val Pro Leu Asn Thr Ser
865                 870                 875                 880

Thr Glu Val Ser Thr Thr Ser Thr Gly Ala Ala Thr Gly Gly Pro Leu
                885                 890                 895

Asp Ser Thr Leu Met Gly Asp Ala Ala Ser Gln Ser Pro Pro Glu Ser
            900                 905                 910

Ser Ala Ala Pro Pro Leu Pro Ser Leu Arg Pro Val Thr Ala Phe Thr
        915                 920                 925

Leu Glu Ala Thr Val Asp Thr Pro Thr Leu Ala Thr Ala Lys Pro Pro
    930                 935                 940

Tyr Val Cys Asp Ile Thr Val Pro Asp Ala Tyr Leu Ile Thr Thr Val
945                 950                 955                 960

Leu Ala Arg Arg Ala Val Gln Glu Tyr Ile Ile Thr Ala Ile Lys Glu
                965                 970                 975

Val Leu Arg Ile His Phe Asn Arg Ala Val Glu Leu Lys Val Tyr Glu
            980                 985                 990

Leu Phe Thr Asp Phe Thr Phe Leu  Val Thr Ser Gly Pro  Phe Val Tyr
        995                 1000                1005

Thr Ala  Ile Ser Val Ile Asn  Val Leu Ile Asn Ser  Lys Leu Val
    1010                1015                1020
```

Arg Asp Gln Thr Pro Leu Ile Leu Ser Val Lys Pro Ser Phe Leu
1025 1030 1035

Val Pro Glu Ser Arg Phe Gln Val Gln Thr Val Leu Gln Phe Val
1040 1045 1050

Pro Pro Ser Val Asp Thr Gly Phe Cys Asn Phe Thr Gln Arg Ile
1055 1060 1065

Glu Lys Gly Leu Met Thr Ala Leu Phe Glu Val Arg Lys His His
1070 1075 1080

Gln Gly Thr Tyr Asn Leu Thr Val Gln Ile Leu Asn Ile Thr Ile
1085 1090 1095

Ser Ser Ser Arg Val Thr Pro Arg Arg Gly Pro Val Asn Ile Ile
1100 1105 1110

Phe Ala Val Lys Ser Thr Gln Gly Phe Leu Asn Gly Ser Glu Val
1115 1120 1125

Ser Glu Leu Leu Arg Asn Leu Ser Val Val Glu Phe Ser Phe Tyr
1130 1135 1140

Leu Gly Tyr Pro Val Leu Gln Ile Ala Glu Pro Phe Gln Tyr Pro
1145 1150 1155

Gln Leu Asn Leu Ser Gln Leu Leu Lys Ser Ser Trp Val Arg Thr
1160 1165 1170

Val Leu Leu Gly Val Met Glu Lys Gln Leu Gln Asn Glu Val Phe
1175 1180 1185

Gln Ala Glu Met Glu Arg Lys Leu Ala Gln Leu Leu Ser Glu Val
1190 1195 1200

Ser Thr Arg Arg Arg Met Trp Arg Arg Ala Thr Val Ala Ala Gly
1205 1210 1215

Asn Ser Val Val Gln Val Val Asn Val Ser Arg Leu Glu Gly Asp
1220 1225 1230

Asp Asn Pro Val Gln Leu Ile Tyr Phe Val Glu Asp Gln Asp Gly
1235 1240 1245

Glu Arg Leu Ser Ala Val Lys Ser Ser Asp Leu Ile Asn Lys Met
1250 1255 1260

Asp Leu Gln Arg Ala Ala Ile Ile Leu Gly Tyr Arg Ile Gln Gly
1265 1270 1275

Val Ile Ala Gln Pro Val Asp Arg Val Lys Arg Pro Ser Pro Glu
1280 1285 1290

Ser Gln Ser Asn Asn Leu Trp Val Ile Val Gly Val Val Ile Pro
1295 1300 1305

Val Leu Val Val Met Val Ile Val Val Ile Leu Tyr Trp Lys Leu
1310 1315 1320

Cys Arg Thr Asp Lys Leu Asp Phe Gln Pro Asp Thr Val Ala Asn
1325 1330 1335

Ile Gln Gln Arg Gln Lys Leu Gln Ile Pro Ser Val Lys Gly Phe
1340 1345 1350

Asp Phe Ala Lys Gln His Leu Gly Gln His Asn Lys Asp Asp Ile
1355 1360 1365

Leu Ile Ile His Glu Pro Ala Pro Leu Pro Gly Pro Leu Lys Asp
1370 1375 1380

His Thr Thr Pro Ser Glu Asn Gly Asp Val Pro Ser Pro Lys Ser
1385 1390 1395

Lys Ile Pro Ser Lys Asn Val Arg His Arg Gly Arg Val Ser Pro
1400 1405 1410

Ser Asp Ala Asp Ser Thr Val Ser Glu Glu Ser Ser Glu Arg Asp
1415 1420 1425

Ala Gly Asp Lys Thr Pro Gly Ala Val Asn Asp Gly Arg Ser His
1430 1435 1440

Arg Ala Pro Gln Ser Gly Pro Pro Leu Pro Ser Ser Gly Asn Glu
1445 1450 1455

Gln His Ser Ser Ala Ser Ile Phe Glu His Val Asp Arg Ile Ser
1460 1465 1470

Arg Pro Pro Glu Ala Ser Arg Arg Val Pro Ser Lys Ile Gln Leu
1475 1480 1485

Ile Ala Met Gln Pro Ile Pro Ala Pro Pro Val Gln Arg Pro Ser

1490 1495 1500

Pro Ala Asp Arg Val Ala Glu Ser Asn Lys Ile Asn Lys Glu Ile
1505 1510 1515

Gln Thr Ala Leu Arg His Lys Ser Glu Ile Glu His His Arg Asn
1520 1525 1530

Lys Ile Arg Leu Arg Ala Lys Arg Arg Gly His Tyr Glu Phe Pro
1535 1540 1545

Val Val Asp Asp Leu Ser Ser Gly Asp Thr Lys Glu Arg His Arg
1550 1555 1560

Val Tyr Arg Arg Ala Gln Met Gln Ile Asp Lys Ile Leu Asp Pro
1565 1570 1575

Thr Ala Ser Val Pro Ser Val Phe Ile Glu Pro Arg Lys Ser Ser
1580 1585 1590

Arg Ile Lys Arg Ser Pro Lys Pro Arg Arg Lys His Gln Val Asn
1595 1600 1605

Gly Cys Pro Ala Asp Ala Glu Lys Asp Arg Leu Ile Thr Thr Asp
1610 1615 1620

Ser Asp Gly Thr Tyr Arg Arg Pro Pro Gly Val His Asn Ser Ala
1625 1630 1635

Tyr Ile Gly Cys Pro Ser Asp Pro Asp Leu Pro Ala Asp Val Gln
1640 1645 1650

Thr Pro Ser Ser Val Glu Leu Gly Arg Tyr Pro Ala Leu Pro Phe
1655 1660 1665

Pro Ala Ser Gln Tyr Ile Pro Pro Gln Pro Ser Ile Glu Glu Ala
1670 1675 1680

Arg Gln Thr Met His Ser Leu Leu Asp Asp Ala Phe Ala Leu Val
1685 1690 1695

Ala Pro Ser Ser Gln Pro Ala Ser Thr Ala Gly Val Gly Pro Gly
1700 1705 1710

Val Pro Pro Gly Leu Pro Ala Asn Ser Thr Pro Ser Gln Glu Glu
1715 1720 1725

```
Arg Arg  Ala Thr Gln Trp Gly  Ser Phe Tyr Ser Pro  Ala Gln Thr
    1730                 1735                 1740

Ala Asn  Asn Pro Cys Ser Arg  Tyr Glu Asp Tyr Gly  Met Thr Pro
    1745                 1750                 1755

Pro Thr  Gly Pro Leu Pro Arg  Pro Gly Phe Gly Pro  Gly Leu Leu
    1760                 1765                 1770

Gln Ser  Thr Glu Leu Val Pro  Pro Asp Pro Gln Gln  Pro Gln Ala
    1775                 1780                 1785

Ser Ala  Glu Ala Pro Phe Ala  Ala Arg Gly Ile Tyr  Ser Glu Glu
    1790                 1795                 1800

Met Pro  Ser Val Ala Arg Pro  Arg Pro Val Gly Gly  Thr Thr Gly
    1805                 1810                 1815

Ser Gln  Ile Gln His Leu Thr  Gln Val Gly Ile Ala  Ser Arg Ile
    1820                 1825                 1830

Gly Ala  Gln Pro Val Glu Ile  Pro Pro Ser Arg Gly  Ser Gln Tyr
    1835                 1840                 1845

Gly Gly  Pro Gly Trp Pro Ser  Tyr Gly Glu Asp Glu  Ala Gly Arg
    1850                 1855                 1860

Arg Glu  Ala Val Pro Arg Thr  Ser Gly Arg Glu Pro  Ser Ala Pro
    1865                 1870                 1875

Ser Gly  Asn Leu Pro His Arg  Gly Leu Gln Gly Pro  Gly Leu Gly
    1880                 1885                 1890

Tyr Pro  Thr Ser Ser Thr Glu  Asp Leu Gln Pro Gly  His Ser Ser
    1895                 1900                 1905

Ala Ser  Leu Ile Lys Ala Ile  Arg Glu Glu Leu Leu  Arg Leu Ser
    1910                 1915                 1920

Gln Lys  Gln Ser Thr Val Gln  Asn Phe His Ser
    1925                 1930

<210>  10
<211>  1950
<212>  PRT
<213>  Homo sapiens

<400>  10
```

```
Met Pro Gly Ala Arg Arg Arg Arg Arg Gly Ala Ala Met Glu Gly Lys
1               5                   10                  15

Pro Arg Ala Gly Val Ala Leu Ala Pro Gly Pro Ser Gly Arg Arg Pro
            20                  25                  30

Ser Ala Arg Cys Ala Arg Arg Arg Arg Pro Gly Leu Leu Leu Pro Gly
        35                  40                  45

Leu Trp Leu Leu Leu Leu Ala Arg Pro Ala Ser Cys Ala Pro Asp Glu
    50                  55                  60

Leu Ser Pro Glu Gln His Asn Leu Ser Leu Tyr Ser Met Glu Leu Val
65                  70                  75                  80

Leu Lys Lys Ser Thr Gly His Ser Ala Ala Gln Val Ala Leu Thr Glu
                85                  90                  95

Thr Ala Pro Gly Ser Gln His Ser Ser Pro Leu His Val Thr Ala Pro
            100                 105                 110

Pro Ser Ala Thr Thr Phe Asp Thr Ala Phe Phe Asn Gln Gly Lys Gln
        115                 120                 125

Thr Lys Ser Thr Ala Asp Pro Ser Ile Phe Val Ala Thr Tyr Val Ser
    130                 135                 140

Val Thr Ser Lys Glu Val Ala Val Asn Asp Asp Glu Met Asp Asn Phe
145                 150                 155                 160

Leu Pro Asp Thr His Trp Thr Thr Pro Arg Met Val Ser Pro Ile Gln
                165                 170                 175

Tyr Ile Thr Val Ser Pro Pro Gly Leu Pro Arg Glu Ala Leu Glu Pro
            180                 185                 190

Met Leu Thr Pro Ser Leu Pro Met Val Ser Leu Gln Asp Glu Glu Val
        195                 200                 205

Thr Ser Gly Trp Gln Asn Thr Thr Arg Gln Pro Ala Ala Tyr Ala Glu
    210                 215                 220

Ser Ala Ser His Phe His Thr Phe Arg Ser Ala Phe Arg Thr Ser Glu
225                 230                 235                 240

Gly Ile Val Pro Thr Pro Gly Arg Asn Leu Val Leu Tyr Pro Thr Asp
                245                 250                 255
```

Ala Tyr Ser His Leu Ser Ser Arg Thr Leu Pro Glu Ile Val Ala Ser
260 265 270

Leu Thr Glu Gly Val Glu Thr Thr Leu Phe Leu Ser Ser Arg Ser Leu
275 280 285

Met Pro Gln Pro Leu Gly Asp Gly Ile Thr Ile Pro Leu Pro Ser Leu
290 295 300

Gly Glu Val Ser Gln Pro Pro Glu Glu Val Trp Ala Thr Ser Ala Asp
305 310 315 320

Arg Tyr Thr Asp Val Thr Thr Val Leu Ser Gln Ser Leu Glu Glu Thr
325 330 335

Ile Ser Pro Arg Thr Tyr Pro Thr Val Thr Ala Ser His Ala Ala Leu
340 345 350

Ala Phe Ser Arg Thr His Ser Pro Leu Leu Ser Thr Pro Leu Ala Phe
355 360 365

Ala Ser Ser Ala Ser Pro Thr Asp Val Ser Ser Asn Pro Phe Leu Pro
370 375 380

Ser Asp Ser Ser Lys Thr Ser Glu Leu His Ser Asn Ser Ala Leu Pro
385 390 395 400

Gly Pro Val Asp Asn Thr His Ile Leu Ser Pro Val Ser Ser Phe Arg
405 410 415

Pro Tyr Thr Trp Cys Ala Ala Cys Thr Val Pro Ser Pro Gln Gln Val
420 425 430

Leu Ala Thr Ser Leu Met Glu Lys Asp Val Gly Ser Gly Asp Gly Ala
435 440 445

Glu Thr Leu Cys Met Thr Val Leu Glu Glu Ser Ser Ile Ser Leu Met
450 455 460

Ser Ser Val Val Ala Asp Phe Ser Glu Phe Glu Glu Asp Pro Gln Val
465 470 475 480

Phe Asn Thr Leu Phe Pro Ser Arg Pro Ile Val Pro Leu Ser Ser Arg
485 490 495

Ser Met Glu Ile Ser Glu Thr Ser Val Gly Ile Ser Ala Glu Val Asp
500 505 510

```
Met Ser Ser Val Thr Thr Thr Gln Val Pro Pro Ala His Gly Arg Leu
        515                 520                 525

Ser Val Pro Ala Ser Leu Asp Pro Thr Ala Gly Ser Leu Ser Val Ala
    530                 535                 540

Glu Thr Gln Val Thr Pro Ser Ser Val Thr Thr Ala Phe Phe Ser Val
545                 550                 555                 560

Ile Thr Ser Ile Leu Leu Asp Ser Ser Phe Ser Val Ile Ala Asn Lys
                565                 570                 575

Asn Thr Pro Ser Leu Ala Val Arg Asp Pro Ser Val Phe Thr Pro Tyr
            580                 585                 590

Ser Leu Val Pro Ser Val Glu Ser Ser Leu Phe Ser Asp Gln Glu Arg
        595                 600                 605

Ser Ser Phe Ser Glu His Lys Pro Arg Gly Ala Leu Asp Phe Ala Ser
    610                 615                 620

Ser Phe Phe Ser Thr Pro Pro Leu Glu Leu Ser Gly Ser Ile Ser Ser
625                 630                 635                 640

Pro Ser Glu Ala Pro Ala Ser Leu Ser Leu Met Pro Ser Asp Leu Ser
                645                 650                 655

Pro Phe Thr Ser Gln Ser Phe Ser Pro Leu Val Glu Thr Phe Thr Leu
            660                 665                 670

Phe Asp Ser Ser Asp Leu Gln Ser Ser Gln Leu Ser Leu Pro Ser Ser
        675                 680                 685

Thr Asn Leu Glu Phe Ser Gln Leu Gln Pro Ser Ser Glu Leu Pro Leu
    690                 695                 700

Asn Thr Ile Met Leu Leu Pro Ser Arg Ser Glu Val Ser Pro Trp Ser
705                 710                 715                 720

Ser Phe Pro Ser Asp Ser Leu Glu Phe Val Glu Ala Ser Thr Val Ser
                725                 730                 735

Leu Thr Asp Ser Glu Ala His Phe Thr Ser Ala Phe Ile Glu Thr Thr
            740                 745                 750

Ser Tyr Leu Glu Ser Ser Leu Ile Ser His Glu Ser Ala Val Thr Ala
```

755 760 765

Leu Val Pro Pro Gly Ser Glu Ser Phe Asp Ile Leu Thr Ala Gly Ile
770 775 780

Gln Ala Thr Ser Pro Leu Thr Thr Val His Thr Thr Pro Ile Leu Thr
785 790 795 800

Glu Ser Ser Leu Phe Ser Thr Leu Thr Pro Pro Asp Asp Gln Ile Ser
805 810 815

Ala Leu Asp Gly His Val Ser Val Leu Ala Ser Phe Ser Lys Ala Ile
820 825 830

Pro Thr Gly Thr Val Leu Ile Thr Asp Ala Tyr Leu Pro Ser Gly Ser
835 840 845

Ser Phe Val Ser Glu Ala Thr Pro Phe Pro Leu Pro Thr Glu Leu Thr
850 855 860

Val Val Gly Pro Ser Leu Thr Pro Thr Glu Val Pro Leu Asn Thr Ser
865 870 875 880

Thr Glu Val Ser Thr Thr Ser Thr Gly Ala Ala Thr Gly Gly Pro Leu
885 890 895

Asp Ser Thr Leu Met Gly Asp Ala Ala Ser Gln Ser Pro Pro Glu Ser
900 905 910

Ser Ala Ala Pro Pro Leu Pro Ser Leu Arg Pro Val Thr Ala Phe Thr
915 920 925

Leu Glu Ala Thr Val Asp Thr Pro Thr Leu Ala Thr Ala Lys Pro Pro
930 935 940

Tyr Val Cys Asp Ile Thr Val Pro Asp Ala Tyr Leu Ile Thr Thr Val
945 950 955 960

Leu Ala Arg Arg Ala Val Gln Glu Tyr Ile Ile Thr Ala Ile Lys Glu
965 970 975

Val Leu Arg Ile His Phe Asn Arg Ala Val Glu Leu Lys Val Tyr Glu
980 985 990

Leu Phe Thr Asp Phe Thr Phe Leu Val Thr Ser Gly Pro Phe Val Tyr
995 1000 1005

Thr Ala Ile Ser Val Ile Asn Val Leu Ile Asn Ser Lys Leu Val
1010 1015 1020

Arg Asp Gln Thr Pro Leu Ile Leu Ser Val Lys Pro Ser Phe Leu
1025 1030 1035

Val Pro Glu Ser Arg Phe Gln Val Gln Thr Val Leu Gln Phe Val
1040 1045 1050

Pro Pro Ser Val Asp Thr Gly Phe Cys Asn Phe Thr Gln Arg Ile
1055 1060 1065

Glu Lys Gly Leu Met Thr Ala Leu Phe Glu Val Arg Lys His His
1070 1075 1080

Gln Gly Thr Tyr Asn Leu Thr Val Gln Ile Leu Asn Ile Thr Ile
1085 1090 1095

Ser Ser Ser Arg Val Thr Pro Arg Arg Gly Pro Val Asn Ile Ile
1100 1105 1110

Phe Ala Val Lys Ser Thr Gln Gly Phe Leu Asn Gly Ser Glu Val
1115 1120 1125

Ser Glu Leu Leu Arg Asn Leu Ser Val Val Glu Phe Ser Phe Tyr
1130 1135 1140

Leu Gly Tyr Pro Val Leu Gln Ile Ala Glu Pro Phe Gln Tyr Pro
1145 1150 1155

Gln Leu Asn Leu Ser Gln Leu Leu Lys Ser Ser Trp Val Arg Thr
1160 1165 1170

Val Leu Leu Gly Val Met Glu Lys Gln Leu Gln Asn Glu Val Phe
1175 1180 1185

Gln Ala Glu Met Glu Arg Lys Leu Ala Gln Leu Leu Ser Glu Val
1190 1195 1200

Ser Thr Arg Arg Arg Met Trp Arg Arg Ala Thr Val Ala Ala Gly
1205 1210 1215

Asn Ser Val Val Gln Val Val Asn Val Ser Arg Leu Glu Gly Asp
1220 1225 1230

Asp Asn Pro Val Gln Leu Ile Tyr Phe Val Glu Asp Gln Asp Gly
1235 1240 1245

```
Glu Arg  Leu Ser Ala Val Lys  Ser Ser Asp Leu Ile  Asn Lys Met
    1250                 1255                 1260

Asp Leu  Gln Arg Ala Ala Ile  Ile Leu Gly Tyr Arg  Ile Gln Gly
    1265                 1270                 1275

Val Ile  Ala Gln Pro Val Asp  Arg Val Lys Arg Pro  Ser Pro Glu
    1280                 1285                 1290

Ser Gln  Ser Asn Asn Leu Trp  Val Ile Val Gly Val  Val Ile Pro
    1295                 1300                 1305

Val Leu  Val Val Met Val Ile  Val Val Ile Leu Tyr  Trp Lys Leu
    1310                 1315                 1320

Cys Arg  Thr Asp Lys Leu Asp  Phe Gln Pro Asp Thr  Val Ala Asn
    1325                 1330                 1335

Ile Gln  Gln Arg Gln Lys Leu  Gln Ile Pro Ser Val  Lys Gly Phe
    1340                 1345                 1350

Asp Phe  Ala Lys Gln His Leu  Gly Gln His Asn Lys  Asp Asp Ile
    1355                 1360                 1365

Leu Ile  Ile His Glu Pro Ala  Pro Leu Pro Gly Pro  Leu Lys Asp
    1370                 1375                 1380

His Thr  Thr Pro Ser Glu Asn  Gly Asp Val Pro Ser  Pro Lys Ser
    1385                 1390                 1395

Lys Ile  Pro Ser Lys Asn Val  Arg His Arg Gly Arg  Val Ser Pro
    1400                 1405                 1410

Ser Asp  Ala Asp Ser Thr Val  Ser Glu Glu Ser Ser  Glu Arg Asp
    1415                 1420                 1425

Ala Gly  Asp Lys Thr Pro Gly  Ala Val Asn Asp Gly  Arg Ser His
    1430                 1435                 1440

Arg Ala  Pro Gln Ser Gly Pro  Pro Leu Pro Ser Ser  Gly Asn Glu
    1445                 1450                 1455

Gln His  Ser Ser Ala Ser Ile  Phe Glu His Val Asp  Arg Ile Ser
    1460                 1465                 1470

Arg Pro  Pro Glu Ala Ser Arg  Arg Val Pro Ser Lys  Ile Gln Leu
    1475                 1480                 1485
```

Ile Ala Met Gln Pro Ile Pro Ala Pro Pro Val Gln Arg Pro Ser
1490 1495 1500

Pro Ala Asp Arg Val Ala Glu Ser Asn Lys Ile Asn Lys Glu Ile
1505 1510 1515

Gln Thr Ala Leu Arg His Lys Ser Glu Ile Glu His His Arg Asn
1520 1525 1530

Lys Ile Arg Leu Arg Ala Lys Arg Arg Gly His Tyr Glu Phe Pro
1535 1540 1545

Val Val Asp Asp Leu Ser Ser Gly Asp Thr Lys Glu Arg His Arg
1550 1555 1560

Val Tyr Arg Arg Ala Gln Met Gln Ile Asp Lys Ile Leu Asp Pro
1565 1570 1575

Thr Ala Ser Val Pro Ser Val Phe Ile Glu Pro Arg Lys Ser Ser
1580 1585 1590

Arg Ile Lys Arg Ser Pro Lys Pro Arg Arg Lys His Gln Val Asn
1595 1600 1605

Gly Cys Pro Ala Asp Ala Glu Lys Asp Arg Leu Ile Thr Thr Asp
1610 1615 1620

Ser Asp Gly Thr Tyr Arg Arg Pro Pro Gly Val His Asn Ser Ala
1625 1630 1635

Tyr Ile Gly Cys Pro Ser Asp Pro Asp Leu Pro Ala Asp Val Gln
1640 1645 1650

Thr Pro Ser Ser Val Glu Leu Gly Arg Tyr Pro Ala Leu Pro Phe
1655 1660 1665

Pro Ala Ser Gln Tyr Ile Pro Pro Gln Pro Ser Ile Glu Glu Ala
1670 1675 1680

Arg Gln Thr Met His Ser Leu Leu Asp Asp Ala Phe Ala Leu Val
1685 1690 1695

Ala Pro Ser Ser Gln Pro Ala Ser Thr Ala Gly Val Gly Pro Gly
1700 1705 1710

Val Pro Pro Gly Leu Pro Ala Asn Ser Thr Pro Ser Gln Glu Glu

1715 1720 1725

Arg Arg Ala Thr Gln Trp Gly Ser Phe Tyr Ser Pro Ala Gln Thr
1730 1735 1740

Ala Asn Asn Pro Cys Ser Arg Tyr Glu Asp Tyr Gly Met Thr Pro
1745 1750 1755

Pro Thr Gly Pro Leu Pro Arg Pro Gly Phe Gly Pro Gly Leu Leu
1760 1765 1770

Gln Ser Thr Glu Leu Val Pro Pro Asp Pro Gln Gln Pro Gln Ala
1775 1780 1785

Ser Ala Glu Ala Pro Phe Ala Ala Arg Gly Ile Tyr Ser Glu Glu
1790 1795 1800

Met Pro Ser Val Ala Arg Pro Arg Pro Val Gly Gly Thr Thr Gly
1805 1810 1815

Ser Gln Ile Gln His Leu Thr Gln Val Gly Ile Ala Ser Arg Ile
1820 1825 1830

Gly Ala Gln Pro Val Glu Ile Pro Pro Ser Arg Gly Ser Gln Tyr
1835 1840 1845

Gly Gly Pro Gly Trp Pro Ser Tyr Gly Glu Asp Glu Ala Gly Arg
1850 1855 1860

Arg Glu Ala Thr His Met Leu Gly His Gln Glu Tyr Ser Ser Ser
1865 1870 1875

Pro Leu Phe Gln Val Pro Arg Thr Ser Gly Arg Glu Pro Ser Ala
1880 1885 1890

Pro Ser Gly Asn Leu Pro His Arg Gly Leu Gln Gly Pro Gly Leu
1895 1900 1905

Gly Tyr Pro Thr Ser Ser Thr Glu Asp Leu Gln Pro Gly His Ser
1910 1915 1920

Ser Ala Ser Leu Ile Lys Ala Ile Arg Glu Glu Leu Leu Arg Leu
1925 1930 1935

Ser Gln Lys Gln Ser Thr Val Gln Asn Phe His Ser
1940 1945 1950

<210> 11
<211> 8240
<212> DNA
<213> Homo sapiens

<400> 11
cccctctcgg tagccctgag gctctggcgc cttcaagtga gaagctaagc accagcctct 60

gctgggctgc agaagcggcg gcggcggcag cagcagcagc agcatcagga aggcgctcgg 120

gccagcgcgg tgaacccggg ctgggcagca ggtcgcggag ccgcgagcca ggatggaggc 180

agagggcagc agcgcgccgg cccgggcggg cagcggagag ggcagcgaca gcgccggcgg 240

ggccacgctc aaagccccca agcatctctg gaggcacgag cagcaccacc agtacccgct 300

ccggcagccc cagttccgcc tcctgcatcc ccatcaccac ctgcccccgc cgccgccacc 360

ctcgccccag ccccagcccc agtgtccgct acagccgccg ccgccgcccc ccctgccgcc 420

gcccccgccg ccgcccgggg ctgcccgcgg ccgctacgcc tcgagcgggg ccaccggccg 480

cgtccggcat cgcggctact cggacaccga gcgctacctg tactgtcgcg ccatggaccg 540

cacctcctac gcggtggaga ccggccaccg gcccggcctg aagaaatcca ggatgtcctg 600

gccctcctcg ttccagggac tcaggcgttt tgatgtggac aatggcacat ctgcgggacg 660

gagtcccttg gatcccatga ccagcccagg atccgggcta attctccaag caaattttgt 720

ccacagtcaa cgacgggagt ccttcctgta tcgatccgac agcgattatg acctctctcc 780

aaagtctatg tcccggaact cctccattgc cagtgatata cacggagatg acttgattgt 840

gactccattt gctcaggtct tggccagtct gcgaactgta cgaaacaact ttgctgcatt 900

aactaatttg caagatcgag cacctagcaa aagatcaccc atgtgcaacc aaccatccat 960

caacaaagcc accataacag aggaggccta ccagaaactg gccagcgaga ccctggagga 1020

gctggactgg tgtctggacc agctagagac cctacagacc aggcactccg tcagtgagat 1080

ggcctccaac aagtttaaaa ggatgcttaa tcgggagctc acccatctct ctgaaatgag 1140

tcggtctgga aatcaagtgt cagagtttat atcaaacaca ttcttagata agcaacatga 1200

agtggaaatt ccttctccaa ctcagaagga aaaggagaaa aagaaaagac caatgtctca 1260

gatcagtgga gtcaagaaat tgatgcacag ctctagtctg actaattcaa gtatcccaag 1320

gtttggagtt aaaactgaac aagaagatgt ccttgccaag gaactagaag atgtgaacaa 1380

atggggtctt catgttttca gaatagcaga gttgtctggt aaccggccct tgactgttat 1440

catgcacacc atttttcagg aacgggattt attaaaaaca tttaaaattc cagtagatac 1500

tttaattaca tatcttatga ctctcgaaga ccattaccat gctgatgtgg cctatcacaa 1560

caatatccat gctgcagatg ttgtccagtc tactcatgtg ctattatcta cacctgcttt 1620

ggaggctgtg tttacagatt tggagattct tgcagcaatt tttgccagtg caatacatga 1680

tgtagatcat cctggtgtgt ccaatcaatt tctgatcaat acaaactctg aacttgcctt 1740

gatgtacaat gattcctcag tcttagagaa ccatcatttg gctgtgggct ttaaattgct 1800
tcaggaagaa aactgtgaca ttttccagaa tttgaccaaa aaacaaagac aatctttaag 1860
gaaaatggtc attgacatcg tacttgcaac agatatgtca aaacacatga atctactggc 1920
tgatttgaag actatggttg aaactaagaa agtgacaagc tctggagttc ttcttcttga 1980
taattattcc gataggattc aggttcttca gaatatggtg cactgtgcag atctgagcaa 2040
cccaacaaag cctctccagc tgtaccgcca gtggacggac cggataatgg aggagttctt 2100
ccgccaagga gaccgagaga gggaacgtgg catggagata agccccatgt gtgacaagca 2160
caatgcttcc gtggaaaaat cacaggtggg cttcatagac tatattgttc atcccctctg 2220
ggagacatgg gcagacctcg tccaccctga cgcccaggat attttggaca ctttggagga 2280
caatcgtgaa tggtaccaga gcacaatccc tcagagcccc tctcctgcac ctgatgaccc 2340
agaggagggc cggcagggtc aaactgagaa attccagttt gaactaactt tagaggaaga 2400
tggtgagtca gacacggaaa aggacagtgg cagtcaagtg gaagaagaca ctagctgcag 2460
tgactccaag actctttgta ctcaagactc agagtctact gaaattcccc ttgatgaaca 2520
ggttgaagag gaggcagtag gggaagaaga ggaaagccag cctgaagcct gtgtcataga 2580
tgatcgttct cctgacacgt aacagtgcaa aaactttcat gccttttttt tttttaagta 2640
gaaaaattgt ttccaaagtg catgtcacat gccacaacca cggtcacacc tcactgtcat 2700
ctgccaggac gtttgttgaa caaaactgac cttgactact cagtccagcg ctcaggaata 2760
tcgtaaccag tttttcacc tccatgtcat ccgagcaagg tggacatctt cacgaacagc 2820
gtttttaaca agatttcagc ttggtagagc tgacaaagca gataaaatct actccaaatt 2880
attttcaaga gagtgtgact catcaggcag cccaaaagtt tattggactt ggggtttcta 2940
ttccttttta tttgtttgca atattttcag aagaaaggca ttgcacagag tgaacttaat 3000
ggacgaagca acaaatatgt caagaacagg acatagcacg aatctgttac cagtaggagg 3060
aggatgagcc acagaaattg cataattttc taatttcaag tcttcctgat acatgactga 3120
atagtgtggt tcagtgagct gcactgacct ctacattttg tatgatatgt aaaacagatt 3180
ttttgtagag cttactttta ttattaaatg tattgaggta ttatatttaa aaaaaactat 3240
gttcagaact tcatctgcca ctggttattt ttttctaagg agtaacttgc aagttttcag 3300
tacaaatctg tgctacactg gataaaaatc taatttatga attttacttg caccttatag 3360
ttcatagcaa ttaactgatt tgtagtgatt cattgtttgt tttatatacc aatgacttcc 3420
atattttaaa agagaaaaac aactttatgt tgcaggaaac cctttttgta agtctttatt 3480
atttactttg cattttgttt cactctttcc agataagcag agttgctctt caccagtgtt 3540
tttcttcatg tgcaaagtga ctatttgttc tataatactt ttatgtgtgt tatatcaaat 3600

gtgtcttaag cttcatgcaa actcagtcat cagttcgtgt tgtctgaagc aagtgggaga 3660
tatataaata cccagtagct aaaatggtca gtcttttta gatgttttcc tacttagtat 3720
ctcctaataa cgttttgctg tgtcactaga tgttcatttc acaagtgcat gtctttctaa 3780
taatccacac atttcatgct ctaataatcc acacatttca tgctcatttt tattgttttt 3840
acagccagtt atagtaagaa aaaggttttt ccccttgtgc tgctttataa tttagcgtgt 3900
gtctgaacct tatccatgtt tgctagatga ggtcttgtca aatatatcac taccattgtc 3960
accggtgaaa agaaacaggt agttaagtta gggttaacat tcatttcaac cacgaggttg 4020
tatatcatga ctagctttta ctcttggttt acagagaaaa gttaaacagc caactaggca 4080
gtttttaaga atattaacaa tatattaaca aacaccaata caactaatcc tatttggttt 4140
taatgatttc accatgggat taagaactat atcaggaaca tccctgagaa acggttttaa 4200
gtgtagcaac tactcttcct taatggacag ccacataacg tgtaggaagt cctttatcac 4260
ttatcctcga tccataagca tatcttgcag aggggaacta cttctttaaa cacatggagg 4320
gaaagaagat gatgccactg gcaccagagg gttagtactg tgatgcatcc taaaatattt 4380
attatattgg taaaaattct ggttaaataa aaaattagag atcactcttg gctgatttca 4440
gcaccaggaa ctgtattaca gttttagaga ttaattccta gtgtttacct gattatagca 4500
gttggcatca tggggcattt aattctgact ttatccccac gtcagcctta ataaagtctt 4560
ctttaccttc tctatgaaga ctttaaagcc caaataatca tttttcacat tgatattcaa 4620
gaattgagat agatagaagc caaagtgggt atctgacaag tggaaaatca aacgtttaag 4680
aagaattaca actctgaaaa gcatttatat gtggaacttc tcaaggagcc tcctggggac 4740
tggaaagtaa gtcatcagcc aggcaaatga ctcatgctga agagagtccc catttcagtc 4800
ccctgagatc tagctgatgc ttagatcctt tgaaataaaa attatgtctt tataactctg 4860
atcttttaca taaagcagaa gaggaatcaa ctagttaatt gcaaggtttc tactctgttt 4920
cctctgtaaa gatcagatgg taatctttca aataagaaaa aaataaagac gtatgtttga 4980
ccaagtagtt tcacaagaat atttgggaac ttgtttcttt taattttatt tgtccctgag 5040
tgaagtctag aaagaaaggt aaagagtcta gagtttattc ctctttccaa aacattctca 5100
ttcctctcct ccctacactt agtatttccc ccacagagtg cctagaatct taataatgaa 5160
taaaataaaa agcagcaata tgtcattaac aaatccagac ctgaaagggt aaagggttta 5220
taactgcact aataaagaga ggctcttttt ttttcttcca gtttgttggt ttttaatggt 5280
accgtgttgt aaagataccc actaatggac aatcaaattg cagaaaaggc tcaatatcca 5340
agagacaggg actaatgcac tgtacaatct gcttatcctt gcccttctct cttgccaaag 5400
tgtgcttcag aaatatatac tgctttaaaa aagaataaaa gaatatcctt ttacaagtgg 5460
ctttacattt cctaaaatgc cataagaaaa tgcaatatct gggtactgta tggggaaaaa 5520

aatgtccaag tttgtgtaaa accagtgcat ttcagcttgc aagttactga acacaataat 5580
gctgttttaa ttttgtttta tatcagttaa aattcacaat aatgtagata gaacaaatta 5640
cagacaagga aagaaaaaac ttgaatgaaa tggattttac agaaagcttt atgataattt 5700
ttgaatgcat tatttatttt ttgtgccatg cattttttt ctcaccaaat gaccttacct 5760
gtaatacagt cttgtttgtc tgtttacaac catgtattta ttgcaatgta catactgtaa 5820
tgttaattgt aaattatctg ttcttattaa aacatcatcc catgatggga tggtgttgat 5880
atatttggaa actcttggtg agagaatgaa tggtgtgtat acatactctg tacatttttc 5940
ttttctcctg taatatagtc ttgtcacctt agagcttgtt tatggaagat tcaagaaaac 6000
tataaaatac ttaaagatat ataaatttaa aaaaacatag ctgcaggtct ttggtcccag 6060
ggctgtgcct taactttaac caatattttc ttctgttttg ctgcatttga aaggtaacag 6120
tggagctagg gctgggcatt ttacatccag gcttttaatt gattagaatt ctgccaatag 6180
gtggatttta caaaaccaca gacaacctct gaaagattct gagacccttt tgagacagaa 6240
gctcttaagt acttcttgcc agggagcagc actgcatgtg tgatggttgt ttgccatctg 6300
ttgatcagga actacttcag ctacttgcat ttgattattt cctttttttt tttttttaac 6360
tcggaaacac aactggggaa atatattctt tcccagtgat tataaacaat ctttttcttt 6420
tttttaagtc cttttggctt ctagagctca taggaaaatg gacttgattt gaaattggag 6480
ccagagttta ctcgtgttgg ttatctattc atcagcttcc tgacatgtta agagaataca 6540
ttaaagagaa aatactgttt tttaatccta aaatttttct tccactaaga taaaccaaat 6600
gtccttacat atatgtaaac ccatctattt aaacgcaaag gtgggttgat gtcagtttac 6660
atagcagaaa gcattcacta tcctctaaga tttgtttctg caaaactttc attgctttag 6720
aattttaaaa tttcaccttg tacaatggcc agccccctaaa gcaggaaaca tttataatgg 6780
attatatgga aacatcctcc cagtacttgc ccagcccttg aatcatgtgg cttttcagtg 6840
aaaggaaaga ttctttttct aggaaaaatg agcctatttt attttatttt attttatttt 6900
ttgacacaaa ctgtagattt tagcagccct ggcccaaagg aatttgatta cttttgtttt 6960
aaacagtaca aaggggacac tataattaca aaaacatcct taactgattt gagttgtttt 7020
tatttctttg gatatatttt cagagtggta aattgtgtgt gagaattaca aatgattatt 7080
cttttagtgg tttcttagcc tctcttacag cccacgggga tagtactgta catcaatacc 7140
ttcatatgaa atttttatat gcaatgaaaa taaaagcatg ggttgattct gcctatttat 7200
gactcaatct tttacaaata aaagattatt cattttaaat tatagttcaa tcagcatgtc 7260
tcttaggata ctgaacgtgg ttgaaatgaa aggatagtga catcataagt tagtactgat 7320
attcataacc aaataaagcc aacttgagta attttgctac attaaaaatt accaaaatta 7380

cttagatggc ctataagatt aagcatggtg ttttctaagc aagctttgaa aggggccttc 7440

catacttact taattgaata ttctgggata ttgaaaatta ttcagatact tgacaattat 7500

ttttggttac ctactccgca aactacaaag ttttaaggac tcaacaataa gttaatgaga 7560

cacagtgttt gctttcatgg agcttacagt ctggagggga caaaggctta aacaatactc 7620

atataattat atatgtgatc agtacaatga aggagctcag tggggtaaat aagcaggaac 7680

ctgaacttga tctgttccgg agggccacag aaggcttcct tgaggccttg agaaagtgat 7740

ttgcatctga gttctgaagg attgtaagag gtaactaggg aaaaagttga caggaagagg 7800

aaggggatcc agacaagaaa catttgcaaa gatcttgagg cataaatgag cttgagacat 7860

ctggagaaac tgaggaaaag tgagagagta ggcagggcct ggagccgcag agccattgct 7920

aaccatcctg tgtgagatat cccccattct gtagctttat tctcataacc ctgctcaatt 7980

ttctttataa cacttctcac agatttatat acgtgtttgt ttttgttatc tgtctctccc 8040

accagaccac agctccatga gagcaaggtc tttgcttacc aatatatcac tagcacttaa 8100

aactatgcct ggtacacagt aggttcttaa tatgtgttga atatagccat caaattgata 8160

ttggatataa ttcaatctga taagatattt tgagatatta aagagttttt aacttgatac 8220

cataaaaaaa aaaaaaaaaa 8240

<210> 12
<211> 8153
<212> DNA
<213> Homo sapiens

<400> 12
aatacttgtt gcaataattg cccacgatag ctgctcaaac aagagagttg gaattcatct 60

gtaaaaatca ctacatgtaa cgtaggagac aagaaaaata ttaatgacag aagatctgcg 120

aacatgatgc acgtgaataa ttttcccttt agaaggcatt cctggatatg ctgatggtaa 180

cagcagaaaa atgtgaactc tgaataaaga ggtgggagtt tttcagcaca taaagaatat 240

ttaaagccaa ttcattggat gcattgacca gtaagtgtag agatcaaaat caagaacaac 300

tccaagaatt gagagcagat gcaaacccca atttttgtgg gtctccagtc cagccttccc 360

aggaatgctg gtctgactac tcagatttgc ttttacttct tgccttttgg atataatgag 420

tttgccaagc agctgtgagt acctgactct ggggaaggtg gctagattcc gaagcgctta 480

tgttcatgga tcaccatacg cgatcaacat gccaattgat attaagccac agaggagacg 540

ttttgatgtg gacaatggca catctgcggg acggagtccc ttggatccca tgaccagccc 600

aggatccggg ctaattctcc aagcaaattt tgtccacagt caacgacggg agtccttcct 660

gtatcgatcc gacagcgatt atgacctctc tccaaagtct atgtcccgga actcctccat 720

tgccagtgat atacacggag atgacttgat tgtgactcca tttgctcagg tcttggccag 780

tctgcgaact gtacgaaaca actttgctgc attaactaat ttgcaagatc gagcacctag 840

caaaagatca cccatgtgca accaaccatc catcaacaaa gccaccataa cagaggaggc 900

ctaccagaaa ctggccagcg agaccctgga ggagctggac tggtgtctgg accagctaga 960

gaccctacag accaggcact ccgtcagtga gatggcctcc aacaagttta aaaggatgct 1020

taatcgggag ctcacccatc tctctgaaat gagtcggtct ggaaatcaag tgtcagagtt 1080

tatatcaaac acattcttag ataagcaaca tgaagtggaa attccttctc caactcagaa 1140

ggaaaaggag aaaaagaaaa gaccaatgtc tcagatcagt ggagtcaaga aattgatgca 1200

cagctctagt ctgactaatt caagtatccc aaggtttgga gttaaaactg aacaagaaga 1260

tgtccttgcc aaggaactag aagatgtgaa caaatggggt cttcatgttt tcagaatagc 1320

agagttgtct ggtaaccggc ccttgactgt tatcatgcac accatttttc aggaacggga 1380

tttattaaaa acatttaaaa ttccagtaga tactttaatt acatatctta tgactctcga 1440

agaccattac catgctgatg tggcctatca caacaatatc catgctgcag atgttgtcca 1500

gtctactcat gtgctattat ctacacctgc tttggaggct gtgtttacag atttggagat 1560

tcttgcagca atttttgcca gtgcaataca tgatgtagat catcctggtg tgtccaatca 1620

atttctgatc aatacaaact ctgaacttgc cttgatgtac aatgattcct cagtcttaga 1680

gaaccatcat ttggctgtgg gctttaaatt gcttcaggaa gaaaactgtg acattttcca 1740

gaatttgacc aaaaaacaaa gacaatcttt aaggaaaatg gtcattgaca tcgtacttgc 1800

aacagatatg tcaaaacaca tgaatctact ggctgatttg aagactatgg ttgaaactaa 1860

gaaagtgaca agctctggag ttcttcttct tgataattat tccgatagga ttcaggttct 1920

tcagaatatg gtgcactgtg cagatctgag caacccaaca aagcctctcc agctgtaccg 1980

ccagtggacg gaccggataa tggaggagtt cttccgccaa ggagaccgag agagggaacg 2040

tggcatggag ataagcccca tgtgtgacaa gcacaatgct tccgtggaaa aatcacaggt 2100

gggcttcata gactatattg ttcatcccct ctgggagaca tgggcagacc tcgtccaccc 2160

tgacgcccag gatattttgg acactttgga ggacaatcgt gaatggtacc agagcacaat 2220

ccctcagagc ccctctcctg cacctgatga cccagaggag ggccggcagg gtcaaactga 2280

gaaattccag tttgaactaa ctttagagga agatggtgag tcagacacgg aaaaggacag 2340

tggcagtcaa gtggaagaag acactagctg cagtgactcc aagactcttt gtactcaaga 2400

ctcagagtct actgaaattc cccttgatga acaggttgaa gaggaggcag taggggaaga 2460

agaggaaagc cagcctgaag cctgtgtcat agatgatcgt tctcctgaca cgtaacagtg 2520

caaaaacttt catgcctttt tttttttaa gtagaaaaat tgtttccaaa gtgcatgtca 2580

catgccacaa ccacggtcac acctcactgt catctgccag gacgtttgtt gaacaaaact 2640

gaccttgact actcagtcca gcgctcagga atatcgtaac cagtttttc acctccatgt 2700

catccgagca aggtggacat cttcacgaac agcgttttta acaagatttc agcttggtag 2760

agctgacaaa gcagataaaa tctactccaa attattttca agagagtgtg actcatcagg 2820

cagcccaaaa gtttattgga cttggggttt ctattccttt ttatttgttt gcaatatttt 2880

cagaagaaag gcattgcaca gagtgaactt aatggacgaa gcaacaaata tgtcaagaac 2940

aggacatagc acgaatctgt taccagtagg aggaggatga gccacagaaa ttgcataatt 3000

ttctaatttc aagtcttcct gatacatgac tgaatagtgt ggttcagtga gctgcactga 3060

cctctacatt ttgtatgata tgtaaaacag attttttgta gagcttactt ttattattaa 3120

atgtattgag gtattatatt taaaaaaaac tatgttcaga acttcatctg ccactggtta 3180

tttttttcta aggagtaact tgcaagtttt cagtacaaat ctgtgctaca ctggataaaa 3240

atctaattta tgaattttac ttgcacctta tagttcatag caattaactg atttgtagtg 3300

attcattgtt tgttttatat accaatgact tccatatttt aaaagagaaa aacaacttta 3360

tgttgcagga aacccttttt gtaagtcttt attatttact ttgcattttg tttcactctt 3420

tccagataag cagagttgct cttcaccagt gtttttcttc atgtgcaaag tgactatttg 3480

ttctataata cttttatgtg tgttatatca aatgtgtctt aagcttcatg caaactcagt 3540

catcagttcg tgttgtctga agcaagtggg agatatataa atacccagta gctaaaatgg 3600

tcagtctttt ttagatgttt tcctacttag tatctcctaa taacgttttg ctgtgtcact 3660

agatgttcat ttcacaagtg catgtctttc taataatcca cacatttcat gctctaataa 3720

tccacacatt tcatgctcat ttttattgtt tttacagcca gttatagtaa gaaaaaggtt 3780

tttccccttg tgctgcttta taatttagcg tgtgtctgaa ccttatccat gtttgctaga 3840

tgaggtcttg tcaaatatat cactaccatt gtcaccggtg aaaagaaaca ggtagttaag 3900

ttagggttaa cattcatttc aaccacgagg ttgtatatca tgactagctt ttactcttgg 3960

tttacagaga aaagttaaac agccaactag gcagttttta agaatattaa caatatatta 4020

acaaacacca atacaactaa tcctatttgg ttttaatgat ttcaccatgg gattaagaac 4080

tatatcagga acatccctga gaaacggttt taagtgtagc aactactctt ccttaatgga 4140

cagccacata acgtgtagga agtcctttat cacttatcct cgatccataa gcatatcttg 4200

cagaggggaa ctacttcttt aaacacatgg agggaaagaa gatgatgcca ctggcaccag 4260

agggttagta ctgtgatgca tcctaaaata tttattatat tggtaaaaat tctggttaaa 4320

taaaaaatta gagatcactc ttggctgatt tcagcaccag gaactgtatt acagttttag 4380

agattaattc ctagtgttta cctgattata gcagttggca tcatggggca tttaattctg 4440

actttatccc cacgtcagcc ttaataaagt cttctttacc ttctctatga agactttaaa 4500

gcccaaataa tcatttttca cattgatatt caagaattga gatagataga agccaaagtg 4560

ggtatctgac aagtggaaaa tcaaacgttt aagaagaatt acaactctga aaagcattta 4620
tatgtggaac ttctcaagga gcctcctggg gactggaaag taagtcatca gccaggcaaa 4680
tgactcatgc tgaagagagt ccccatttca gtcccctgag atctagctga tgcttagatc 4740
ctttgaaata aaaattatgt ctttataact ctgatctttt acataaagca gaagaggaat 4800
caactagtta attgcaaggt ttctactctg tttcctctgt aaagatcaga tggtaatctt 4860
tcaaataaga aaaaaataaa gacgtatgtt tgaccaagta gtttcacaag aatatttggg 4920
aacttgtttc ttttaatttt atttgtccct gagtgaagtc tagaaagaaa ggtaaagagt 4980
ctagagttta ttcctctttc caaaacattc tcattcctct cctccctaca cttagtattt 5040
cccccacaga gtgcctagaa tcttaataat gaataaaata aaaagcagca atatgtcatt 5100
aacaaatcca gacctgaaag ggtaaagggt ttataactgc actaataaag agaggctctt 5160
ttttttcttt ccagtttgtt ggtttttaat ggtaccgtgt tgtaaagata cccactaatg 5220
gacaatcaaa ttgcagaaaa ggctcaatat ccaagagaca gggactaatg cactgtacaa 5280
tctgcttatc cttgcccttc tctcttgcca aagtgtgctt cagaaatata tactgcttta 5340
aaaaagaata aaagaatatc cttttacaag tggctttaca tttcctaaaa tgccataaga 5400
aaatgcaata tctgggtact gtatggggaa aaaaatgtcc aagtttgtgt aaaaccagtg 5460
catttcagct tgcaagttac tgaacacaat aatgctgttt taattttgtt ttatatcagt 5520
taaaattcac aataatgtag atagaacaaa ttacagacaa ggaaagaaaa aacttgaatg 5580
aaatggattt tacagaaagc tttatgataa tttttgaatg cattatttat tttttgtgcc 5640
atgcattttt tttctcacca aatgacctta cctgtaatac agtcttgttt gtctgtttac 5700
aaccatgtat ttattgcaat gtacatactg taatgttaat tgtaaattat ctgttcttat 5760
taaaacatca tcccatgatg ggatggtgtt gatatatttg gaaactcttg gtgagagaat 5820
gaatggtgtg tatacatact ctgtacattt ttcttttctc ctgtaatata gtcttgtcac 5880
cttagagctt gtttatggaa gattcaagaa aactataaaa tacttaaaga tatataaatt 5940
taaaaaaaca tagctgcagg tctttggtcc cagggctgtg ccttaacttt aaccaatatt 6000
ttcttctgtt ttgctgcatt tgaaaggtaa cagtggagct agggctgggc attttacatc 6060
caggctttta attgattaga attctgccaa taggtggatt ttacaaaacc acagacaacc 6120
tctgaaagat tctgagaccc ttttgagaca gaagctctta agtacttctt gccagggagc 6180
agcactgcat gtgtgatggt tgtttgccat ctgttgatca ggaactactt cagctacttg 6240
catttgatta tttccttttt tttttttttt aactcggaaa cacaactggg gaaatatatt 6300
ctttcccagt gattataaac aatctttttc ttttttttaa gtccttttgg cttctagagc 6360
tcataggaaa atggacttga tttgaaattg gagccagagt ttactcgtgt tggttatcta 6420
ttcatcagct tcctgacatg ttaagagaat acattaaaga gaaaatactg tttttttaatc 6480

ctaaaatttt tcttccacta agataaacca aatgtcctta catatatgta aacccatcta 6540
tttaaacgca aaggtgggtt gatgtcagtt tacatagcag aaagcattca ctatcctcta 6600
agatttgttt ctgcaaaact ttcattgctt tagaatttta aaatttcacc ttgtacaatg 6660
gccagcccct aaagcaggaa acatttataa tggattatat ggaaacatcc tcccagtact 6720
tgcccagccc ttgaatcatg tggcttttca gtgaaaggaa agattctttt tctaggaaaa 6780
atgagcctat tttattttat tttattttat tttttgacac aaactgtaga ttttagcagc 6840
cctggcccaa aggaatttga ttacttttgt tttaaacagt acaaagggga cactataatt 6900
acaaaaacat ccttaactga tttgagttgt ttttatttct ttggatatat tttcagagtg 6960
gtaaattgtg tgtgagaatt acaaatgatt attcttttag tggtttctta gcctctctta 7020
cagcccacgg ggatagtact gtacatcaat accttcatat gaaattttta tatgcaatga 7080
aaataaaagc atgggttgat tctgcctatt tatgactcaa tcttttacaa ataaaagatt 7140
attcatttta aattatagtt caatcagcat gtctcttagg atactgaacg tggttgaaat 7200
gaaaggatag tgacatcata agttagtact gatattcata accaaataaa gccaacttga 7260
gtaattttgc tacattaaaa attaccaaaa ttacttagat ggcctataag attaagcatg 7320
gtgttttcta agcaagcttt gaaaggggcc ttccatactt acttaattga atattctggg 7380
atattgaaaa ttattcagat acttgacaat tatttttggt tacctactcc gcaaactaca 7440
aagttttaag gactcaacaa taagttaatg agacacagtg tttgctttca tggagcttac 7500
agtctggagg ggacaaaggc ttaaacaata ctcatataat tatatatgtg atcagtacaa 7560
tgaaggagct cagtggggta aataagcagg aacctgaact tgatctgttc cggagggcca 7620
cagaaggctt ccttgaggcc ttgagaaagt gatttgcatc tgagttctga aggattgtaa 7680
gaggtaacta gggaaaaagt tgacaggaag aggaagggga tccagacaag aaacatttgc 7740
aaagatcttg aggcataaat gagcttgaga catctggaga aactgaggaa aagtgagaga 7800
gtaggcaggg cctggagccg cagagccatt gctaaccatc ctgtgtgaga tatcccccat 7860
tctgtagctt tattctcata accctgctca attttcttta taacacttct cacagattta 7920
tatacgtgtt tgtttttgtt atctgtctct cccaccagac cacagctcca tgagagcaag 7980
gtctttgctt accaatatat cactagcact taaaactatg cctggtacac agtaggttct 8040
taatatgtgt tgaatatagc catcaaattg atattggata taattcaatc tgataagata 8100
ttttgagata ttaaagagtt tttaacttga taccataaaa aaaaaaaaaa aaa 8153

<210> 13
<211> 8203
<212> DNA
<213> Homo sapiens

<400> 13
gcggcgcatt agttccaata gtttaacagg ctgctttgta gcacggacaa agccgtgcga 60
gcgcggtgct ttccctcctt gttcatttgc tatgcgagct tgtcagtgat ctttggcagg 120
ggcttgggag aggaggggtg acgtttaata cgcttccgcg gaggagtgcg ctcgcctcct 180
ctgcacccag ccccaggctc tacagagaga ctgaggcagg cgactgaatg cactaacagc 240
agcaggctca gacctgcttc cctggacatt tccgggaccg tgagcgaggg aaccacgttg 300
ccctggattc ttgccagctg tacaaagttg accaggaaaa tggctcagca gacaagcccg 360
gacactttaa cagtacctga agtggataat ccgcattgtc caaacccgtg gctgaacgaa 420
gaccttgtga aatccttgcg agaaaacctg ttgcagcatg agaagtccaa gacagcgagg 480
aaatcggttt ctcccaagct ctctccagtg atctctccga gaaattcccc caggcttctg 540
cgcagaatgc ttctcagcag caacatcccc aaacagcggc gtttcacggt ggcacataca 600
tgttttgatg tggacaatgg cacatctgcg ggacggagtc ccttggatcc catgaccagc 660
ccaggatccg ggctaattct ccaagcaaat tttgtccaca gtcaacgacg ggagtccttc 720
ctgtatcgat ccgacagcga ttatgacctc tctccaaagt ctatgtcccg gaactcctcc 780
attgccagtg atatacacgg agatgacttg attgtgactc catttgctca ggtcttggcc 840
agtctgcgaa ctgtacgaaa caactttgct gcattaacta atttgcaaga tcgagcacct 900
agcaaaagat cacccatgtg caaccaacca tccatcaaca aagccaccat aacagaggag 960
gcctaccaga aactggccag cgagaccctg gaggagctgg actggtgtct ggaccagcta 1020
gagaccctac agaccaggca ctccgtcagt gagatggcct ccaacaagtt taaaaggatg 1080
cttaatcggg agctcaccca tctctctgaa atgagtcggt ctggaaatca agtgtcagag 1140
tttatatcaa acacattctt agataagcaa catgaagtgg aaattccttc tccaactcag 1200
aaggaaaagg agaaaaagaa aagaccaatg tctcagatca gtggagtcaa gaaattgatg 1260
cacagctcta gtctgactaa ttcaagtatc ccaaggtttg gagttaaaac tgaacaagaa 1320
gatgtccttg ccaaggaact agaagatgtg aacaaatggg gtcttcatgt tttcagaata 1380
gcagagttgt ctggtaaccg gcccttgact gttatcatgc acaccatttt tcaggaacgg 1440
gatttattaa aaacatttaa aattccagta gatactttaa ttacatatct tatgactctc 1500
gaagaccatt accatgctga tgtggcctat cacaacaata tccatgctgc agatgttgtc 1560
cagtctactc atgtgctatt atctacacct gctttggagg ctgtgtttac agatttggag 1620
attcttgcag caatttttgc cagtgcaata catgatgtag atcatcctgg tgtgtccaat 1680
caatttctga tcaatacaaa ctctgaactt gccttgatgt acaatgattc ctcagtctta 1740
gagaaccatc atttggctgt gggctttaaa ttgcttcagg aagaaaactg tgacattttc 1800
cagaatttga ccaaaaaaca aagacaatct ttaaggaaaa tggtcattga catcgtactt 1860

gcaacagata tgtcaaaaca catgaatcta ctggctgatt tgaagactat ggttgaaact 1920

aagaaagtga caagctctgg agttcttctt cttgataatt attccgatag gattcaggtt 1980

cttcagaata tggtgcactg tgcagatctg agcaacccaa caaagcctct ccagctgtac 2040

cgccagtgga cggaccggat aatggaggag ttcttccgcc aaggagaccg agagagggaa 2100

cgtggcatgg agataagccc catgtgtgac aagcacaatg cttccgtgga aaaatcacag 2160

gtgggcttca tagactatat tgttcatccc ctctgggaga catgggcaga cctcgtccac 2220

cctgacgccc aggatatttt ggacactttg gaggacaatc gtgaatggta ccagagcaca 2280

atccctcaga gcccctctcc tgcacctgat gacccagagg agggccggca gggtcaaact 2340

gagaaattcc agtttgaact aactttagag gaagatggtg agtcagacac ggaaaaggac 2400

agtggcagtc aagtggaaga agacactagc tgcagtgact ccaagactct ttgtactcaa 2460

gactcagagt ctactgaaat tccccttgat gaacaggttg aagaggaggc agtaggggaa 2520

gaagaggaaa gccagcctga agcctgtgtc atagatgatc gttctcctga cacgtaacag 2580

tgcaaaaact ttcatgcctt tttttttttt aagtagaaaa attgtttcca aagtgcatgt 2640

cacatgccac aaccacggtc acacctcact gtcatctgcc aggacgtttg ttgaacaaaa 2700

ctgaccttga ctactcagtc cagcgctcag gaatatcgta accagttttt tcacctccat 2760

gtcatccgag caaggtggac atcttcacga acagcgtttt taacaagatt tcagcttggt 2820

agagctgaca aagcagataa aatctactcc aaattatttt caagagagtg tgactcatca 2880

ggcagcccaa aagtttattg gacttggggt ttctattcct ttttatttgt ttgcaatatt 2940

ttcagaagaa aggcattgca cagagtgaac ttaatggacg aagcaacaaa tatgtcaaga 3000

acaggacata gcacgaatct gttaccagta ggaggaggat gagccacaga aattgcataa 3060

ttttctaatt tcaagtcttc ctgatacatg actgaatagt gtggttcagt gagctgcact 3120

gacctctaca ttttgtatga tatgtaaaac agattttttg tagagcttac ttttattatt 3180

aaatgtattg aggtattata tttaaaaaaa actatgttca gaacttcatc tgccactggt 3240

tatttttttc taaggagtaa cttgcaagtt ttcagtacaa atctgtgcta cactggataa 3300

aaatctaatt tatgaatttt acttgcacct tatagttcat agcaattaac tgatttgtag 3360

tgattcattg tttgttttat ataccaatga cttccatatt ttaaaagaga aaaacaactt 3420

tatgttgcag gaaacccttt ttgtaagtct ttattattta ctttgcattt tgtttcactc 3480

tttccagata agcagagttg ctcttcacca gtgtttttct tcatgtgcaa agtgactatt 3540

tgttctataa tacttttatg tgtgttatat caaatgtgtc ttaagcttca tgcaaactca 3600

gtcatcagtt cgtgttgtct gaagcaagtg ggagatatat aaatacccag tagctaaaat 3660

ggtcagtctt ttttagatgt tttcctactt agtatctcct aataacgttt tgctgtgtca 3720

ctagatgttc atttcacaag tgcatgtctt tctaataatc cacacatttc atgctctaat 3780

aatccacaca tttcatgctc atttttattg tttttacagc cagttatagt aagaaaaagg 3840
tttttcccct tgtgctgctt tataatttag cgtgtgtctg aaccttatcc atgtttgcta 3900
gatgaggtct tgtcaaatat atcactacca ttgtcaccgg tgaaaagaaa caggtagtta 3960
agttagggtt aacattcatt tcaaccacga ggttgtatat catgactagc ttttactctt 4020
ggtttacaga gaaaagttaa acagccaact aggcagtttt taagaatatt aacaatatat 4080
taacaaacac caatacaact aatcctattt ggttttaatg atttcaccat gggattaaga 4140
actatatcag gaacatccct gagaaacggt tttaagtgta gcaactactc ttccttaatg 4200
gacagccaca taacgtgtag gaagtccttt atcacttatc ctcgatccat aagcatatct 4260
tgcagagggg aactacttct ttaaacacat ggagggaaag aagatgatgc cactggcacc 4320
agagggttag tactgtgatg catcctaaaa tatttattat attggtaaaa attctggtta 4380
aataaaaaat tagagatcac tcttggctga tttcagcacc aggaactgta ttacagtttt 4440
agagattaat tcctagtgtt tacctgatta tagcagttgg catcatgggg catttaattc 4500
tgactttatc cccacgtcag ccttaataaa gtcttcttta ccttctctat gaagacttta 4560
aagcccaaat aatcattttt cacattgata ttcaagaatt gagatagata gaagccaaag 4620
tgggtatctg acaagtggaa aatcaaacgt ttaagaagaa ttacaactct gaaaagcatt 4680
tatatgtgga acttctcaag gagcctcctg gggactggaa agtaagtcat cagccaggca 4740
aatgactcat gctgaagaga gtccccattt cagtcccctg agatctagct gatgcttaga 4800
tcctttgaaa taaaaattat gtctttataa ctctgatctt ttacataaag cagaagagga 4860
atcaactagt taattgcaag gtttctactc tgtttcctct gtaaagatca gatggtaatc 4920
tttcaaataa gaaaaaaata aagacgtatg tttgaccaag tagtttcaca agaatatttg 4980
ggaacttgtt tcttttaatt ttatttgtcc ctgagtgaag tctagaaaga aaggtaaaga 5040
gtctagagtt tattcctctt tccaaaacat tctcattcct ctcctcccta cacttagtat 5100
ttcccccaca gagtgcctag aatcttaata atgaataaaa taaaaagcag caatatgtca 5160
ttaacaaatc cagacctgaa agggtaaagg gtttataact gcactaataa agagaggctc 5220
tttttttttc ttccagtttg ttggttttta atggtaccgt gttgtaaaga tacccactaa 5280
tggacaatca aattgcagaa aaggctcaat atccaagaga cagggactaa tgcactgtac 5340
aatctgctta tccttgccct tctctcttgc caaagtgtgc ttcagaaata tatactgctt 5400
taaaaaagaa taaaagaata tccttttaca agtggcttta catttcctaa aatgccataa 5460
gaaaatgcaa tatctgggta ctgtatgggg aaaaaaatgt ccaagtttgt gtaaaaccag 5520
tgcatttcag cttgcaagtt actgaacaca ataatgctgt tttaattttg ttttatatca 5580
gttaaaattc acaataatgt agatagaaca aattacagac aaggaaagaa aaaacttgaa 5640

tgaaatggat tttacagaaa gctttatgat aatttttgaa tgcattattt atttttttgtg 5700
ccatgcattt tttttctcac caaatgacct tacctgtaat acagtcttgt ttgtctgttt 5760
acaaccatgt atttattgca atgtacatac tgtaatgtta attgtaaatt atctgttctt 5820
attaaaacat catcccatga tgggatggtg ttgatatatt tggaaactct tggtgagaga 5880
atgaatggtg tgtatacata ctctgtacat ttttcttttc tcctgtaata tagtcttgtc 5940
accttagagc ttgtttatgg aagattcaag aaaactataa aatacttaaa gatatataaa 6000
tttaaaaaaa catagctgca ggtctttggt cccagggctg tgccttaact ttaaccaata 6060
ttttcttctg ttttgctgca tttgaaaggt aacagtggag ctagggctgg gcattttaca 6120
tccaggcttt taattgatta gaattctgcc aataggtgga ttttacaaaa ccacagacaa 6180
cctctgaaag attctgagac ccttttgaga cagaagctct taagtacttc ttgccaggga 6240
gcagcactgc atgtgtgatg gttgtttgcc atctgttgat caggaactac ttcagctact 6300
tgcatttgat tatttccttt tttttttttt ttaactcgga aacacaactg gggaaatata 6360
ttctttccca gtgattataa acaatctttt tctttttttt aagtcctttt ggcttctaga 6420
gctcatagga aaatggactt gatttgaaat tggagccaga gtttactcgt gttggttatc 6480
tattcatcag cttcctgaca tgttaagaga atacattaaa gagaaaatac tgttttttaa 6540
tcctaaaatt tttcttccac taagataaac caaatgtcct tacatatatg taaacccatc 6600
tatttaaacg caaaggtggg ttgatgtcag tttacatagc agaaagcatt cactatcctc 6660
taagatttgt ttctgcaaaa ctttcattgc tttagaattt taaaatttca ccttgtacaa 6720
tggccagccc ctaaagcagg aaacatttat aatggattat atggaaacat cctcccagta 6780
cttgcccagc ccttgaatca tgtggctttt cagtgaaagg aaagattctt tttctaggaa 6840
aaatgagcct attttatttt attttatttt attttttgac acaaactgta gattttagca 6900
gccctggccc aaaggaattt gattactttt gttttaaaca gtacaaaggg gacactataa 6960
ttacaaaaac atccttaact gatttgagtt gtttttattt ctttggatat attttcagag 7020
tggtaaattg tgtgtgagaa ttacaaatga ttattctttt agtggtttct tagcctctct 7080
tacagcccac ggggatagta ctgtacatca ataccttcat atgaaatttt tatatgcaat 7140
gaaaataaaa gcatgggttg attctgccta tttatgactc aatcttttac aaataaaaga 7200
ttattcattt taaattatag ttcaatcagc atgtctctta ggatactgaa cgtggttgaa 7260
atgaaaggat agtgacatca taagttagta ctgatattca taaccaaata aagccaactt 7320
gagtaatttt gctacattaa aaattaccaa aattacttag atggcctata agattaagca 7380
tggtgttttc taagcaagct ttgaaagggg ccttccatac ttacttaatt gaatattctg 7440
ggatattgaa aattattcag atacttgaca attatttttg gttacctact ccgcaaacta 7500
caaagtttta aggactcaac aataagttaa tgagacacag tgtttgcttt catggagctt 7560

acagtctgga ggggacaaag gcttaaacaa tactcatata attatatatg tgatcagtac 7620

aatgaaggag ctcagtgggg taaataagca ggaacctgaa cttgatctgt tccggagggc 7680

cacagaaggc ttccttgagg ccttgagaaa gtgatttgca tctgagttct gaaggattgt 7740

aagaggtaac tagggaaaaa gttgacagga agaggaaggg gatccagaca agaaacattt 7800

gcaaagatct tgaggcataa atgagcttga gacatctgga gaaactgagg aaaagtgaga 7860

gagtaggcag ggcctggagc cgcagagcca ttgctaacca tcctgtgtga gatatccccc 7920

attctgtagc tttattctca taaccctgct caattttctt tataacactt ctcacagatt 7980

tatatacgtg tttgtttttg ttatctgtct ctcccaccag accacagctc catgagagca 8040

aggtctttgc ttaccaatat atcactagca cttaaaacta tgcctggtac acagtaggtt 8100

cttaatatgt gttgaatata gccatcaaat tgatattgga tataattcaa tctgataaga 8160

tattttgaga tattaaagag tttttaactt gataccataa aaa 8203

<210> 14
<211> 7783
<212> DNA
<213> Homo sapiens

<400> 14
aatacttgtt gcaataattg cccacgatag ctgctcaaac aagagagttg gaattcatct 60

gtaaaaatca ctacatgtaa cgtaggagac aagaaaaata ttaatgacag aagatctgcg 120

aacatgatgc acgtgaataa ttttcccttt agaaggcatt cctggatatg ttttgatgtg 180

gacaatggca catctgcggg acggagtccc ttggatccca tgaccagccc aggatccggg 240

ctaattctcc aagcaaattt tgtccacagt caacgacggg agtccttcct gtatcgatcc 300

gacagcgatt atgacctctc tccaaagtct atgtcccgga actcctccat tgccagtgat 360

atacacggag atgacttgat tgtgactcca tttgctcagg tcttggccag tctgcgaact 420

gtacgaaaca actttgctgc attaactaat ttgcaagatc gagcacctag caaaagatca 480

cccatgtgca accaaccatc catcaacaaa gccaccataa cagaggaggc ctaccagaaa 540

ctggccagcg agaccctgga ggagctggac tggtgtctgg accagctaga gaccctacag 600

accaggcact ccgtcagtga gatggcctcc aacaagttta aaaggatgct taatcgggag 660

ctcacccatc tctctgaaat gagtcggtct ggaaatcaag tgtcagagtt tatatcaaac 720

acattcttag ataagcaaca tgaagtggaa attccttctc caactcagaa ggaaaaggag 780

aaaaagaaaa gaccaatgtc tcagatcagt ggagtcaaga aattgatgca cagctctagt 840

ctgactaatt caagtatccc aaggtttgga gttaaaactg aacaagaaga tgtccttgcc 900

aaggaactag aagatgtgaa caaatggggt cttcatgttt tcagaatagc agagttgtct 960

ggtaaccggc ccttgactgt tatcatgcac accatttttc aggaacggga tttattaaaa 1020

acatttaaaa ttccagtaga tactttaatt acatatctta tgactctcga agaccattac 1080
catgctgatg tggcctatca caacaatatc catgctgcag atgttgtcca gtctactcat 1140
gtgctattat ctacacctgc tttggaggct gtgtttacag atttggagat tcttgcagca 1200
atttttgcca gtgcaataca tgatgtagat catcctggtg tgtccaatca atttctgatc 1260
aatacaaact ctgaacttgc cttgatgtac aatgattcct cagtcttaga gaaccatcat 1320
ttggctgtgg gctttaaatt gcttcaggaa gaaaactgtg acattttcca gaatttgacc 1380
aaaaaacaaa gacaatcttt aaggaaaatg gtcattgaca tcgtacttgc aacagatatg 1440
tcaaaacaca tgaatctact ggctgatttg aagactatgg ttgaaactaa gaaagtgaca 1500
agctctggag ttcttcttct tgataattat tccgatagga ttcaggttct tcagaatatg 1560
gtgcactgtg cagatctgag caacccaaca aagcctctcc agctgtaccg ccagtggacg 1620
gaccggataa tggaggagtt cttccgccaa ggagaccgag agagggaacg tggcatggag 1680
ataagcccca tgtgtgacaa gcacaatgct tccgtggaaa aatcacaggt gggcttcata 1740
gactatattg ttcatcccct ctgggagaca tgggcagacc tcgtccaccc tgacgcccag 1800
gatattttgg acactttgga ggacaatcgt gaatggtacc agagcacaat ccctcagagc 1860
ccctctcctg cacctgatga cccagaggag ggccggcagg gtcaaactga gaaattccag 1920
tttgaactaa ctttagagga agatggtgag tcagacacgg aaaaggacag tggcagtcaa 1980
gtggaagaag acactagctg cagtgactcc aagactcttt gtactcaaga ctcagagtct 2040
actgaaattc cccttgatga acaggttgaa gaggaggcag taggggaaga agaggaaagc 2100
cagcctgaag cctgtgtcat agatgatcgt tctcctgaca cgtaacagtg caaaaacttt 2160
catgcctttt tttttttaa gtagaaaaat tgtttccaaa gtgcatgtca catgccacaa 2220
ccacggtcac acctcactgt catctgccag gacgtttgtt gaacaaaact gaccttgact 2280
actcagtcca gcgctcagga atatcgtaac cagtttttc acctccatgt catccgagca 2340
aggtggacat cttcacgaac agcgttttta acaagatttc agcttggtag agctgacaaa 2400
gcagataaaa tctactccaa attattttca agagagtgtg actcatcagg cagcccaaaa 2460
gtttattgga cttggggttt ctattccttt ttatttgttt gcaatatttt cagaagaaag 2520
gcattgcaca gagtgaactt aatggacgaa gcaacaaata tgtcaagaac aggacatagc 2580
acgaatctgt taccagtagg aggaggatga gccacagaaa ttgcataatt ttctaatttc 2640
aagtcttcct gatacatgac tgaatagtgt ggttcagtga gctgcactga cctctacatt 2700
ttgtatgata tgtaaaacag atttttgta gagcttactt ttattattaa atgtattgag 2760
gtattatatt taaaaaaaac tatgttcaga acttcatctg ccactggtta tttttttcta 2820
aggagtaact tgcaagtttt cagtacaaat ctgtgctaca ctggataaaa atctaattta 2880

tgaattttac ttgcacctta tagttcatag caattaactg atttgtagtg attcattgtt 2940

tgttttatat accaatgact tccatatttt aaaagagaaa aacaacttta tgttgcagga 3000

aaccctttttt gtaagtcttt attatttact ttgcattttg tttcactctt tccagataag 3060

cagagttgct cttcaccagt gtttttcttc atgtgcaaag tgactatttg ttctataata 3120

cttttatgtg tgttatatca aatgtgtctt aagcttcatg caaactcagt catcagttcg 3180

tgttgtctga agcaagtggg agatatataa atacccagta gctaaaatgg tcagtctttt 3240

ttagatgttt tcctacttag tatctcctaa taacgttttg ctgtgtcact agatgttcat 3300

ttcacaagtg catgtctttc taataatcca cacatttcat gctctaataa tccacacatt 3360

tcatgctcat ttttattgtt tttacagcca gttatagtaa gaaaaaggtt tttccccttg 3420

tgctgcttta taatttagcg tgtgtctgaa ccttatccat gtttgctaga tgaggtcttg 3480

tcaaatatat cactaccatt gtcaccggtg aaaagaaaca ggtagttaag ttagggttaa 3540

cattcatttc aaccacgagg ttgtatatca tgactagctt ttactcttgg tttacagaga 3600

aaagttaaac agccaactag gcagttttta agaatattaa caatatatta acaaacacca 3660

atacaactaa tcctatttgg ttttaatgat ttcaccatgg gattaagaac tatatcagga 3720

acatccctga gaaacggttt taagtgtagc aactactctt ccttaatgga cagccacata 3780

acgtgtagga agtcctttat cacttatcct cgatccataa gcatatcttg cagaggggaa 3840

ctacttcttt aaacacatgg agggaaagaa gatgatgcca ctggcaccag agggttagta 3900

ctgtgatgca tcctaaaata tttattatat tggtaaaaat tctggttaaa taaaaaatta 3960

gagatcactc ttggctgatt tcagcaccag gaactgtatt acagttttag agattaattc 4020

ctagtgttta cctgattata gcagttggca tcatggggca tttaattctg actttatccc 4080

cacgtcagcc ttaataaagt cttctttacc ttctctatga agactttaaa gcccaaataa 4140

tcatttttca cattgatatt caagaattga gatagataga agccaaagtg ggtatctgac 4200

aagtggaaaa tcaaacgttt aagaagaatt acaactctga aaagcattta tatgtggaac 4260

ttctcaagga gcctcctggg gactggaaag taagtcatca gccaggcaaa tgactcatgc 4320

tgaagagagt ccccatttca gtcccctgag atctagctga tgcttagatc ctttgaaata 4380

aaaattatgt ctttataact ctgatctttt acataaagca gaagaggaat caactagtta 4440

attgcaaggt ttctactctg tttcctctgt aaagatcaga tggtaatctt tcaaataaga 4500

aaaaaataaa gacgtatgtt tgaccaagta gtttcacaag aatatttggg aacttgtttc 4560

ttttaatttt atttgtccct gagtgaagtc tagaaagaaa ggtaaagagt ctagagttta 4620

ttcctctttc caaaacattc tcattcctct cctccctaca cttagtattt cccccacaga 4680

gtgcctagaa tcttaataat gaataaaata aaaagcagca atatgtcatt aacaaatcca 4740

gacctgaaag ggtaaagggt ttataactgc actaataaag agaggctctt ttttttctt 4800

ccagtttgtt ggtttttaat ggtaccgtgt tgtaaagata cccactaatg gacaatcaaa 4860

ttgcagaaaa ggctcaatat ccaagagaca gggactaatg cactgtacaa tctgcttatc 4920

cttgcccttc tctcttgcca aagtgtgctt cagaaatata tactgcttta aaaaagaata 4980

aaagaatatc cttttacaag tggctttaca tttcctaaaa tgccataaga aaatgcaata 5040

tctgggtact gtatggggaa aaaaatgtcc aagtttgtgt aaaaccagtg catttcagct 5100

tgcaagttac tgaacacaat aatgctgttt taattttgtt ttatatcagt taaaattcac 5160

aataatgtag atagaacaaa ttacagacaa ggaaagaaaa aacttgaatg aaatggattt 5220

tacagaaagc tttatgataa tttttgaatg cattatttat tttttgtgcc atgcattttt 5280

tttctcacca aatgacctta cctgtaatac agtcttgttt gtctgtttac aaccatgtat 5340

ttattgcaat gtacatactg taatgttaat tgtaaattat ctgttcttat taaaacatca 5400

tcccatgatg ggatggtgtt gatatatttg gaaactcttg gtgagagaat gaatggtgtg 5460

tatacatact ctgtacattt ttcttttctc ctgtaatata gtcttgtcac cttagagctt 5520

gtttatggaa gattcaagaa aactataaaa tacttaaaga tatataaatt taaaaaaaca 5580

tagctgcagg tctttggtcc cagggctgtg ccttaacttt aaccaatatt ttcttctgtt 5640

ttgctgcatt tgaaaggtaa cagtggagct agggctgggc attttacatc caggctttta 5700

attgattaga attctgccaa taggtggatt ttacaaaacc acagacaacc tctgaaagat 5760

tctgagaccc ttttgagaca gaagctctta agtacttctt gccagggagc agcactgcat 5820

gtgtgatggt tgtttgccat ctgttgatca ggaactactt cagctacttg catttgatta 5880

tttccttttt tttttttttt aactcggaaa cacaactggg gaaatatatt ctttcccagt 5940

gattataaac aatctttttc tttttttaa gtccttttgg cttctagagc tcataggaaa 6000

atggacttga tttgaaattg gagccagagt ttactcgtgt tggttatcta ttcatcagct 6060

tcctgacatg ttaagagaat acattaaaga gaaaatactg tttttaatc ctaaaatttt 6120

tcttccacta agataaacca aatgtcctta catatatgta aacccatcta tttaaacgca 6180

aaggtgggtt gatgtcagtt tacatagcag aaagcattca ctatcctcta agatttgttt 6240

ctgcaaaact ttcattgctt tagaatttta aaatttcacc ttgtacaatg gccagcccct 6300

aaagcaggaa acatttataa tggattatat ggaaacatcc tcccagtact tgcccagccc 6360

ttgaatcatg tggcttttca gtgaaaggaa agattctttt tctaggaaaa atgagcctat 6420

tttattttat tttattttat tttttgacac aaactgtaga ttttagcagc cctggcccaa 6480

aggaatttga ttacttttgt tttaaacagt acaaagggga cactataatt acaaaaacat 6540

ccttaactga tttgagttgt ttttatttct ttggatatat tttcagagtg gtaaattgtg 6600

tgtgagaatt acaaatgatt attcttttag tggtttctta gcctctctta cagcccacgg 6660

ggatagtact gtacatcaat accttcatat gaaattttta tatgcaatga aaataaaagc 6720

atgggttgat tctgcctatt tatgactcaa tcttttacaa ataaaagatt attcatttta 6780

aattatagtt caatcagcat gtctcttagg atactgaacg tggttgaaat gaaaggatag 6840

tgacatcata agttagtact gatattcata accaaataaa gccaacttga gtaattttgc 6900

tacattaaaa attaccaaaa ttacttagat ggcctataag attaagcatg gtgttttcta 6960

agcaagcttt gaaaggggcc ttccatactt acttaattga atattctggg atattgaaaa 7020

ttattcagat acttgacaat tatttttggt tacctactcc gcaaactaca aagttttaag 7080

gactcaacaa taagttaatg agacacagtg tttgctttca tggagcttac agtctggagg 7140

ggacaaaggc ttaaacaata ctcatataat tatatatgtg atcagtacaa tgaaggagct 7200

cagtggggta aataagcagg aacctgaact tgatctgttc cggagggcca cagaaggctt 7260

ccttgaggcc ttgagaaagt gatttgcatc tgagttctga aggattgtaa gaggtaacta 7320

gggaaaaagt tgacaggaag aggaagggga tccagacaag aaacatttgc aaagatcttg 7380

aggcataaat gagcttgaga catctggaga aactgaggaa aagtgagaga gtaggcaggg 7440

cctggagccg cagagccatt gctaaccatc ctgtgtgaga tatcccccat tctgtagctt 7500

tattctcata accctgctca attttcttta taacacttct cacagattta tatacgtgtt 7560

tgtttttgtt atctgtctct cccaccagac cacagctcca tgagagcaag gtctttgctt 7620

accaatatat cactagcact taaaactatg cctggtacac agtaggttct taatatgtgt 7680

tgaatatagc catcaaattg atattggata taattcaatc tgataagata ttttgagata 7740

ttaaagagtt tttaacttga taccataaaa aaaaaaaaaa aaa 7783

<210> 15
<211> 7667
<212> DNA
<213> Homo sapiens

<400> 15
acttcaagtg ccgtgcagaa ggctcggcag gcggggcggg cgtggggccg cggctccggg 60

ttggggaccg aggagatccg gctgtggacc agacgctcct ctgcggggcg ggcacccaag 120

cgcgctcgcc accccctcgc catccgctag agccgggctc ctggactggg actcgggccc 180

gccgcacagt tgaaaagtcg catagtggtt tttccgctcg cgtcgctgtg tgaaagttgg 240

ctcgccgctc tttgcacgcc ctccctggag gccgacccga gacgccaagc tggagagacc 300

gtgcctcccc gaggccggcc gccccgcgag cacagcctcc gcccccgttg cactgccggg 360

ctgggcaata tgaaggagca gccctcatgt gccggcaccg ggcatccgag catggcggga 420

ggaggcctac cagaaactgg ccagcgagac cctggaggag ctggactggt gtctggacca 480

gctagagacc ctacagacca ggcactccgt cagtgagatg gcctccaaca agtttaaaag 540

gatgcttaat cgggagctca cccatctctc tgaaatgagt cggtctggaa atcaagtgtc 600
agagtttata tcaaacacat tcttagataa gcaacatgaa gtggaaattc cttctccaac 660
tcagaaggaa aaggagaaaa agaaaagacc aatgtctcag atcagtggag tcaagaaatt 720
gatgcacagc tctagtctga ctaattcaag tatcccaagg tttggagtta aaactgaaca 780
agaagatgtc cttgccaagg aactagaaga tgtgaacaaa tggggtcttc atgttttcag 840
aatagcagag ttgtctggta accggccctt gactgttatc atgcacacca tttttcagga 900
acgggattta ttaaaaacat ttaaaattcc agtagatact ttaattacat atcttatgac 960
tctcgaagac cattaccatg ctgatgtggc ctatcacaac aatatccatg ctgcagatgt 1020
tgtccagtct actcatgtgc tattatctac acctgctttg gaggctgtgt ttacagattt 1080
ggagattctt gcagcaattt ttgccagtgc aatacatgat gtagatcatc ctggtgtgtc 1140
caatcaattt ctgatcaata caaactctga acttgccttg atgtacaatg attcctcagt 1200
cttagagaac catcatttgg ctgtgggctt taaattgctt caggaagaaa actgtgacat 1260
tttccagaat ttgaccaaaa aacaaagaca atctttaagg aaaatggtca ttgacatcgt 1320
acttgcaaca gatatgtcaa aacacatgaa tctactggct gatttgaaga ctatggttga 1380
aactaagaaa gtgacaagct ctggagttct tcttcttgat aattattccg ataggattca 1440
ggttcttcag aatatggtgc actgtgcaga tctgagcaac ccaacaaagc ctctccagct 1500
gtaccgccag tggacggacc ggataatgga ggagttcttc cgccaaggag accgagagag 1560
ggaacgtggc atggagataa gccccatgtg tgacaagcac aatgcttccg tggaaaaatc 1620
acaggtgggc ttcatagact atattgttca tcccctctgg gagacatggg cagacctcgt 1680
ccaccctgac gcccaggata ttttggacac tttggaggac aatcgtgaat ggtaccagag 1740
cacaatccct cagagcccct ctcctgcacc tgatgaccca gaggagggcc ggcagggtca 1800
aactgagaaa ttccagtttg aactaacttt agaggaagat ggtgagtcag acacggaaaa 1860
ggacagtggc agtcaagtgg aagaagacac tagctgcagt gactccaaga ctctttgtac 1920
tcaagactca gagtctactg aaattcccct tgatgaacag gttgaagagg aggcagtagg 1980
ggaagaagag gaaagccagc ctgaagcctg tgtcatagat gatcgttctc ctgacacgta 2040
acagtgcaaa aactttcatg cctttttttt ttttaagtag aaaaattgtt tccaaagtgc 2100
atgtcacatg ccacaaccac ggtcacacct cactgtcatc tgccaggacg tttgttgaac 2160
aaaactgacc ttgactactc agtccagcgc tcaggaatat cgtaaccagt tttttcacct 2220
ccatgtcatc cgagcaaggt ggacatcttc acgaacagcg tttttaacaa gatttcagct 2280
tggtagagct gacaaagcag ataaaatcta ctccaaatta ttttcaagag agtgtgactc 2340
atcaggcagc ccaaaagttt attggacttg gggtttctat tcctttttat ttgtttgcaa 2400
tattttcaga agaaaggcat tgcacagagt gaacttaatg gacgaagcaa caaatatgtc 2460

aagaacagga catagcacga atctgttacc agtaggagga ggatgagcca cagaaattgc 2520

ataattttct aatttcaagt cttcctgata catgactgaa tagtgtggtt cagtgagctg 2580

cactgacctc tacattttgt atgatatgta aaacagattt tttgtagagc ttacttttat 2640

tattaaatgt attgaggtat tatatttaaa aaaaactatg ttcagaactt catctgccac 2700

tggttatttt tttctaagga gtaacttgca agttttcagt acaaatctgt gctacactgg 2760

ataaaaatct aatttatgaa ttttacttgc accttatagt tcatagcaat taactgattt 2820

gtagtgattc attgtttgtt ttatatacca atgacttcca tattttaaaa gagaaaaaca 2880

actttatgtt gcaggaaacc ctttttgtaa gtctttatta tttactttgc attttgtttc 2940

actctttcca gataagcaga gttgctcttc accagtgttt ttcttcatgt gcaaagtgac 3000

tatttgttct ataatacttt tatgtgtgtt atatcaaatg tgtcttaagc ttcatgcaaa 3060

ctcagtcatc agttcgtgtt gtctgaagca agtgggagat atataaatac ccagtagcta 3120

aaatggtcag tctttttag atgttttcct acttagtatc tcctaataac gttttgctgt 3180

gtcactagat gttcatttca caagtgcatg tctttctaat aatccacaca tttcatgctc 3240

taataatcca cacatttcat gctcattttt attgttttta cagccagtta tagtaagaaa 3300

aaggtttttc cccttgtgct gctttataat ttagcgtgtg tctgaacctt atccatgttt 3360

gctagatgag gtcttgtcaa atatatcact accattgtca ccggtgaaaa gaaacaggta 3420

gttaagttag ggttaacatt catttcaacc acgaggttgt atatcatgac tagcttttac 3480

tcttggttta cagagaaaag ttaaacagcc aactaggcag tttttaagaa tattaacaat 3540

atattaacaa acaccaatac aactaatcct atttggtttt aatgatttca ccatgggatt 3600

aagaactata tcaggaacat ccctgagaaa cggttttaag tgtagcaact actcttcctt 3660

aatggacagc cacataacgt gtaggaagtc ctttatcact tatcctcgat ccataagcat 3720

atcttgcaga ggggaactac ttctttaaac acatggaggg aaagaagatg atgccactgg 3780

caccagaggg ttagtactgt gatgcatcct aaaatattta ttatattggt aaaaattctg 3840

gttaaataaa aaattagaga tcactcttgg ctgatttcag caccaggaac tgtattacag 3900

ttttagagat taattcctag tgtttacctg attatagcag ttggcatcat ggggcattta 3960

attctgactt tatccccacg tcagccttaa taaagtcttc tttaccttct ctatgaagac 4020

tttaaagccc aaataatcat ttttcacatt gatattcaag aattgagata gatagaagcc 4080

aaagtgggta tctgacaagt ggaaaatcaa acgtttaaga agaattacaa ctctgaaaag 4140

catttatatg tggaacttct caaggagcct cctggggact ggaaagtaag tcatcagcca 4200

ggcaaatgac tcatgctgaa gagagtcccc atttcagtcc cctgagatct agctgatgct 4260

tagatccttt gaaataaaaa ttatgtcttt ataactctga tcttttacat aaagcagaag 4320

aggaatcaac tagttaattg caaggtttct actctgtttc ctctgtaaag atcagatggt 4380

aatctttcaa ataagaaaaa aataaagacg tatgtttgac caagtagttt cacaagaata 4440

tttgggaact tgtttctttt aattttattt gtccctgagt gaagtctaga aagaaaggta 4500

aagagtctag agtttattcc tctttccaaa acattctcat tcctctcctc cctacactta 4560

gtatttcccc cacagagtgc ctagaatctt aataatgaat aaaataaaaa gcagcaatat 4620

gtcattaaca aatccagacc tgaaagggta aagggtttat aactgcacta ataaagagag 4680

gctctttttt tttcttccag tttgttggtt tttaatggta ccgtgttgta aagataccca 4740

ctaatggaca atcaaattgc agaaaaggct caatatccaa gagacaggga ctaatgcact 4800

gtacaatctg cttatccttg cccttctctc ttgccaaagt gtgcttcaga aatatatact 4860

gctttaaaaa agaataaaag aatatccttt tacaagtggc tttacatttc ctaaaatgcc 4920

ataagaaaat gcaatatctg ggtactgtat ggggaaaaaa atgtccaagt ttgtgtaaaa 4980

ccagtgcatt tcagcttgca agttactgaa cacaataatg ctgttttaat tttgttttat 5040

atcagttaaa attcacaata atgtagatag aacaaattac agacaaggaa agaaaaaact 5100

tgaatgaaat ggattttaca gaaagcttta tgataatttt tgaatgcatt atttattttt 5160

tgtgccatgc attttttttc tcaccaaatg accttacctg taatacagtc ttgtttgtct 5220

gtttacaacc atgtatttat tgcaatgtac atactgtaat gttaattgta aattatctgt 5280

tcttattaaa acatcatccc atgatgggat ggtgttgata tatttggaaa ctcttggtga 5340

gagaatgaat ggtgtgtata catactctgt acatttttct tttctcctgt aatatagtct 5400

tgtcacctta gagcttgttt atggaagatt caagaaaact ataaaatact taaagatata 5460

taaatttaaa aaaacatagc tgcaggtctt tggtcccagg gctgtgcctt aactttaacc 5520

aatattttct tctgttttgc tgcatttgaa aggtaacagt ggagctaggg ctgggcattt 5580

tacatccagg cttttaattg attagaattc tgccaatagg tggattttac aaaaccacag 5640

acaacctctg aaagattctg agaccctttt gagacagaag ctcttaagta cttcttgcca 5700

gggagcagca ctgcatgtgt gatggttgtt tgccatctgt tgatcaggaa ctacttcagc 5760

tacttgcatt tgattatttc cttttttttt ttttttaact cggaaacaca actggggaaa 5820

tatattcttt cccagtgatt ataaacaatc tttttctttt ttttaagtcc ttttggcttc 5880

tagagctcat aggaaaatgg acttgatttg aaattggagc cagagtttac tcgtgttggt 5940

tatctattca tcagcttcct gacatgttaa gagaatacat taaagagaaa atactgtttt 6000

ttaatcctaa aatttttctt ccactaagat aaaccaaatg tccttacata tatgtaaacc 6060

catctattta aacgcaaagg tgggttgatg tcagtttaca tagcagaaag cattcactat 6120

cctctaagat ttgtttctgc aaaactttca ttgctttaga attttaaaat ttcaccttgt 6180

acaatggcca gcccctaaag caggaaacat ttataatgga ttatatggaa acatcctccc 6240

agtacttgcc cagcccttga atcatgtggc ttttcagtga aaggaaagat tctttttcta 6300

ggaaaaatga gcctatttta ttttatttta ttttattttt tgacacaaac tgtagatttt 6360

agcagccctg gcccaaagga atttgattac ttttgtttta aacagtacaa aggggacact 6420

ataattacaa aaacatcctt aactgatttg agttgttttt atttctttgg atatattttc 6480

agagtggtaa attgtgtgtg agaattacaa atgattattc ttttagtggt ttcttagcct 6540

ctcttacagc ccacggggat agtactgtac atcaatacct tcatatgaaa tttttatatg 6600

caatgaaaat aaaagcatgg gttgattctg cctatttatg actcaatctt ttacaaataa 6660

aagattattc attttaaatt atagttcaat cagcatgtct cttaggatac tgaacgtggt 6720

tgaaatgaaa ggatagtgac atcataagtt agtactgata ttcataacca aataaagcca 6780

acttgagtaa ttttgctaca ttaaaaatta ccaaaattac ttagatggcc tataagatta 6840

agcatggtgt tttctaagca agctttgaaa ggggccttcc atacttactt aattgaatat 6900

tctgggatat tgaaaattat tcagatactt gacaattatt tttggttacc tactccgcaa 6960

actacaaagt tttaaggact caacaataag ttaatgagac acagtgtttg ctttcatgga 7020

gcttacagtc tggaggggac aaaggcttaa acaatactca tataattata tatgtgatca 7080

gtacaatgaa ggagctcagt ggggtaaata agcaggaacc tgaacttgat ctgttccgga 7140

gggccacaga aggcttcctt gaggccttga gaaagtgatt tgcatctgag ttctgaagga 7200

ttgtaagagg taactaggga aaaagttgac aggaagagga aggggatcca gacaagaaac 7260

atttgcaaag atcttgaggc ataaatgagc ttgagacatc tggagaaact gaggaaaagt 7320

gagagagtag gcagggcctg gagccgcaga gccattgcta accatcctgt gtgagatatc 7380

ccccattctg tagctttatt ctcataaccc tgctcaattt tctttataac acttctcaca 7440

gatttatata cgtgtttgtt tttgttatct gtctctccca ccagaccaca gctccatgag 7500

agcaaggtct ttgcttacca atatatcact agcacttaaa actatgcctg gtacacagta 7560

ggttcttaat atgtgttgaa tatagccatc aaattgatat tggatataat tcaatctgat 7620

aagatatttt gagatattaa agagttttta acttgatacc ataaaaa 7667

<210> 16
<211> 8379
<212> DNA
<213> Homo sapiens

<400> 16
cactgccctg cggtgttttg aactgccttc ttacagacgt catacagccc ttgaggaata 60

gtttctgcct ggtgagattg aatgatagtt ctcattcaca aaaccctgga ttctaagcag 120

ggacacacag aaattacttt cgcaggtaaa tcagcccacc cagccaaagt gtggagagat 180

ttgttccttg gctgacttct ttgctccacg gagaggagtg ttttcctgtg cttgccctga 240

aatggaactt ccttgacagc tctcccgtgt tacagtacct cccggtcatt ttctttttct 300

ctctctctac ctgcgctctt cgagtgtcag aaacctttaa agctgttact atggaattgc 360

aaaaaagaga tcaagtgact ctttcactat gctggtttcc cttgtgaccc agatgaagaa 420

tcaattcaga attcagttcc tcccttggca ttgcaagaca cagaagaaac tgtcacttcc 480

taacagccta gtactggagt aaattcagta tgaaggaaga aagcgctcct gcgtgttaga 540

accttgccca tgagctggac cgaggacagg agatggactc caggaaaatt ggatttcttc 600

aagcagcctc ccttggaaat ggaatatctt taaaatcttc tttgcagaaa gacagttaga 660

atgtattaat cagaatagtt gaagacttat tttccttttt atttttttc aaaatgagca 720

ttattatgaa gccaagatcc cgatctacaa gttccctaag gactgcagag gcagtttgtt 780

ttgatgtgga caatggcaca tctgcgggac ggagtccctt ggatcccatg accagcccag 840

gatccgggct aattctccaa gcaaattttg tccacagtca acgacgggag tccttcctgt 900

atcgatccga cagcgattat gacctctctc caaagtctat gtcccggaac tcctccattg 960

ccagtgatat acacggagat gacttgattg tgactccatt tgctcaggtc ttggccagtc 1020

tgcgaactgt acgaaacaac tttgctgcat taactaattt gcaagatcga gcacctagca 1080

aaagatcacc catgtgcaac caaccatcca tcaacaaagc caccataaca gaggaggcct 1140

accagaaact ggccagcgag accctggagg agctggactg gtgtctggac cagctagaga 1200

ccctacagac caggcactcc gtcagtgaga tggcctccaa caagtttaaa aggatgctta 1260

atcgggagct cacccatctc tctgaaatga gtcggtctgg aaatcaagtg tcagagttta 1320

tatcaaacac attcttagat aagcaacatg aagtggaaat tccttctcca actcagaagg 1380

aaaaggagaa aaagaaaaga ccaatgtctc agatcagtgg agtcaagaaa ttgatgcaca 1440

gctctagtct gactaattca agtatcccaa ggtttggagt taaaactgaa caagaagatg 1500

tccttgccaa ggaactagaa gatgtgaaca aatggggtct tcatgttttc agaatagcag 1560

agttgtctgg taaccggccc ttgactgtta tcatgcacac catttttcag gaacgggatt 1620

tattaaaaac atttaaaatt ccagtagata ctttaattac atatcttatg actctcgaag 1680

accattacca tgctgatgtg gcctatcaca acaatatcca tgctgcagat gttgtccagt 1740

ctactcatgt gctattatct acacctgctt tggaggctgt gtttacagat ttggagattc 1800

ttgcagcaat ttttgccagt gcaatacatg atgtagatca tcctggtgtg tccaatcaat 1860

ttctgatcaa tacaaactct gaacttgcct tgatgtacaa tgattcctca gtcttagaga 1920

accatcattt ggctgtgggc tttaaattgc ttcaggaaga aaactgtgac attttccaga 1980

atttgaccaa aaaacaaaga caatctttaa ggaaaatggt cattgacatc gtacttgcaa 2040

cagatatgtc aaaacacatg aatctactgg ctgatttgaa gactatggtt gaaactaaga 2100

aagtgacaag ctctggagtt cttcttcttg ataattattc cgataggatt caggttcttc 2160

agaatatggt gcactgtgca gatctgagca acccaacaaa gcctctccag ctgtaccgcc 2220

agtggacgga ccggataatg gaggagttct tccgccaagg agaccgagag agggaacgtg 2280

gcatggagat aagccccatg tgtgacaagc acaatgcttc cgtggaaaaa tcacaggtgg 2340

gcttcataga ctatattgtt catcccctct gggagacatg ggcagacctc gtccaccctg 2400

acgcccagga tattttggac actttggagg acaatcgtga atggtaccag agcacaatcc 2460

ctcagagccc ctctcctgca cctgatgacc cagaggaggg ccggcagggt caaactgaga 2520

aattccagtt tgaactaact ttagaggaag atggtgagtc agacacggaa aaggacagtg 2580

gcagtcaagt ggaagaagac actagctgca gtgactccaa gactctttgt actcaagact 2640

cagagtctac tgaaattccc cttgatgaac aggttgaaga ggaggcagta ggggaagaag 2700

aggaaagcca gcctgaagcc tgtgtcatag atgatcgttc tcctgacacg taacagtgca 2760

aaaactttca tgcctttttt ttttttaagt agaaaaattg tttccaaagt gcatgtcaca 2820

tgccacaacc acggtcacac ctcactgtca tctgccagga cgtttgttga acaaaactga 2880

ccttgactac tcagtccagc gctcaggaat atcgtaacca gttttttcac ctccatgtca 2940

tccgagcaag gtggacatct tcacgaacag cgtttttaac aagatttcag cttggtagag 3000

ctgacaaagc agataaaatc tactccaaat tattttcaag agagtgtgac tcatcaggca 3060

gcccaaaagt ttattggact tggggtttct attccttttt atttgtttgc aatattttca 3120

gaagaaaggc attgcacaga gtgaacttaa tggacgaagc aacaaatatg tcaagaacag 3180

gacatagcac gaatctgtta ccagtaggag gaggatgagc cacagaaatt gcataatttt 3240

ctaatttcaa gtcttcctga tacatgactg aatagtgtgg ttcagtgagc tgcactgacc 3300

tctacatttt gtatgatatg taaaacagat tttttgtaga gcttactttt attattaaat 3360

gtattgaggt attatattta aaaaaaacta tgttcagaac ttcatctgcc actggttatt 3420

tttttctaag gagtaacttg caagttttca gtacaaatct gtgctacact ggataaaaat 3480

ctaatttatg aattttactt gcaccttata gttcatagca attaactgat ttgtagtgat 3540

tcattgtttg ttttatatac caatgacttc catattttaa aagagaaaaa caactttatg 3600

ttgcaggaaa cccttttttgt aagtctttat tatttacttt gcattttgtt tcactctttc 3660

cagataagca gagttgctct tcaccagtgt ttttcttcat gtgcaaagtg actatttgtt 3720

ctataatact tttatgtgtg ttatatcaaa tgtgtcttaa gcttcatgca aactcagtca 3780

tcagttcgtg ttgtctgaag caagtgggag atatataaat acccagtagc taaaatggtc 3840

agtctttttt agatgttttc ctacttagta tctcctaata acgttttgct gtgtcactag 3900

atgttcattt cacaagtgca tgtctttcta ataatccaca catttcatgc tctaataatc 3960

cacacatttc atgctcattt ttattgtttt tacagccagt tatagtaaga aaaaggtttt 4020

tccccttgtg ctgctttata atttagcgtg tgtctgaacc ttatccatgt ttgctagatg 4080
aggtcttgtc aaatatatca ctaccattgt caccggtgaa aagaaacagg tagttaagtt 4140
agggttaaca ttcatttcaa ccacgaggtt gtatatcatg actagctttt actcttggtt 4200
tacagagaaa agttaaacag ccaactaggc agtttttaag aatattaaca atatattaac 4260
aaacaccaat acaactaatc ctatttggtt ttaatgattt caccatggga ttaagaacta 4320
tatcaggaac atccctgaga aacggtttta agtgtagcaa ctactcttcc ttaatggaca 4380
gccacataac gtgtaggaag tcctttatca cttatcctcg atccataagc atatcttgca 4440
gaggggaact acttctttaa acacatggag ggaaagaaga tgatgccact ggcaccagag 4500
ggttagtact gtgatgcatc ctaaaatatt tattatattg gtaaaaattc tggttaaata 4560
aaaaattaga gatcactctt ggctgatttc agcaccagga actgtattac agttttagag 4620
attaattcct agtgtttacc tgattatagc agttggcatc atggggcatt taattctgac 4680
tttatcccca cgtcagcctt aataaagtct tctttacctt ctctatgaag actttaaagc 4740
ccaaataatc atttttcaca ttgatattca agaattgaga tagatagaag ccaaagtggg 4800
tatctgacaa gtggaaaatc aaacgtttaa gaagaattac aactctgaaa agcatttata 4860
tgtggaactt ctcaaggagc ctcctgggga ctggaaagta agtcatcagc caggcaaatg 4920
actcatgctg aagagagtcc ccatttcagt cccctgagat ctagctgatg cttagatcct 4980
ttgaaataaa aattatgtct ttataactct gatcttttac ataaagcaga agaggaatca 5040
actagttaat tgcaaggttt ctactctgtt tcctctgtaa agatcagatg gtaatctttc 5100
aaataagaaa aaaataaaga cgtatgtttg accaagtagt ttcacaagaa tatttgggaa 5160
cttgtttctt ttaattttat ttgtccctga gtgaagtcta gaaagaaagg taaagagtct 5220
agagtttatt cctctttcca aaacattctc attcctctcc tccctacact tagtatttcc 5280
cccacagagt gcctagaatc ttaataatga ataaaataaa aagcagcaat atgtcattaa 5340
caaatccaga cctgaaaggg taaagggttt ataactgcac taataaagag aggctctttt 5400
tttttcttcc agtttgttgg tttttaatgg taccgtgttg taaagatacc cactaatgga 5460
caatcaaatt gcagaaaagg ctcaatatcc aagagacagg gactaatgca ctgtacaatc 5520
tgcttatcct tgcccttctc tcttgccaaa gtgtgcttca gaaatatata ctgctttaaa 5580
aaagaataaa agaatatcct tttacaagtg gctttacatt tcctaaaatg ccataagaaa 5640
atgcaatatc tgggtactgt atggggaaaa aaatgtccaa gtttgtgtaa aaccagtgca 5700
tttcagcttg caagttactg aacacaataa tgctgtttta attttgtttt atatcagtta 5760
aaattcacaa taatgtagat agaacaaatt acagacaagg aaagaaaaaa cttgaatgaa 5820
atggatttta cagaaagctt tatgataatt tttgaatgca ttatttattt tttgtgccat 5880

gcattttttt tctcaccaaa tgaccttacc tgtaatacag tcttgtttgt ctgtttacaa 5940

ccatgtattt attgcaatgt acatactgta atgttaattg taaattatct gttcttatta 6000

aaacatcatc ccatgatggg atggtgttga tatatttgga aactcttggt gagagaatga 6060

atggtgtgta tacatactct gtacattttt cttttctcct gtaatatagt cttgtcacct 6120

tagagcttgt ttatggaaga ttcaagaaaa ctataaaata cttaaagata tataaattta 6180

aaaaaacata gctgcaggtc tttggtccca gggctgtgcc ttaactttaa ccaatatttt 6240

cttctgtttt gctgcatttg aaaggtaaca gtggagctag ggctgggcat tttacatcca 6300

ggcttttaat tgattagaat tctgccaata ggtggatttt acaaaaccac agacaacctc 6360

tgaaagattc tgagaccctt ttgagacaga agctcttaag tacttcttgc cagggagcag 6420

cactgcatgt gtgatggttg tttgccatct gttgatcagg aactacttca gctacttgca 6480

tttgattatt tccttttttt tttttttaa ctcggaaaca caactgggga aatatattct 6540

ttcccagtga ttataaacaa tctttttctt ttttttaagt ccttttggct tctagagctc 6600

ataggaaaat ggacttgatt tgaaattgga gccagagttt actcgtgttg gttatctatt 6660

catcagcttc ctgacatgtt aagagaatac attaaagaga aaatactgtt ttttaatcct 6720

aaaatttttc ttccactaag ataaaccaaa tgtccttaca tatatgtaaa cccatctatt 6780

taaacgcaaa ggtgggttga tgtcagttta catagcagaa agcattcact atcctctaag 6840

atttgtttct gcaaaacttt cattgcttta gaattttaaa atttcacctt gtacaatggc 6900

cagcccctaa agcaggaaac atttataatg gattatatgg aaacatcctc ccagtacttg 6960

cccagccctt gaatcatgtg gcttttcagt gaaaggaaag attctttttc taggaaaaat 7020

gagcctattt tattttattt tattttattt tttgacacaa actgtagatt ttagcagccc 7080

tggcccaaag gaatttgatt acttttgttt taaacagtac aaaggggaca ctataattac 7140

aaaaacatcc ttaactgatt tgagttgttt ttatttcttt ggatatattt tcagagtggt 7200

aaattgtgtg tgagaattac aaatgattat tcttttagtg gtttcttagc ctctcttaca 7260

gcccacgggg atagtactgt acatcaatac cttcatatga aatttttata tgcaatgaaa 7320

ataaaagcat gggttgattc tgcctattta tgactcaatc ttttacaaat aaaagattat 7380

tcattttaaa ttatagttca atcagcatgt ctcttaggat actgaacgtg gttgaaatga 7440

aaggatagtg acatcataag ttagtactga tattcataac caaataaagc caacttgagt 7500

aattttgcta cattaaaaat taccaaaatt acttagatgg cctataagat taagcatggt 7560

gttttctaag caagctttga aaggggcctt ccatacttac ttaattgaat attctgggat 7620

attgaaaatt attcagatac ttgacaatta tttttggtta cctactccgc aaactacaaa 7680

gttttaagga ctcaacaata agttaatgag acacagtgtt tgctttcatg gagcttacag 7740

tctggagggg acaaaggctt aaacaatact catataatta tatatgtgat cagtacaatg 7800

aaggagctca gtggggtaaa taagcaggaa cctgaacttg atctgttccg gagggccaca 7860

gaaggcttcc ttgaggcctt gagaaagtga tttgcatctg agttctgaag gattgtaaga 7920

ggtaactagg gaaaaagttg acaggaagag gaaggggatc cagacaagaa acatttgcaa 7980

agatcttgag gcataaatga gcttgagaca tctggagaaa ctgaggaaaa gtgagagagt 8040

aggcagggcc tggagccgca gagccattgc taaccatcct gtgtgagata tcccccattc 8100

tgtagcttta ttctcataac cctgctcaat tttctttata acacttctca cagatttata 8160

tacgtgtttg tttttgttat ctgtctctcc caccagacca cagctccatg agagcaaggt 8220

ctttgcttac caatatatca ctagcactta aaactatgcc tggtacacag taggttctta 8280

atatgtgttg aatatagcca tcaaattgat attggatata attcaatctg ataagatatt 8340

ttgagatatt aaagagtttt taacttgata ccataaaaa 8379

<210> 17
<211> 7545
<212> DNA
<213> Homo sapiens

<400> 17
ctttcttgca actaagcatc ttgacataca ttattcatta agccctggag ctcgggagag 60

aaagatgcag acccttagat ctttagatat tcctttatca cgtggatttt ctttattcag 120

aatagttgct gaattttgtg ccattctgga gtcttacaaa tggcatgtat tcgatgggaa 180

gacggctgga tgggatttaa tgcgaggctt tcttatgtat acttaattac caaaaatctt 240

taaaaactca tactctgcgt ggcttgtgga ggttgttaaa gtgtcgagat tttgaagcta 300

aatacatttt agagcttact atatatatac atatatatat atatacatat aatcaatcaa 360

aaatgcctga agcaaactat ttactgtcag tgtcttgggg ctacataaag tttaaaagga 420

tgcttaatcg ggagctcacc catctctctg aaatgagtcg gtctggaaat caagtgtcag 480

agtttatatc aaacacattc ttagataagc aacatgaagt ggaaattcct tctccaactc 540

agaaggaaaa ggagaaaaag aaaagaccaa tgtctcagat cagtggagtc aagaaattga 600

tgcacagctc tagtctgact aattcaagta tcccaaggtt tggagttaaa actgaacaag 660

aagatgtcct tgccaaggaa ctagaagatg tgaacaaatg gggtcttcat gttttcagaa 720

tagcagagtt gtctggtaac cggcccttga ctgttatcat gcacaccatt tttcaggaac 780

gggatttatt aaaaacattt aaaattccag tagatacttt aattacatat cttatgactc 840

tcgaagacca ttaccatgct gatgtggcct atcacaacaa tatccatgct gcagatgttg 900

tccagtctac tcatgtgcta ttatctacac ctgctttgga ggctgtgttt acagatttgg 960

agattcttgc agcaattttt gccagtgcaa tacatgatgt agatcatcct ggtgtgtcca 1020

atcaatttct gatcaataca aactctgaac ttgccttgat gtacaatgat tcctcagtct 1080

tagagaacca tcatttggct gtgggcttta aattgcttca ggaagaaaac tgtgacattt 1140
tccagaattt gaccaaaaaa caaagacaat ctttaaggaa aatggtcatt gacatcgtac 1200
ttgcaacaga tatgtcaaaa cacatgaatc tactggctga tttgaagact atggttgaaa 1260
ctaagaaagt gacaagctct ggagttcttc ttcttgataa ttattccgat aggattcagg 1320
ttcttcagaa tatggtgcac tgtgcagatc tgagcaaccc aacaaagcct ctccagctgt 1380
accgccagtg gacggaccgg ataatggagg agttcttccg ccaaggagac cgagagaggg 1440
aacgtggcat ggagataagc cccatgtgtg acaagcacaa tgcttccgtg gaaaaatcac 1500
aggtgggctt catagactat attgttcatc ccctctggga gacatgggca gacctcgtcc 1560
accctgacgc ccaggatatt ttggacactt tggaggacaa tcgtgaatgg taccagagca 1620
caatccctca gagcccctct cctgcacctg atgacccaga ggagggccgg cagggtcaaa 1680
ctgagaaatt ccagtttgaa ctaactttag aggaagatgg tgagtcagac acggaaaagg 1740
acagtggcag tcaagtggaa gaagacacta gctgcagtga ctccaagact ctttgtactc 1800
aagactcaga gtctactgaa attccccttg atgaacaggt tgaagaggag gcagtagggg 1860
aagaagagga aagccagcct gaagcctgtg tcatagatga tcgttctcct gacacgtaac 1920
agtgcaaaaa ctttcatgcc tttttttttt ttaagtagaa aaattgtttc caaagtgcat 1980
gtcacatgcc acaaccacgg tcacacctca ctgtcatctg ccaggacgtt tgttgaacaa 2040
aactgacctt gactactcag tccagcgctc aggaatatcg taaccagttt tttcacctcc 2100
atgtcatccg agcaaggtgg acatcttcac gaacagcgtt tttaacaaga tttcagcttg 2160
gtagagctga caaagcagat aaaatctact ccaaattatt ttcaagagag tgtgactcat 2220
caggcagccc aaaagtttat tggacttggg gtttctattc ctttttattt gtttgcaata 2280
ttttcagaag aaaggcattg cacagagtga acttaatgga cgaagcaaca aatatgtcaa 2340
gaacaggaca tagcacgaat ctgttaccag taggaggagg atgagccaca gaaattgcat 2400
aattttctaa tttcaagtct tcctgataca tgactgaata gtgtggttca gtgagctgca 2460
ctgacctcta cattttgtat gatatgtaaa acagattttt tgtagagctt acttttatta 2520
ttaaatgtat tgaggtatta tatttaaaaa aaactatgtt cagaacttca tctgccactg 2580
gttatttttt tctaaggagt aacttgcaag ttttcagtac aaatctgtgc tacactggat 2640
aaaaatctaa tttatgaatt ttacttgcac cttatagttc atagcaatta actgatttgt 2700
agtgattcat tgtttgtttt atataccaat gacttccata ttttaaaaga gaaaaacaac 2760
tttatgttgc aggaaaccct ttttgtaagt ctttattatt tactttgcat tttgtttcac 2820
tctttccaga taagcagagt tgctcttcac cagtgttttt cttcatgtgc aaagtgacta 2880
tttgttctat aatactttta tgtgtgttat atcaaatgtg tcttaagctt catgcaaact 2940

cagtcatcag ttcgtgttgt ctgaagcaag tgggagatat ataaataccc agtagctaaa 3000
atggtcagtc ttttttagat gttttcctac ttagtatctc ctaataacgt tttgctgtgt 3060
cactagatgt tcatttcaca agtgcatgtc tttctaataa tccacacatt tcatgctcta 3120
ataatccaca catttcatgc tcatttttat tgtttttaca gccagttata gtaagaaaaa 3180
ggtttttccc cttgtgctgc tttataattt agcgtgtgtc tgaaccttat ccatgtttgc 3240
tagatgaggt cttgtcaaat atatcactac cattgtcacc ggtgaaaaga aacaggtagt 3300
taagttaggg ttaacattca tttcaaccac gaggttgtat atcatgacta gcttttactc 3360
ttggtttaca gagaaaagtt aaacagccaa ctaggcagtt tttaagaata ttaacaatat 3420
attaacaaac accaatacaa ctaatcctat ttggttttaa tgatttcacc atgggattaa 3480
gaactatatc aggaacatcc ctgagaaacg gttttaagtg tagcaactac tcttccttaa 3540
tggacagcca cataacgtgt aggaagtcct ttatcactta tcctcgatcc ataagcatat 3600
cttgcagagg ggaactactt ctttaaacac atggagggaa agaagatgat gccactggca 3660
ccagagggtt agtactgtga tgcatcctaa aatatttatt atattggtaa aaattctggt 3720
taaataaaaa attagagatc actcttggct gatttcagca ccaggaactg tattacagtt 3780
ttagagatta attcctagtg tttacctgat tatagcagtt ggcatcatgg ggcatttaat 3840
tctgacttta tccccacgtc agccttaata aagtcttctt taccttctct atgaagactt 3900
taaagcccaa ataatcattt ttcacattga tattcaagaa ttgagataga tagaagccaa 3960
agtgggtatc tgacaagtgg aaaatcaaac gtttaagaag aattacaact ctgaaaagca 4020
tttatatgtg gaacttctca aggagcctcc tggggactgg aaagtaagtc atcagccagg 4080
caaatgactc atgctgaaga gagtccccat ttcagtcccc tgagatctag ctgatgctta 4140
gatcctttga aataaaaatt atgtctttat aactctgatc ttttacataa agcagaagag 4200
gaatcaacta gttaattgca aggtttctac tctgtttcct ctgtaaagat cagatggtaa 4260
tctttcaaat aagaaaaaaa taaagacgta tgtttgacca agtagtttca caagaatatt 4320
tgggaacttg tttcttttaa ttttatttgt ccctgagtga agtctagaaa gaaaggtaaa 4380
gagtctagag tttattcctc tttccaaaac attctcattc ctctcctccc tacacttagt 4440
atttccccca cagagtgcct agaatcttaa taatgaataa aataaaaagc agcaatatgt 4500
cattaacaaa tccagacctg aaagggtaaa gggtttataa ctgcactaat aaagagaggc 4560
tctttttttt tcttccagtt tgttggtttt taatggtacc gtgttgtaaa gatacccact 4620
aatggacaat caaattgcag aaaaggctca atatccaaga gacagggact aatgcactgt 4680
acaatctgct tatccttgcc cttctctctt gccaaagtgt gcttcagaaa tatatactgc 4740
tttaaaaaag aataaaagaa tatcctttta caagtggctt tacatttcct aaaatgccat 4800
aagaaaatgc aatatctggg tactgtatgg ggaaaaaaat gtccaagttt gtgtaaaacc 4860

agtgcatttc agcttgcaag ttactgaaca caataatgct gttttaattt tgttttatat 4920
cagttaaaat tcacaataat gtagatagaa caaattacag acaaggaaag aaaaaacttg 4980
aatgaaatgg attttacaga aagctttatg ataatttttg aatgcattat ttattttttg 5040
tgccatgcat tttttttctc accaaatgac cttacctgta atacagtctt gtttgtctgt 5100
ttacaaccat gtatttattg caatgtacat actgtaatgt taattgtaaa ttatctgttc 5160
ttattaaaac atcatcccat gatgggatgg tgttgatata tttggaaact cttggtgaga 5220
gaatgaatgg tgtgtataca tactctgtac atttttcttt tctcctgtaa tatagtcttg 5280
tcaccttaga gcttgtttat ggaagattca agaaaactat aaaatactta aagatatata 5340
aatttaaaaa aacatagctg caggtctttg gtcccagggc tgtgccttaa ctttaaccaa 5400
tattttcttc tgttttgctg catttgaaag gtaacagtgg agctagggct gggcatttta 5460
catccaggct tttaattgat tagaattctg ccaataggtg gattttacaa aaccacagac 5520
aacctctgaa agattctgag accctttga gacagaagct cttaagtact tcttgccagg 5580
gagcagcact gcatgtgtga tggttgtttg ccatctgttg atcaggaact acttcagcta 5640
cttgcatttg attatttcct tttttttttt ttttaactcg gaaacacaac tggggaaata 5700
tattctttcc cagtgattat aaacaatctt tttctttttt ttaagtcctt ttggcttcta 5760
gagctcatag gaaaatggac ttgatttgaa attggagcca gagtttactc gtgttggtta 5820
tctattcatc agcttcctga catgttaaga gaatacatta aagagaaaat actgtttttt 5880
aatcctaaaa tttttcttcc actaagataa accaaatgtc cttacatata tgtaaaccca 5940
tctatttaaa cgcaaaggtg ggttgatgtc agtttacata gcagaaagca ttcactatcc 6000
tctaagattt gtttctgcaa aactttcatt gctttagaat tttaaaattt caccttgtac 6060
aatggccagc ccctaaagca ggaaacattt ataatggatt atatggaaac atcctcccag 6120
tacttgccca gcccttgaat catgtggctt ttcagtgaaa ggaaagattc tttttctagg 6180
aaaaatgagc ctattttatt ttattttatt ttattttttg acacaaactg tagattttag 6240
cagccctggc ccaaaggaat ttgattactt ttgttttaaa cagtacaaag gggacactat 6300
aattacaaaa acatccttaa ctgatttgag ttgtttttat ttctttggat atattttcag 6360
agtggtaaat tgtgtgtgag aattacaaat gattattctt ttagtggttt cttagcctct 6420
cttacagccc acggggatag tactgtacat caataccttc atatgaaatt tttatatgca 6480
atgaaaataa aagcatgggt tgattctgcc tatttatgac tcaatctttt acaaataaaa 6540
gattattcat tttaaattat agttcaatca gcatgtctct taggatactg aacgtggttg 6600
aaatgaaagg atagtgacat cataagttag tactgatatt cataaccaaa taaagccaac 6660
ttgagtaatt ttgctacatt aaaaattacc aaaattactt agatggccta taagattaag 6720

catggtgttt tctaagcaag ctttgaaagg ggccttccat acttacttaa ttgaatattc 6780

tgggatattg aaaattattc agatacttga caattatttt tggttaccta ctccgcaaac 6840

tacaaagttt taaggactca acaataagtt aatgagacac agtgtttgct ttcatggagc 6900

ttacagtctg gaggggacaa aggcttaaac aatactcata taattatata tgtgatcagt 6960

acaatgaagg agctcagtgg ggtaaataag caggaacctg aacttgatct gttccggagg 7020

gccacagaag gcttccttga ggccttgaga aagtgatttg catctgagtt ctgaaggatt 7080

gtaagaggta actagggaaa aagttgacag gaagaggaag gggatccaga caagaaacat 7140

ttgcaaagat cttgaggcat aaatgagctt gagacatctg gagaaactga ggaaaagtga 7200

gagagtaggc agggcctgga gccgcagagc cattgctaac catcctgtgt gagatatccc 7260

ccattctgta gctttattct cataaccctg ctcaattttc tttataacac ttctcacaga 7320

tttatatacg tgtttgtttt tgttatctgt ctctcccacc agaccacagc tccatgagag 7380

caaggtcttt gcttaccaat atatcactag cacttaaaac tatgcctggt acacagtagg 7440

ttcttaatat gtgttgaata tagccatcaa attgatattg gatataattc aatctgataa 7500

gatattttga gatattaaag agtttttaac ttgataccat aaaaa 7545

<210> 18
<211> 8130
<212> DNA
<213> Homo sapiens

<400> 18
agattatagc ccagcgtacg agaagcacga gtcctatagt tggcgtaccc tgaggcctgc 60

cagttcctgc cttaatgcat atgtagtcgt aattgagttc tgacacggcc ttggatgttt 120

ctgtcctaaa tagctgacat tgcatcttca agactgtcat tccagttggc ttttgagtgg 180

atacgtgcag tgagatcatt gacactggaa acactagttc ccattttaat tacttaaaac 240

accacgatga aaagaaatac ctgtgatttg ctttctcgga gcaaaagtgc ctctgaggaa 300

acactacatt ccagtaatga agaggaagac cctttccgcg gaatggaacc ctatcttgtc 360

cggagacttt catgtcgcaa tattcagctt ccccctctcg ccttcagaca gttggaacaa 420

gctgacttga aaagtgaatc agagaacatt caacgaccaa ccagcctccc cctgaagatt 480

ctgccgctga ttgctatcac ttctgcagaa tccagtggtt ttgatgtgga caatggcaca 540

tctgcgggac ggagtccctt ggatcccatg accagcccag gatccgggct aattctccaa 600

gcaaattttg tccacagtca acgacgggag tccttcctgt atcgatccga cagcgattat 660

gacctctctc caaagtctat gtcccggaac tcctccattg ccagtgatat acacggagat 720

gacttgattg tgactccatt tgctcaggtc ttggccagtc tgcgaactgt acgaaacaac 780

tttgctgcat taactaattt gcaagatcga gcacctagca aaagatcacc catgtgcaac 840

caaccatcca tcaacaaagc caccataaca gaggaggcct accagaaact ggccagcgag 900

accctggagg agctggactg gtgtctggac cagctagaga ccctacagac caggcactcc 960

gtcagtgaga tggcctccaa caagtttaaa aggatgctta atcgggagct cacccatctc 1020

tctgaaatga gtcggtctgg aaatcaagtg tcagagttta tatcaaacac attcttagat 1080

aagcaacatg aagtggaaat tccttctcca actcagaagg aaaaggagaa aaagaaaaga 1140

ccaatgtctc agatcagtgg agtcaagaaa ttgatgcaca gctctagtct gactaattca 1200

agtatcccaa ggtttggagt taaaactgaa caagaagatg tccttgccaa ggaactagaa 1260

gatgtgaaca aatggggtct tcatgttttc agaatagcag agttgtctgg taaccggccc 1320

ttgactgtta tcatgcacac catttttcag gaacgggatt tattaaaaac atttaaaatt 1380

ccagtagata ctttaattac atatcttatg actctcgaag accattacca tgctgatgtg 1440

gcctatcaca acaatatcca tgctgcagat gttgtccagt ctactcatgt gctattatct 1500

acacctgctt tggaggctgt gtttacagat ttggagattc ttgcagcaat ttttgccagt 1560

gcaatacatg atgtagatca tcctggtgtg tccaatcaat ttctgatcaa tacaaactct 1620

gaacttgcct tgatgtacaa tgattcctca gtcttagaga accatcattt ggctgtgggc 1680

tttaaattgc ttcaggaaga aaactgtgac attttccaga atttgaccaa aaaacaaaga 1740

caatctttaa ggaaaatggt cattgacatc gtacttgcaa cagatatgtc aaaacacatg 1800

aatctactgg ctgatttgaa gactatggtt gaaactaaga aagtgacaag ctctggagtt 1860

cttcttcttg ataattattc cgataggatt caggttcttc agaatatggt gcactgtgca 1920

gatctgagca acccaacaaa gcctctccag ctgtaccgcc agtggacgga ccggataatg 1980

gaggagttct tccgccaagg agaccgagag agggaacgtg gcatggagat aagccccatg 2040

tgtgacaagc acaatgcttc cgtggaaaaa tcacaggtgg gcttcataga ctatattgtt 2100

catcccctct gggagacatg ggcagacctc gtccaccctg acgcccagga tattttggac 2160

actttggagg acaatcgtga atggtaccag agcacaatcc ctcagagccc ctctcctgca 2220

cctgatgacc cagaggaggg ccggcagggt caaactgaga aattccagtt tgaactaact 2280

ttagaggaag atggtgagtc agacacggaa aaggacagtg gcagtcaagt ggaagaagac 2340

actagctgca gtgactccaa gactctttgt actcaagact cagagtctac tgaaattccc 2400

cttgatgaac aggttgaaga ggaggcagta ggggaagaag aggaaagcca gcctgaagcc 2460

tgtgtcatag atgatcgttc tcctgacacg taacagtgca aaaactttca tgcctttttt 2520

ttttttaagt agaaaaattg tttccaaagt gcatgtcaca tgccacaacc acggtcacac 2580

ctcactgtca tctgccagga cgtttgttga acaaaactga ccttgactac tcagtccagc 2640

gctcaggaat atcgtaacca gttttttcac ctccatgtca tccgagcaag gtggacatct 2700

tcacgaacag cgtttttaac aagatttcag cttggtagag ctgacaaagc agataaaatc 2760

tactccaaat tattttcaag agagtgtgac tcatcaggca gcccaaaagt ttattggact 2820
tggggtttct attccttttt atttgtttgc aatattttca gaagaaaggc attgcacaga 2880
gtgaacttaa tggacgaagc aacaaatatg tcaagaacag gacatagcac gaatctgtta 2940
ccagtaggag gaggatgagc cacagaaatt gcataatttt ctaatttcaa gtcttcctga 3000
tacatgactg aatagtgtgg ttcagtgagc tgcactgacc tctacatttt gtatgatatg 3060
taaaacagat tttttgtaga gcttactttt attattaaat gtattgaggt attatattta 3120
aaaaaaacta tgttcagaac ttcatctgcc actggttatt tttttctaag gagtaacttg 3180
caagttttca gtacaaatct gtgctacact ggataaaaat ctaatttatg aattttactt 3240
gcaccttata gttcatagca attaactgat ttgtagtgat tcattgtttg ttttatatac 3300
caatgacttc catattttaa aagagaaaaa caactttatg ttgcaggaaa ccctttttgt 3360
aagtctttat tatttacttt gcattttgtt tcactctttc cagataagca gagttgctct 3420
tcaccagtgt ttttcttcat gtgcaaagtg actatttgtt ctataatact tttatgtgtg 3480
ttatatcaaa tgtgtcttaa gcttcatgca aactcagtca tcagttcgtg ttgtctgaag 3540
caagtgggag atatataaat acccagtagc taaaatggtc agtctttttt agatgttttc 3600
ctacttagta tctcctaata acgttttgct gtgtcactag atgttcattt cacaagtgca 3660
tgtctttcta ataatccaca catttcatgc tctaataatc cacacatttc atgctcattt 3720
ttattgtttt tacagccagt tatagtaaga aaaaggtttt tccccttgtg ctgctttata 3780
atttagcgtg tgtctgaacc ttatccatgt ttgctagatg aggtcttgtc aaatatatca 3840
ctaccattgt caccggtgaa aagaaacagg tagttaagtt agggttaaca ttcatttcaa 3900
ccacgaggtt gtatatcatg actagctttt actcttggtt tacagagaaa agttaaacag 3960
ccaactaggc agtttttaag aatattaaca atatattaac aaacaccaat acaactaatc 4020
ctatttggtt ttaatgattt caccatggga ttaagaacta tatcaggaac atccctgaga 4080
aacggtttta agtgtagcaa ctactcttcc ttaatggaca gccacataac gtgtaggaag 4140
tcctttatca cttatcctcg atccataagc atatcttgca gaggggaact acttctttaa 4200
acacatggag ggaaagaaga tgatgccact ggcaccagag ggttagtact gtgatgcatc 4260
ctaaaatatt tattatattg gtaaaaattc tggttaaata aaaaattaga gatcactctt 4320
ggctgatttc agcaccagga actgtattac agttttagag attaattcct agtgtttacc 4380
tgattatagc agttggcatc atggggcatt taattctgac tttatcccca cgtcagcctt 4440
aataaagtct tctttacctt ctctatgaag actttaaagc ccaaataatc atttttcaca 4500
ttgatattca agaattgaga tagatagaag ccaaagtggg tatctgacaa gtggaaaatc 4560
aaacgtttaa gaagaattac aactctgaaa agcatttata tgtggaactt ctcaaggagc 4620

ctcctgggga ctggaaagta agtcatcagc caggcaaatg actcatgctg aagagagtcc 4680
ccatttcagt cccctgagat ctagctgatg cttagatcct ttgaaataaa aattatgtct 4740
ttataactct gatcttttac ataaagcaga agaggaatca actagttaat tgcaaggttt 4800
ctactctgtt tcctctgtaa agatcagatg gtaatctttc aaataagaaa aaaataaaga 4860
cgtatgtttg accaagtagt ttcacaagaa tatttgggaa cttgtttctt ttaattttat 4920
ttgtccctga gtgaagtcta gaaagaaagg taaagagtct agagtttatt cctctttcca 4980
aaacattctc attcctctcc tccctacact tagtatttcc cccacagagt gcctagaatc 5040
ttaataatga ataaaataaa aagcagcaat atgtcattaa caaatccaga cctgaaaggg 5100
taaagggttt ataactgcac taataaagag aggctctttt tttttcttcc agtttgttgg 5160
tttttaatgg taccgtgttg taaagatacc cactaatgga caatcaaatt gcagaaaagg 5220
ctcaatatcc aagagacagg gactaatgca ctgtacaatc tgcttatcct tgcccttctc 5280
tcttgccaaa gtgtgcttca gaaatatata ctgctttaaa aaagaataaa agaatatcct 5340
tttacaagtg gctttacatt tcctaaaatg ccataagaaa atgcaatatc tgggtactgt 5400
atggggaaaa aaatgtccaa gtttgtgtaa aaccagtgca tttcagcttg caagttactg 5460
aacacaataa tgctgtttta attttgtttt atatcagtta aaattcacaa taatgtagat 5520
agaacaaatt acagacaagg aaagaaaaaa cttgaatgaa atggatttta cagaaagctt 5580
tatgataatt tttgaatgca ttatttattt tttgtgccat gcattttttt tctcaccaaa 5640
tgaccttacc tgtaatacag tcttgtttgt ctgtttacaa ccatgtattt attgcaatgt 5700
acatactgta atgttaattg taaattatct gttcttatta aaacatcatc ccatgatggg 5760
atggtgttga tatatttgga aactcttggt gagagaatga atggtgtgta tacatactct 5820
gtacattttt cttttctcct gtaatatagt cttgtcacct tagagcttgt ttatggaaga 5880
ttcaagaaaa ctataaaata cttaaagata tataaattta aaaaaacata gctgcaggtc 5940
tttggtccca gggctgtgcc ttaactttaa ccaatatttt cttctgtttt gctgcatttg 6000
aaaggtaaca gtggagctag ggctgggcat tttacatcca ggcttttaat tgattagaat 6060
tctgccaata ggtggatttt acaaaaccac agacaacctc tgaaagattc tgagaccctt 6120
ttgagacaga agctcttaag tacttcttgc cagggagcag cactgcatgt gtgatggttg 6180
tttgccatct gttgatcagg aactacttca gctacttgca tttgattatt tccttttttt 6240
ttttttttaa ctcggaaaca caactgggga aatatattct ttcccagtga ttataaacaa 6300
tctttttctt ttttttaagt ccttttggct tctagagctc ataggaaaat ggacttgatt 6360
tgaaattgga gccagagttt actcgtgttg gttatctatt catcagcttc ctgacatgtt 6420
aagagaatac attaaagaga aaatactgtt ttttaatcct aaaatttttc ttccactaag 6480
ataaaccaaa tgtccttaca tatatgtaaa cccatctatt taaacgcaaa ggtgggttga 6540

tgtcagttta catagcagaa agcattcact atcctctaag atttgtttct gcaaaacttt 6600

cattgcttta gaattttaaa atttcacctt gtacaatggc cagcccctaa agcaggaaac 6660

atttataatg gattatatgg aaacatcctc ccagtacttg cccagccctt gaatcatgtg 6720

gcttttcagt gaaaggaaag attctttttc taggaaaaat gagcctattt tattttattt 6780

tattttattt tttgacacaa actgtagatt ttagcagccc tggcccaaag gaatttgatt 6840

acttttgttt taaacagtac aaaggggaca ctataattac aaaaacatcc ttaactgatt 6900

tgagttgttt ttatttcttt ggatatattt tcagagtggt aaattgtgtg tgagaattac 6960

aaatgattat tcttttagtg gtttcttagc ctctcttaca gcccacgggg atagtactgt 7020

acatcaatac cttcatatga aatttttata tgcaatgaaa ataaaagcat gggttgattc 7080

tgcctattta tgactcaatc ttttacaaat aaaagattat tcattttaaa ttatagttca 7140

atcagcatgt ctcttaggat actgaacgtg gttgaaatga aaggatagtg acatcataag 7200

ttagtactga tattcataac caaataaagc caacttgagt aattttgcta cattaaaaat 7260

taccaaaatt acttagatgg cctataagat taagcatggt gttttctaag caagctttga 7320

aaggggcctt ccatacttac ttaattgaat attctgggat attgaaaatt attcagatac 7380

ttgacaatta tttttggtta cctactccgc aaactacaaa gttttaagga ctcaacaata 7440

agttaatgag acacagtgtt tgctttcatg gagcttacag tctggagggg acaaaggctt 7500

aaacaatact catataatta tatatgtgat cagtacaatg aaggagctca gtggggtaaa 7560

taagcaggaa cctgaacttg atctgttccg gagggccaca gaaggcttcc ttgaggcctt 7620

gagaaagtga tttgcatctg agttctgaag gattgtaaga ggtaactagg gaaaaagttg 7680

acaggaagag gaaggggatc cagacaagaa acatttgcaa agatcttgag gcataaatga 7740

gcttgagaca tctggagaaa ctgaggaaaa gtgagagagt aggcagggcc tggagccgca 7800

gagccattgc taaccatcct gtgtgagata tcccccattc tgtagcttta ttctcataac 7860

cctgctcaat tttctttata acacttctca cagatttata tacgtgtttg tttttgttat 7920

ctgtctctcc caccagacca cagctccatg agagcaaggt ctttgcttac caatatatca 7980

ctagcactta aaactatgcc tggtacacag taggttctta atatgtgttg aatatagcca 8040

tcaaattgat attggatata attcaatctg ataagatatt ttgagatatt aaagagtttt 8100

taacttgata ccataaaaaa aaaaaaaaaa 8130

<210> 19
<211> 7814
<212> DNA
<213> Homo sapiens

<400> 19
acaaaggaaa tgccctcact cagctacaag tgcctcctgc tccgtgcggg gcctcagggc 60

cccagagccc cgcaccagct ctgacttctc gtggttcctg gggatcttgg catcgtcctt 120
aaaaatggct tttgtttggg atcctctggg agccacggtg ccaggaccat ctacaagagc 180
caaatcaaga ttgcgtttct caaagtccta cagttttgat gtggacaatg gcacatctgc 240
gggacggagt cccttggatc ccatgaccag cccaggatcc gggctaattc tccaagcaaa 300
ttttgtccac agtcaacgac gggagtcctt cctgtatcga tccgacagcg attatgacct 360
ctctccaaag tctatgtccc ggaactcctc cattgccagt gatatacacg gagatgactt 420
gattgtgact ccatttgctc aggtcttggc cagtctgcga actgtacgaa acaactttgc 480
tgcattaact aatttgcaag atcgagcacc tagcaaaaga tcacccatgt gcaaccaacc 540
atccatcaac aaagccacca taacagagga ggcctaccag aaactggcca gcgagaccct 600
ggaggagctg gactggtgtc tggaccagct agagacccta cagaccaggc actccgtcag 660
tgagatggcc tccaacaagt ttaaaaggat gcttaatcgg gagctcaccc atctctctga 720
aatgagtcgg tctggaaatc aagtgtcaga gtttatatca aacacattct tagataagca 780
acatgaagtg gaaattcctt ctccaactca gaaggaaaag gagaaaaaga aaagaccaat 840
gtctcagatc agtggagtca agaaattgat gcacagctct agtctgacta attcaagtat 900
cccaaggttt ggagttaaaa ctgaacaaga agatgtcctt gccaaggaac tagaagatgt 960
gaacaaatgg ggtcttcatg ttttcagaat agcagagttg tctggtaacc ggcccttgac 1020
tgttatcatg cacaccattt ttcaggaacg ggatttatta aaaacattta aaattccagt 1080
agatacttta attacatatc ttatgactct cgaagaccat taccatgctg atgtggccta 1140
tcacaacaat atccatgctg cagatgttgt ccagtctact catgtgctat tatctacacc 1200
tgctttggag gctgtgttta cagatttgga gattcttgca gcaatttttg ccagtgcaat 1260
acatgatgta gatcatcctg gtgtgtccaa tcaatttctg atcaatacaa actctgaact 1320
tgccttgatg tacaatgatt cctcagtctt agagaaccat catttggctg tgggctttaa 1380
attgcttcag gaagaaaact gtgacatttt ccagaatttg accaaaaaac aaagacaatc 1440
tttaaggaaa atggtcattg acatcgtact tgcaacagat atgtcaaaac acatgaatct 1500
actggctgat ttgaagacta tggttgaaac taagaaagtg acaagctctg gagttcttct 1560
tcttgataat tattccgata ggattcaggt tcttcagaat atggtgcact gtgcagatct 1620
gagcaaccca acaaagcctc tccagctgta ccgccagtgg acggaccgga taatggagga 1680
gttcttccgc caaggagacc gagagaggga acgtggcatg gagataagcc ccatgtgtga 1740
caagcacaat gcttccgtgg aaaaatcaca ggtgggcttc atagactata ttgttcatcc 1800
cctctgggag acatgggcag acctcgtcca ccctgacgcc caggatattt tggacacttt 1860
ggaggacaat cgtgaatggt accagagcac aatccctcag agcccctctc ctgcacctga 1920

tgacccagag gagggccggc agggtcaaac tgagaaattc cagtttgaac taactttaga 1980
ggaagatggt gagtcagaca cggaaaagga cagtggcagt caagtggaag aagacactag 2040
ctgcagtgac tccaagactc tttgtactca agactcagag tctactgaaa ttccccttga 2100
tgaacaggtt gaagaggagg cagtagggga agaagaggaa agccagcctg aagcctgtgt 2160
catagatgat cgttctcctg acacgtaaca gtgcaaaaac tttcatgcct tttttttttt 2220
taagtagaaa aattgtttcc aaagtgcatg tcacatgcca caaccacggt cacacctcac 2280
tgtcatctgc caggacgttt gttgaacaaa actgaccttg actactcagt ccagcgctca 2340
ggaatatcgt aaccagtttt ttcacctcca tgtcatccga gcaaggtgga catcttcacg 2400
aacagcgttt ttaacaagat ttcagcttgg tagagctgac aaagcagata aaatctactc 2460
caaattattt tcaagagagt gtgactcatc aggcagccca aaagtttatt ggacttgggg 2520
tttctattcc tttttatttg tttgcaatat tttcagaaga aaggcattgc acagagtgaa 2580
cttaatggac gaagcaacaa atatgtcaag aacaggacat agcacgaatc tgttaccagt 2640
aggaggagga tgagccacag aaattgcata attttctaat ttcaagtctt cctgatacat 2700
gactgaatag tgtggttcag tgagctgcac tgacctctac attttgtatg atatgtaaaa 2760
cagatttttt gtagagctta cttttattat taaatgtatt gaggtattat atttaaaaaa 2820
aactatgttc agaacttcat ctgccactgg ttattttttt ctaaggagta acttgcaagt 2880
tttcagtaca aatctgtgct acactggata aaaatctaat ttatgaattt tacttgcacc 2940
ttatagttca tagcaattaa ctgatttgta gtgattcatt gtttgtttta tataccaatg 3000
acttccatat tttaaaagag aaaaacaact ttatgttgca ggaaaccctt tttgtaagtc 3060
tttattattt actttgcatt ttgtttcact ctttccagat aagcagagtt gctcttcacc 3120
agtgtttttc ttcatgtgca aagtgactat ttgttctata atacttttat gtgtgttata 3180
tcaaatgtgt cttaagcttc atgcaaactc agtcatcagt tcgtgttgtc tgaagcaagt 3240
gggagatata taaataccca gtagctaaaa tggtcagtct tttttagatg ttttcctact 3300
tagtatctcc taataacgtt ttgctgtgtc actagatgtt catttcacaa gtgcatgtct 3360
ttctaataat ccacacattt catgctctaa taatccacac atttcatgct catttttatt 3420
gtttttacag ccagttatag taagaaaaag gtttttcccc ttgtgctgct ttataattta 3480
gcgtgtgtct gaaccttatc catgtttgct agatgaggtc ttgtcaaata tatcactacc 3540
attgtcaccg gtgaaaagaa acaggtagtt aagttagggt taacattcat ttcaaccacg 3600
aggttgtata tcatgactag cttttactct tggtttacag agaaaagtta aacagccaac 3660
taggcagttt ttaagaatat taacaatata ttaacaaaca ccaatacaac taatcctatt 3720
tggttttaat gatttcacca tgggattaag aactatatca ggaacatccc tgagaaacgg 3780
ttttaagtgt agcaactact cttccttaat ggacagccac ataacgtgta ggaagtcctt 3840

tatcacttat cctcgatcca taagcatatc ttgcagaggg gaactacttc tttaaacaca 3900
tggagggaaa gaagatgatg ccactggcac cagagggtta gtactgtgat gcatcctaaa 3960
atatttatta tattggtaaa aattctggtt aaataaaaaa ttagagatca ctcttggctg 4020
atttcagcac caggaactgt attacagttt tagagattaa ttcctagtgt ttacctgatt 4080
atagcagttg gcatcatggg gcatttaatt ctgactttat ccccacgtca gccttaataa 4140
agtcttcttt accttctcta tgaagacttt aaagcccaaa taatcatttt tcacattgat 4200
attcaagaat tgagatagat agaagccaaa gtgggtatct gacaagtgga aaatcaaacg 4260
tttaagaaga attacaactc tgaaaagcat ttatatgtgg aacttctcaa ggagcctcct 4320
ggggactgga aagtaagtca tcagccaggc aaatgactca tgctgaagag agtccccatt 4380
tcagtcccct gagatctagc tgatgcttag atcctttgaa ataaaaatta tgtctttata 4440
actctgatct tttacataaa gcagaagagg aatcaactag ttaattgcaa ggtttctact 4500
ctgtttcctc tgtaaagatc agatggtaat ctttcaaata agaaaaaaat aaagacgtat 4560
gtttgaccaa gtagtttcac aagaatattt gggaacttgt ttcttttaat tttatttgtc 4620
cctgagtgaa gtctagaaag aaaggtaaag agtctagagt ttattcctct ttccaaaaca 4680
ttctcattcc tctcctccct acacttagta tttcccccac agagtgccta gaatcttaat 4740
aatgaataaa ataaaaagca gcaatatgtc attaacaaat ccagacctga aagggtaaag 4800
ggtttataac tgcactaata aagagaggct cttttttttt cttccagttt gttggttttt 4860
aatggtaccg tgttgtaaag atacccacta atggacaatc aaattgcaga aaaggctcaa 4920
tatccaagag acagggacta atgcactgta caatctgctt atccttgccc ttctctcttg 4980
ccaaagtgtg cttcagaaat atatactgct ttaaaaaaga ataaaagaat atccttttac 5040
aagtggcttt acatttccta aaatgccata agaaaatgca atatctgggt actgtatggg 5100
gaaaaaaatg tccaagtttg tgtaaaacca gtgcatttca gcttgcaagt tactgaacac 5160
aataatgctg ttttaatttt gttttatatc agttaaaatt cacaataatg tagatagaac 5220
aaattacaga caaggaaaga aaaaacttga atgaaatgga ttttacagaa agctttatga 5280
taatttttga atgcattatt tattttttgt gccatgcatt ttttttctca ccaaatgacc 5340
ttacctgtaa tacagtcttg tttgtctgtt tacaaccatg tatttattgc aatgtacata 5400
ctgtaatgtt aattgtaaat tatctgttct tattaaaaca tcatcccatg atgggatggt 5460
gttgatatat ttggaaactc ttggtgagag aatgaatggt gtgtatacat actctgtaca 5520
tttttctttt ctcctgtaat atagtcttgt caccttagag cttgtttatg gaagattcaa 5580
gaaaactata aaatacttaa agatatataa atttaaaaaa acatagctgc aggtctttgg 5640
tcccagggct gtgccttaac tttaaccaat attttcttct gttttgctgc atttgaaagg 5700

taacagtgga gctagggctg ggcattttac atccaggctt ttaattgatt agaattctgc 5760
caataggtgg attttacaaa accacagaca acctctgaaa gattctgaga ccctttttgag 5820
acagaagctc ttaagtactt cttgccaggg agcagcactg catgtgtgat ggttgtttgc 5880
catctgttga tcaggaacta cttcagctac ttgcatttga ttatttcctt tttttttttt 5940
tttaactcgg aaacacaact ggggaaatat attctttccc agtgattata aacaatcttt 6000
ttcttttttt taagtccttt tggcttctag agctcatagg aaaatggact tgatttgaaa 6060
ttggagccag agtttactcg tgttggttat ctattcatca gcttcctgac atgttaagag 6120
aatacattaa agagaaaata ctgtttttta atcctaaaat tttcttccca ctaagataaa 6180
ccaaatgtcc ttacatatat gtaaacccat ctatttaaac gcaaaggtgg gttgatgtca 6240
gtttacatag cagaaagcat tcactatcct ctaagatttg tttctgcaaa actttcattg 6300
ctttagaatt ttaaaatttc accttgtaca atggccagcc cctaaagcag gaaacattta 6360
taatggatta tatggaaaca tcctcccagt acttgcccag cccttgaatc atgtggcttt 6420
tcagtgaaag gaaagattct ttttctagga aaaatgagcc tattttattt tattttattt 6480
tattttttga cacaaactgt agattttagc agccctggcc caaaggaatt tgattacttt 6540
tgttttaaac agtacaaagg ggacactata attacaaaaa catccttaac tgatttgagt 6600
tgtttttatt tctttggata tattttcaga gtggtaaatt gtgtgtgaga attacaaatg 6660
attattcttt tagtggtttc ttagcctctc ttacagccca cggggatagt actgtacatc 6720
aataccttca tatgaaattt ttatatgcaa tgaaaataaa agcatgggtt gattctgcct 6780
atttatgact caatctttta caaataaaag attattcatt ttaaattata gttcaatcag 6840
catgtctctt aggatactga acgtggttga aatgaaagga tagtgacatc ataagttagt 6900
actgatattc ataaccaaat aaagccaact tgagtaattt tgctacatta aaaattacca 6960
aaattactta gatggcctat aagattaagc atggtgtttt ctaagcaagc tttgaaaggg 7020
gccttccata cttacttaat tgaatattct gggatattga aaattattca gatacttgac 7080
aattattttt ggttacctac tccgcaaact acaaagtttt aaggactcaa caataagtta 7140
atgagacaca gtgtttgctt tcatggagct tacagtctgg aggggacaaa ggcttaaaca 7200
atactcatat aattatatat gtgatcagta caatgaagga gctcagtggg gtaaataagc 7260
aggaacctga acttgatctg ttccggaggg ccacagaagg cttccttgag gccttgagaa 7320
agtgatttgc atctgagttc tgaaggattg taagaggtaa ctagggaaaa agttgacagg 7380
aagaggaagg ggatccagac aagaaacatt tgcaaagatc ttgaggcata aatgagcttg 7440
agacatctgg agaaactgag gaaaagtgag agagtaggca gggcctggag ccgcagagcc 7500
attgctaacc atcctgtgtg agatatcccc cattctgtag ctttattctc ataaccctgc 7560
tcaattttct ttataacact tctcacagat ttatatacgt gtttgttttt gttatctgtc 7620

tctcccacca gaccacagct ccatgagagc aaggtctttg cttaccaata tatcactagc 7680

acttaaaact atgcctggta cacagtaggt tcttaatatg tgttgaatat agccatcaaa 7740

ttgatattgg atataattca atctgataag atattttgag atattaaaga gtttttaact 7800

tgataccata aaaa 7814

<210> 20
<211> 809
<212> PRT
<213> Homo sapiens

<400> 20

Met Glu Ala Glu Gly Ser Ser Ala Pro Ala Arg Ala Gly Ser Gly Glu
1               5                   10                  15

Gly Ser Asp Ser Ala Gly Gly Ala Thr Leu Lys Ala Pro Lys His Leu
            20                  25                  30

Trp Arg His Glu Gln His His Gln Tyr Pro Leu Arg Gln Pro Gln Phe
        35                  40                  45

Arg Leu Leu His Pro His His His Leu Pro Pro Pro Pro Pro Pro Ser
    50                  55                  60

Pro Gln Pro Gln Pro Gln Cys Pro Leu Gln Pro Pro Pro Pro Pro Pro
65                  70                  75                  80

Leu Pro Pro Pro Pro Pro Pro Pro Gly Ala Ala Arg Gly Arg Tyr Ala
                85                  90                  95

Ser Ser Gly Ala Thr Gly Arg Val Arg His Arg Gly Tyr Ser Asp Thr
            100                 105                 110

Glu Arg Tyr Leu Tyr Cys Arg Ala Met Asp Arg Thr Ser Tyr Ala Val
        115                 120                 125

Glu Thr Gly His Arg Pro Gly Leu Lys Lys Ser Arg Met Ser Trp Pro
    130                 135                 140

Ser Ser Phe Gln Gly Leu Arg Arg Phe Asp Val Asp Asn Gly Thr Ser
145                 150                 155                 160

Ala Gly Arg Ser Pro Leu Asp Pro Met Thr Ser Pro Gly Ser Gly Leu
                165                 170                 175

Ile Leu Gln Ala Asn Phe Val His Ser Gln Arg Arg Glu Ser Phe Leu
            180                 185                 190

```
Tyr Arg Ser Asp Ser Asp Tyr Asp Leu Ser Pro Lys Ser Met Ser Arg
        195                 200                 205

Asn Ser Ser Ile Ala Ser Asp Ile His Gly Asp Asp Leu Ile Val Thr
    210                 215                 220

Pro Phe Ala Gln Val Leu Ala Ser Leu Arg Thr Val Arg Asn Asn Phe
225                 230                 235                 240

Ala Ala Leu Thr Asn Leu Gln Asp Arg Ala Pro Ser Lys Arg Ser Pro
                245                 250                 255

Met Cys Asn Gln Pro Ser Ile Asn Lys Ala Thr Ile Thr Glu Glu Ala
            260                 265                 270

Tyr Gln Lys Leu Ala Ser Glu Thr Leu Glu Glu Leu Asp Trp Cys Leu
        275                 280                 285

Asp Gln Leu Glu Thr Leu Gln Thr Arg His Ser Val Ser Glu Met Ala
    290                 295                 300

Ser Asn Lys Phe Lys Arg Met Leu Asn Arg Glu Leu Thr His Leu Ser
305                 310                 315                 320

Glu Met Ser Arg Ser Gly Asn Gln Val Ser Glu Phe Ile Ser Asn Thr
                325                 330                 335

Phe Leu Asp Lys Gln His Glu Val Glu Ile Pro Ser Pro Thr Gln Lys
            340                 345                 350

Glu Lys Glu Lys Lys Lys Arg Pro Met Ser Gln Ile Ser Gly Val Lys
        355                 360                 365

Lys Leu Met His Ser Ser Ser Leu Thr Asn Ser Ser Ile Pro Arg Phe
    370                 375                 380

Gly Val Lys Thr Glu Gln Glu Asp Val Leu Ala Lys Glu Leu Glu Asp
385                 390                 395                 400

Val Asn Lys Trp Gly Leu His Val Phe Arg Ile Ala Glu Leu Ser Gly
                405                 410                 415

Asn Arg Pro Leu Thr Val Ile Met His Thr Ile Phe Gln Glu Arg Asp
            420                 425                 430

Leu Leu Lys Thr Phe Lys Ile Pro Val Asp Thr Leu Ile Thr Tyr Leu
```

435 440 445

Met Thr Leu Glu Asp His Tyr His Ala Asp Val Ala Tyr His Asn Asn
450 455 460

Ile His Ala Ala Asp Val Val Gln Ser Thr His Val Leu Leu Ser Thr
465 470 475 480

Pro Ala Leu Glu Ala Val Phe Thr Asp Leu Glu Ile Leu Ala Ala Ile
485 490 495

Phe Ala Ser Ala Ile His Asp Val Asp His Pro Gly Val Ser Asn Gln
500 505 510

Phe Leu Ile Asn Thr Asn Ser Glu Leu Ala Leu Met Tyr Asn Asp Ser
515 520 525

Ser Val Leu Glu Asn His His Leu Ala Val Gly Phe Lys Leu Leu Gln
530 535 540

Glu Glu Asn Cys Asp Ile Phe Gln Asn Leu Thr Lys Lys Gln Arg Gln
545 550 555 560

Ser Leu Arg Lys Met Val Ile Asp Ile Val Leu Ala Thr Asp Met Ser
565 570 575

Lys His Met Asn Leu Leu Ala Asp Leu Lys Thr Met Val Glu Thr Lys
580 585 590

Lys Val Thr Ser Ser Gly Val Leu Leu Leu Asp Asn Tyr Ser Asp Arg
595 600 605

Ile Gln Val Leu Gln Asn Met Val His Cys Ala Asp Leu Ser Asn Pro
610 615 620

Thr Lys Pro Leu Gln Leu Tyr Arg Gln Trp Thr Asp Arg Ile Met Glu
625 630 635 640

Glu Phe Phe Arg Gln Gly Asp Arg Glu Arg Glu Arg Gly Met Glu Ile
645 650 655

Ser Pro Met Cys Asp Lys His Asn Ala Ser Val Glu Lys Ser Gln Val
660 665 670

Gly Phe Ile Asp Tyr Ile Val His Pro Leu Trp Glu Thr Trp Ala Asp
675 680 685

```
Leu Val His Pro Asp Ala Gln Asp Ile Leu Asp Thr Leu Glu Asp Asn
    690                 695                 700

Arg Glu Trp Tyr Gln Ser Thr Ile Pro Gln Ser Pro Ser Pro Ala Pro
705                 710                 715                 720

Asp Asp Pro Glu Glu Gly Arg Gln Gly Gln Thr Glu Lys Phe Gln Phe
                725                 730                 735

Glu Leu Thr Leu Glu Glu Asp Gly Glu Ser Asp Thr Glu Lys Asp Ser
            740                 745                 750

Gly Ser Gln Val Glu Glu Asp Thr Ser Cys Ser Asp Ser Lys Thr Leu
        755                 760                 765

Cys Thr Gln Asp Ser Glu Ser Thr Glu Ile Pro Leu Asp Glu Gln Val
    770                 775                 780

Glu Glu Glu Ala Val Gly Glu Glu Glu Glu Ser Gln Pro Glu Ala Cys
785                 790                 795                 800

Val Ile Asp Asp Arg Ser Pro Asp Thr
                805


<210>  21
<211>  699
<212>  PRT
<213>  Homo sapiens

<400>  21

Met Ser Leu Pro Ser Ser Cys Glu Tyr Leu Thr Leu Gly Lys Val Ala
1               5                   10                  15

Arg Phe Arg Ser Ala Tyr Val His Gly Ser Pro Tyr Ala Ile Asn Met
            20                  25                  30

Pro Ile Asp Ile Lys Pro Gln Arg Arg Arg Phe Asp Val Asp Asn Gly
        35                  40                  45

Thr Ser Ala Gly Arg Ser Pro Leu Asp Pro Met Thr Ser Pro Gly Ser
    50                  55                  60

Gly Leu Ile Leu Gln Ala Asn Phe Val His Ser Gln Arg Arg Glu Ser
65                  70                  75                  80

Phe Leu Tyr Arg Ser Asp Ser Asp Tyr Asp Leu Ser Pro Lys Ser Met
                85                  90                  95
```

```
Ser Arg Asn Ser Ser Ile Ala Ser Asp Ile His Gly Asp Asp Leu Ile
            100                 105                 110

Val Thr Pro Phe Ala Gln Val Leu Ala Ser Leu Arg Thr Val Arg Asn
        115                 120                 125

Asn Phe Ala Ala Leu Thr Asn Leu Gln Asp Arg Ala Pro Ser Lys Arg
    130                 135                 140

Ser Pro Met Cys Asn Gln Pro Ser Ile Asn Lys Ala Thr Ile Thr Glu
145                 150                 155                 160

Glu Ala Tyr Gln Lys Leu Ala Ser Glu Thr Leu Glu Glu Leu Asp Trp
                165                 170                 175

Cys Leu Asp Gln Leu Glu Thr Leu Gln Thr Arg His Ser Val Ser Glu
            180                 185                 190

Met Ala Ser Asn Lys Phe Lys Arg Met Leu Asn Arg Glu Leu Thr His
        195                 200                 205

Leu Ser Glu Met Ser Arg Ser Gly Asn Gln Val Ser Glu Phe Ile Ser
    210                 215                 220

Asn Thr Phe Leu Asp Lys Gln His Glu Val Glu Ile Pro Ser Pro Thr
225                 230                 235                 240

Gln Lys Glu Lys Glu Lys Lys Lys Arg Pro Met Ser Gln Ile Ser Gly
                245                 250                 255

Val Lys Lys Leu Met His Ser Ser Ser Leu Thr Asn Ser Ser Ile Pro
            260                 265                 270

Arg Phe Gly Val Lys Thr Glu Gln Glu Asp Val Leu Ala Lys Glu Leu
        275                 280                 285

Glu Asp Val Asn Lys Trp Gly Leu His Val Phe Arg Ile Ala Glu Leu
    290                 295                 300

Ser Gly Asn Arg Pro Leu Thr Val Ile Met His Thr Ile Phe Gln Glu
305                 310                 315                 320

Arg Asp Leu Leu Lys Thr Phe Lys Ile Pro Val Asp Thr Leu Ile Thr
                325                 330                 335

Tyr Leu Met Thr Leu Glu Asp His Tyr His Ala Asp Val Ala Tyr His
            340                 345                 350
```

Asn Asn Ile His Ala Ala Asp Val Val Gln Ser Thr His Val Leu Leu
355 360 365

Ser Thr Pro Ala Leu Glu Ala Val Phe Thr Asp Leu Glu Ile Leu Ala
370 375 380

Ala Ile Phe Ala Ser Ala Ile His Asp Val Asp His Pro Gly Val Ser
385 390 395 400

Asn Gln Phe Leu Ile Asn Thr Asn Ser Glu Leu Ala Leu Met Tyr Asn
405 410 415

Asp Ser Ser Val Leu Glu Asn His His Leu Ala Val Gly Phe Lys Leu
420 425 430

Leu Gln Glu Glu Asn Cys Asp Ile Phe Gln Asn Leu Thr Lys Lys Gln
435 440 445

Arg Gln Ser Leu Arg Lys Met Val Ile Asp Ile Val Leu Ala Thr Asp
450 455 460

Met Ser Lys His Met Asn Leu Leu Ala Asp Leu Lys Thr Met Val Glu
465 470 475 480

Thr Lys Lys Val Thr Ser Ser Gly Val Leu Leu Leu Asp Asn Tyr Ser
485 490 495

Asp Arg Ile Gln Val Leu Gln Asn Met Val His Cys Ala Asp Leu Ser
500 505 510

Asn Pro Thr Lys Pro Leu Gln Leu Tyr Arg Gln Trp Thr Asp Arg Ile
515 520 525

Met Glu Glu Phe Phe Arg Gln Gly Asp Arg Glu Arg Glu Arg Gly Met
530 535 540

Glu Ile Ser Pro Met Cys Asp Lys His Asn Ala Ser Val Glu Lys Ser
545 550 555 560

Gln Val Gly Phe Ile Asp Tyr Ile Val His Pro Leu Trp Glu Thr Trp
565 570 575

Ala Asp Leu Val His Pro Asp Ala Gln Asp Ile Leu Asp Thr Leu Glu
580 585 590

Asp Asn Arg Glu Trp Tyr Gln Ser Thr Ile Pro Gln Ser Pro Ser Pro
595 600 605

```
Ala Pro Asp Asp Pro Glu Glu Gly Arg Gln Gly Gln Thr Glu Lys Phe
    610                 615                 620

Gln Phe Glu Leu Thr Leu Glu Glu Asp Gly Glu Ser Asp Thr Glu Lys
625                 630                 635                 640

Asp Ser Gly Ser Gln Val Glu Glu Asp Thr Ser Cys Ser Asp Ser Lys
                645                 650                 655

Thr Leu Cys Thr Gln Asp Ser Glu Ser Thr Glu Ile Pro Leu Asp Glu
            660                 665                 670

Gln Val Glu Glu Glu Ala Val Gly Glu Glu Glu Glu Ser Gln Pro Glu
        675                 680                 685

Ala Cys Val Ile Asp Asp Arg Ser Pro Asp Thr
    690                 695


<210>  22
<211>  745
<212>  PRT
<213>  Homo sapiens

<400>  22

Met Ala Gln Gln Thr Ser Pro Asp Thr Leu Thr Val Pro Glu Val Asp
1               5                   10                  15

Asn Pro His Cys Pro Asn Pro Trp Leu Asn Glu Asp Leu Val Lys Ser
            20                  25                  30

Leu Arg Glu Asn Leu Leu Gln His Glu Lys Ser Lys Thr Ala Arg Lys
        35                  40                  45

Ser Val Ser Pro Lys Leu Ser Pro Val Ile Ser Pro Arg Asn Ser Pro
    50                  55                  60

Arg Leu Leu Arg Arg Met Leu Leu Ser Ser Asn Ile Pro Lys Gln Arg
65                  70                  75                  80

Arg Phe Thr Val Ala His Thr Cys Phe Asp Val Asp Asn Gly Thr Ser
                85                  90                  95

Ala Gly Arg Ser Pro Leu Asp Pro Met Thr Ser Pro Gly Ser Gly Leu
            100                 105                 110

Ile Leu Gln Ala Asn Phe Val His Ser Gln Arg Arg Glu Ser Phe Leu
        115                 120                 125
```

Tyr Arg Ser Asp Ser Asp Tyr Asp Leu Ser Pro Lys Ser Met Ser Arg
130 135 140

Asn Ser Ser Ile Ala Ser Asp Ile His Gly Asp Asp Leu Ile Val Thr
145 150 155 160

Pro Phe Ala Gln Val Leu Ala Ser Leu Arg Thr Val Arg Asn Asn Phe
165 170 175

Ala Ala Leu Thr Asn Leu Gln Asp Arg Ala Pro Ser Lys Arg Ser Pro
180 185 190

Met Cys Asn Gln Pro Ser Ile Asn Lys Ala Thr Ile Thr Glu Glu Ala
195 200 205

Tyr Gln Lys Leu Ala Ser Glu Thr Leu Glu Glu Leu Asp Trp Cys Leu
210 215 220

Asp Gln Leu Glu Thr Leu Gln Thr Arg His Ser Val Ser Glu Met Ala
225 230 235 240

Ser Asn Lys Phe Lys Arg Met Leu Asn Arg Glu Leu Thr His Leu Ser
245 250 255

Glu Met Ser Arg Ser Gly Asn Gln Val Ser Glu Phe Ile Ser Asn Thr
260 265 270

Phe Leu Asp Lys Gln His Glu Val Glu Ile Pro Ser Pro Thr Gln Lys
275 280 285

Glu Lys Glu Lys Lys Lys Arg Pro Met Ser Gln Ile Ser Gly Val Lys
290 295 300

Lys Leu Met His Ser Ser Ser Leu Thr Asn Ser Ser Ile Pro Arg Phe
305 310 315 320

Gly Val Lys Thr Glu Gln Glu Asp Val Leu Ala Lys Glu Leu Glu Asp
325 330 335

Val Asn Lys Trp Gly Leu His Val Phe Arg Ile Ala Glu Leu Ser Gly
340 345 350

Asn Arg Pro Leu Thr Val Ile Met His Thr Ile Phe Gln Glu Arg Asp
355 360 365

Leu Leu Lys Thr Phe Lys Ile Pro Val Asp Thr Leu Ile Thr Tyr Leu

```
    370                 375                 380

Met Thr Leu Glu Asp His Tyr His Ala Asp Val Ala Tyr His Asn Asn
385                 390                 395                 400

Ile His Ala Ala Asp Val Val Gln Ser Thr His Val Leu Leu Ser Thr
                405                 410                 415

Pro Ala Leu Glu Ala Val Phe Thr Asp Leu Glu Ile Leu Ala Ala Ile
            420                 425                 430

Phe Ala Ser Ala Ile His Asp Val Asp His Pro Gly Val Ser Asn Gln
        435                 440                 445

Phe Leu Ile Asn Thr Asn Ser Glu Leu Ala Leu Met Tyr Asn Asp Ser
    450                 455                 460

Ser Val Leu Glu Asn His His Leu Ala Val Gly Phe Lys Leu Leu Gln
465                 470                 475                 480

Glu Glu Asn Cys Asp Ile Phe Gln Asn Leu Thr Lys Lys Gln Arg Gln
                485                 490                 495

Ser Leu Arg Lys Met Val Ile Asp Ile Val Leu Ala Thr Asp Met Ser
            500                 505                 510

Lys His Met Asn Leu Leu Ala Asp Leu Lys Thr Met Val Glu Thr Lys
        515                 520                 525

Lys Val Thr Ser Ser Gly Val Leu Leu Leu Asp Asn Tyr Ser Asp Arg
    530                 535                 540

Ile Gln Val Leu Gln Asn Met Val His Cys Ala Asp Leu Ser Asn Pro
545                 550                 555                 560

Thr Lys Pro Leu Gln Leu Tyr Arg Gln Trp Thr Asp Arg Ile Met Glu
                565                 570                 575

Glu Phe Phe Arg Gln Gly Asp Arg Glu Arg Glu Arg Gly Met Glu Ile
            580                 585                 590

Ser Pro Met Cys Asp Lys His Asn Ala Ser Val Glu Lys Ser Gln Val
        595                 600                 605

Gly Phe Ile Asp Tyr Ile Val His Pro Leu Trp Glu Thr Trp Ala Asp
    610                 615                 620
```

Leu Val His Pro Asp Ala Gln Asp Ile Leu Asp Thr Leu Glu Asp Asn
625 630 635 640

Arg Glu Trp Tyr Gln Ser Thr Ile Pro Gln Ser Pro Ser Pro Ala Pro
645 650 655

Asp Asp Pro Glu Glu Gly Arg Gln Gly Gln Thr Glu Lys Phe Gln Phe
660 665 670

Glu Leu Thr Leu Glu Glu Asp Gly Glu Ser Asp Thr Glu Lys Asp Ser
675 680 685

Gly Ser Gln Val Glu Glu Asp Thr Ser Cys Ser Asp Ser Lys Thr Leu
690 695 700

Cys Thr Gln Asp Ser Glu Ser Thr Glu Ile Pro Leu Asp Glu Gln Val
705 710 715 720

Glu Glu Glu Ala Val Gly Glu Glu Glu Glu Ser Gln Pro Glu Ala Cys
725 730 735

Val Ile Asp Asp Arg Ser Pro Asp Thr
740 745

<210> 23
<211> 673
<212> PRT
<213> Homo sapiens

<400> 23

Met Met His Val Asn Asn Phe Pro Phe Arg Arg His Ser Trp Ile Cys
1 5 10 15

Phe Asp Val Asp Asn Gly Thr Ser Ala Gly Arg Ser Pro Leu Asp Pro
20 25 30

Met Thr Ser Pro Gly Ser Gly Leu Ile Leu Gln Ala Asn Phe Val His
35 40 45

Ser Gln Arg Arg Glu Ser Phe Leu Tyr Arg Ser Asp Ser Asp Tyr Asp
50 55 60

Leu Ser Pro Lys Ser Met Ser Arg Asn Ser Ser Ile Ala Ser Asp Ile
65 70 75 80

His Gly Asp Asp Leu Ile Val Thr Pro Phe Ala Gln Val Leu Ala Ser
85 90 95

```
Leu Arg Thr Val Arg Asn Asn Phe Ala Ala Leu Thr Asn Leu Gln Asp
            100                 105                 110

Arg Ala Pro Ser Lys Arg Ser Pro Met Cys Asn Gln Pro Ser Ile Asn
        115                 120                 125

Lys Ala Thr Ile Thr Glu Glu Ala Tyr Gln Lys Leu Ala Ser Glu Thr
    130                 135                 140

Leu Glu Glu Leu Asp Trp Cys Leu Asp Gln Leu Glu Thr Leu Gln Thr
145                 150                 155                 160

Arg His Ser Val Ser Glu Met Ala Ser Asn Lys Phe Lys Arg Met Leu
                165                 170                 175

Asn Arg Glu Leu Thr His Leu Ser Glu Met Ser Arg Ser Gly Asn Gln
            180                 185                 190

Val Ser Glu Phe Ile Ser Asn Thr Phe Leu Asp Lys Gln His Glu Val
        195                 200                 205

Glu Ile Pro Ser Pro Thr Gln Lys Glu Lys Glu Lys Lys Lys Arg Pro
    210                 215                 220

Met Ser Gln Ile Ser Gly Val Lys Lys Leu Met His Ser Ser Ser Leu
225                 230                 235                 240

Thr Asn Ser Ser Ile Pro Arg Phe Gly Val Lys Thr Glu Gln Glu Asp
                245                 250                 255

Val Leu Ala Lys Glu Leu Glu Asp Val Asn Lys Trp Gly Leu His Val
            260                 265                 270

Phe Arg Ile Ala Glu Leu Ser Gly Asn Arg Pro Leu Thr Val Ile Met
        275                 280                 285

His Thr Ile Phe Gln Glu Arg Asp Leu Leu Lys Thr Phe Lys Ile Pro
    290                 295                 300

Val Asp Thr Leu Ile Thr Tyr Leu Met Thr Leu Glu Asp His Tyr His
305                 310                 315                 320

Ala Asp Val Ala Tyr His Asn Asn Ile His Ala Ala Asp Val Val Gln
                325                 330                 335

Ser Thr His Val Leu Leu Ser Thr Pro Ala Leu Glu Ala Val Phe Thr
            340                 345                 350
```

Asp Leu Glu Ile Leu Ala Ala Ile Phe Ala Ser Ala Ile His Asp Val
355 360 365

Asp His Pro Gly Val Ser Asn Gln Phe Leu Ile Asn Thr Asn Ser Glu
370 375 380

Leu Ala Leu Met Tyr Asn Asp Ser Ser Val Leu Glu Asn His His Leu
385 390 395 400

Ala Val Gly Phe Lys Leu Leu Gln Glu Glu Asn Cys Asp Ile Phe Gln
405 410 415

Asn Leu Thr Lys Lys Gln Arg Gln Ser Leu Arg Lys Met Val Ile Asp
420 425 430

Ile Val Leu Ala Thr Asp Met Ser Lys His Met Asn Leu Leu Ala Asp
435 440 445

Leu Lys Thr Met Val Glu Thr Lys Lys Val Thr Ser Ser Gly Val Leu
450 455 460

Leu Leu Asp Asn Tyr Ser Asp Arg Ile Gln Val Leu Gln Asn Met Val
465 470 475 480

His Cys Ala Asp Leu Ser Asn Pro Thr Lys Pro Leu Gln Leu Tyr Arg
485 490 495

Gln Trp Thr Asp Arg Ile Met Glu Glu Phe Phe Arg Gln Gly Asp Arg
500 505 510

Glu Arg Glu Arg Gly Met Glu Ile Ser Pro Met Cys Asp Lys His Asn
515 520 525

Ala Ser Val Glu Lys Ser Gln Val Gly Phe Ile Asp Tyr Ile Val His
530 535 540

Pro Leu Trp Glu Thr Trp Ala Asp Leu Val His Pro Asp Ala Gln Asp
545 550 555 560

Ile Leu Asp Thr Leu Glu Asp Asn Arg Glu Trp Tyr Gln Ser Thr Ile
565 570 575

Pro Gln Ser Pro Ser Pro Ala Pro Asp Asp Pro Glu Glu Gly Arg Gln
580 585 590

Gly Gln Thr Glu Lys Phe Gln Phe Glu Leu Thr Leu Glu Glu Asp Gly
595 600 605

Glu Ser Asp Thr Glu Lys Asp Ser Gly Ser Gln Val Glu Glu Asp Thr
610 615 620

Ser Cys Ser Asp Ser Lys Thr Leu Cys Thr Gln Asp Ser Glu Ser Thr
625 630 635 640

Glu Ile Pro Leu Asp Glu Gln Val Glu Glu Glu Ala Val Gly Glu Glu
645 650 655

Glu Glu Ser Gln Pro Glu Ala Cys Val Ile Asp Asp Arg Ser Pro Asp
660 665 670

Thr

<210> 24
<211> 507
<212> PRT
<213> Homo sapiens

<400> 24

Met Ala Ser Asn Lys Phe Lys Arg Met Leu Asn Arg Glu Leu Thr His
1 5 10 15

Leu Ser Glu Met Ser Arg Ser Gly Asn Gln Val Ser Glu Phe Ile Ser
20 25 30

Asn Thr Phe Leu Asp Lys Gln His Glu Val Glu Ile Pro Ser Pro Thr
35 40 45

Gln Lys Glu Lys Glu Lys Lys Lys Arg Pro Met Ser Gln Ile Ser Gly
50 55 60

Val Lys Lys Leu Met His Ser Ser Ser Leu Thr Asn Ser Ser Ile Pro
65 70 75 80

Arg Phe Gly Val Lys Thr Glu Gln Glu Asp Val Leu Ala Lys Glu Leu
85 90 95

Glu Asp Val Asn Lys Trp Gly Leu His Val Phe Arg Ile Ala Glu Leu
100 105 110

Ser Gly Asn Arg Pro Leu Thr Val Ile Met His Thr Ile Phe Gln Glu
115 120 125

Arg Asp Leu Leu Lys Thr Phe Lys Ile Pro Val Asp Thr Leu Ile Thr
130 135 140

Tyr Leu Met Thr Leu Glu Asp His Tyr His Ala Asp Val Ala Tyr His
145 150 155 160

Asn Asn Ile His Ala Ala Asp Val Val Gln Ser Thr His Val Leu Leu
165 170 175

Ser Thr Pro Ala Leu Glu Ala Val Phe Thr Asp Leu Glu Ile Leu Ala
180 185 190

Ala Ile Phe Ala Ser Ala Ile His Asp Val Asp His Pro Gly Val Ser
195 200 205

Asn Gln Phe Leu Ile Asn Thr Asn Ser Glu Leu Ala Leu Met Tyr Asn
210 215 220

Asp Ser Ser Val Leu Glu Asn His His Leu Ala Val Gly Phe Lys Leu
225 230 235 240

Leu Gln Glu Glu Asn Cys Asp Ile Phe Gln Asn Leu Thr Lys Lys Gln
245 250 255

Arg Gln Ser Leu Arg Lys Met Val Ile Asp Ile Val Leu Ala Thr Asp
260 265 270

Met Ser Lys His Met Asn Leu Leu Ala Asp Leu Lys Thr Met Val Glu
275 280 285

Thr Lys Lys Val Thr Ser Ser Gly Val Leu Leu Leu Asp Asn Tyr Ser
290 295 300

Asp Arg Ile Gln Val Leu Gln Asn Met Val His Cys Ala Asp Leu Ser
305 310 315 320

Asn Pro Thr Lys Pro Leu Gln Leu Tyr Arg Gln Trp Thr Asp Arg Ile
325 330 335

Met Glu Glu Phe Phe Arg Gln Gly Asp Arg Glu Arg Glu Arg Gly Met
340 345 350

Glu Ile Ser Pro Met Cys Asp Lys His Asn Ala Ser Val Glu Lys Ser
355 360 365

Gln Val Gly Phe Ile Asp Tyr Ile Val His Pro Leu Trp Glu Thr Trp
370 375 380

Ala Asp Leu Val His Pro Asp Ala Gln Asp Ile Leu Asp Thr Leu Glu

385 390 395 400

Asp Asn Arg Glu Trp Tyr Gln Ser Thr Ile Pro Gln Ser Pro Ser Pro
405 410 415

Ala Pro Asp Asp Pro Glu Glu Gly Arg Gln Gly Gln Thr Glu Lys Phe
420 425 430

Gln Phe Glu Leu Thr Leu Glu Glu Asp Gly Glu Ser Asp Thr Glu Lys
435 440 445

Asp Ser Gly Ser Gln Val Glu Glu Asp Thr Ser Cys Ser Asp Ser Lys
450 455 460

Thr Leu Cys Thr Gln Asp Ser Glu Ser Thr Glu Ile Pro Leu Asp Glu
465 470 475 480

Gln Val Glu Glu Glu Ala Val Gly Glu Glu Glu Glu Ser Gln Pro Glu
485 490 495

Ala Cys Val Ile Asp Asp Arg Ser Pro Asp Thr
500 505

<210> 25
<211> 679
<212> PRT
<213> Homo sapiens

<400> 25

Met Ser Ile Ile Met Lys Pro Arg Ser Arg Ser Thr Ser Ser Leu Arg
1 5 10 15

Thr Ala Glu Ala Val Cys Phe Asp Val Asp Asn Gly Thr Ser Ala Gly
20 25 30

Arg Ser Pro Leu Asp Pro Met Thr Ser Pro Gly Ser Gly Leu Ile Leu
35 40 45

Gln Ala Asn Phe Val His Ser Gln Arg Arg Glu Ser Phe Leu Tyr Arg
50 55 60

Ser Asp Ser Asp Tyr Asp Leu Ser Pro Lys Ser Met Ser Arg Asn Ser
65 70 75 80

Ser Ile Ala Ser Asp Ile His Gly Asp Asp Leu Ile Val Thr Pro Phe
85 90 95

Ala Gln Val Leu Ala Ser Leu Arg Thr Val Arg Asn Asn Phe Ala Ala

100 105 110

Leu Thr Asn Leu Gln Asp Arg Ala Pro Ser Lys Arg Ser Pro Met Cys
115 120 125

Asn Gln Pro Ser Ile Asn Lys Ala Thr Ile Thr Glu Glu Ala Tyr Gln
130 135 140

Lys Leu Ala Ser Glu Thr Leu Glu Glu Leu Asp Trp Cys Leu Asp Gln
145 150 155 160

Leu Glu Thr Leu Gln Thr Arg His Ser Val Ser Glu Met Ala Ser Asn
165 170 175

Lys Phe Lys Arg Met Leu Asn Arg Glu Leu Thr His Leu Ser Glu Met
180 185 190

Ser Arg Ser Gly Asn Gln Val Ser Glu Phe Ile Ser Asn Thr Phe Leu
195 200 205

Asp Lys Gln His Glu Val Glu Ile Pro Ser Pro Thr Gln Lys Glu Lys
210 215 220

Glu Lys Lys Lys Arg Pro Met Ser Gln Ile Ser Gly Val Lys Lys Leu
225 230 235 240

Met His Ser Ser Ser Leu Thr Asn Ser Ser Ile Pro Arg Phe Gly Val
245 250 255

Lys Thr Glu Gln Glu Asp Val Leu Ala Lys Glu Leu Glu Asp Val Asn
260 265 270

Lys Trp Gly Leu His Val Phe Arg Ile Ala Glu Leu Ser Gly Asn Arg
275 280 285

Pro Leu Thr Val Ile Met His Thr Ile Phe Gln Glu Arg Asp Leu Leu
290 295 300

Lys Thr Phe Lys Ile Pro Val Asp Thr Leu Ile Thr Tyr Leu Met Thr
305 310 315 320

Leu Glu Asp His Tyr His Ala Asp Val Ala Tyr His Asn Asn Ile His
325 330 335

Ala Ala Asp Val Val Gln Ser Thr His Val Leu Leu Ser Thr Pro Ala
340 345 350

Leu Glu Ala Val Phe Thr Asp Leu Glu Ile Leu Ala Ala Ile Phe Ala
355 360 365

Ser Ala Ile His Asp Val Asp His Pro Gly Val Ser Asn Gln Phe Leu
370 375 380

Ile Asn Thr Asn Ser Glu Leu Ala Leu Met Tyr Asn Asp Ser Ser Val
385 390 395 400

Leu Glu Asn His His Leu Ala Val Gly Phe Lys Leu Leu Gln Glu Glu
405 410 415

Asn Cys Asp Ile Phe Gln Asn Leu Thr Lys Lys Gln Arg Gln Ser Leu
420 425 430

Arg Lys Met Val Ile Asp Ile Val Leu Ala Thr Asp Met Ser Lys His
435 440 445

Met Asn Leu Leu Ala Asp Leu Lys Thr Met Val Glu Thr Lys Lys Val
450 455 460

Thr Ser Ser Gly Val Leu Leu Leu Asp Asn Tyr Ser Asp Arg Ile Gln
465 470 475 480

Val Leu Gln Asn Met Val His Cys Ala Asp Leu Ser Asn Pro Thr Lys
485 490 495

Pro Leu Gln Leu Tyr Arg Gln Trp Thr Asp Arg Ile Met Glu Glu Phe
500 505 510

Phe Arg Gln Gly Asp Arg Glu Arg Glu Arg Gly Met Glu Ile Ser Pro
515 520 525

Met Cys Asp Lys His Asn Ala Ser Val Glu Lys Ser Gln Val Gly Phe
530 535 540

Ile Asp Tyr Ile Val His Pro Leu Trp Glu Thr Trp Ala Asp Leu Val
545 550 555 560

His Pro Asp Ala Gln Asp Ile Leu Asp Thr Leu Glu Asp Asn Arg Glu
565 570 575

Trp Tyr Gln Ser Thr Ile Pro Gln Ser Pro Ser Pro Ala Pro Asp Asp
580 585 590

Pro Glu Glu Gly Arg Gln Gly Gln Thr Glu Lys Phe Gln Phe Glu Leu
595 600 605

Thr Leu Glu Glu Asp Gly Glu Ser Asp Thr Glu Lys Asp Ser Gly Ser
610 615 620

Gln Val Glu Glu Asp Thr Ser Cys Ser Asp Ser Lys Thr Leu Cys Thr
625 630 635 640

Gln Asp Ser Glu Ser Thr Glu Ile Pro Leu Asp Glu Gln Val Glu Glu
645 650 655

Glu Ala Val Gly Glu Glu Glu Glu Ser Gln Pro Glu Ala Cys Val Ile
660 665 670

Asp Asp Arg Ser Pro Asp Thr
675

<210> 26
<211> 518
<212> PRT
<213> Homo sapiens

<400> 26

Met Pro Glu Ala Asn Tyr Leu Leu Ser Val Ser Trp Gly Tyr Ile Lys
1 5 10 15

Phe Lys Arg Met Leu Asn Arg Glu Leu Thr His Leu Ser Glu Met Ser
20 25 30

Arg Ser Gly Asn Gln Val Ser Glu Phe Ile Ser Asn Thr Phe Leu Asp
35 40 45

Lys Gln His Glu Val Glu Ile Pro Ser Pro Thr Gln Lys Glu Lys Glu
50 55 60

Lys Lys Lys Arg Pro Met Ser Gln Ile Ser Gly Val Lys Lys Leu Met
65 70 75 80

His Ser Ser Ser Leu Thr Asn Ser Ser Ile Pro Arg Phe Gly Val Lys
85 90 95

Thr Glu Gln Glu Asp Val Leu Ala Lys Glu Leu Glu Asp Val Asn Lys
100 105 110

Trp Gly Leu His Val Phe Arg Ile Ala Glu Leu Ser Gly Asn Arg Pro
115 120 125

Leu Thr Val Ile Met His Thr Ile Phe Gln Glu Arg Asp Leu Leu Lys
130 135 140

Thr Phe Lys Ile Pro Val Asp Thr Leu Ile Thr Tyr Leu Met Thr Leu
145 150 155 160

Glu Asp His Tyr His Ala Asp Val Ala Tyr His Asn Asn Ile His Ala
165 170 175

Ala Asp Val Val Gln Ser Thr His Val Leu Leu Ser Thr Pro Ala Leu
180 185 190

Glu Ala Val Phe Thr Asp Leu Glu Ile Leu Ala Ala Ile Phe Ala Ser
195 200 205

Ala Ile His Asp Val Asp His Pro Gly Val Ser Asn Gln Phe Leu Ile
210 215 220

Asn Thr Asn Ser Glu Leu Ala Leu Met Tyr Asn Asp Ser Ser Val Leu
225 230 235 240

Glu Asn His His Leu Ala Val Gly Phe Lys Leu Leu Gln Glu Glu Asn
245 250 255

Cys Asp Ile Phe Gln Asn Leu Thr Lys Lys Gln Arg Gln Ser Leu Arg
260 265 270

Lys Met Val Ile Asp Ile Val Leu Ala Thr Asp Met Ser Lys His Met
275 280 285

Asn Leu Leu Ala Asp Leu Lys Thr Met Val Glu Thr Lys Lys Val Thr
290 295 300

Ser Ser Gly Val Leu Leu Leu Asp Asn Tyr Ser Asp Arg Ile Gln Val
305 310 315 320

Leu Gln Asn Met Val His Cys Ala Asp Leu Ser Asn Pro Thr Lys Pro
325 330 335

Leu Gln Leu Tyr Arg Gln Trp Thr Asp Arg Ile Met Glu Glu Phe Phe
340 345 350

Arg Gln Gly Asp Arg Glu Arg Glu Arg Gly Met Glu Ile Ser Pro Met
355 360 365

Cys Asp Lys His Asn Ala Ser Val Glu Lys Ser Gln Val Gly Phe Ile
370 375 380

Asp Tyr Ile Val His Pro Leu Trp Glu Thr Trp Ala Asp Leu Val His
385 390 395 400

Pro Asp Ala Gln Asp Ile Leu Asp Thr Leu Glu Asp Asn Arg Glu Trp
405 410 415

Tyr Gln Ser Thr Ile Pro Gln Ser Pro Ser Pro Ala Pro Asp Asp Pro
420 425 430

Glu Glu Gly Arg Gln Gly Gln Thr Glu Lys Phe Gln Phe Glu Leu Thr
435 440 445

Leu Glu Glu Asp Gly Glu Ser Asp Thr Glu Lys Asp Ser Gly Ser Gln
450 455 460

Val Glu Glu Asp Thr Ser Cys Ser Asp Ser Lys Thr Leu Cys Thr Gln
465 470 475 480

Asp Ser Glu Ser Thr Glu Ile Pro Leu Asp Glu Gln Val Glu Glu Glu
485 490 495

Ala Val Gly Glu Glu Glu Glu Ser Gln Pro Glu Ala Cys Val Ile Asp
500 505 510

Asp Arg Ser Pro Asp Thr
515

<210> 27
<211> 748
<212> PRT
<213> Homo sapiens

<400> 27

Met Lys Arg Asn Thr Cys Asp Leu Leu Ser Arg Ser Lys Ser Ala Ser
1 5 10 15

Glu Glu Thr Leu His Ser Ser Asn Glu Glu Glu Asp Pro Phe Arg Gly
20 25 30

Met Glu Pro Tyr Leu Val Arg Arg Leu Ser Cys Arg Asn Ile Gln Leu
35 40 45

Pro Pro Leu Ala Phe Arg Gln Leu Glu Gln Ala Asp Leu Lys Ser Glu
50 55 60

Ser Glu Asn Ile Gln Arg Pro Thr Ser Leu Pro Leu Lys Ile Leu Pro
65 70 75 80

Leu Ile Ala Ile Thr Ser Ala Glu Ser Ser Gly Phe Asp Val Asp Asn
85 90 95

```
Gly Thr Ser Ala Gly Arg Ser Pro Leu Asp Pro Met Thr Ser Pro Gly
            100                 105                 110

Ser Gly Leu Ile Leu Gln Ala Asn Phe Val His Ser Gln Arg Arg Glu
        115                 120                 125

Ser Phe Leu Tyr Arg Ser Asp Ser Asp Tyr Asp Leu Ser Pro Lys Ser
    130                 135                 140

Met Ser Arg Asn Ser Ser Ile Ala Ser Asp Ile His Gly Asp Asp Leu
145                 150                 155                 160

Ile Val Thr Pro Phe Ala Gln Val Leu Ala Ser Leu Arg Thr Val Arg
                165                 170                 175

Asn Asn Phe Ala Ala Leu Thr Asn Leu Gln Asp Arg Ala Pro Ser Lys
            180                 185                 190

Arg Ser Pro Met Cys Asn Gln Pro Ser Ile Asn Lys Ala Thr Ile Thr
        195                 200                 205

Glu Glu Ala Tyr Gln Lys Leu Ala Ser Glu Thr Leu Glu Glu Leu Asp
    210                 215                 220

Trp Cys Leu Asp Gln Leu Glu Thr Leu Gln Thr Arg His Ser Val Ser
225                 230                 235                 240

Glu Met Ala Ser Asn Lys Phe Lys Arg Met Leu Asn Arg Glu Leu Thr
                245                 250                 255

His Leu Ser Glu Met Ser Arg Ser Gly Asn Gln Val Ser Glu Phe Ile
            260                 265                 270

Ser Asn Thr Phe Leu Asp Lys Gln His Glu Val Glu Ile Pro Ser Pro
        275                 280                 285

Thr Gln Lys Glu Lys Glu Lys Lys Lys Arg Pro Met Ser Gln Ile Ser
    290                 295                 300

Gly Val Lys Lys Leu Met His Ser Ser Ser Leu Thr Asn Ser Ser Ile
305                 310                 315                 320

Pro Arg Phe Gly Val Lys Thr Glu Gln Glu Asp Val Leu Ala Lys Glu
                325                 330                 335

Leu Glu Asp Val Asn Lys Trp Gly Leu His Val Phe Arg Ile Ala Glu
```

340 345 350

Leu Ser Gly Asn Arg Pro Leu Thr Val Ile Met His Thr Ile Phe Gln
355 360 365

Glu Arg Asp Leu Leu Lys Thr Phe Lys Ile Pro Val Asp Thr Leu Ile
370 375 380

Thr Tyr Leu Met Thr Leu Glu Asp His Tyr His Ala Asp Val Ala Tyr
385 390 395 400

His Asn Asn Ile His Ala Ala Asp Val Val Gln Ser Thr His Val Leu
405 410 415

Leu Ser Thr Pro Ala Leu Glu Ala Val Phe Thr Asp Leu Glu Ile Leu
420 425 430

Ala Ala Ile Phe Ala Ser Ala Ile His Asp Val Asp His Pro Gly Val
435 440 445

Ser Asn Gln Phe Leu Ile Asn Thr Asn Ser Glu Leu Ala Leu Met Tyr
450 455 460

Asn Asp Ser Ser Val Leu Glu Asn His His Leu Ala Val Gly Phe Lys
465 470 475 480

Leu Leu Gln Glu Glu Asn Cys Asp Ile Phe Gln Asn Leu Thr Lys Lys
485 490 495

Gln Arg Gln Ser Leu Arg Lys Met Val Ile Asp Ile Val Leu Ala Thr
500 505 510

Asp Met Ser Lys His Met Asn Leu Leu Ala Asp Leu Lys Thr Met Val
515 520 525

Glu Thr Lys Lys Val Thr Ser Ser Gly Val Leu Leu Leu Asp Asn Tyr
530 535 540

Ser Asp Arg Ile Gln Val Leu Gln Asn Met Val His Cys Ala Asp Leu
545 550 555 560

Ser Asn Pro Thr Lys Pro Leu Gln Leu Tyr Arg Gln Trp Thr Asp Arg
565 570 575

Ile Met Glu Glu Phe Phe Arg Gln Gly Asp Arg Glu Arg Glu Arg Gly
580 585 590

Met Glu Ile Ser Pro Met Cys Asp Lys His Asn Ala Ser Val Glu Lys
595 600 605

Ser Gln Val Gly Phe Ile Asp Tyr Ile Val His Pro Leu Trp Glu Thr
610 615 620

Trp Ala Asp Leu Val His Pro Asp Ala Gln Asp Ile Leu Asp Thr Leu
625 630 635 640

Glu Asp Asn Arg Glu Trp Tyr Gln Ser Thr Ile Pro Gln Ser Pro Ser
645 650 655

Pro Ala Pro Asp Asp Pro Glu Glu Gly Arg Gln Gly Gln Thr Glu Lys
660 665 670

Phe Gln Phe Glu Leu Thr Leu Glu Glu Asp Gly Glu Ser Asp Thr Glu
675 680 685

Lys Asp Ser Gly Ser Gln Val Glu Glu Asp Thr Ser Cys Ser Asp Ser
690 695 700

Lys Thr Leu Cys Thr Gln Asp Ser Glu Ser Thr Glu Ile Pro Leu Asp
705 710 715 720

Glu Gln Val Glu Glu Glu Ala Val Gly Glu Glu Glu Glu Ser Gln Pro
725 730 735

Glu Ala Cys Val Ile Asp Asp Arg Ser Pro Asp Thr
740 745

<210> 28
<211> 687
<212> PRT
<213> Homo sapiens

<400> 28

Met Ala Phe Val Trp Asp Pro Leu Gly Ala Thr Val Pro Gly Pro Ser
1 5 10 15

Thr Arg Ala Lys Ser Arg Leu Arg Phe Ser Lys Ser Tyr Ser Phe Asp
20 25 30

Val Asp Asn Gly Thr Ser Ala Gly Arg Ser Pro Leu Asp Pro Met Thr
35 40 45

Ser Pro Gly Ser Gly Leu Ile Leu Gln Ala Asn Phe Val His Ser Gln
50 55 60

Arg Arg Glu Ser Phe Leu Tyr Arg Ser Asp Ser Asp Tyr Asp Leu Ser
65 70 75 80

Pro Lys Ser Met Ser Arg Asn Ser Ser Ile Ala Ser Asp Ile His Gly
85 90 95

Asp Asp Leu Ile Val Thr Pro Phe Ala Gln Val Leu Ala Ser Leu Arg
100 105 110

Thr Val Arg Asn Asn Phe Ala Ala Leu Thr Asn Leu Gln Asp Arg Ala
115 120 125

Pro Ser Lys Arg Ser Pro Met Cys Asn Gln Pro Ser Ile Asn Lys Ala
130 135 140

Thr Ile Thr Glu Glu Ala Tyr Gln Lys Leu Ala Ser Glu Thr Leu Glu
145 150 155 160

Glu Leu Asp Trp Cys Leu Asp Gln Leu Glu Thr Leu Gln Thr Arg His
165 170 175

Ser Val Ser Glu Met Ala Ser Asn Lys Phe Lys Arg Met Leu Asn Arg
180 185 190

Glu Leu Thr His Leu Ser Glu Met Ser Arg Ser Gly Asn Gln Val Ser
195 200 205

Glu Phe Ile Ser Asn Thr Phe Leu Asp Lys Gln His Glu Val Glu Ile
210 215 220

Pro Ser Pro Thr Gln Lys Glu Lys Glu Lys Lys Lys Arg Pro Met Ser
225 230 235 240

Gln Ile Ser Gly Val Lys Lys Leu Met His Ser Ser Ser Leu Thr Asn
245 250 255

Ser Ser Ile Pro Arg Phe Gly Val Lys Thr Glu Gln Glu Asp Val Leu
260 265 270

Ala Lys Glu Leu Glu Asp Val Asn Lys Trp Gly Leu His Val Phe Arg
275 280 285

Ile Ala Glu Leu Ser Gly Asn Arg Pro Leu Thr Val Ile Met His Thr
290 295 300

Ile Phe Gln Glu Arg Asp Leu Leu Lys Thr Phe Lys Ile Pro Val Asp
305 310 315 320

Thr Leu Ile Thr Tyr Leu Met Thr Leu Glu Asp His Tyr His Ala Asp
325 330 335

Val Ala Tyr His Asn Asn Ile His Ala Ala Asp Val Val Gln Ser Thr
340 345 350

His Val Leu Leu Ser Thr Pro Ala Leu Glu Ala Val Phe Thr Asp Leu
355 360 365

Glu Ile Leu Ala Ala Ile Phe Ala Ser Ala Ile His Asp Val Asp His
370 375 380

Pro Gly Val Ser Asn Gln Phe Leu Ile Asn Thr Asn Ser Glu Leu Ala
385 390 395 400

Leu Met Tyr Asn Asp Ser Ser Val Leu Glu Asn His His Leu Ala Val
405 410 415

Gly Phe Lys Leu Leu Gln Glu Glu Asn Cys Asp Ile Phe Gln Asn Leu
420 425 430

Thr Lys Lys Gln Arg Gln Ser Leu Arg Lys Met Val Ile Asp Ile Val
435 440 445

Leu Ala Thr Asp Met Ser Lys His Met Asn Leu Leu Ala Asp Leu Lys
450 455 460

Thr Met Val Glu Thr Lys Lys Val Thr Ser Ser Gly Val Leu Leu Leu
465 470 475 480

Asp Asn Tyr Ser Asp Arg Ile Gln Val Leu Gln Asn Met Val His Cys
485 490 495

Ala Asp Leu Ser Asn Pro Thr Lys Pro Leu Gln Leu Tyr Arg Gln Trp
500 505 510

Thr Asp Arg Ile Met Glu Glu Phe Phe Arg Gln Gly Asp Arg Glu Arg
515 520 525

Glu Arg Gly Met Glu Ile Ser Pro Met Cys Asp Lys His Asn Ala Ser
530 535 540

Val Glu Lys Ser Gln Val Gly Phe Ile Asp Tyr Ile Val His Pro Leu
545 550 555 560

Trp Glu Thr Trp Ala Asp Leu Val His Pro Asp Ala Gln Asp Ile Leu
565 570 575

Asp Thr Leu Glu Asp Asn Arg Glu Trp Tyr Gln Ser Thr Ile Pro Gln
580 585 590

Ser Pro Ser Pro Ala Pro Asp Asp Pro Glu Glu Gly Arg Gln Gly Gln
595 600 605

Thr Glu Lys Phe Gln Phe Glu Leu Thr Leu Glu Glu Asp Gly Glu Ser
610 615 620

Asp Thr Glu Lys Asp Ser Gly Ser Gln Val Glu Glu Asp Thr Ser Cys
625 630 635 640

Ser Asp Ser Lys Thr Leu Cys Thr Gln Asp Ser Glu Ser Thr Glu Ile
645 650 655

Pro Leu Asp Glu Gln Val Glu Glu Glu Ala Val Gly Glu Glu Glu Glu
660 665 670

Ser Gln Pro Glu Ala Cys Val Ile Asp Asp Arg Ser Pro Asp Thr
675 680 685

<210> 29
<211> 6663
<212> DNA
<213> Homo sapiens

<400> 29
actgaccccg ctgtaccacg gccctcttgc ggacagcccc ggggacgtcg ttgggacatc 60

gctgggaccc cgggctctgc agccacaacc atgtttggcc ggaagcgcag tgtctccttt 120

gggggcttcg gatggatcga caagaccatg ctggcaagtc tgaaggtcaa gaagcaggag 180

ctggccaaca gctcggatgc gaccctccca gaccggccgc tctcccctcc tctcacggca 240

cctcccacca tgaagtcgtc ggagttcttt gagatgctgg agaaaatgca ggggatcaag 300

cttgaagagc agaagccggg accccagaag aacaaggacg actatatccc ataccccagc 360

atcgacgagg ttgtggagaa gggaggcccg taccctcagg tcatcctgcc acagtttggg 420

ggctattgga tcgaggaccc ggagaacgtg ggcaccccaa catcgctggg gagcagcatc 480

tgtgaggagg aggaagagga caacctcagc cccaacacat ttggctacaa gctcgagtgc 540

aagggtgaag ccagggccta ccggaggcac ttcctgggga aggatcatct aaacttttac 600

tgtaccggca gcagcctggg gaacttgatc ctgtccgtca agtgcgagga agcagagggg 660

atcgagtacc tccgggtcat ccttaggtcc aaactgaaga cggtacatga gcggatcccc 720

ttggctggac tgagcaagct tcccagtgtc cctcagattg caaaggcttt ctgtgatgat 780

gcagtgggac tgagattcaa tcctgtcctg taccccaagg cctcccaaat gattgtgtcc 840

tatgatgagc atgaagtcaa caacacattc aaattcggag tcatttatca aaaagccagg 900

cagaccctgg aggaggagct atttgggaac aatgaggaga gcccagcttt taaggagttc 960

ttggacctgc tgggggacac gatcacactg caggatttca aaggtttccg aggaggcctg 1020

gacgtgaccc acggacagac aggggtggaa tcagtgtaca caacattccg ggacagggag 1080

atcatgtttc acgtttccac aaagctgcca tttaccgacg gagacgccca gcagctccag 1140

agaaagagac acattggaaa tgacatcgtg gccatcatct tccaagagga aaacacgccg 1200

tttgtcccag acatgatagc ctccaatttc ttacatgcct acatcgtcgt gcaggtcgag 1260

accccaggca cagagacccc atcctacaag gtctctgtca ctgcgcggga agatgtgccc 1320

acctttggtc cacctctgcc cagtcccccc gttttccaga agggcccgga attcagggag 1380

tttctgctca ccaagctcac caatgccgag aacgcctgct gcaagtcgga caagtttgca 1440

aagctggagg accggaccag ggctgccctc ctggacaacc ttcacgatga gctccacgcc 1500

cacacacagg ccatgctggg actgggccca gaggaggaca agtttgagaa tggaggccac 1560

gggggggttcc tggagtcttt taagagggcc atccgcgtac gcagccactc catggagacc 1620

atggtgggcg gccagaagaa gtcgcacagt gggggcatcc ctggcagcct cagcgggggc 1680

atctcccaca acagcatgga ggtcaccaag accaccttct cgcctccagt ggtggcggca 1740

acggtgaaga accagtcacg gagtcccatc aagcgacgct cggggctctt cccccgcctg 1800

cacacgggct cagaaggcca gggcgacagc cgggcacgat gtgacagcac atccagcaca 1860

cccaagaccc cagatggtgg acactcctct caggagataa agtctgagac ctcatccaat 1920

cccagctctc cggaaatctg ccccaacaag gagaagccct tcatgaagtt gaaggaaaac 1980

ggccgtgcca tctcccgctc ctcctccagc accagcagcg tcagcagcac tgcaggggag 2040

ggcgaggcca tggaggaggg cgacagtggg ggcagccagc cgtccacgac ctcacccttc 2100

aagcaggagg tgtttgtcta cagcccgtcc ccgagcagcg agagccccag cctgggggca 2160

gctgccaccc cgatcatcat gagccggagt cccacagatg ccaaaagcag aaactccccg 2220

agatcgaacc tgaaattccg ctttgacaag ctcagccatg ccagctctgg tgcgggtcac 2280

taatgtgaaa gtggagtcct tcgcctgtcc aagggaatcc cctcttctgt cctggaaaag 2340

gctcctgtac cagcagtttg ggagtgccgt ccacgaccct gacagtccca gccctgctgc 2400

cccatggcca cgtgcccaca gatgtgctgt tggtccaggt gtcccagtct ggccacagcc 2460

ctgcctccgc cctcacctac atgccctccc agcccctccc atctctggac gaggcctcct 2520

tcctcaggtt cctcctgctc ctgacctccc agtgtgatgt ccgggtcctt tatcatccta 2580

ttcatcctgg agaggaaaag tgtcgggcaa aggggggatct ggggggagct cagcagtgac 2640

tggggagctg gtctgcctca gagacagagt agggggtggg agcagagcct cggtgagggt 2700

cttggccaca gggcagtgcc ttcctgaacg tggcaggctt tactaccagg aacgcactcg 2760
gtggtggagg ccccatgttc ccaggagcca agattcgtag catccttgag gccatcctga 2820
taaaattcgg cgctattgcc cccgtagctc tggagctcta aaccgtctat ctgcttctgt 2880
gctgaacgcc tttcccatct gctgacgtag gcccagggct gccctgcccc tgctgccagt 2940
gtaccgtgag cggggctcca gccagttcaa gctcagagcc agagctggac gggccagaac 3000
tgcgctgcac acttcctgga ctgaggcggg gactttgggt cccacccggt ttctcctgat 3060
tatggctgct gtggggtgag ggagggaggg ggcagccccg aggcagtctc ttccctttga 3120
gaagatattt tcccacaaag ggtgggaag ccaggagtga gaaggaattc agggagagca 3180
aaggagccag tgctgagatg ctgctgtgtt ggttgaggaa aacctcgggc ctgagggcca 3240
ggccggagcc caggtctctg ctgacaatgg gggttcaagg aagacgtcgt tatctcccct 3300
ccccacttac tcgaggagag aggtgagggg gggatgactt gcgggttctg atcaggcccc 3360
tgggtgggga aggggcacag tgtccctcag cagcttacgc ccctggagtc ttggggggcc 3420
cagcctggcc ctggggcctt ttccagctac tgtgcccttg ggcagctgcg tctggggctc 3480
aacccccaa tcctgttccc ctctccagct gcgggtctgt aggcagctgt cacatctgaa 3540
gggtttctgc aacctggacc ccatctgggt gtgggtcaga ccctgtgacc cacatgccac 3600
ccccaccctc cacagagccc ccttgctggg acagccagct cacctccaag gacatcccct 3660
cctggcttct ccccttccg agtctgcagc gccgtgggct tctctgccga tgggcccggg 3720
ttggggttaa ggtgggcatc ctccaggtac aacgagcctg aagagcccct ttcagtgcag 3780
acggggctgc agagtgacac tggctgggca cctgccccac gaccaatgac aaggatttcc 3840
agctgaatgc tttattccca tagggatctg gacctgtgcc caagatataa atactacact 3900
tttttttttt ttttttaact gacattgtga aattctccct atagcttttg ccattcaagc 3960
aacattgtga tctttcttcc ccgccacgtg tgtgggaatg attgagtcct gtttgcaagc 4020
tggagaggag ctctcccttt gctagtactt tctctaaagt actagtctag taaaatttat 4080
tcttgttaga aggtcaacaa aatatctgtt tagcttttat gaagagtcac cgtagcagcc 4140
cccacggctg gaaagaggcc tgtacgttct ggacgcgttt tgttggctgg gcttctggag 4200
gcactggcaa ggtcaaactg catttcttta agaacagttg caggatctgg ctcgcctctg 4260
tgggaagccg gcattacagg tgcttggtgg atgggccgtg tcacattgcc atctggggtc 4320
ctttggggtt tccaggttgt caccatgctg tcccatttgg gaatcccata cctgcctgtc 4380
cccactgcgc tggctgaccc ttgctgcctg ctgcctcttg ggagggcttt gtccctgcct 4440
ctgagctggt gggcaggatg gctgggtggc ccccagagaa gcacagacct gagatggggt 4500
ctccatgccc ggtttgctgt tggaatgatc tgaacaggac cccaaatgcc tcttccctct 4560
ggtcatgcct cactatctct aggagctcca tcctgtggct tccagagtgt gcacttccag 4620

cccacccggg cagtgctgag agggaggagg agaacaagga tggcccagcc tcccctccct 4680
cccctagacc acggggcggg cagctcgggt tcctggaggg ctgtttcccc cacgctgtcc 4740
ctacatctgc tctgatctaa aatgtctttc cttttatgct gcgcccagtc ttggggctca 4800
aagatttgcc caaacctcat tggccctcgt gactaggctc atctagatgg tgctcacgct 4860
ggtgtttgag gcatttccac tgtgatctcc acgaggggat gttttccggg acacatctct 4920
ggctctggga actgcctgac tcactgaaga aactactttt caggcactgt agggtcaccc 4980
atatgcctcc agctcagttg acgcttaaaa acaggtgcag aaaagctcgc gatggaaggt 5040
cttaatgaga gtgtctgtct atgccaatca tgtaaaatga cgtttcttga aaaagattca 5100
gtggttcagc tttgtcagca tcatctcaac acaagcctgc tggctctttt tagcatctca 5160
tccaaccatg tcatcgtcca gatgagaaat cttagcccag gtgaggggag taacttgctt 5220
gaggtcacac agctggctgt tggcaaagct gggattagaa ccctcaaccc agggtccctt 5280
cctctgcagc gcctacatgg ttggttgaat aagtggctgc gtttcctggg gccctgggtt 5340
ttggggaagc cagttagctg ctgctttggc actggcatgg aggtgagcag tcaaggatgc 5400
tggtgaggct gcagtttctg ctctttttca tcagggggga tagtctctag gatttttcag 5460
tgaggaccct tgggctttgg atgcagcttg aaccaagaaa acgaggaggg aaagggattc 5520
agtgaactat tcctcagtgg gatcggttct tcagctcctg atgggggctg tgtaatgggg 5580
gcagaggcca gggaaaaaga tgctgttcac ccaccctcag cttccctttt cctaaattaa 5640
gaggaaaagt ggtcaaagaa aaactcttca tttctccctg attcttaaac gaaggtggtt 5700
aatagaaact caggctcccg tgacaaggca ggacaagagc ctgtttcgct ttcctccctg 5760
accctgccag gtgccaactc aaacactacc tttctcattg gtttctaagt cagtagagac 5820
agatctgttt taagcagttg gggttcgag tagatctcat gggtacagga ggccagcagg 5880
gaccaggcca gtcagccatg ctcaggaccc ctcggctcct cccccagcct ctagctaccc 5940
tgtatcgagg caaggggagg ccagtaaagt ttgccaagcc tgatcctgca gcctggtggg 6000
gctggctggg gtattctttt accaaactct gttttaccgc cagccccttg tacaccccaa 6060
tcccatgtct ccctcccttc agctggaccg tgtgcccctt tgggaggaag aagacaagcc 6120
ccactagggc caagggcagc agagccctgc cgagtgagag gctgtggggc agcggctctg 6180
tcctgtgcct taccagccct ggggaggggg gcatttggct ggaagactgg aatttaattg 6240
ccatcgtctt tgattttgtg acatttctgc ttggaagtgt gaactacccc cccccccccg 6300
cttcctgctc cttagcatgc gtgcagctct ctcctgtttt gggtgttccc cttggacact 6360
ccagctcggg gactgctggc gtgtgaatgt gcagattccc ctgtgtggtc gaacctaaga 6420
actgtggctt ggaagtgatg ctccatgtga cgacgacttt gctttctttc ctcttagtga 6480

ggaggtgatt cgtagatccc aactgcctat gtaatgtaaa taatgtacat ttaatttatt 6540

gctatggtag cacattgtat ttgttaatgt acaaaacaaa ttctaaaagg ttgacaaatg 6600

tatattttgt tgcttaaatg tgtctttgca gaaattgaca ataaataaca tattttgtgt 6660

cca 6663

<210> 30
<211> 6618
<212> DNA
<213> Homo sapiens

<400> 30
actgaccccg ctgtaccacg gccctcttgc ggacagcccc ggggacgtcg ttgggacatc 60

gctgggaccc cgggctctgc agccacaacc atgtttggcc ggaagcgcag tgtctccttt 120

gggggcttcg gatggatcga caagaccatg ctggcaagtc tgaaggtcaa gaagcaggag 180

ctggccaaca gctcggatgc gaccctccca gaccggccgc tctcccctcc tctcacggca 240

cctcccacca tgaagtcgtc ggagttcttt gagatgctgg agaaaatgca ggacgactat 300

atcccatacc ccagcatcga cgaggttgtg gagaagggag gcccgtaccc tcaggtcatc 360

ctgccacagt ttgggggcta ttggatcgag gacccggaga acgtgggcac cccaacatcg 420

ctggggagca gcatctgtga ggaggaggaa gaggacaacc tcagccccaa cacatttggc 480

tacaagctcg agtgcaaggg tgaagccagg gcctaccgga ggcacttcct ggggaaggat 540

catctaaact tttactgtac cggcagcagc ctggggaact tgatcctgtc cgtcaagtgc 600

gaggaagcag aggggatcga gtacctccgg gtcatcctta ggtccaaact gaagacggta 660

catgagcgga tccccttggc tggactgagc aagcttccca gtgtccctca gattgcaaag 720

gctttctgtg atgatgcagt gggactgaga ttcaatcctg tcctgtaccc caaggcctcc 780

caaatgattg tgtcctatga tgagcatgaa gtcaacaaca cattcaaatt cggagtcatt 840

tatcaaaaag ccaggcagac cctggaggag gagctatttg ggaacaatga ggagagccca 900

gcttttaagg agttcttgga cctgctgggg gacacgatca cactgcagga tttcaaaggt 960

ttccgaggag gcctggacgt gacccacgga cagacagggg tggaatcagt gtacacaaca 1020

ttccgggaca gggagatcat gtttcacgtt tccacaaagc tgccatttac cgacggagac 1080

gcccagcagc tccagagaaa gagacacatt ggaaatgaca tcgtggccat catcttccaa 1140

gaggaaaaca cgccgtttgt cccagacatg atagcctcca atttcttaca tgcctacatc 1200

gtcgtgcagg tcgagacccc aggcacagag accccatcct acaaggtctc tgtcactgcg 1260

cgggaagatg tgcccacctt tggtccacct ctgcccagtc cccccgtttt ccagaagggc 1320

ccggaattca gggagtttct gctcaccaag ctcaccaatg ccgagaacgc ctgctgcaag 1380

tcggacaagt ttgcaaagct ggaggaccgg accagggctg ccctcctgga caaccttcac 1440

gatgagctcc acgcccacac acaggccatg ctgggactgg gcccagagga ggacaagttt 1500
gagaatggag gccacggggg gttcctggag tcttttaaga gggccatccg cgtacgcagc 1560
cactccatgg agaccatggt gggcggccag aagaagtcgc acagtggggg catccctggc 1620
agcctcagcg gggcatctc ccacaacagc atggaggtca ccaagaccac cttctcgcct 1680
ccagtggtgg cggcaacggt gaagaaccag tcacggagtc ccatcaagcg acgctcgggg 1740
ctcttccccc gcctgcacac gggctcagaa ggccagggcg acagccgggc acgatgtgac 1800
agcacatcca gcacacccaa gaccccagat ggtggacact cctctcagga gataaagtct 1860
gagacctcat ccaatcccag ctctccggaa atctgcccca acaaggagaa gcccttcatg 1920
aagttgaagg aaaacggccg tgccatctcc cgctcctcct ccagcaccag cagcgtcagc 1980
agcactgcag gggagggcga ggccatggag gagggcgaca gtgggggcag ccagccgtcc 2040
acgacctcac ccttcaagca ggaggtgttt gtctacagcc cgtccccgag cagcgagagc 2100
cccagcctgg gggcagctgc caccccgatc atcatgagcc ggagtcccac agatgccaaa 2160
agcagaaact ccccgagatc gaacctgaaa ttccgctttg acaagctcag ccatgccagc 2220
tctggtgcgg gtcactaatg tgaaagtgga gtccttcgcc tgtccaaggg aatcccctct 2280
tctgtcctgg aaaaggctcc tgtaccagca gtttgggagt gccgtccacg accctgacag 2340
tcccagccct gctgccccat ggccacgtgc ccacagatgt gctgttggtc caggtgtccc 2400
agtctggcca cagccctgcc tccgccctca cctacatgcc ctcccagccc ctcccatctc 2460
tggacgaggc ctccttcctc aggttcctcc tgctcctgac ctcccagtgt gatgtccggg 2520
tcctttatca tcctattcat cctggagagg aaaagtgtcg ggcaaagggg gatctggggg 2580
gagctcagca gtgactgggg agctggtctg cctcagagac agagtagggg gtgggagcag 2640
agcctcggtg agggtcttgg ccacagggca gtgccttcct gaacgtggca ggctttacta 2700
ccaggaacgc actcggtggt ggaggcccca tgttcccagg agccaagatt cgtagcatcc 2760
ttgaggccat cctgataaaa ttcggcgcta ttgcccccgt agctctggag ctctaaaccg 2820
tctatctgct tctgtgctga acgcctttcc catctgctga cgtaggccca gggctgccct 2880
gcccctgctg ccagtgtacc gtgagcgggg ctccagccag ttcaagctca gagccagagc 2940
tggacgggcc agaactgcgc tgcacacttc ctggactgag gcggggactt tgggtcccac 3000
ccggtttctc ctgattatgg ctgctgtggg gtgaggggag ggagggggcag ccccgaggca 3060
gtctcttccc tttgagaaga tattttccca caaaggggtg ggaagccagg agtgagaagg 3120
aattcaggga gagcaaagga gccagtgctg agatgctgct gtgttggttg aggaaaacct 3180
cgggcctgag ggccaggccg gagcccaggt ctctgctgac aatggggggtt caaggaagac 3240
gtcgttatct cccctcccca cttactcgag gagagaggtg agggggggat gacttgcggg 3300
ttctgatcag gcccctgggt ggggaagggg cacagtgtcc ctcagcagct tacgcccctg 3360

gagtcttggg gggcccagcc tggccctggg gccttttcca gctactgtgc ccttgggcag 3420
ctgcgtctgg ggctcaaccc cccaatcctg ttcccctctc cagctgcggg tctgtaggca 3480
gctgtcacat ctgaagggtt tctgcaacct ggaccccatc tgggtgtggg tcagaccctg 3540
tgacccacat gccaccccca ccctccacag agccccottg ctgggacagc cagctcacct 3600
ccaaggacat cccctcctgg cttctccccc ttccgagtct gcagcgccgt gggcttctct 3660
gccgatgggc ccgggttggg gttaaggtgg gcatcctcca ggtacaacga gcctgaagag 3720
cccctttcag tgcagacggg gctgcagagt gacactggct gggcacctgc cccacgacca 3780
atgacaagga tttccagctg aatgctttat tcccataggg atctggacct gtgcccaaga 3840
tataaatact acactttttt tttttttttt taactgacat tgtgaaattc tccctatagc 3900
ttttgccatt caagcaacat tgtgatcttt cttccccgcc acgtgtgtgg gaatgattga 3960
gtcctgtttg caagctggag aggagctctc cctttgctag tactttctct aaagtactag 4020
tctagtaaaa tttattcttg ttagaaggtc aacaaaatat ctgtttagct tttatgaaga 4080
gtcaccgtag cagcccccac ggctggaaag aggcctgtac gttctggacg cgttttgttg 4140
gctgggcttc tggaggcact ggcaaggtca aactgcattt ctttaagaac agttgcagga 4200
tctggctcgc ctctgtggga agccggcatt acaggtgctt ggtggatggg ccgtgtcaca 4260
ttgccatctg ggtcctttgg gggtttccag gttgtcacca tgctgtccca tttgggaatc 4320
ccatacctgc ctgtccccac tgcgctggct gacccttgct gcctgctgcc tcttgggagg 4380
gctttgtccc tgcctctgag ctggtgggca ggatggctgg gtggccccca gagaagcaca 4440
gacctgagat ggggtctcca tgcccggttt gctgttggaa tgatctgaac aggaccccaa 4500
atgcctcttc cctctggtca tgcctcacta tctctaggag ctccatcctg tggcttccag 4560
agtgtgcact tccagcccac ccgggcagtg ctgagaggga ggaggagaac aaggatggcc 4620
cagcctcccc tccctcccct agaccacggg gcgggcagct cgggttcctg gagggctgtt 4680
tcccccacgc tgtccctaca tctgctctga tctaaaatgt ctttcctttt atgctgcgcc 4740
cagtcttggg gctcaaagat ttgcccaaac ctcattggcc ctcgtgacta ggctcatcta 4800
gatggtgctc acgctggtgt ttgaggcatt tccactgtga tctccacgag ggatgtttt 4860
ccgggacaca tctctggctc tgggaactgc ctgactcact gaagaaacta cttttcaggc 4920
actgtagggt cacccatatg cctccagctc agttgacgct taaaaacagg tgcagaaaag 4980
ctcgcgatgg aaggtcttaa tgagagtgtc tgtctatgcc aatcatgtaa aatgacgttt 5040
cttgaaaaag attcagtggt tcagctttgt cagcatcatc tcaacacaag cctgctggct 5100
ctttttagca tctcatccaa ccatgtcatc gtccagatga gaaatcttag cccaggtgag 5160
gggagtaact tgcttgaggt cacacagctg gctgttggca aagctgggat tagaaccctc 5220

aacccagggt cccttcctct gcagcgccta catggttggt tgaataagtg gctgcgtttc 5280

ctggggccct gggttttggg gaagccagtt agctgctgct ttggcactgg catggaggtg 5340

agcagtcaag gatgctggtg aggctgcagt ttctgctctt tttcatcagg ggggatagtc 5400

tctaggattt ttcagtgagg acccttgggc tttggatgca gcttgaacca agaaaacgag 5460

gagggaaagg gattcagtga actattcctc agtgggatcg gttcttcagc tcctgatggg 5520

ggctgtgtaa tgggggcaga ggccagggaa aaagatgctg ttcacccacc ctcagcttcc 5580

cttttcctaa attaagagga aaagtggtca aagaaaaact cttcatttct ccctgattct 5640

taaacgaagg tggttaatag aaactcaggc tcccgtgaca aggcaggaca agagcctgtt 5700

tcgctttcct ccctgaccct gccaggtgcc aactcaaaca ctacctttct cattggtttc 5760

taagtcagta gagacagatc tgttttaagc agttgggggt tcgagtagat ctcatgggta 5820

caggaggcca gcagggacca ggccagtcag ccatgctcag gacccctcgg ctcctccccc 5880

agcctctagc taccctgtat cgaggcaagg ggaggccagt aaagtttgcc aagcctgatc 5940

ctgcagcctg gtggggctgg ctggggtatt cttttaccaa actctgtttt accgccagcc 6000

ccttgtacac cccaatccca tgtctccctc ccttcagctg gaccgtgtgc cccttttggga 6060

ggaagaagac aagccccact agggccaagg gcagcagagc cctgccgagt gagaggctgt 6120

ggggcagcgg ctctgtcctg tgccttacca gccctgggga gggggggcatt tggctggaag 6180

actggaattt aattgccatc gtctttgatt ttgtgacatt tctgcttgga agtgtgaact 6240

acccccccccc ccccgcttcc tgctccttag catgcgtgca gctctctcct gttttgggtg 6300

ttccccttgg acactccagc tcggggactg ctggcgtgtg aatgtgcaga ttcccctgtg 6360

tggtcgaacc taagaactgt ggcttggaag tgatgctcca tgtgacgacg actttgcttt 6420

ctttcctctt agtgaggagg tgattcgtag atcccaactg cctatgtaat gtaaataatg 6480

tacatttaat ttattgctat ggtagcacat tgtatttgtt aatgtacaaa acaaattcta 6540

aaaggttgac aaatgtatat tttgttgctt aaatgtgtct ttgcagaaat tgacaataaa 6600

taacatattt tgtgtcca 6618

<210> 31
<211> 6719
<212> DNA
<213> Homo sapiens

<400> 31
gtgcgctggg gccggccggg gtgcgcgcgt gcgcggcagg actgctgcga gggaccccgc 60

cgccccggag gaggaggagg aggaggagga ggaggaggag ggggctgggc cgagggaggg 120

ggcgaccgcg gggactgagg tgcccttcgc tccgggtcca gaggcagccg cgcgccaggc 180

ggcgcagaag gatcgacaag accatgctgg caagtctgaa ggtcaagaag caggagctgg 240

ccaacagctc ggatgcgacc ctcccagacc ggccgctctc ccctcctctc acggcacctc 300
ccaccatgaa gtcgtcggag ttctttgaga tgctggagaa aatgcagggg atcaagcttg 360
aagagcagaa gccgggaccc cagaagaaca aggacgacta tatcccatac cccagcatcg 420
acgaggttgt ggagaaggga ggcccgtacc ctcaggtcat cctgccacag tttgggggct 480
attggatcga ggacccggag aacgtgggca ccccaacatc gctggggagc agcatctgtg 540
aggaggagga agaggacaac ctcagcccca acacatttgg ctacaagctc gagtgcaagg 600
gtgaagccag ggcctaccgg aggcacttcc tggggaagga tcatctaaac ttttactgta 660
ccggcagcag cctggggaac ttgatcctgt ccgtcaagtg cgaggaagca gaggggatcg 720
agtacctccg ggtcatcctt aggtccaaac tgaagacggt acatgagcgg atccccttgg 780
ctggactgag caagcttccc agtgtccctc agattgcaaa ggctttctgt gatgatgcag 840
tgggactgag attcaatcct gtcctgtacc ccaaggcctc ccaaatgatt gtgtcctatg 900
atgagcatga agtcaacaac acattcaaat tcggagtcat ttatcaaaaa gccaggcaga 960
ccctggagga ggagctattt gggaacaatg aggagagccc agcttttaag gagttcttgg 1020
acctgctggg ggacacgatc acactgcagg atttcaaagg tttccgagga ggcctggacg 1080
tgacccacgg acagacaggg gtggaatcag tgtacacaac attccgggac agggagatca 1140
tgtttcacgt ttccacaaag ctgccattta ccgacggaga cgcccagcag ctccagagaa 1200
agagacacat tggaaatgac atcgtggcca tcatcttcca agaggaaaac acgccgtttg 1260
tcccagacat gatagcctcc aatttcttac atgcctacat cgtcgtgcag gtcgagaccc 1320
caggcacaga gaccccatcc tacaaggtct ctgtcactgc gcgggaagat gtgcccacct 1380
ttggtccacc tctgcccagt ccccccgttt tccagaaggg cccggaattc agggagtttc 1440
tgctcaccaa gctcaccaat gccgagaacg cctgctgcaa gtcggacaag tttgcaaagc 1500
tggaggaccg gaccagggct gccctcctgg acaaccttca cgatgagctc cacgcccaca 1560
cacaggccat gctgggactg ggcccagagg aggacaagtt tgagaatgga ggccacgggg 1620
ggttcctgga gtcttttaag agggccatcc gcgtacgcag ccactccatg gagaccatgg 1680
tgggcggcca gaagaagtcg cacagtgggg gcatccctgg cagcctcagc gggggcatct 1740
cccacaacag catggaggtc accaagacca ccttctcgcc tccagtggtg gcggcaacgg 1800
tgaagaacca gtcacggagt cccatcaagc gacgctcggg gctcttcccc cgcctgcaca 1860
cgggctcaga aggccagggc gacagccggg cacgatgtga cagcacatcc agcacaccca 1920
agaccccaga tggtggacac tcctctcagg agataaagtc tgagacctca tccaatccca 1980
gctctccgga aatctgcccc aacaaggaga agcccttcat gaagttgaag gaaaacggcc 2040
gtgccatctc ccgctcctcc tccagcacca gcagcgtcag cagcactgca ggggagggcg 2100
aggccatgga ggagggcgac agtgggggca gccagccgtc cacgacctca cccttcaagc 2160

aggaggtgtt tgtctacagc ccgtccccga gcagcgagag ccccagcctg ggggcagctg 2220
ccacccccgat catcatgagc cggagtccca cagatgccaa aagcagaaac tccccgagat 2280
cgaacctgaa attccgcttt gacaagctca gccatgccag ctctggtgcg ggtcactaat 2340
gtgaaagtgg agtccttcgc ctgtccaagg gaatcccctc ttctgtcctg gaaaaggctc 2400
ctgtaccagc agtttgggag tgccgtccac gaccctgaca gtcccagccc tgctgcccca 2460
tggccacgtg cccacagatg tgctgttggt ccaggtgtcc cagtctggcc acagccctgc 2520
ctccgccctc acctacatgc cctcccagcc cctcccatct ctggacgagg cctccttcct 2580
caggttcctc ctgctcctga cctcccagtg tgatgtccgg gtcctttatc atcctattca 2640
tcctggagag gaaaagtgtc gggcaaaggg ggatctgggg ggagctcagc agtgactggg 2700
gagctggtct gcctcagaga cagagtaggg ggtgggagca gagcctcggt gagggtcttg 2760
gccacagggc agtgccttcc tgaacgtggc aggctttact accaggaacg cactcggtgg 2820
tggaggcccc atgttcccag gagccaagat tcgtagcatc cttgaggcca tcctgataaa 2880
attcggcgct attgcccccg tagctctgga gctctaaacc gtctatctgc ttctgtgctg 2940
aacgcctttc ccatctgctg acgtaggccc agggctgccc tgcccctgct gccagtgtac 3000
cgtgagcggg gctccagcca gttcaagctc agagccagag ctggacgggc cagaactgcg 3060
ctgcacactt cctggactga ggcggggact ttgggtccca cccggtttct cctgattatg 3120
gctgctgtgg ggtgagggga gggaggggca gccccgaggc agtctcttcc ctttgagaag 3180
atattttccc acaaaggggt gggaagccag gagtgagaag gaattcaggg agagcaaagg 3240
agccagtgct gagatgctgc tgtgttggtt gaggaaaacc tcgggcctga gggccaggcc 3300
ggagcccagg tctctgctga caatgggggt tcaaggaaga cgtcgttatc tcccctcccc 3360
acttactcga ggagagaggt gagggggga tgacttgcgg gttctgatca ggcccctggg 3420
tggggaaggg gcacagtgtc cctcagcagc ttacgcccct ggagtcttgg ggggcccagc 3480
ctggccctgg ggcctttcc agctactgtg cccttgggca gctgcgtctg gggctcaacc 3540
ccccaatcct gttcccctct ccagctgcgg gtctgtaggc agctgtcaca tctgaagggt 3600
ttctgcaacc tggaccccat ctgggtgtgg gtcagaccct gtgacccaca tgccaccccc 3660
accctccaca gagcccctt gctgggacag ccagctcacc tccaaggaca tcccctcctg 3720
gcttctcccc cttccgagtc tgcagcgccg tgggcttctc tgccgatggg cccgggttgg 3780
ggttaaggtg ggcatcctcc aggtacaacg agcctgaaga gcccctttca gtgcagacgg 3840
ggctgcagag tgacactggc tgggcacctg ccccacgacc aatgacaagg atttccagct 3900
gaatgcttta ttcccatagg gatctggacc tgtgcccaag atataaatac tacacttttt 3960
tttttttttt ttaactgaca ttgtgaaatt ctccctatag cttttgccat tcaagcaaca 4020

ttgtgatctt tcttccccgc cacgtgtgtg ggaatgattg agtcctgttt gcaagctgga 4080
gaggagctct ccctttgcta gtactttctc taaagtacta gtctagtaaa atttattctt 4140
gttagaaggt caacaaaata tctgtttagc ttttatgaag agtcaccgta gcagccccca 4200
cggctggaaa gaggcctgta cgttctggac gcgttttgtt ggctgggctt ctggaggcac 4260
tggcaaggtc aaactgcatt tctttaagaa cagttgcagg atctggctcg cctctgtggg 4320
aagccggcat tacaggtgct tggtggatgg gccgtgtcac attgccatct ggggtccttt 4380
ggggtttcca ggttgtcacc atgctgtccc atttgggaat cccatacctg cctgtcccca 4440
ctgcgctggc tgacccttgc tgcctgctgc ctcttgggag ggctttgtcc ctgcctctga 4500
gctggtgggc aggatggctg ggtggccccc agagaagcac agacctgaga tggggtctcc 4560
atgcccggtt tgctgttgga atgatctgaa caggacccca aatgcctctt ccctctggtc 4620
atgcctcact atctctagga gctccatcct gtggcttcca gagtgtgcac ttccagccca 4680
cccgggcagt gctgagaggg aggaggagaa caaggatggc ccagcctccc ctccctcccc 4740
tagaccacgg ggcgggcagc tcgggttcct ggagggctgt ttcccccacg ctgtccctac 4800
atctgctctg atctaaaatg tctttccttt tatgctgcgc ccagtcttgg ggctcaaaga 4860
tttgcccaaa cctcattggc cctcgtgact aggctcatct agatggtgct cacgctggtg 4920
tttgaggcat ttccactgtg atctccacga ggggatgttt tccgggacac atctctggct 4980
ctgggaactg cctgactcac tgaagaaact acttttcagg cactgtaggg tcacccatat 5040
gcctccagct cagttgacgc ttaaaaacag gtgcagaaaa gctcgcgatg gaaggtctta 5100
atgagagtgt ctgtctatgc caatcatgta aaatgacgtt tcttgaaaaa gattcagtgg 5160
ttcagctttg tcagcatcat ctcaacacaa gcctgctggc tctttttagc atctcatcca 5220
accatgtcat cgtccagatg agaaatctta gcccaggtga ggggagtaac ttgcttgagg 5280
tcacacagct ggctgttggc aaagctggga ttagaaccct caacccaggg tcccttcctc 5340
tgcagcgcct acatggttgg ttgaataagt ggctgcgttt cctggggccc tgggttttgg 5400
ggaagccagt tagctgctgc tttggcactg gcatggaggt gagcagtcaa ggatgctggt 5460
gaggctgcag tttctgctct ttttcatcag gggggatagt ctctaggatt tttcagtgag 5520
gacccttggg ctttggatgc agcttgaacc aagaaaacga ggagggaaag ggattcagtg 5580
aactattcct cagtgggatc ggttcttcag ctcctgatgg gggctgtgta atgggggcag 5640
aggccaggga aaaagatgct gttcacccac cctcagcttc ccttttccta aattaagagg 5700
aaaagtggtc aaagaaaaac tcttcatttc tccctgattc ttaaacgaag gtggttaata 5760
gaaactcagg ctcccgtgac aaggcaggac aagagcctgt ttcgctttcc tccctgaccc 5820
tgccaggtgc caactcaaac actacctttc tcattggttt ctaagtcagt agagacagat 5880
ctgttttaag cagttggggg ttcgagtaga tctcatgggt acaggaggcc agcagggacc 5940

```
aggccagtca gccatgctca ggacccctcg gctcctcccc cagcctctag ctaccctgta      6000
tcgaggcaag gggaggccag taaagtttgc caagcctgat cctgcagcct ggtggggctg      6060
gctggggtat tcttttacca aactctgttt taccgccagc cccttgtaca ccccaatccc      6120
atgtctccct cccttcagct ggaccgtgtg ccccttttggg aggaagaaga caagccccac      6180
tagggccaag ggcagcagag ccctgccgag tgagaggctg tggggcagcg gctctgtcct      6240
gtgccttacc agccctgggg aggggggcat ttggctggaa gactggaatt taattgccat      6300
cgtctttgat tttgtgacat ttctgcttgg aagtgtgaac tacccccccc cccccgcttc      6360
ctgctcctta gcatgcgtgc agctctctcc tgttttgggt gttccccttg gacactccag      6420
ctcggggact gctggcgtgt gaatgtgcag attcccctgt gtggtcgaac ctaagaactg      6480
tggcttggaa gtgatgctcc atgtgacgac gactttgctt tctttcctct tagtgaggag      6540
gtgattcgta gatcccaact gcctatgtaa tgtaaataat gtacatttaa tttattgcta      6600
tggtagcaca ttgtatttgt taatgtacaa aacaaattct aaaaggttga caaatgtata      6660
ttttgttgct taaatgtgtc tttgcagaaa ttgacaataa ataacatatt ttgtgtcca       6719
```

<210> 32
<211> 730
<212> PRT
<213> Homo sapiens

<400> 32

```
Met Phe Gly Arg Lys Arg Ser Val Ser Phe Gly Gly Phe Gly Trp Ile
1               5                   10                  15

Asp Lys Thr Met Leu Ala Ser Leu Lys Val Lys Lys Gln Glu Leu Ala
            20                  25                  30

Asn Ser Ser Asp Ala Thr Leu Pro Asp Arg Pro Leu Ser Pro Pro Leu
        35                  40                  45

Thr Ala Pro Pro Thr Met Lys Ser Ser Glu Phe Phe Glu Met Leu Glu
    50                  55                  60

Lys Met Gln Gly Ile Lys Leu Glu Glu Gln Lys Pro Gly Pro Gln Lys
65                  70                  75                  80

Asn Lys Asp Asp Tyr Ile Pro Tyr Pro Ser Ile Asp Glu Val Val Glu
                85                  90                  95

Lys Gly Gly Pro Tyr Pro Gln Val Ile Leu Pro Gln Phe Gly Gly Tyr
            100                 105                 110
```

```
Trp Ile Glu Asp Pro Glu Asn Val Gly Thr Pro Thr Ser Leu Gly Ser
        115                 120                 125

Ser Ile Cys Glu Glu Glu Glu Glu Asp Asn Leu Ser Pro Asn Thr Phe
    130                 135                 140

Gly Tyr Lys Leu Glu Cys Lys Gly Glu Ala Arg Ala Tyr Arg Arg His
145                 150                 155                 160

Phe Leu Gly Lys Asp His Leu Asn Phe Tyr Cys Thr Gly Ser Ser Leu
                165                 170                 175

Gly Asn Leu Ile Leu Ser Val Lys Cys Glu Glu Ala Glu Gly Ile Glu
            180                 185                 190

Tyr Leu Arg Val Ile Leu Arg Ser Lys Leu Lys Thr Val His Glu Arg
        195                 200                 205

Ile Pro Leu Ala Gly Leu Ser Lys Leu Pro Ser Val Pro Gln Ile Ala
    210                 215                 220

Lys Ala Phe Cys Asp Asp Ala Val Gly Leu Arg Phe Asn Pro Val Leu
225                 230                 235                 240

Tyr Pro Lys Ala Ser Gln Met Ile Val Ser Tyr Asp Glu His Glu Val
                245                 250                 255

Asn Asn Thr Phe Lys Phe Gly Val Ile Tyr Gln Lys Ala Arg Gln Thr
            260                 265                 270

Leu Glu Glu Glu Leu Phe Gly Asn Asn Glu Glu Ser Pro Ala Phe Lys
        275                 280                 285

Glu Phe Leu Asp Leu Leu Gly Asp Thr Ile Thr Leu Gln Asp Phe Lys
    290                 295                 300

Gly Phe Arg Gly Gly Leu Asp Val Thr His Gly Gln Thr Gly Val Glu
305                 310                 315                 320

Ser Val Tyr Thr Thr Phe Arg Asp Arg Glu Ile Met Phe His Val Ser
                325                 330                 335

Thr Lys Leu Pro Phe Thr Asp Gly Asp Ala Gln Gln Leu Gln Arg Lys
            340                 345                 350

Arg His Ile Gly Asn Asp Ile Val Ala Ile Ile Phe Gln Glu Glu Asn
        355                 360                 365
```

```
Thr Pro Phe Val Pro Asp Met Ile Ala Ser Asn Phe Leu His Ala Tyr
    370                 375                 380

Ile Val Val Gln Val Glu Thr Pro Gly Thr Glu Thr Pro Ser Tyr Lys
385                 390                 395                 400

Val Ser Val Thr Ala Arg Glu Asp Val Pro Thr Phe Gly Pro Pro Leu
                405                 410                 415

Pro Ser Pro Pro Val Phe Gln Lys Gly Pro Glu Phe Arg Glu Phe Leu
            420                 425                 430

Leu Thr Lys Leu Thr Asn Ala Glu Asn Ala Cys Cys Lys Ser Asp Lys
        435                 440                 445

Phe Ala Lys Leu Glu Asp Arg Thr Arg Ala Ala Leu Leu Asp Asn Leu
    450                 455                 460

His Asp Glu Leu His Ala His Thr Gln Ala Met Leu Gly Leu Gly Pro
465                 470                 475                 480

Glu Glu Asp Lys Phe Glu Asn Gly Gly His Gly Gly Phe Leu Glu Ser
                485                 490                 495

Phe Lys Arg Ala Ile Arg Val Arg Ser His Ser Met Glu Thr Met Val
            500                 505                 510

Gly Gly Gln Lys Lys Ser His Ser Gly Gly Ile Pro Gly Ser Leu Ser
        515                 520                 525

Gly Gly Ile Ser His Asn Ser Met Glu Val Thr Lys Thr Thr Phe Ser
    530                 535                 540

Pro Pro Val Val Ala Ala Thr Val Lys Asn Gln Ser Arg Ser Pro Ile
545                 550                 555                 560

Lys Arg Arg Ser Gly Leu Phe Pro Arg Leu His Thr Gly Ser Glu Gly
                565                 570                 575

Gln Gly Asp Ser Arg Ala Arg Cys Asp Ser Thr Ser Ser Thr Pro Lys
            580                 585                 590

Thr Pro Asp Gly Gly His Ser Ser Gln Glu Ile Lys Ser Glu Thr Ser
        595                 600                 605

Ser Asn Pro Ser Ser Pro Glu Ile Cys Pro Asn Lys Glu Lys Pro Phe
    610                 615                 620
```

Met Lys Leu Lys Glu Asn Gly Arg Ala Ile Ser Arg Ser Ser Ser Ser
625 630 635 640

Thr Ser Ser Val Ser Ser Thr Ala Gly Glu Gly Glu Ala Met Glu Glu
645 650 655

Gly Asp Ser Gly Gly Ser Gln Pro Ser Thr Thr Ser Pro Phe Lys Gln
660 665 670

Glu Val Phe Val Tyr Ser Pro Ser Pro Ser Ser Glu Ser Pro Ser Leu
675 680 685

Gly Ala Ala Ala Thr Pro Ile Ile Met Ser Arg Ser Pro Thr Asp Ala
690 695 700

Lys Ser Arg Asn Ser Pro Arg Ser Asn Leu Lys Phe Arg Phe Asp Lys
705 710 715 720

Leu Ser His Ala Ser Ser Gly Ala Gly His
725 730

<210> 33
<211> 715
<212> PRT
<213> Homo sapiens

<400> 33

Met Phe Gly Arg Lys Arg Ser Val Ser Phe Gly Gly Phe Gly Trp Ile
1 5 10 15

Asp Lys Thr Met Leu Ala Ser Leu Lys Val Lys Lys Gln Glu Leu Ala
20 25 30

Asn Ser Ser Asp Ala Thr Leu Pro Asp Arg Pro Leu Ser Pro Pro Leu
35 40 45

Thr Ala Pro Pro Thr Met Lys Ser Ser Glu Phe Phe Glu Met Leu Glu
50 55 60

Lys Met Gln Asp Asp Tyr Ile Pro Tyr Pro Ser Ile Asp Glu Val Val
65 70 75 80

Glu Lys Gly Gly Pro Tyr Pro Gln Val Ile Leu Pro Gln Phe Gly Gly
85 90 95

Tyr Trp Ile Glu Asp Pro Glu Asn Val Gly Thr Pro Thr Ser Leu Gly
100 105 110

Ser Ser Ile Cys Glu Glu Glu Glu Glu Asp Asn Leu Ser Pro Asn Thr
115 120 125

Phe Gly Tyr Lys Leu Glu Cys Lys Gly Glu Ala Arg Ala Tyr Arg Arg
130 135 140

His Phe Leu Gly Lys Asp His Leu Asn Phe Tyr Cys Thr Gly Ser Ser
145 150 155 160

Leu Gly Asn Leu Ile Leu Ser Val Lys Cys Glu Glu Ala Glu Gly Ile
165 170 175

Glu Tyr Leu Arg Val Ile Leu Arg Ser Lys Leu Lys Thr Val His Glu
180 185 190

Arg Ile Pro Leu Ala Gly Leu Ser Lys Leu Pro Ser Val Pro Gln Ile
195 200 205

Ala Lys Ala Phe Cys Asp Asp Ala Val Gly Leu Arg Phe Asn Pro Val
210 215 220

Leu Tyr Pro Lys Ala Ser Gln Met Ile Val Ser Tyr Asp Glu His Glu
225 230 235 240

Val Asn Asn Thr Phe Lys Phe Gly Val Ile Tyr Gln Lys Ala Arg Gln
245 250 255

Thr Leu Glu Glu Glu Leu Phe Gly Asn Asn Glu Glu Ser Pro Ala Phe
260 265 270

Lys Glu Phe Leu Asp Leu Leu Gly Asp Thr Ile Thr Leu Gln Asp Phe
275 280 285

Lys Gly Phe Arg Gly Gly Leu Asp Val Thr His Gly Gln Thr Gly Val
290 295 300

Glu Ser Val Tyr Thr Thr Phe Arg Asp Arg Glu Ile Met Phe His Val
305 310 315 320

Ser Thr Lys Leu Pro Phe Thr Asp Gly Asp Ala Gln Gln Leu Gln Arg
325 330 335

Lys Arg His Ile Gly Asn Asp Ile Val Ala Ile Ile Phe Gln Glu Glu
340 345 350

Asn Thr Pro Phe Val Pro Asp Met Ile Ala Ser Asn Phe Leu His Ala

```
        355                 360                 365

Tyr Ile Val Val Gln Val Glu Thr Pro Gly Thr Glu Thr Pro Ser Tyr
    370                 375                 380

Lys Val Ser Val Thr Ala Arg Glu Asp Val Pro Thr Phe Gly Pro Pro
385                 390                 395                 400

Leu Pro Ser Pro Pro Val Phe Gln Lys Gly Pro Glu Phe Arg Glu Phe
                405                 410                 415

Leu Leu Thr Lys Leu Thr Asn Ala Glu Asn Ala Cys Cys Lys Ser Asp
            420                 425                 430

Lys Phe Ala Lys Leu Glu Asp Arg Thr Arg Ala Ala Leu Leu Asp Asn
        435                 440                 445

Leu His Asp Glu Leu His Ala His Thr Gln Ala Met Leu Gly Leu Gly
    450                 455                 460

Pro Glu Glu Asp Lys Phe Glu Asn Gly Gly His Gly Gly Phe Leu Glu
465                 470                 475                 480

Ser Phe Lys Arg Ala Ile Arg Val Arg Ser His Ser Met Glu Thr Met
                485                 490                 495

Val Gly Gly Gln Lys Lys Ser His Ser Gly Gly Ile Pro Gly Ser Leu
            500                 505                 510

Ser Gly Gly Ile Ser His Asn Ser Met Glu Val Thr Lys Thr Thr Phe
        515                 520                 525

Ser Pro Pro Val Val Ala Ala Thr Val Lys Asn Gln Ser Arg Ser Pro
    530                 535                 540

Ile Lys Arg Arg Ser Gly Leu Phe Pro Arg Leu His Thr Gly Ser Glu
545                 550                 555                 560

Gly Gln Gly Asp Ser Arg Ala Arg Cys Asp Ser Thr Ser Ser Thr Pro
                565                 570                 575

Lys Thr Pro Asp Gly Gly His Ser Ser Gln Glu Ile Lys Ser Glu Thr
            580                 585                 590

Ser Ser Asn Pro Ser Ser Pro Glu Ile Cys Pro Asn Lys Glu Lys Pro
        595                 600                 605
```

Phe Met Lys Leu Lys Glu Asn Gly Arg Ala Ile Ser Arg Ser Ser Ser
610 615 620

Ser Thr Ser Ser Val Ser Ser Thr Ala Gly Glu Gly Glu Ala Met Glu
625 630 635 640

Glu Gly Asp Ser Gly Gly Ser Gln Pro Ser Thr Thr Ser Pro Phe Lys
645 650 655

Gln Glu Val Phe Val Tyr Ser Pro Ser Pro Ser Ser Glu Ser Pro Ser
660 665 670

Leu Gly Ala Ala Ala Thr Pro Ile Ile Met Ser Arg Ser Pro Thr Asp
675 680 685

Ala Lys Ser Arg Asn Ser Pro Arg Ser Asn Leu Lys Phe Arg Phe Asp
690 695 700

Lys Leu Ser His Ala Ser Ser Gly Ala Gly His
705 710 715

<210> 34
<211> 711
<212> PRT
<213> Homo sapiens

<400> 34

Met Leu Ala Ser Leu Lys Val Lys Lys Gln Glu Leu Ala Asn Ser Ser
1 5 10 15

Asp Ala Thr Leu Pro Asp Arg Pro Leu Ser Pro Pro Leu Thr Ala Pro
20 25 30

Pro Thr Met Lys Ser Ser Glu Phe Phe Glu Met Leu Glu Lys Met Gln
35 40 45

Gly Ile Lys Leu Glu Glu Gln Lys Pro Gly Pro Gln Lys Asn Lys Asp
50 55 60

Asp Tyr Ile Pro Tyr Pro Ser Ile Asp Glu Val Val Glu Lys Gly Gly
65 70 75 80

Pro Tyr Pro Gln Val Ile Leu Pro Gln Phe Gly Gly Tyr Trp Ile Glu
85 90 95

Asp Pro Glu Asn Val Gly Thr Pro Thr Ser Leu Gly Ser Ser Ile Cys
100 105 110

Glu Glu Glu Glu Glu Asp Asn Leu Ser Pro Asn Thr Phe Gly Tyr Lys
115 120 125

Leu Glu Cys Lys Gly Glu Ala Arg Ala Tyr Arg Arg His Phe Leu Gly
130 135 140

Lys Asp His Leu Asn Phe Tyr Cys Thr Gly Ser Ser Leu Gly Asn Leu
145 150 155 160

Ile Leu Ser Val Lys Cys Glu Glu Ala Glu Gly Ile Glu Tyr Leu Arg
165 170 175

Val Ile Leu Arg Ser Lys Leu Lys Thr Val His Glu Arg Ile Pro Leu
180 185 190

Ala Gly Leu Ser Lys Leu Pro Ser Val Pro Gln Ile Ala Lys Ala Phe
195 200 205

Cys Asp Asp Ala Val Gly Leu Arg Phe Asn Pro Val Leu Tyr Pro Lys
210 215 220

Ala Ser Gln Met Ile Val Ser Tyr Asp Glu His Glu Val Asn Asn Thr
225 230 235 240

Phe Lys Phe Gly Val Ile Tyr Gln Lys Ala Arg Gln Thr Leu Glu Glu
245 250 255

Glu Leu Phe Gly Asn Asn Glu Glu Ser Pro Ala Phe Lys Glu Phe Leu
260 265 270

Asp Leu Leu Gly Asp Thr Ile Thr Leu Gln Asp Phe Lys Gly Phe Arg
275 280 285

Gly Gly Leu Asp Val Thr His Gly Gln Thr Gly Val Glu Ser Val Tyr
290 295 300

Thr Thr Phe Arg Asp Arg Glu Ile Met Phe His Val Ser Thr Lys Leu
305 310 315 320

Pro Phe Thr Asp Gly Asp Ala Gln Gln Leu Gln Arg Lys Arg His Ile
325 330 335

Gly Asn Asp Ile Val Ala Ile Ile Phe Gln Glu Glu Asn Thr Pro Phe
340 345 350

Val Pro Asp Met Ile Ala Ser Asn Phe Leu His Ala Tyr Ile Val Val
355 360 365

Gln Val Glu Thr Pro Gly Thr Glu Thr Pro Ser Tyr Lys Val Ser Val
370 375 380

Thr Ala Arg Glu Asp Val Pro Thr Phe Gly Pro Pro Leu Pro Ser Pro
385 390 395 400

Pro Val Phe Gln Lys Gly Pro Glu Phe Arg Glu Phe Leu Leu Thr Lys
405 410 415

Leu Thr Asn Ala Glu Asn Ala Cys Cys Lys Ser Asp Lys Phe Ala Lys
420 425 430

Leu Glu Asp Arg Thr Arg Ala Ala Leu Leu Asp Asn Leu His Asp Glu
435 440 445

Leu His Ala His Thr Gln Ala Met Leu Gly Leu Gly Pro Glu Glu Asp
450 455 460

Lys Phe Glu Asn Gly Gly His Gly Gly Phe Leu Glu Ser Phe Lys Arg
465 470 475 480

Ala Ile Arg Val Arg Ser His Ser Met Glu Thr Met Val Gly Gly Gln
485 490 495

Lys Lys Ser His Ser Gly Gly Ile Pro Gly Ser Leu Ser Gly Gly Ile
500 505 510

Ser His Asn Ser Met Glu Val Thr Lys Thr Thr Phe Ser Pro Pro Val
515 520 525

Val Ala Ala Thr Val Lys Asn Gln Ser Arg Ser Pro Ile Lys Arg Arg
530 535 540

Ser Gly Leu Phe Pro Arg Leu His Thr Gly Ser Glu Gly Gln Gly Asp
545 550 555 560

Ser Arg Ala Arg Cys Asp Ser Thr Ser Ser Thr Pro Lys Thr Pro Asp
565 570 575

Gly Gly His Ser Ser Gln Glu Ile Lys Ser Glu Thr Ser Ser Asn Pro
580 585 590

Ser Ser Pro Glu Ile Cys Pro Asn Lys Glu Lys Pro Phe Met Lys Leu
595 600 605

Lys Glu Asn Gly Arg Ala Ile Ser Arg Ser Ser Ser Ser Thr Ser Ser
610 615 620

Val Ser Ser Thr Ala Gly Glu Gly Glu Ala Met Glu Glu Gly Asp Ser
625 630 635 640

Gly Gly Ser Gln Pro Ser Thr Thr Ser Pro Phe Lys Gln Glu Val Phe
645 650 655

Val Tyr Ser Pro Ser Pro Ser Ser Glu Ser Pro Ser Leu Gly Ala Ala
660 665 670

Ala Thr Pro Ile Ile Met Ser Arg Ser Pro Thr Asp Ala Lys Ser Arg
675 680 685

Asn Ser Pro Arg Ser Asn Leu Lys Phe Arg Phe Asp Lys Leu Ser His
690 695 700

Ala Ser Ser Gly Ala Gly His
705 710

<210> 35
<211> 2732
<212> DNA
<213> Homo sapiens

<400> 35
gactgcggga gtggagccgg cgagagagtg gcagcggggg ctgatggaag tgcagtgggg 60
gctggagagg gcaccctact gtatccagca tgctccaagg ccacagctct gtgttccagg 120
ccttgctggg gaccttcttc acctggggga tgacagcagc tggggcagct ctcgtgttcg 180
tattctctag tggacagagg cggatcttag atggaagtct tggctttgct gcaggggtca 240
tgttggcagc ttcctattgg tctcttctgg ccccagcagt tgagatggcc acgtcctctg 300
ggggcttcgg tgcctttgcc ttcttccctg tggctgttgg cttcaccctt ggagcggctt 360
ttgtctactt ggctgacctc ctgatgcctc acttgggtgc agcagaagac ccccagacga 420
ccctggcact gaacttcggc tctacgttga tgaagaagaa gtctgatcct gagggtcccg 480
cgctgctctt ccctgagagt gaactttcca tccggataga caagagtgag aatggtgagg 540
catatcagag aaagaaggcg gcagccactg gccttccaga gggtcctgct gtccctgtgc 600
cttctcgagg gaatctggca cagcccggcg gcagcagctg gaggaggatc gcactgctca 660
tcttggccat cactatacac aacgttccag agggtctcgc tgttggagtt ggatttgggg 720
ctatagaaaa gacggcatct gctacctttg agagtgccag gaatttggcc attggaatcg 780
ggatccagaa tttccccgag ggcctggctg tcagccttcc cttgcgaggg gcaggcttct 840
ccacctggag agctttctgg tatgggcagc tgagcggcat ggtggagccc ctggccgggg 900
tctttggtgc ctttgccgtg gtgctggctg agcccatcct gccctacgct ctggcctttg 960

ctgccggtgc catggtctac gtggtcatgg acgacatcat ccccgaagcc cagatcagtg 1020

gtaatgggaa actggcatcc tgggcctcca tcctgggatt tgtagtgatg atgtcactgg 1080

acgttggcct gggctagggc tgagacgctt cggaccccgg gaaaggccat acgaagaaac 1140

agcagtggtt ggcttctatg ggacaacaag cttctttctt cacattaaaa ctttttttcct 1200

tcctctcttc ttcatctcat tatcctgatt gactctgatt ataatagaac catttttact 1260

ttgctttgag ggagattttt gatttaatgg ggaattttaa ggtgtcatgg aaatacagat 1320

tctttgtttt ggccactgaa tggactctct cttcagtggg attatcaagg aacttcagat 1380

cagggaaatc tccacttcgg gaccttctat ctgcctccca actcctcaag gtcacctata 1440

gaagcgagct accaaaagac gtctcctaag cattttggtg gcctagtgac tcagggcaga 1500

gtggccagca cacctctcat ccgcccctcc tgctccatca ctgctgagcc tctccccatc 1560

tagaatgttg gaactggagc atcataaaga tagcaagcta ccttccaagg ccgagccagc 1620

ccagagagga gcatgtcttc ctttacctcc ccctaaggag atactacatg ggagggggac 1680

acagaaaaag ggaaggaaat tggctagtct ggcttttttt tttttttttt ttaaaggcaa 1740

agattgacat tattgaagga aaggggatga ggacaactgt gaactcacag tgagccctgt 1800

ggaaagaaga gacagacaga gtgtgggttt gttcggaggc ctctgctgtc aatggattcc 1860

aggagcaagg ccatttgtcg cgctttccaa atttcttagg catttatttt gataagttta 1920

tagccatcat gtttctaaga gacttggaga caccagcaaa ctgctagaac tcaaactctt 1980

caattactca aagaaggagc catttcagtt aactcaagtg aatgaaagag ttttggaatc 2040

tgctgtgggt ccttccctgt tgaccatttg gtaacttata atctgacaaa aactcttgag 2100

ctgcaacagg ccttgccaga gggctcagga tgggaaagga agaaggggat aggaaaagaa 2160

gaggtaattt tacatttccc ctttaaagta aattttagcc aactcatcat tctgaaatgt 2220

ccctataaag aatgagtcga actagaccag aagccagcct actccttctt acatagcttc 2280

tccaacaggg gtagcaatga cctgtccact tcaaacacag ataaggcctg ccatcctcat 2340

tggttaaagg cacacgtgag actttcagtg ggctctgctg agaaggaagg cagcccagga 2400

gtcaggtatg caggcattgc attgtcagtg tctgctctca gagtttacac attcaattgc 2460

ttccaagggt gaatctcctg ctctgtgaat gctatcagac cccaaaggcc aaccttgggc 2520

tgggtctatg tacgttcttc cgaagcactg atgatcaaaa ttgaagacac attcagaggt 2580

ttgattggtt gagattaact ggtgtggtgg ttggtgtatg tatgttttat ttttatgtct 2640

ttgtatgtag ttctacataa tgcaaattgt gctttctgat ggacaagacc tcataactgt 2700

gattaatatc aataaaaagg ggatgttgtg ga 2732

<210> 36

<211> 2869
<212> DNA
<213> Homo sapiens

<400> 36

```
gactgcggga gtggagccgg cgagagagtg gcagcggggg ctgatggaag tgcagtgggg      60
gctggagagg gcaccctagt gagttggctt ttctctcctt cggtgccttt ggttgggctc     120
gaggcccggg gtcggaggcc atcaggacaa gagtgtggga cttggaaggg cagccacctg     180
gagcttggaa ggggctgtat ccagcatgct ccaaggccac agctctgtgt tccaggcctt     240
gctggggacc ttcttcacct gggggatgac agcagctggg gcagctctcg tgttcgtatt     300
ctctagtgga cagaggcgga tcttagatgg aagtcttggc tttgctgcag ggtcatgtt      360
ggcagcttcc tattggtctc ttctggcccc agcagttgag atggccacgt cctctggggg     420
cttcggtgcc tttgccttct tccctgtggc tgttggcttc acccttggag cggcttttgt     480
ctacttggct gacctcctga tgcctcactt gggtgcagca gaagaccccc agacgaccct     540
ggcactgaac ttcggctcta cgttgatgaa gaagaagtct gatcctgagg gtcccgcgct     600
gctcttccct gagagtgaac tttccatccg gataggtaga gctgggcttc tttcagacaa     660
gagtgagaat ggtgaggcat atcagagaaa gaaggcggca gccactggcc ttccagaggg     720
tcctgctgtc cctgtgcctt ctcgagggaa tctggcacag cccggcggca gcagctggag     780
gaggatcgca ctgctcatct tggccatcac tatacacaac gttccagagg gtctcgctgt     840
tggagttgga tttggggcta tagaaaagac ggcatctgct acctttgaga gtgccaggaa     900
tttggccatt ggaatcggga tccagaattt ccccgagggc ctggctgtca gccttccctt     960
gcgaggggca ggcttctcca cctggagagc tttctggtat gggcagctga gcggcatggt    1020
ggagcccctg gccggggtct ttggtgcctt tgccgtggtg ctggctgagc ccatcctgcc    1080
ctacgctctg gcctttgctg ccggtgccat ggtctacgtg gtcatggacg acatcatccc    1140
cgaagcccag atcagtggta atgggaaact ggcatcctgg gcctccatcc tgggatttgt    1200
agtgatgatg tcactggacg ttggcctggg ctagggctga gacgcttcgg accccgggaa    1260
aggccatacg aagaaacagc agtggttggc ttctatggga caacaagctt ctttcttcac    1320
attaaaactt ttttccttcc tctcttcttc atctcattat cctgattgac tctgattata    1380
atagaaccat ttttactttg ctttgaggga gatttttgat ttaatgggga attttaaggt    1440
gtcatggaaa tacagattct ttgttttggc cactgaatgg actctctctt cagtgggatt    1500
atcaaggaac ttcagatcag ggaaatctcc acttcgggac cttctatctg cctcccaact    1560
cctcaaggtc acctatagaa gcgagctacc aaaagacgtc tcctaagcat tttggtggcc    1620
tagtgactca gggcagagtg gccagcacac ctctcatccg cccctcctgc tccatcactg    1680
ctgagcctct ccccatctag aatgttggaa ctggagcatc ataaagatag caagctacct    1740
```

```
tccaaggccg agccagccca gagaggagca tgtcttcctt tacctccccc taaggagata      1800

ctacatggga gggggacaca gaaaaaggga aggaaattgg ctagtctggc tttttttttt      1860

tttttttta aaggcaaaga ttgacattat tgaaggaaag gggatgagga caactgtgaa       1920

ctcacagtga gccctgtgga aagaagagac agacagagtg tgggtttgtt cggaggcctc      1980

tgctgtcaat ggattccagg agcaaggcca tttgtcgcgc tttccaaatt tcttaggcat      2040

ttattttgat aagtttatag ccatcatgtt tctaagagac ttggagacac cagcaaactg      2100

ctagaactca aactcttcaa ttactcaaag aaggagccat ttcagttaac tcaagtgaat      2160

gaaagagttt tggaatctgc tgtgggtcct tccctgttga ccatttggta acttataatc      2220

tgacaaaaac tcttgagctg caacaggcct tgccagaggg ctcaggatgg gaaaggaaga      2280

aggggatagg aaaagaagag gtaattttac atttcccctt taaagtaaat tttagccaac      2340

tcatcattct gaaatgtccc tataaagaat gagtcgaact agaccagaag ccagcctact      2400

ccttcttaca tagcttctcc aacaggggta gcaatgacct gtccacttca aacacagata      2460

aggcctgcca tcctcattgg ttaaaggcac acgtgagact ttcagtgggc tctgctgaga      2520

aggaaggcag cccaggagtc aggtatgcag gcattgcatt gtcagtgtct gctctcagag      2580

tttacacatt caattgcttc caagggtgaa tctcctgctc tgtgaatgct atcagacccc      2640

aaaggccaac cttgggctgg gtctatgtac gttcttccga agcactgatg atcaaaattg      2700

aagacacatt cagaggtttg attggttgag attaactggt gtggtggttg gtgtatgtat      2760

gttttatttt tatgtctttg tatgtagttc tacataatgc aaattgtgct ttctgatgga      2820

caagacctca taactgtgat taatatcaat aaaaagggga tgttgtgga                 2869
```

<210> 37
<211> 335
<212> PRT
<213> Homo sapiens

<400> 37

```
Met Leu Gln Gly His Ser Ser Val Phe Gln Ala Leu Leu Gly Thr Phe
1               5                   10                  15

Phe Thr Trp Gly Met Thr Ala Ala Gly Ala Ala Leu Val Phe Val Phe
            20                  25                  30

Ser Ser Gly Gln Arg Arg Ile Leu Asp Gly Ser Leu Gly Phe Ala Ala
        35                  40                  45

Gly Val Met Leu Ala Ala Ser Tyr Trp Ser Leu Leu Ala Pro Ala Val
    50                  55                  60

Glu Met Ala Thr Ser Ser Gly Gly Phe Gly Ala Phe Ala Phe Phe Pro
```

```
65                  70                  75                  80

Val Ala Val Gly Phe Thr Leu Gly Ala Ala Phe Val Tyr Leu Ala Asp
                85                  90                  95

Leu Leu Met Pro His Leu Gly Ala Ala Glu Asp Pro Gln Thr Thr Leu
            100                 105                 110

Ala Leu Asn Phe Gly Ser Thr Leu Met Lys Lys Lys Ser Asp Pro Glu
        115                 120                 125

Gly Pro Ala Leu Leu Phe Pro Glu Ser Glu Leu Ser Ile Arg Ile Asp
    130                 135                 140

Lys Ser Glu Asn Gly Glu Ala Tyr Gln Arg Lys Lys Ala Ala Ala Thr
145                 150                 155                 160

Gly Leu Pro Glu Gly Pro Ala Val Pro Val Pro Ser Arg Gly Asn Leu
                165                 170                 175

Ala Gln Pro Gly Gly Ser Ser Trp Arg Arg Ile Ala Leu Leu Ile Leu
            180                 185                 190

Ala Ile Thr Ile His Asn Val Pro Glu Gly Leu Ala Val Gly Val Gly
        195                 200                 205

Phe Gly Ala Ile Glu Lys Thr Ala Ser Ala Thr Phe Glu Ser Ala Arg
    210                 215                 220

Asn Leu Ala Ile Gly Ile Gly Ile Gln Asn Phe Pro Glu Gly Leu Ala
225                 230                 235                 240

Val Ser Leu Pro Leu Arg Gly Ala Gly Phe Ser Thr Trp Arg Ala Phe
                245                 250                 255

Trp Tyr Gly Gln Leu Ser Gly Met Val Glu Pro Leu Ala Gly Val Phe
            260                 265                 270

Gly Ala Phe Ala Val Val Leu Ala Glu Pro Ile Leu Pro Tyr Ala Leu
        275                 280                 285

Ala Phe Ala Ala Gly Ala Met Val Tyr Val Val Met Asp Asp Ile Ile
    290                 295                 300

Pro Glu Ala Gln Ile Ser Gly Asn Gly Lys Leu Ala Ser Trp Ala Ser
305                 310                 315                 320
```

```
Ile Leu Gly Phe Val Val Met Met Ser Leu Asp Val Gly Leu Gly
                325                 330                 335


<210>  38
<211>  342
<212>  PRT
<213>  Homo sapiens

<400>  38

Met Leu Gln Gly His Ser Ser Val Phe Gln Ala Leu Leu Gly Thr Phe
1               5                   10                  15


Phe Thr Trp Gly Met Thr Ala Ala Gly Ala Ala Leu Val Phe Val Phe
            20                  25                  30


Ser Ser Gly Gln Arg Arg Ile Leu Asp Gly Ser Leu Gly Phe Ala Ala
        35                  40                  45


Gly Val Met Leu Ala Ala Ser Tyr Trp Ser Leu Leu Ala Pro Ala Val
    50                  55                  60


Glu Met Ala Thr Ser Ser Gly Gly Phe Gly Ala Phe Ala Phe Phe Pro
65                  70                  75                  80


Val Ala Val Gly Phe Thr Leu Gly Ala Ala Phe Val Tyr Leu Ala Asp
                85                  90                  95


Leu Leu Met Pro His Leu Gly Ala Ala Glu Asp Pro Gln Thr Thr Leu
            100                 105                 110


Ala Leu Asn Phe Gly Ser Thr Leu Met Lys Lys Lys Ser Asp Pro Glu
        115                 120                 125


Gly Pro Ala Leu Leu Phe Pro Glu Ser Glu Leu Ser Ile Arg Ile Gly
    130                 135                 140


Arg Ala Gly Leu Leu Ser Asp Lys Ser Glu Asn Gly Glu Ala Tyr Gln
145                 150                 155                 160


Arg Lys Lys Ala Ala Ala Thr Gly Leu Pro Glu Gly Pro Ala Val Pro
                165                 170                 175


Val Pro Ser Arg Gly Asn Leu Ala Gln Pro Gly Gly Ser Ser Trp Arg
            180                 185                 190


Arg Ile Ala Leu Leu Ile Leu Ala Ile Thr Ile His Asn Val Pro Glu
        195                 200                 205
```

Gly Leu Ala Val Gly Val Gly Phe Gly Ala Ile Glu Lys Thr Ala Ser
    210                 215                 220

Ala Thr Phe Glu Ser Ala Arg Asn Leu Ala Ile Gly Ile Gly Ile Gln
225                 230                 235                 240

Asn Phe Pro Glu Gly Leu Ala Val Ser Leu Pro Leu Arg Gly Ala Gly
                245                 250                 255

Phe Ser Thr Trp Arg Ala Phe Trp Tyr Gly Gln Leu Ser Gly Met Val
            260                 265                 270

Glu Pro Leu Ala Gly Val Phe Gly Ala Phe Ala Val Val Leu Ala Glu
        275                 280                 285

Pro Ile Leu Pro Tyr Ala Leu Ala Phe Ala Ala Gly Ala Met Val Tyr
    290                 295                 300

Val Val Met Asp Asp Ile Ile Pro Glu Ala Gln Ile Ser Gly Asn Gly
305                 310                 315                 320

Lys Leu Ala Ser Trp Ala Ser Ile Leu Gly Phe Val Val Met Met Ser
                325                 330                 335

Leu Asp Val Gly Leu Gly
            340

<210> 39
<211> 4510
<212> DNA
<213> Homo sapiens

<400> 39
gctgaggcca ggagggcgca ctggggattg gaggcgaggg aagtgcaggg cgcatcccag 60

gcggcagggc tcccagcatc ggcagtcgcc atcaccgcca gaccgcagag acaggttcgg 120

atccgcggtc ctcttgcctc tttccaggcc tcgatgagtg ttaaatcgcc atttaatgtg 180

atgtcaagaa ataatttgga agcacctcct tgtaagatga cagagccatt taattttgag 240

aaaaatgaaa acaagcttcc accacatgag tctttaagaa gtcctggaac acttcctaac 300

caccctaatt tcaggctgaa aagctcagag aatggaaata aaaagaacaa tttttttgctt 360

tgtgagcaaa ccaaacaata tttggctagt caggaagaca attcagtttc ttcaaacccg 420

aatggcatca acggagaagt agttggctcc aaaggagaca ggaaaaaatt gccagcagga 480

aactcagtgt caccaccaag tgctgaaagt aattcaccac ccaaagaagt gaatattaag 540

cctggaaata atgtacgtcc tgcaaaatca aaaaaactaa acaagttggt cgagaattcc 600

ttgtccataa gtaatccagg gctcttcacc tccttaggac ctcctcttcg gtccacaact 660

tgccatcgct gtggcctatt tggatcgctg aggtgctctc agtgcaagca gacctactat 720
tgctccacag catgtcaaag aagagactgg tctgcacaca gcatcgtgtg caggcctgtt 780
cagccaaatt tccacaaact tgaaaataaa tcatctattg aaacaaagga tgtggaggta 840
aacaataaga gtgactgtcc acttggagtt actaaggaaa tagccatttg ggctgagaga 900
ataatgtttt ctgatttgag aagtctacaa ctcaagaaaa ccatggaaat aaagggtacg 960
gttaccgaat tcaaacaccc aggggacttc tacgtgcagt tatattcttc agaagtttta 1020
gaatacatga accaactctc tgccagctta aaagaaacat atgcaaatgt gcatgaaaaa 1080
gactatattc ctgttaaggg ggaagtttgt attgccaagt acactgttga tcagacctgg 1140
aacagagcaa tcatacaaaa cgttgatgtg cagcaaaaga aggcacatgt cttatatatt 1200
gattatggaa atgaagaaat aattccatta aacagaattt accacctcaa caggaacatt 1260
gacttgtttc ctccttgtgc cataaagtgc tttgtagcca atgttatccc agcagaaggg 1320
aattggagca gtgattgtat caaagctact aaaccactgt taatggagca gtactgctcc 1380
ataaagattg tcgacatctt ggaagaggaa gtggttacct ttgctgtaga agttgagctg 1440
ccaaattcag gaaaactttt agaccatgtg cttatagaaa tgggatatgg cttgaaaccc 1500
agtggacaag attctaagaa ggaaaatgca gatcaaagtg atcctgaaga tgttggaaaa 1560
atgacaactg aaaacaacat tgtcgtagac aaaagtgacc taatcccaaa agtgttaact 1620
ttgaatgtag gtgatgagtt ttgtggtgtg gttgcccaca ttcaaacacc agaagacttc 1680
ttttgtcaac aactgcaaag tggccgaaag cttgctgaac ttcaggcatc ccttagcaag 1740
tactgtgatc agttgcctcc acgctctgat ttttatccag ccattggtga tatatgttgt 1800
gctcagttct cagaggatga tcagtggtac cgtgcctctg ttttggctta cgcttctgaa 1860
gaatctgtac tggtcggata tgtagattat ggaaactttg aaatccttag tttgatgaga 1920
ctttgtccca taatcccaaa gttgttggaa ttgccaatgc aagctataaa gtgtgtacta 1980
gcaggagtaa agccatcatt aggaatttgg actccagaag ctatttgtct catgaaaaaa 2040
cttgtacaga acaaaataat cacagtgaaa gtggtggaca agttggaaaa cagttccctg 2100
gtggagctta ttgataaatc cgagacgcct catgtcagtg ttagcaaagt tctcctagat 2160
gcaggctttg ctgtgggaga acagagtatg gtgacagata aacccagtga cgtgaaagaa 2220
accagtgttc ccttgggtgt ggaaggaaaa gtaaatccat tggagtggac atgggttgaa 2280
cttggtgttg accaaacagt agatgttgtg gtctgtgtga tatatagtcc tggagaattt 2340
tattgccatg tgcttaaaga ggatgcttta aagaaactca atgatttgaa caagtcatta 2400
gcagaacact gccagcagaa gttacctaat ggtttcaagg cagagatagg acaaccttgt 2460
tgtgcttttt ttgcaggtga tggtagttgg tatcgtgctt tagtcaagga aatcttacca 2520

aatggacatg ttaaagtaca ttttgtggat tatggaaaca tcgaagaagt tactgcagat 2580

gaactccgaa tgatatcatc aacattttta aaccttccct ttcagggaat acggtgccag 2640

ttagcagata tacagtctag aaacaaacat tggtctgaag aagccataac aagattccag 2700

atgtgtgttg ctgggataaa attgcaagcc agagtggttg aagtcactga aaatgggata 2760

ggagttgaac tcaccgatct ctccacttgt tatcccagaa taattagtga tgttctgatt 2820

gatgaacatc tggttttaaa atctgcttca ccacataaag acttaccaaa tgacagactt 2880

gttaataaac atgagcttca agttcatgta cagggacttc aagctacctc ttcagctgag 2940

caatggaaga cgatagaatt gccagtggat aaaactatac aagcaaatgt attagaaatc 3000

ataagcccaa acttgtttta tgctctacca aaagggatgc cagaaaatca ggaaaagctg 3060

tgcatgttga cagctgaatt attagaatac tgcaatgctc cgaaaagtcg accaccctat 3120

agaccaagaa ttggagacgc atgctgtgcc aaatacacaa gtgatgattt ttggtatcgt 3180

gcagttgttc tggggacatc agacactgat gtggaagtgc tctatgcaga ctatggaaac 3240

attgaaaccc tgcctctttg cagagtgcaa ccaatcacct ctagccacct ggcgcttcct 3300

ttccaaatta ttagatgttc acttgaagga ttaatggaat tgaatggaag ctcttctcaa 3360

ttaataataa tgctattaaa aaatttcatg ttgaatcaga atgtaatgct ttctgtgaaa 3420

ggaattacaa agaatgtcca tacagtgtca gttgagaaat gttctgagaa tgggactgtc 3480

gatgtagctg ataagctagt gacatttggt ctggcaaaaa acatcacacc tcaaaggcag 3540

agtgctttaa atacagaaaa gatgtatagg atgaattgct gctgcacaga gttacagaaa 3600

caagttgaaa aacatgaaca tattcttctc ttcctcttaa acaattcaac caatcaaaat 3660

aaatttattg aaatgaaaaa actgttaaaa aaaacagcat ctcttggagg taaaccctta 3720

tgagacagga aacagcaaag gctagcttta ggagagaaag tacagcacct ggtgttttta 3780

tttatgagaa ccttttcttt gtccactttc tctgtaatga ccttctatcc ctccgttttt 3840

gcctgcctgc cattctccta ttaggttggt ggtttttatt ttcctctaag ttccttccac 3900

caaataaata ttacgtaaaa aattcatacc aaatcaatga gaatactggc aaggaataca 3960

tagggacttt ctgctatata tgtaactttt tattacttaa aggtaccgaa ggaaggccag 4020

gtgcagtggc tcacgcccag cactttggga ggctgaggtg ggaggatccc ttgaggccag 4080

gagttcaagg ttacagtgag ctatgatagt gccactgcac tccagcctgg gtgacagatt 4140

ttgtcttaaa aaaaaaaaaa aaaaagttga tatgagtttt attttctgtc cgtttgaaat 4200

attttgtaat attccctgca ttctctgtcg tctgcctctt ccacataatg tcctttgctt 4260

tcatgtttgt tatcttcttt ttctgttcac tcagaggtca tcaatttctt tctctccgtc 4320

cttaattgga ttatttttct tttggccttt gggcacagag tctgacctct ggaccactct 4380

aactggagaa ggaactttat gttccctctc ctgctgtgtc cacaacctta gaaatctgta 4440

gctagatttt tgttgttata gatagaattt actgtttctg aaacccaaat acagttatca 4500

gtttaaggtt 4510

<210> 40
<211> 1189
<212> PRT
<213> Homo sapiens

<400> 40

Met Ser Val Lys Ser Pro Phe Asn Val Met Ser Arg Asn Asn Leu Glu
1 5 10 15

Ala Pro Pro Cys Lys Met Thr Glu Pro Phe Asn Phe Glu Lys Asn Glu
20 25 30

Asn Lys Leu Pro Pro His Glu Ser Leu Arg Ser Pro Gly Thr Leu Pro
35 40 45

Asn His Pro Asn Phe Arg Leu Lys Ser Ser Glu Asn Gly Asn Lys Lys
50 55 60

Asn Asn Phe Leu Leu Cys Glu Gln Thr Lys Gln Tyr Leu Ala Ser Gln
65 70 75 80

Glu Asp Asn Ser Val Ser Ser Asn Pro Asn Gly Ile Asn Gly Glu Val
85 90 95

Val Gly Ser Lys Gly Asp Arg Lys Lys Leu Pro Ala Gly Asn Ser Val
100 105 110

Ser Pro Pro Ser Ala Glu Ser Asn Ser Pro Pro Lys Glu Val Asn Ile
115 120 125

Lys Pro Gly Asn Asn Val Arg Pro Ala Lys Ser Lys Lys Leu Asn Lys
130 135 140

Leu Val Glu Asn Ser Leu Ser Ile Ser Asn Pro Gly Leu Phe Thr Ser
145 150 155 160

Leu Gly Pro Pro Leu Arg Ser Thr Thr Cys His Arg Cys Gly Leu Phe
165 170 175

Gly Ser Leu Arg Cys Ser Gln Cys Lys Gln Thr Tyr Tyr Cys Ser Thr
180 185 190

Ala Cys Gln Arg Arg Asp Trp Ser Ala His Ser Ile Val Cys Arg Pro
195 200 205

Val Gln Pro Asn Phe His Lys Leu Glu Asn Lys Ser Ser Ile Glu Thr
210 215 220

Lys Asp Val Glu Val Asn Asn Lys Ser Asp Cys Pro Leu Gly Val Thr
225 230 235 240

Lys Glu Ile Ala Ile Trp Ala Glu Arg Ile Met Phe Ser Asp Leu Arg
245 250 255

Ser Leu Gln Leu Lys Lys Thr Met Glu Ile Lys Gly Thr Val Thr Glu
260 265 270

Phe Lys His Pro Gly Asp Phe Tyr Val Gln Leu Tyr Ser Ser Glu Val
275 280 285

Leu Glu Tyr Met Asn Gln Leu Ser Ala Ser Leu Lys Glu Thr Tyr Ala
290 295 300

Asn Val His Glu Lys Asp Tyr Ile Pro Val Lys Gly Glu Val Cys Ile
305 310 315 320

Ala Lys Tyr Thr Val Asp Gln Thr Trp Asn Arg Ala Ile Ile Gln Asn
325 330 335

Val Asp Val Gln Gln Lys Lys Ala His Val Leu Tyr Ile Asp Tyr Gly
340 345 350

Asn Glu Glu Ile Ile Pro Leu Asn Arg Ile Tyr His Leu Asn Arg Asn
355 360 365

Ile Asp Leu Phe Pro Pro Cys Ala Ile Lys Cys Phe Val Ala Asn Val
370 375 380

Ile Pro Ala Glu Gly Asn Trp Ser Ser Asp Cys Ile Lys Ala Thr Lys
385 390 395 400

Pro Leu Leu Met Glu Gln Tyr Cys Ser Ile Lys Ile Val Asp Ile Leu
405 410 415

Glu Glu Glu Val Val Thr Phe Ala Val Glu Val Glu Leu Pro Asn Ser
420 425 430

Gly Lys Leu Leu Asp His Val Leu Ile Glu Met Gly Tyr Gly Leu Lys
435 440 445

Pro Ser Gly Gln Asp Ser Lys Lys Glu Asn Ala Asp Gln Ser Asp Pro

450 455 460

Glu Asp Val Gly Lys Met Thr Thr Glu Asn Asn Ile Val Val Asp Lys
465 470 475 480

Ser Asp Leu Ile Pro Lys Val Leu Thr Leu Asn Val Gly Asp Glu Phe
485 490 495

Cys Gly Val Val Ala His Ile Gln Thr Pro Glu Asp Phe Phe Cys Gln
500 505 510

Gln Leu Gln Ser Gly Arg Lys Leu Ala Glu Leu Gln Ala Ser Leu Ser
515 520 525

Lys Tyr Cys Asp Gln Leu Pro Pro Arg Ser Asp Phe Tyr Pro Ala Ile
530 535 540

Gly Asp Ile Cys Cys Ala Gln Phe Ser Glu Asp Asp Gln Trp Tyr Arg
545 550 555 560

Ala Ser Val Leu Ala Tyr Ala Ser Glu Glu Ser Val Leu Val Gly Tyr
565 570 575

Val Asp Tyr Gly Asn Phe Glu Ile Leu Ser Leu Met Arg Leu Cys Pro
580 585 590

Ile Ile Pro Lys Leu Leu Glu Leu Pro Met Gln Ala Ile Lys Cys Val
595 600 605

Leu Ala Gly Val Lys Pro Ser Leu Gly Ile Trp Thr Pro Glu Ala Ile
610 615 620

Cys Leu Met Lys Lys Leu Val Gln Asn Lys Ile Ile Thr Val Lys Val
625 630 635 640

Val Asp Lys Leu Glu Asn Ser Ser Leu Val Glu Leu Ile Asp Lys Ser
645 650 655

Glu Thr Pro His Val Ser Val Ser Lys Val Leu Leu Asp Ala Gly Phe
660 665 670

Ala Val Gly Glu Gln Ser Met Val Thr Asp Lys Pro Ser Asp Val Lys
675 680 685

Glu Thr Ser Val Pro Leu Gly Val Glu Gly Lys Val Asn Pro Leu Glu
690 695 700

```
Trp Thr Trp Val Glu Leu Gly Val Asp Gln Thr Val Asp Val Val Val
705                 710                 715                 720

Cys Val Ile Tyr Ser Pro Gly Glu Phe Tyr Cys His Val Leu Lys Glu
                725                 730                 735

Asp Ala Leu Lys Lys Leu Asn Asp Leu Asn Lys Ser Leu Ala Glu His
            740                 745                 750

Cys Gln Gln Lys Leu Pro Asn Gly Phe Lys Ala Glu Ile Gly Gln Pro
        755                 760                 765

Cys Cys Ala Phe Phe Ala Gly Asp Gly Ser Trp Tyr Arg Ala Leu Val
    770                 775                 780

Lys Glu Ile Leu Pro Asn Gly His Val Lys Val His Phe Val Asp Tyr
785                 790                 795                 800

Gly Asn Ile Glu Glu Val Thr Ala Asp Glu Leu Arg Met Ile Ser Ser
                805                 810                 815

Thr Phe Leu Asn Leu Pro Phe Gln Gly Ile Arg Cys Gln Leu Ala Asp
            820                 825                 830

Ile Gln Ser Arg Asn Lys His Trp Ser Glu Glu Ala Ile Thr Arg Phe
        835                 840                 845

Gln Met Cys Val Ala Gly Ile Lys Leu Gln Ala Arg Val Val Glu Val
    850                 855                 860

Thr Glu Asn Gly Ile Gly Val Glu Leu Thr Asp Leu Ser Thr Cys Tyr
865                 870                 875                 880

Pro Arg Ile Ile Ser Asp Val Leu Ile Asp Glu His Leu Val Leu Lys
                885                 890                 895

Ser Ala Ser Pro His Lys Asp Leu Pro Asn Asp Arg Leu Val Asn Lys
            900                 905                 910

His Glu Leu Gln Val His Val Gln Gly Leu Gln Ala Thr Ser Ser Ala
        915                 920                 925

Glu Gln Trp Lys Thr Ile Glu Leu Pro Val Asp Lys Thr Ile Gln Ala
    930                 935                 940

Asn Val Leu Glu Ile Ile Ser Pro Asn Leu Phe Tyr Ala Leu Pro Lys
945                 950                 955                 960
```

Gly Met Pro Glu Asn Gln Glu Lys Leu Cys Met Leu Thr Ala Glu Leu
965 970 975

Leu Glu Tyr Cys Asn Ala Pro Lys Ser Arg Pro Pro Tyr Arg Pro Arg
980 985 990

Ile Gly Asp Ala Cys Cys Ala Lys Tyr Thr Ser Asp Asp Phe Trp Tyr
995 1000 1005

Arg Ala Val Val Leu Gly Thr Ser Asp Thr Asp Val Glu Val Leu
1010 1015 1020

Tyr Ala Asp Tyr Gly Asn Ile Glu Thr Leu Pro Leu Cys Arg Val
1025 1030 1035

Gln Pro Ile Thr Ser Ser His Leu Ala Leu Pro Phe Gln Ile Ile
1040 1045 1050

Arg Cys Ser Leu Glu Gly Leu Met Glu Leu Asn Gly Ser Ser Ser
1055 1060 1065

Gln Leu Ile Ile Met Leu Leu Lys Asn Phe Met Leu Asn Gln Asn
1070 1075 1080

Val Met Leu Ser Val Lys Gly Ile Thr Lys Asn Val His Thr Val
1085 1090 1095

Ser Val Glu Lys Cys Ser Glu Asn Gly Thr Val Asp Val Ala Asp
1100 1105 1110

Lys Leu Val Thr Phe Gly Leu Ala Lys Asn Ile Thr Pro Gln Arg
1115 1120 1125

Gln Ser Ala Leu Asn Thr Glu Lys Met Tyr Arg Met Asn Cys Cys
1130 1135 1140

Cys Thr Glu Leu Gln Lys Gln Val Glu Lys His Glu His Ile Leu
1145 1150 1155

Leu Phe Leu Leu Asn Asn Ser Thr Asn Gln Asn Lys Phe Ile Glu
1160 1165 1170

Met Lys Lys Leu Leu Lys Lys Thr Ala Ser Leu Gly Gly Lys Pro
1175 1180 1185

Leu

<210> 41
<211> 5799
<212> DNA
<213> Homo sapiens

<400> 41
agtgccggct gccgatgacc gattcacgct cccggtactg gggccccctc tgcccagcca 60

cccctaccca gagcacccgg gctgcgcggc cccgccgagg gtgcgggctt tgttcgcatt 120

gtctgcgcgc acctgagcgc ggccttcctg gcacggcggc gtcgggggaa gagcgcacct 180

ggcgcgcgcc tccctcgtgg ccactcgcgg tccgtcccgg gcgagctggc ggggttttgg 240

gaggggtgcg gtcagcagtt atatcaacat gccccctttc ctgttgctgg aagccgtctg 300

tgttttcctg ttttccagag tgcccccatc tctccctctc caggaagtcc atgtaagcaa 360

agaaaccatc gggaagattt cagctgccag caaaatgatg tggtgctcgg ctgcagtgga 420

catcatgttt ctgttagatg ggtctaacag cgtcgggaaa gggagctttg aaaggtccaa 480

gcactttgcc atcacagtct gtgacggtct ggacatcagc cccgagaggg tcagagtggg 540

agcattccag ttcagttcca ctcctcatct ggaattcccc ttggattcat tttcaaccca 600

acaggaagtg aaggcaagaa tcaagaggat ggttttcaaa ggagggcgca cggagacgga 660

acttgctctg aaataccttc tgcacagagg gttgcctgga ggcagaaatg cttctgtgcc 720

ccagatcctc atcatcgtca ctgatgggaa gtcccagggg gatgtggcac tgccatccaa 780

gcagctgaag gaaaggggtg tcactgtgtt tgctgtgggg gtcaggtttc ccaggtggga 840

ggagctgcat gcactggcca gcgagcctag agggcagcac gtgctgttgg ctgagcaggt 900

ggaggatgcc accaacggcc tcttcagcac cctcagcagc tcggccatct gctccagcgc 960

cacgccagac tgcagggtcg aggctcaccc ctgtgagcac aggacgctgg agatggtccg 1020

ggagttcgct ggcaatgccc catgctggag aggatcgcgg cggacccttg cggtgctggc 1080

tgcacactgt cccttctaca gctggaagag agtgttccta acccaccctg ccacctgcta 1140

caggaccacc tgcccaggcc cctgtgactc gcagccctgc cagaatggag gcacatgtgt 1200

tccagaagga ctggacggct accagtgcct ctgcccgctg gcctttggag gggaggctaa 1260

ctgtgccctg aagctgagcc tggaatgcag ggtcgacctc ctcttcctgc tggacagctc 1320

tgcgggcacc actctggacg gcttcctgcg ggccaaagtc ttcgtgaagc ggtttgtgcg 1380

ggccgtgctg agcgaggact ctcgggcccg agtgggtgtg gccacataca gcagggagct 1440

gctggtggcg gtgcctgtgg gggagtacca ggatgtgcct gacctggtct ggagcctcga 1500

tggcattccc ttccgtggtg gccccaccct gacgggcagt gccttgcggc aggcggcaga 1560

gcgtggcttc gggagcgcca ccaggacagg ccaggaccgg ccacgtagag tggtggtttt 1620

gctcactgag tcacactccg aggatgaggt tgcgggccca gcgcgtcacg caagggcgcg 1680

agagctgctc ctgctgggtg taggcagtga ggccgtgcgg gcagagctgg aggagatcac 1740
aggcagccca aagcatgtga tggtctactc ggatcctcag gatctgttca accaaatccc 1800
tgagctgcag ggaagctgt gcagccggca gcggccaggg tgccggacac aagccctgga 1860
cctcgtcttc atgttggaca cctctgcctc agtagggccc gagaatttg ctcagatgca 1920
gagctttgtg agaagctgtg ccctccagtt tgaggtgaac cctgacgtga cacaggtcgg 1980
cctggtggtg tatggcagcc aggtgcagac tgccttcggg ctggacacca aacccacccg 2040
ggctgcgatg ctgcgggcca ttagccaggc cccctaccta ggtggggtgg gctcagccgg 2100
caccgccctg ctgcacatct atgacaaagt gatgaccgtc cagaggggtg cccggcctgg 2160
tgtccccaaa gctgtggtgg tgctcacagg cgggagaggc gcagaggatg cagccgttcc 2220
tgcccagaag ctgaggaaca atggcatctc tgtcttggtc gtgggcgtgg ggcctgtcct 2280
aagtgagggt ctgcggaggc ttgcaggtcc ccgggattcc ctgatccacg tggcagctta 2340
cgccgacctg cggtaccacc aggacgtgct cattgagtgg ctgtgtggag aagccaagca 2400
gccagtcaac ctctgcaaac ccagcccgtg catgaatgag ggcagctgcg tcctgcagaa 2460
tgggagctac cgctgcaagt gtcgggatgg ctgggagggc cccactgcg agaaccgatt 2520
cttgagacgc ccctgaggca catggctccc gtgcaggagg gcagcagccg taccctccc 2580
agcaactaca gagaaggcct gggcactgaa atggtgccta ccttctggaa tgtctgtgcc 2640
ccaggtcctt agaatgtctg cttcccgccg tggccaggac cactattctc actgagggag 2700
gaggatgtcc caactgcagc catgctgctt agagacaaga aagcagctga tgtcacccac 2760
aaacgatgtt gttgaaaagt tttgatgtgt aagtaaatac ccactttctg tacctgctgt 2820
gccttgttga ggctatgtca tctgccacct ttcccttgag gataaacaag gggtcctgaa 2880
gacttaaatt tagcggcctg acgttccttt gcacacaatc aatgctcgcc agaatgttgt 2940
tgacacagta atgcccagca gaggccttta ctagagcatc ctttggacgg cgaaggccac 3000
ggcctttcaa gatggaaagc agcagctttt ccacttcccc agagacattc tggatgcatt 3060
tgcattgagt ctgaaagggg gcttgaggga cgtttgtgac ttctggcgac tgccttttgt 3120
gtgtggaaga gacttggaaa ggtctcagac tgaaatgtga ccaattaacc agcttgtttg 3180
atgatggggg aggggctgag tgtgcaatgg gcccaggtct ggaggggcca cgtaaaatcg 3240
ttctgagtcg tgagcagtgt ccacctgaag ggtcttcctt tcaaaagagg ctgcggccag 3300
agactgtggc tcatgcctgt aatcccagca ctttggaggc tgaggcaggt ggatcacccg 3360
aggtcaggag tttgagacca gcctggccaa cgtggtgaaa gtttgtcttt actaaaaata 3420
caaaaggtag ccgggggtgg tggtggatgc ctataatccc agctactcgg gaggctgaga 3480
caggagaatg gcttgaacct gggaggcgga agttacagtg agccgagatc tcaccactgc 3540

actccagcct gggcaacaag agtaaaaatc tgtctcaaaa agaaaaaaat gtacttagga 3600
ggggttaatt gtggcgtgtt tatggaattc tttccttatt ctccttttag tggggcaaag 3660
agaagtaaga ttcttaaact caaaaatata ggataaagaa acttacagag attttgcttt 3720
ttaaagcatt gatcttacgc tgtctaggtt ttaattttgt tttgctttgc ttttctacac 3780
agtttttaaa gaaatatttc aagaaatgtt ggttatttat taaacaggga tatttgtacc 3840
tatgtggcaa agaggcatat ttggaatatt ctctggcaaa ctagatactt acttccctat 3900
cgctgcagta tttaggaatt acttcttctc cttggttgtg ttgtttagag ttggattttc 3960
tgtagaaatc tttctagagc tctgatgtga ctccagacac tttatcgttt ttcttttttt 4020
tgagacggag ttttgctctt gttgcccagg ctagagtgca gtggtacaat cttggctcac 4080
tgcaaccttt gcctcccagg ttgaagtgat tctcatgcct cagcctcttg agtagctggg 4140
attagaggca ggtgccacca tgcctagcta atttttgtat ttttagttag agacagggtt 4200
tcaccatgtt ggccaggcta gtctcgaact tctgacctca ggtgatcccc ctgccttggc 4260
ctcccaaagt tctgggatta caggggtgaa ccactgtgcc tggcccattt ttctttataa 4320
atattgtaac ataatgtttt atagacaaac attcaagggt actttggctt taagaacttc 4380
aggatttctg gtgctagaaa agcgcttgaa gcagtatcac caagatttta gatattaaaa 4440
agtctggtgt accagacatt gagtcataat catctatatt caagggatac tttcattgat 4500
aactttgtta ttatgctgcc cttcacagaa gacaacgtcc ggggcaggat cacatgctcc 4560
ctagcagatg ctgatcagtg atgtcataga aattacatga atgcattgtc tttaaatagc 4620
agtttaacca tgttataatg taggcttttt gtcttgttcc gggctggtat ttgggtgccc 4680
tgattgaatt acttggattt aaacagcaaa ctgtgggctc tcgacttaca tagtaagggc 4740
ctttactgtt tctttgtagg aaatgggttt ctcgcctttg aaacattttt tccccttttg 4800
tagtgacagt gccactaaat agttcagctt ttgtcagtcc cccaggaaag tgctatccta 4860
tggcctaact agccaagcct tttttctttc atttaaaaga aattagcttt aatttttacc 4920
tttaattact tattcaaata agacagaaat atattttcct tgcaataatt aaaacattgc 4980
atataggcca taaatttcct tattttctct gaacgatcct gattccagtc atcttgttga 5040
ataccctagt tctaataatt gactcttgct tttctagaga aatatttcca aatgatgcta 5100
gttttgtctc ttcctttcaa agttgtatac cacttctttt tcttgtcatt ttgcattgcc 5160
tgggacctcc agaataatgt ttcatgaagt agcatgtatc catatctggt tcttgacttt 5220
ttcatcataa taattgtttt ctatgggtta cttatcagtt taagaatgct taattcctag 5280
atgaactaag agtgtttatt acatgttgag atttatggta tgctttttct tcctcaagat 5340
aatgcatttt ttgtattatc tgttaatgtg ataggttatc catttgtgta ttttcaatca 5400
ttgaacaacc cttgattttt ttggataaac tctatttggt cattatgcat cattctataa 5460

accctgctga atttttcatt tgccaacatc ttatttcaga ttcttttaat ctgtgtccaa 5520

caatgagatt tgttttcttt tgcaatttgt tttgaatttt tggtatcaga gctatactaa 5580

ccttataatg gaaaatacat atttctcaaa cttttacact gatatattca tagtattttt 5640

ttataatttg aaaaatcttg tcagtatctg tattaaggcc tccatttcag ttctgctatt 5700

tcatattgcc ttaggttgtc tatttgtctc tttaatgaac ccgattttga ttttgtcatt 5760

tttaaaataa acacatttat gctataaact tccttaatt 5799

<210> 42
<211> 755
<212> PRT
<213> Homo sapiens

<400> 42

Met Pro Pro Phe Leu Leu Leu Glu Ala Val Cys Val Phe Leu Phe Ser
1 5 10 15

Arg Val Pro Pro Ser Leu Pro Leu Gln Glu Val His Val Ser Lys Glu
20 25 30

Thr Ile Gly Lys Ile Ser Ala Ala Ser Lys Met Met Trp Cys Ser Ala
35 40 45

Ala Val Asp Ile Met Phe Leu Leu Asp Gly Ser Asn Ser Val Gly Lys
50 55 60

Gly Ser Phe Glu Arg Ser Lys His Phe Ala Ile Thr Val Cys Asp Gly
65 70 75 80

Leu Asp Ile Ser Pro Glu Arg Val Arg Val Gly Ala Phe Gln Phe Ser
85 90 95

Ser Thr Pro His Leu Glu Phe Pro Leu Asp Ser Phe Ser Thr Gln Gln
100 105 110

Glu Val Lys Ala Arg Ile Lys Arg Met Val Phe Lys Gly Gly Arg Thr
115 120 125

Glu Thr Glu Leu Ala Leu Lys Tyr Leu Leu His Arg Gly Leu Pro Gly
130 135 140

Gly Arg Asn Ala Ser Val Pro Gln Ile Leu Ile Ile Val Thr Asp Gly
145 150 155 160

Lys Ser Gln Gly Asp Val Ala Leu Pro Ser Lys Gln Leu Lys Glu Arg
165 170 175

Gly Val Thr Val Phe Ala Val Gly Val Arg Phe Pro Arg Trp Glu Glu
180 185 190

Leu His Ala Leu Ala Ser Glu Pro Arg Gly Gln His Val Leu Leu Ala
195 200 205

Glu Gln Val Glu Asp Ala Thr Asn Gly Leu Phe Ser Thr Leu Ser Ser
210 215 220

Ser Ala Ile Cys Ser Ser Ala Thr Pro Asp Cys Arg Val Glu Ala His
225 230 235 240

Pro Cys Glu His Arg Thr Leu Glu Met Val Arg Glu Phe Ala Gly Asn
245 250 255

Ala Pro Cys Trp Arg Gly Ser Arg Arg Thr Leu Ala Val Leu Ala Ala
260 265 270

His Cys Pro Phe Tyr Ser Trp Lys Arg Val Phe Leu Thr His Pro Ala
275 280 285

Thr Cys Tyr Arg Thr Thr Cys Pro Gly Pro Cys Asp Ser Gln Pro Cys
290 295 300

Gln Asn Gly Gly Thr Cys Val Pro Glu Gly Leu Asp Gly Tyr Gln Cys
305 310 315 320

Leu Cys Pro Leu Ala Phe Gly Gly Glu Ala Asn Cys Ala Leu Lys Leu
325 330 335

Ser Leu Glu Cys Arg Val Asp Leu Leu Phe Leu Leu Asp Ser Ser Ala
340 345 350

Gly Thr Thr Leu Asp Gly Phe Leu Arg Ala Lys Val Phe Val Lys Arg
355 360 365

Phe Val Arg Ala Val Leu Ser Glu Asp Ser Arg Ala Arg Val Gly Val
370 375 380

Ala Thr Tyr Ser Arg Glu Leu Leu Val Ala Val Pro Val Gly Glu Tyr
385 390 395 400

Gln Asp Val Pro Asp Leu Val Trp Ser Leu Asp Gly Ile Pro Phe Arg
405 410 415

Gly Gly Pro Thr Leu Thr Gly Ser Ala Leu Arg Gln Ala Ala Glu Arg

420 425 430

Gly Phe Gly Ser Ala Thr Arg Thr Gly Gln Asp Arg Pro Arg Arg Val
435 440 445

Val Val Leu Leu Thr Glu Ser His Ser Glu Asp Glu Val Ala Gly Pro
450 455 460

Ala Arg His Ala Arg Ala Arg Glu Leu Leu Leu Leu Gly Val Gly Ser
465 470 475 480

Glu Ala Val Arg Ala Glu Leu Glu Glu Ile Thr Gly Ser Pro Lys His
485 490 495

Val Met Val Tyr Ser Asp Pro Gln Asp Leu Phe Asn Gln Ile Pro Glu
500 505 510

Leu Gln Gly Lys Leu Cys Ser Arg Gln Arg Pro Gly Cys Arg Thr Gln
515 520 525

Ala Leu Asp Leu Val Phe Met Leu Asp Thr Ser Ala Ser Val Gly Pro
530 535 540

Glu Asn Phe Ala Gln Met Gln Ser Phe Val Arg Ser Cys Ala Leu Gln
545 550 555 560

Phe Glu Val Asn Pro Asp Val Thr Gln Val Gly Leu Val Val Tyr Gly
565 570 575

Ser Gln Val Gln Thr Ala Phe Gly Leu Asp Thr Lys Pro Thr Arg Ala
580 585 590

Ala Met Leu Arg Ala Ile Ser Gln Ala Pro Tyr Leu Gly Gly Val Gly
595 600 605

Ser Ala Gly Thr Ala Leu Leu His Ile Tyr Asp Lys Val Met Thr Val
610 615 620

Gln Arg Gly Ala Arg Pro Gly Val Pro Lys Ala Val Val Val Leu Thr
625 630 635 640

Gly Gly Arg Gly Ala Glu Asp Ala Ala Val Pro Ala Gln Lys Leu Arg
645 650 655

Asn Asn Gly Ile Ser Val Leu Val Val Gly Val Gly Pro Val Leu Ser
660 665 670

```
Glu Gly Leu Arg Arg Leu Ala Gly Pro Arg Asp Ser Leu Ile His Val
        675                 680                 685

Ala Ala Tyr Ala Asp Leu Arg Tyr His Gln Asp Val Leu Ile Glu Trp
    690                 695                 700

Leu Cys Gly Glu Ala Lys Gln Pro Val Asn Leu Cys Lys Pro Ser Pro
705                 710                 715                 720

Cys Met Asn Glu Gly Ser Cys Val Leu Gln Asn Gly Ser Tyr Arg Cys
                725                 730                 735

Lys Cys Arg Asp Gly Trp Glu Gly Pro His Cys Glu Asn Arg Phe Leu
            740                 745                 750

Arg Arg Pro
        755
```

**Description**

FIELD OF THE INVENTION

The invention relates to a method of predicting a response of a prostate cancer subject to radiotherapy, and to an apparatus for predicting a response of a prostate cancer subject to radiotherapy. Moreover, the invention relates to a diagnostic kit, to a use of the kit, to a use of the kit in a method of predicting a response of a prostate cancer subject to radiotherapy, to a use of a gene expression profile for each of one or more PDE4D7 correlated genes in a method of predicting a response of a prostate cancer subject to radiotherapy, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize. Prostate Cancer (PCa) is the second most commonly-occurring non-skin malignancy in men, with an estimated 1.3 million new cases diagnosed and 360,000 deaths world-wide in 2018 (see Bray F. et al., "Global cancer statistics 2018: GLOBOCAN estimates of incidence and mortality worldwide for 36 cancers in 185 countries", CA Cancer J Clin, Vol. 68, No. 6, pages 394-424, 2018). In the US, about 90% of the new cases concern localized cancer, meaning that metastases have not yet been formed (see ACS (American Cancer Society), "Cancer Facts & Figures 2010", 2010).

For the treatment of primary localized prostate cancer, several radical therapies are available, of which surgery (radical prostatectomy, RP) and radiation therapy (RT) are most commonly used. RT is administered via an external beam or via the implantation of radioactive seeds into the prostate (brachytherapy) or a combination of both. It is especially preferable for patients who are not eligible for surgery or have been diagnosed with a tumour in an advanced localized or regional stage. Radical RT is provided to up to 50% of patients diagnosed with localized prostate cancer in the US (see ACS, 2010, ibid).

After treatment, prostate cancer antigen (PSA) levels in the blood are measured for disease monitoring. An increase of the blood PSA level provides a biochemical surrogate measure for cancer recurrence or progression. However, the variation in reported biochemical progression-free survival (bPFS) is large (see Grimm P. et al., "Comparative analysis of prostate-specific antigen free survival outcomes for patients with low, intermediate and high risk prostate cancer treatment by radical therapy. Results from the Prostate Cancer Results Study Group", BJU Int, Suppl. 1, pages 22-29, 2012). For many patients, the bPFS at 5 or even 10 years after radical RT may lie above 90%. Unfortunately, for the group of patients at medium and especially higher risk of recurrence, the bPFS can drop to around 40% at 5 years, depending on the type of RT used (see Grimm P. et al., 2012, ibid).

A large number of the patients with primary localized prostate cancer that are not treated with RT will undergo RP (see ACS, 2010, ibid). After RP, an average of 60% of patients in the highest risk group experience biochemical recurrence after 5 and 10 years (see Grimm P. et al., 2012, ibid). In case of biochemical progression after RP, one of the main challenges is the uncertainty whether this is due to recurring localized disease, one or more metastases or even an indolent disease that will not lead to clinical disease progression (see Dal Pra A. et al., "Contemporary role of postoperative radiotherapy for prostate cancer", Transl Androl Urol, Vo. 7, No. 3, pages 399-413, 2018, and Herrera F.G. and Berthold D.R., "Radiation therapy after radical prostatectomy: Implications for clinicians", Front Oncol, Vol. 6, No. 117, 2016). RT to eradicate remaining cancer cells in the prostate bed is one of the main treatment options to salvage survival after a PSA increase following RP. The effectiveness of salvage radiotherapy (SRT) results in 5-year bPFS for 18% to 90% of patients, depending on multiple factors (see Herrera F.G. and Berthold D.R., 2016, ibid, and Pisansky T.M. et al., "Salvage radiation therapy dose response for biochemical failure of prostate cancer after prostatectomy - A multi-institutional observational study", Int J Radiat Oncol Biol Phys, Vol. 96, No. 5, pages 1046-1053, 2016).

It is clear that for certain patient groups, radical or salvage RT is not effective. Their situation is even worsened by the serious side effects that RT can cause, such as bowel inflammation and dysfunction, urinary incontinence and erectile dysfunction (see Resnick M.J. et al., "Long-term functional outcomes after treatment for localized prostate cancer", N Engl J Med, Vol. 368, No. 5, pages 436-445, 2013, and Hegarty S.E. et al., "Radiation therapy after radical prostatectomy for prostate cancer: Evaluation of complications and influence of radiation timing on outcomes in a large, population-based cohort", PLoS One, Vol. 10, No. 2, 2015). In addition, the median cost of one course of RT based on Medicare reimbursement is $ 18,000, with a wide variation up to about $ 40,000 (see Paravati A.J. et al., "Variation in the cost of radiation therapy among medicare patients with cancer", J Oncol Pract, Vol. 11, No. 5, pages 403-409, 2015). These figures do not include the considerable longitudinal costs of follow-up care after radical and salvage RT.

An improved prediction of effectiveness of RT for each patient, be it in the radical or the salvage setting, would improve therapy selection and potentially survival. This can be achieved by 1) optimizing RT for those patients where RT is predicted to be effective (e.g., by dose escalation or a different starting time) and 2) guiding patients where RT is predicted not to be effective to an alternative, potentially more effective form of treatment. Further, this would reduce suffering for those patients who would be spared ineffective therapy and would reduce costs spent on ineffective therapies.

Numerous investigations have been conducted into measures for response prediction of radical RT (see Hall W.A. et al., "Biomarkers of outcome in patients with localized prostate cancer treated with radiotherapy", Semin Radiat Oncol, Vol. 27, pages 11-20, 2016, and Raymond E. et al., "An appraisal of analytical tools used in predicting clinical outcomes following radiation therapy treatment of men with prostate cancer: A systematic review", Radiat Oncol, Vol. 12, No. 1, page 56, 2017) and SRT (see Herrera F.G. and Berthold D.R., 2016, ibid). Many of these measures depend on the concentration of the blood-based biomarker PSA. Metrics investigated for prediction of response before start of RT (radical as well as salvage) include the absolute value of the PSA concentration, its absolute value relative to the prostate volume, the absolute increase over a certain time and the doubling time. Other frequently considered factors are the Gleason score and the clinical tumour stage. For the SRT setting, additional factors are relevant, e.g., surgical margin status, time to recurrence after RP, pre-/peri-surgical PSA values and clinico-pathological parameters.

Although these clinical variables provide limited improvements in patient stratification in various risk groups, there is a need for better predictive tools.

A wide range of biomarker candidates in tissue and bodily fluids has been investigated, but validation is often limited and generally demonstrates prognostic information and not a predictive (therapy-specific) value (see Hall W.A. et al., 2016, ibid). A small number of gene expression panels is currently being validated by commercial organizations. One or a few of these may show predictive value for RT in future (see Dal Pra A. et al., 2018, ibid).

In conclusion, a strong need for better prediction of response to RT remains, for primary prostate cancer as well as for the post-surgery setting.

SUMMARY OF THE INVENTION

It is an objective of the invention to provide a method of predicting a response of a prostate cancer subject to radiotherapy, and an apparatus for predicting a response of a prostate cancer subject to radiotherapy, which allow to make better treatment decisions. It is a further objective of the invention to provide a diagnostic kit, a use of the kit, a use of the kit in a method of predicting a response of a prostate cancer subject to radiotherapy, a use of a gene expression profile for each of one or more PDE4D7 correlated genes in a method of predicting a response of a prostate cancer subject to radiotherapy, and a corresponding computer program product.

In a first aspect of the present invention, a method of predicting a response of a prostate cancer subject to radiotherapy is presented, comprising:

- determining or receiving the result of a determination of a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said gene expression profile(s) being determined in a biological sample obtained from the subject,
- determining the prediction of the radiotherapy response based on the gene expression profile(s) for the one or more PDE4D7 correlated genes, and
- optionally, providing the prediction or a therapy recommendation based on the prediction to a medical caregiver or the subject.

Phosphodiesterases (PDEs) provide the sole means for the degradation of the second messenger 3'-5'-cyclic AMP. As such they are poised to provide a key regulatory role. Thus, aberrant changes in their expression, activity and intracellular location may all contribute to the underlying molecular pathology of particular disease states. Indeed, it has recently been shown that mutations in PDE genes are enriched in prostate cancer patients leading to elevated cAMP signalling and a potential predisposition to prostate cancer. However, varied expression profiles in different cell types coupled with complex arrays of isoform variants within each PDE family makes understanding the links between aberrant changes in PDE expression and functionality during disease progression challenging. Several studies have endeavored to describe the complement of PDEs in prostate, all of which identified significant levels of PDE4 expression alongside other PDEs.

Using sequence information on currently identified PDE isoforms, we have analysed their expression in 19 prostate cancer cell lines and xenografts (see Henderson D.J. et al., "The cAMP phosphodiesterase-4D7 (PDE4D7) is downregulated in androgen-independent prostate cancer cells and mediates proliferation by compartmentalizing cAMP at the plasma membrane of VCaP prostate cancer cells", Br J Cancer, Vol. 110, No. 5, pages 1278-1287, 2014). Such studies identified PDE3B, PDE4B, PDE4D, PDE7A, PDE8A, PDE8B and PDE9A isoforms as being abundantly expressed at the mRNA level in cancerous prostate cells (see Henderson D.J. et al., 2014, ibid), while PDE1, PDE3A, PDE5A, PDE10A and PDE11A mRNA are present at lower levels (unpublished data), highlighting the complexity of cyclic nucleotide signalling in the prostate epithelium. Importantly, by separating the prostate cancer cell samples into androgen sensitive and androgen insensitive, castration resistant prostate cancer (CRPC), cellular phenotypes, we discovered that the expression of PDE4D isoforms was down-regulated in CRPC samples. In particular, we found that the most abundant PDE4 isoform in many of the androgen sensitive samples, PDE4D7, exhibited a significant degree of down-regulation in the CRPC cell models, presenting a scenario where the down-regulation of PDE4D7 could directly contribute to the exacerbation of disease driving cAMP signalling changes. Moreover, these observations suggested that measurement of PDE4D7 may inform on prostate cancer disease progression where low levels of expression may be connected with a more aggressive phenotype.

Based on the correlation between PDE4D7 expression and pathological features of the disease, our defined aim was to identify prognostic associations between the expression of PDE4D7 in a patient prostate tissue, collected by either biopsy or surgery, and clinically useful information relevant to the outcome of individual patients. Clinically relevant endpoints, or surrogate endpoints that are significantly correlated to the development of metastases, cancer specific or overall mortality have, typically, been evaluated as prognostic cancer biomarkers. The most relevant rational for using a surrogate endpoint relates to situations where either data on established clinical endpoints are not available or when the number of events in the data cohort is too limited for statistical data analysis. For the development of the PDE4D7 prognostic biomarker we evaluated either BCR (biochemical relapse) progression-free survival or start of post-surgical secondary treatment as surrogate endpoints for metastases and prostate cancer death. Using these particular endpoints we identified a relevant number of events in our clinical cohorts (e.g., >30% for BCR), which is particularly relevant for multivariable data analysis.

In our evaluation, we selected standard methods of multivariable analysis such as Cox regression and Kaplan-Meier survival analysis in order to investigate the added and independent value of the continuous and/or the categorical 'PDE4D7 score' compared to established prognostic clinical variables such as PSA and Gleason score (see Alves de Inda M. et al., "Validation of Cyclic Adenosine Monophosphate Phosphodiesterase-4D7 for its Independent Contribution to Risk Stratification in a Prostate Cancer Patient Cohort with Longitudinal Biological Outcomes", Eur Urol Focus, Vol. 4, No. 3, pages 376-384, 2018). We thus built risk models where we combined the 'PDE4D7 score' with either pre- or post-surgical clinical predictors of post-surgical progression using logistic regression. The resulting models were subsequently tested on multiple independent patient cohorts in Kaplan-Meier survival and ROC curve analysis in order to predict post-treatment progression free survival (see Alves de Inda M. et al., 2018, ibid).

Using such a strategy, we set out to test the prognostic value of the PDE4D7 score on a biopsy from retrospectively collected, resected prostate tissue in a consecutively managed patient cohort from a single surgery center in a post-surgical setting (see Alves de Inda M. et al., 2018, ibid). The patient population comprised some 500 individuals where longitudinal follow-up, of both pathology and biological outcomes, was undertaken. These clinical data were available for all patients and collected during a follow-up of a median 120 months after treatment. The 'PDE4D7 score' was determined as described above and then tested in both uni- and multivariable analyses using the available post-surgical co-variates (i.e. pathology Gleason score, pT stage, surgical margin status, seminal vesicle invasion status, and lymph node invasion status) in order to adjust for the multivariable setting. In this instance, biochemical progression-free survival after primary intervention was set as the evaluated clinical endpoint. The univariable analysis of these clinical samples (see Alves de Inda M. et al., 2018, ibid), showing the inverse association between PDE4D7 expression (in terms of 'PDE4D7 score') and post-surgical biological relapse (HR=0.53 per unit change; 95% CI 0.41-0.67; p<0.0001), robustly confirmed our previous data (see Böttcher R. et al., "Human phosphodiesterase 4D7 (PDE4D7) expression is increased in TMPRSS2-ERG-positive primary prostate cancer and independently adds to a reduced risk of post-surgical disease progression". Br J Cancer, Vo. 113, NO. 10, pages 1502-1511, 2015, and Böttcher R. et al., "Human PDE4D isoform composition is deregulated in primary prostate cancer and indicative for disease progression and development of distant metastases", Oncotarget, Vol. 7, No. 43, pages 70669-70684, 2016). In multivariable analysis with such clinical variables, the 'PDE4D7 score' remained as an independent and effective means for predicting clinical outcome (HR=0.56 per unit change; 95% CI 0.43-0.73; p<0.0001). Furthermore, we obtained a very similar outcome when we evaluated the 'PDE4D7 score' in multivariable analysis (HR=0.54 95% CI 0.42-0.69; p<0.0001) with the validated and clinically-used risk model CAPRA-S. The CAPRA-S score, which is based on pre-operative PSA and pathologic parameters determined at the time of surgery, was developed to provide clinicians with information aimed to help predict disease recurrence, including BCR, systemic progression, and PCSM and has been validated in US and other populations.

Interestingly, when assessing the hazard ratio (HR) compared to the continuous 'PDE4D7 score' we uncovered a linear increase in risk with decreasing 'PDE4D7 score' for score values lying between 2 and 5. However, at PDE4D7 scores less than 2, then the risk of post-surgical progression increases steeply (see Alves de Inda M. et al., 2018, ibid). This is also evident in the Kaplan-Meier survival curves where patients that are grouped within the lowest 'PDE4D7 scores' category exhibit the highest risk of disease recurrence. Using logistic regression analysis we then combined the CAPRA-S score with the continuous 'PDE4D7 score'. Testing this model using ROC curve analysis we noticed a 4-6% significant improvement in AUC compared to the CAPRA-S alone for both 2- and 5-year predictions of post-treatment progression to BCR. Thus we evaluated a combined CAPRA-S & 'PDE4D7 score' Cox regression combination model in Kaplan-Meier survival analysis and compared this to the CAPRA-S score categories alone. Undertaking this, we confirmed the added value in risk prediction when using a model the combined ’PDE4D7 & CAPRA-S’ score, compared to using the clinical metric of CAPRA-S score alone (see Alves de Inda M. et al., 2018, ibid).

Subsequent to the diagnosis of prostate cancer, an accurate risk assessment needs to be undertaken before stratification to a defined primary treatment. With this in mind, we set out to see if we could translate the prognostic use of the ’PDE4D7 score’ in a pre-surgery situation testing tumour tissue obtained from diagnostic needle biopsy samples (see van Strijp D. et al., "The Prognostic PDE4D7 Score in a Diagnostic Biopsy Prostate Cancer Patient Cohort with Longitudinal Biological Outcomes", Prostate Cancer, 2018:5821616). In this, needle biopsies were performed on 168 patients, from a single diagnostic clinical centre, who had undergone surgery as a primary treatment. The minimum follow-up period for each patient was 60 months after this intervention. The clinical co-variates used to adjust the ’PDE4D7 score’ in the multivariable analysis were age at surgery, pre-operative PSA, PSA density, biopsy Gleason score, percentage of tumor positive biopsy cores, percentage of tumour in the biopsy and clinical cT stage. In this we evaluated the utility of the ’PDE4D7 score’ and the combined ’PDE4D7 & CAPRA’ scores compared to the pre-surgical CAPRA score in Cox regression analysis for biochemical relapse (see van Strijp D. et al., 2018, ibid). Evaluating this patient cohort we found (see van Strijp D. et al., 2018, ibid) that the ’PDE4D7 score’ was inversely associated with BCR in multivariable analysis when adjusting for clinical variables (HR=0.43; 95% CI 0.29-0.63; p<0.0001) as well as for the clinical CAPRA score (HR=0.53; 95% CI 0.38-0.74; p=0.0001). Kaplan-Meier analysis demonstrated that, as before, in a post-surgical setting, the ’PDE4D7 score’ categories were significantly associated with BCR progression free survival (logrank p<0.0001) and secondary treatment free survival (logrank p=0.01). We then employed a combination logistic regression model, which was developed on the previous cohort. This consisted of the combined ’CAPRA & PDE4D7’ score, demonstrating that patients within the highest combined ’CAPRA & PDE4D7’ combined score category have virtually no risk of biochemical progression or transfer to any secondary treatment after surgery. This logistic regression model was also evaluated using ROC curve analysis in order to predict 5-year BCR after surgery. This revealed an increase in AUC of 5% over the CAPRA score alone (AUC=0.82 vs. 0.77, respectively; p=0.004). Decision curve analysis of the combined ’CAPRA & PDE4D7’ score model confirmed the superior net benefit of using this combined score, compared to either score alone, across all decision thresholds in order to decide on whether to undertake intervention (e.g. surgery) based on the risk threshold of an individual patient to experience post-surgical disease progression (see van Strijp D. et al., 2018, ibid).

The present invention is based on the idea that, since the PDE4D7 biomarker has been proven to be a good predictor of radiotherapy response, the ability to identify markers that are highly correlated with the PDE47 biomarker might help to be better able to predict overall RT response.

The identified genes ABCC5, CUX2, KIAA1549, PDE4D, RAP1GAP2, SLC39A11, TDRD1, and VWA2 were identified as follows: We have identified in RNAseq data generated on 571 prostate cancer patients on close to 60,000 transcripts a range of genes that are correlated to the expression of the known biomarker PDE4D7 in this data. The correlation between the expression of any of these genes and PDE4D7 across the 571 samples was done by Pearson correlation and is expressed as a value between 0 to 1 in case of positive correlation or a value between -1 to 0 in case of negative correlation. The equation for the correlation coefficient is:

$$Corr(X,Y) = \frac{\sum(x-\bar{x})(y-\bar{y})}{\sqrt{\sum(x-\bar{x})^2 \sum(y-\bar{y})^2}} \quad (1)$$

where $\bar{x}$ and $\bar{y}$ are the sample means AVERAGE(PDE4D7) and AVERAGE(gene) across all samples, respectively. As input data for the calculation of the correlation coefficient we used the PDE4D7 score (see Alves de Inda M. et al., 2018, ibid) and the RNAseq determined TPM gene expression value per gene of interest (see below).

The maximum negative correlation coefficient identified between the expression of any of the approximately 60,000 transcripts and the expression of PDE4D7 was -0.38 while the maximum positive correlation coefficient identified between the expression of any of the approximately 60,000 transcripts and the expression of PDE4D7 was +0.56. We selected genes in the range of correlation -0.31 to -0.38 as well as +0.41 to +0.56. We identified in total 77 transcripts matching these characteristics which are listed in the following TABLE 1 (sorted alphabetically).

TABLE 1: Selection of 77 transcripts of which the expression shows strong negative or positive correlation to PDE4D7 expression.

| Gene Symbol | Ensembl_ID | Corr | p-value | 95% CI |
|---|---|---|---|---|
| ABCC5 | ENSG00000114770 | -0.32 | p<0.0001 | -0.3938 to -0.2410 |

(continued)

| Gene Symbol | Ensembl_ID | Corr | p-value | 95% CI |
|---|---|---|---|---|
| ABI3BP | ENSG00000154175 | -0.31 | p<0.0001 | -0.3850 to -0.2313 |
| AC011487.1 | ENSG00000213777 | 0.48 | p<0.0001 | 0.4100 to 0.5413 |
| AC109486.1 | ENSG00000213896 | 0.45 | p<0.0001 | 0.3785 to 0.5145 |
| ACSL3 | ENSG00000123983 | 0.46 | p<0.0001 | 0.3882 to 0.5227 |
| ADA2 | ENSG00000093072 | -0.31 | p<0.0001 | -0.3818 to -0.2277 |
| AL117329.1 | ENSG00000224271 | 0.41 | p<0.0001 | 0.3406 to 0.4817 |
| ANKRD13A | ENSG00000076513 | -0.32 | p<0.0001 | -0.3951 to -0.2425 |
| ANTXR1 | ENSG00000169604 | -0.37 | p<0.0001 | -0.4401 to -0.2931 |
| ARHGEF2 | ENSG00000116584 | -0.32 | p<0.0001 | -0.3906 to -0.2375 |
| ARSD | ENSG00000006756 | 0.43 | p<0.0001 | 0.3631 to 0.5011 |
| AUTS2 | ENSG00000158321 | -0.38 | p<0.0001 | -0.4469 to -0.3008 |
| BCAT1 | ENSG00000060982 | -0.32 | p<0.0001 | -0.3980 to -0.2457 |
| BCL2 | ENSG00000171791 | -0.31 | p<0.0001 | -0.3856 to -0.2319 |
| CACNA1D | ENSG00000157388 | 0.48 | p<0.0001 | 0.4123 to 0.5433 |
| CD74 | ENSG00000019582 | -0.36 | p<0.0001 | -0.4297 to -0.2815 |
| CELF2 | ENSG00000048740 | -0.31 | p<0.0001 | -0.3892 to -0.2361 |
| CHRM3 | ENSG00000133019 | 0.47 | p<0.0001 | 0.3968 to 0.5301 |
| CIITA | ENSG00000179583 | -0.32 | p<0.0001 | -0.3943 to -0.2416 |
| COL8A1 | ENSG00000144810 | -0.37 | p<0.0001 | -0.4375 to -0.2901 |
| CSGALNACT1 | ENSG00000147408 | 0.50 | p<0.0001 | 0.4358 to 0.5632 |
| CTSB | ENSG00000164733 | -0.31 | p<0.0001 | -0.3794 to -0.2250 |
| CUX2 | ENSG00000111249 | 0.50 | p<0.0001 | 0.4317 to 0.5597 |

(continued)

| Gene Symbol | Ensembl_ID | Corr | p-value | 95% CI |
|---|---|---|---|---|
| CXCR4 | ENSG00000121966 | -0.33 | p<0.0001 | -0.3987 to -0.2465 |
| CYBB | ENSG00000165168 | -0.31 | p<0.0001 | -0.3804 to -0.2261 |
| DOCK11 | ENSG00000147251 | -0.34 | p<0.0001 | -0.4100 to -0.2591 |
| DOCK2 | ENSG00000134516 | -0.32 | p<0.0001 | -0.3925 to -0.2395 |
| DRAM1 | ENSG00000136048 | -0.31 | p<0.0001 | -0.3808 to -0.2265 |
| ERG | ENSG00000157554 | 0.50 | p<0.0001 | 0.4337 to 0.5614 |
| FNIP2 | ENSG00000052795 | 0.44 | p<0.0001 | 0.3675 to 0.5050 |
| FRMD4A | ENSG00000151474 | 0.45 | p<0.0001 | 0.3867 to 0.5215 |
| FRY | ENSG00000073910 | -0.31 | p<0.0001 | -0.3842 to -0.2303 |
| GJB1 | ENSG00000169562 | 0.44 | p<0.0001 | 0.3676 to 0.5051 |
| GNB4 | ENSG00000114450 | -0.31 | p<0.0001 | -0.3871 to -0.2336 |
| GPRIN3 | ENSG00000185477 | -0.31 | p<0.0001 | -0.3799 to -0.2256 |
| GUCY1A1 | ENSG00000164116 | 0.45 | p<0.0001 | 0.3790 to 0.5149 |
| HLA-DPA1 | ENSG00000231389 | -0.34 | p<0.0001 | -0.4138 to -0.2634 |
| HLA-DRA | ENSG00000204287 | -0.34 | p<0.0001 | -0.4084 to -0.2573 |
| IGFBP3 | ENSG00000146674 | -0.36 | p<0.0001 | -0.4330 to -0.2851 |
| INHBA | ENSG00000122641 | -0.32 | p<0.0001 | -0.3939 to -0.2411 |
| ITGA4 | ENSG00000115232 | -0.33 | p<0.0001 | -0.4052 to -0.2538 |
| KCNH8 | ENSG00000183960 | 0.43 | p<0.0001 | 0.3570 to 0.4959 |
| KIAA1324 | ENSG00000116299 | 0.44 | p<0.0001 | 0.3689 to 0.5062 |
| KIAA1549 | ENSG00000122778 | 0.44 | p<0.0001 | 0.3684 to 0.5058 |
| KIF13B | ENSG00000197892 | 0.42 | p<0.0001 | 0.3491 to 0.4891 |

(continued)

| Gene Symbol | Ensembl_ID | Corr | p-value | 95% CI |
|---|---|---|---|---|
| LAPTM5 | ENSG00000162511 | -0.33 | p<0.0001 | -0.4069 to -0.2557 |
| LTBP2 | ENSG00000119681 | -0.37 | p<0.0001 | -0.4375 to -0.2901 |
| MAML3 | ENSG00000196782 | 0.43 | p<0.0001 | 0.3592 to 0.4978 |
| MAP7 | ENSG00000135525 | 0.44 | p<0.0001 | 0.3707 to 0.5078 |
| MFAP4 | ENSG00000166482 | -0.31 | p<0.0001 | -0.3844 to -0.2306 |
| MSN | ENSG00000147065 | -0.32 | p<0.0001 | -0.3935 to -0.2407 |
| NPNT | ENSG00000168743 | -0.31 | p<0.0001 | -0.3832 to -0.2292 |
| OSBPL3 | ENSG00000070882 | -0.31 | p<0.0001 | -0.3876 to -0.2341 |
| PART1 | ENSG00000152931 | 0.56 | p<0.0001 | 0.4969 to 0.6142 |
| PDE3B | ENSG00000152270 | 0.43 | p<0.0001 | 0.3565 to 0.4955 |
| PDE4D | ENSG00000113448 | 0.48 | p<0.0001 | 0.4144 to 0.5450 |
| PLS3 | ENSG00000102024 | -0.31 | p<0.0001 | -0.3787 to -0.2242 |
| PLXNC1 | ENSG00000136040 | -0.36 | p<0.0001 | -0.4306 to -0.2823 |
| PTPRC | ENSG00000081237 | -0.31 | p<0.0001 | -0.3828 to -0.2288 |
| RAB3B | ENSG00000169213 | 0.41 | p<0.0001 | 0.3372 to 0.4788 |
| RAP1GAP2 | ENSG00000132359 | 0.47 | p<0.0001 | 0.3984 to 0.5315 |
| RNU6-806P | ENSG00000202017 | 0.48 | p<0.0001 | 0.4088 to 0.5403 |
| SERPING1 | ENSG00000149131 | -0.31 | p<0.0001 | -0.3852 to -0.2315 |
| SETP21 | ENSG00000214244 | 0.45 | p<0.0001 | 0.3814 to 0.5169 |
| SH3KBP1 | ENSG00000147010 | -0.32 | p<0.0001 | -0.3947 to -0.2421 |
| SLC25A42 | ENSG00000181035 | 0.43 | p<0.0001 | 0.3590 to 0.4976 |
| SLC39A11 | ENSG00000133195 | -0.37 | p<0.0001 | -0.4377 to -0.2904 |

(continued)

| Gene Symbol | Ensembl_ID | Corr | p-value | 95% CI |
|---|---|---|---|---|
| SPARC | ENSG00000113140 | -0.31 | p<0.0001 | -0.3811 to -0.2269 |
| SULF1 | ENSG00000137573 | -0.36 | p<0.0001 | -0.4302 to -0.2819 |
| SYK | ENSG00000165025 | -0.31 | p<0.0001 | -0.3875 to -0.2340 |
| TDRD1 | ENSG00000095627 | 0.41 | p<0.0001 | 0.3412 to 0.4822 |
| THBS2 | ENSG00000186340 | -0.35 | p<0.0001 | -0.4229 to -0.2737 |
| VWA2 | ENSG00000165816 | 0.43 | p<0.0001 | 0.3629 to 0.5010 |
| WIPF1 | ENSG00000115935 | -0.32 | p<0.0001 | -0.3910 to -0.2379 |
| ZFP36L1 | ENSG00000185650 | -0.33 | p<0.0001 | -0.4049 to -0.2535 |
| ZNF614 | ENSG00000142556 | 0.41 | p<0.0001 | 0.3434 to 0.4842 |
| ZNF875 | ENSG00000181666 | 0.41 | p<0.0001 | 0.3359 to 0.4776 |

From those 77 transcripts we selected the eight genes ABCC5, CUX2, KIAA1549, PDE4D, RAP1GAP2, SLC39A11, TDRD1, and VWA2 by testing Cox regression combination models iteratively in a sub-cohort of 186 patients who were undergoing salvage radiation treatment (SRT) due to post-surgical biochemical relapse. The clinical endpoint tested was prostate cancer specific death after start of SRT.

The boundary condition for the selection of the eight genes was given by the restriction that the p-values in the multivariate Cox-regression were <0.1 for all genes retained in the model.

The term "ABCC5" refers to the human ATP binding cassette subfamily C member 5 gene (Ensembl: ENSG00000114770), for example, to the sequence as defined in NCBI Reference Sequence NM_001023587.2 or in NCBI Reference Sequence NM_005688.3, specifically, to the nucleotide sequence as set forth in SEQ ID NO:1 or in SEQ ID NO:2, which correspond to the sequences of the above indicated NCBI Reference Sequences of the ABCC5 transcript, and also relates to the corresponding amino acid sequence for example as set forth in SEQ ID NO:3 or in SEQ ID NO:4, which correspond to the protein sequences defined in NCBI Protein Accession Reference Sequence NP_001018881.1 and in NCBI Protein Accession Reference Sequence NP_005679 encoding the ABCC5 polypeptide.

The term "ABCC5" also comprises nucleotide sequences showing a high degree of homology to ABCC5, e.g., nucleic acid sequences being at least 75%, 80%, 85%, 90%, 91%, 92%, 93%, 94%, 95%, 96%, 97%, 98% or 99% identical to the sequence as set forth in SEQ ID NO:1 or in SEQ ID NO:2 or amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:3 or in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:1 or in SEQ ID NO:2.

The term "CUX2" refers to the human Cut Like Homeobox 2 gene (Ensembl: ENSG00000111249), for example, to the sequence as defined in NCBI Reference Sequence NM_015267.3, specifically, to the nucleotide sequence as set forth in SEQ ID NO:5, which corresponds to the sequence of the above indicated NCBI Reference Sequence of the CUX2 transcript, and also relates to the corresponding amino acid sequence for example as set forth in SEQ ID NO:6, which corresponds to the protein sequence defined in NCBI Protein Accession Reference Sequence NP_056082.2 encoding the CUX2 polypeptide.

The term "CUX2" also comprises nucleotide sequences showing a high degree of homology to CUX2, e.g.,

nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "KIAA1549" refers to the human KIAA1549 gene (Ensembl: ENSG00000122778), for example, to the sequence as defined in NCBI Reference Sequence NM_020910 or in NCBI Reference Sequence NM_001164665, specifically, to the nucleotide sequence as set forth in SEQ ID NO:7 or in SEQ ID NO:8, which correspond to the sequences of the above indicated NCBI Reference Sequence of the KIAA1549 transcript, and also relates to the corresponding amino acid sequence for example as set forth in SEQ ID NO:9 or in SEQ ID NO:10, which correspond to the protein sequences defined in NCBI Protein Accession Reference Sequence NP_065961 and in NCBI Protein Accession Reference Sequence NP_001158137 encoding the KIAA1549 polypeptide.

The term "KIAA1549" also comprises nucleotide sequences showing a high degree of homology to KIAA1549, e.g., nucleic acid sequences being at least 75%, 80%, 85%, 90%, 91%, 92%, 93%, 94%, 95%, 96%, 97%, 98% or 99% identical to the sequence as set forth in SEQ ID NO:7 or in SEQ ID NO:8 or amino acid sequences being at least 75%, 80%, 85%, 90%, 91%, 92%, 93%, 94%, 95%, 96%, 97%, 98% or 99% identical to the sequence as set forth in SEQ ID NO:9 or in SEQ ID NO:10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:9 or in SEQ ID NO:10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7 or in SEQ ID NO:8.

The term "PDE4D" refers to the human Phosphodiesterase 4D gene (Ensembl: ENSG00000113448), for example, to the sequence as defined in NCBI Reference Sequence NM_001104631 or in NCBI Reference Sequence NM_001349242 or in NCBI Reference Sequence NM_001197218 or in NCBI Reference Sequence NM_006203 or in NCBI Reference Sequence NM_001197221 or in NCBI Reference Sequence NM_001197220 or in NCBI Reference Sequence NM_001197223 or in NCBI Reference Sequence NM_001165899 or in NCBI Reference Sequence NM_001165899, specifically, to the nucleotide sequence as set forth in SEQ ID NO: 11 or in SEQ ID NO:12 or in SEQ ID NO:13 or in SEQ ID NO:14 or in SEQ ID NO:15 or in SEQ ID NO:16 or in SEQ ID NO:17 or in SEQ ID NO:18 or in SEQ ID NO:19, which correspond to the sequences of the above indicated NCBI Reference Sequence of the PDE4D transcript, and also relates to the corresponding amino acid sequence for example as set forth in SEQ ID NO:20 or in SEQ ID NO:21 or in SEQ ID NO:22 or in SEQ ID NO:23 or in SEQ ID NO:24 or in SEQ ID NO:25 or in SEQ ID NO:26 or in SEQ ID NO:27 or in SEQ ID NO:28, which correspond to the protein sequences defined in NCBI Protein Accession Reference Sequence NP_001098101 and in NCBI Protein Accession Reference Sequence NP_001336171 and in NCBI Protein Accession Reference Sequence NP_001184147 and in NCBI Protein Accession Reference Sequence NP_006194 and in NCBI Protein Accession Reference Sequence NP_001184150 and in NCBI Protein Accession Reference Sequence NP_001184149 and in NCBI Protein Accession Reference Sequence NP_001184152 and in NCBI Protein Accession Reference Sequence NP_001159371 and in NCBI Protein Accession Reference Sequence NP_001184148 encoding the PDE4D polypeptide.

The term "PDE4D" also comprises nucleotide sequences showing a high degree of homology to PDE4D, e.g., nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO:12 or in SEQ ID NO:13 or in SEQ ID NO:14 or in SEQ ID NO: 15 or in SEQ ID NO:16 or in SEQ ID NO:17 or in SEQ ID NO:18 or in SEQ ID NO:19 or amino acid sequences being at least 75%, 80%, 85%, 90%, 91%, 92%, 93%, 94%, 95%, 96%, 97%, 98% or 99% identical to the sequence as set forth in SEQ ID NO:20 or in SEQ ID NO:21 or in SEQ ID NO:22 or in SEQ ID NO:23 or in SEQ ID NO:24 or in SEQ ID NO:25 or in SEQ ID NO:26 or in SEQ ID NO:27 or in SEQ ID NO:28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or in SEQ ID NO:21 or in SEQ ID NO:22 or in SEQ ID NO:23 or in SEQ ID NO:24 or in SEQ ID NO:25 or in SEQ ID NO:26 or in SEQ ID NO:27 or in SEQ ID NO:28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO:12 or in SEQ ID NO:13 or in SEQ ID NO:14 or in SEQ ID NO: 15 or in SEQ ID NO:16 or in SEQ ID NO:17 or in SEQ ID NO:18 or in SEQ ID NO:19.

The term "RAP1GAP2" refers to the human RAP1 GTPase Activating Protein 2 gene (ENSG00000132359), for example, to the sequence as defined in NCBI Reference Sequence NM_015085 or in NCBI Reference Sequence NM_001100398 or in NCBI Reference Sequence NM_001330058, specifically, to the nucleotide sequence as set forth in SEQ ID NO:29 or in SEQ ID NO:30 or in SEQ ID NO:31, which correspond to the sequences of the above indicated NCBI Reference Sequences of the RAP1GAP2 transcript, and also relates to the corresponding amino acid sequence for example as set forth in SEQ ID NO:32 or in SEQ ID NO:33 or in SEQ ID NO:34, which correspond to the protein sequences defined in NCBI Protein Accession Reference Sequence NP_055900 and in NCBI Protein Accession Reference Sequence NP_001093868 and in NCBI Protein Accession Reference Sequence NP_001316987 encoding the RAP1GAP2 polypeptide.

The term "RAP1GAP2" also comprises nucleotide sequences showing a high degree of homology to RAP1GAP2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:29 or in SEQ ID NO:30 or in SEQ ID NO:31 or amino acid sequences being at least 75%, 80%, 85%, 90%, 91%, 92%, 93%, 94%, 95%, 96%, 97%, 98% or 99% identical to the sequence as set forth in SEQ ID NO:32 or in SEQ ID NO:33 or in SEQ ID NO:34 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:32 or in SEQ ID NO:33 or in SEQ ID NO:34 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29 or in SEQ ID NO:30 or in SEQ ID NO:31.

The term "SLC39A11" refers to the human Solute Carrier Family 39 Member 11 gene (Ensembl: ENSG00000133195), for example, to the sequence as defined in NCBI Reference Sequence NM_139177 or in NCBI Reference Sequence NM_001352692, specifically, to the nucleotide sequence as set forth in SEQ ID NO:35 or in SEQ ID NO:36, which correspond to the sequences of the above indicated NCBI Reference Sequences of the SLC39A11 transcript, and also relates to the corresponding amino acid sequence for example as set forth in SEQ ID NO:37 or in SEQ ID NO:38, which correspond to the protein sequences defined in NCBI Protein Accession Reference Sequence NP_631916 and in NCBI Protein Accession Reference Sequence NP_001339621 encoding the SLC39A11 polypeptide.

The term "SLC39A11" also comprises nucleotide sequences showing a high degree of homology to SLC39A11, e.g., nucleic acid sequences being at least 75%, 80%, 85%, 90%, 91%, 92%, 93%, 94%, 95%, 96%, 97%, 98% or 99% identical to the sequence as set forth in SEQ ID NO:35 or in SEQ ID NO:36 or amino acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:37 or in SEQ ID NO:38 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:35 or in SEQ ID NO:36.

The term "TDRD1" refers to the human Tudor Domain Containing 1 gene (Ensembl: ENSG00000095627), for example, to the sequence as defined in NCBI Reference Sequence NM_198795, specifically, to the nucleotide sequence as set forth in SEQ ID NO:39, which corresponds to the sequence of the above indicated NCBI Reference Sequence of the TDRD1 transcript, and also relates to the corresponding amino acid sequence for example as set forth in SEQ ID NO:40, which corresponds to the protein sequence defined in NCBI Protein Accession Reference Sequence NP_942090 encoding the TDRD1 polypeptide.

The term "TDRD1" also comprises nucleotide sequences showing a high degree of homology to TDRD1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:39 or amino acid sequences being at least 75%, 80%, 85%, 90%, 91%, 92%, 93%, 94%, 95%, 96%, 97%, 98% or 99% identical to the sequence as set forth in SEQ ID NO:40 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:40 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:39.

The term "VWA2" refers to the human Von Willebrand Factor A Domain Containing 2 gene (Ensembl: ENSG00000165816), for example, to the sequence as defined in NCBI Reference Sequence NM_001320804, specifically, to the nucleotide sequence as set forth in SEQ ID NO:41, which corresponds to the sequence of the above indicated NCBI Reference Sequence of the VWA2 transcript, and also relates to the corresponding amino acid sequence for example as set forth in SEQ ID NO:42, which corresponds to the protein sequence defined in NCBI Protein Accession Reference Sequence NP_001307733 encoding the VWA2 polypeptide.

The term "VWA2" also comprises nucleotide sequences showing a high degree of homology to VWA2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:41 or amino acid sequences being at least 75%, 80%, 85%, 90%, 91%, 92%, 93%, 94%, 95%, 96%, 97%, 98% or 99% identical to the sequence as set forth in SEQ ID NO:42 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:42 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:41.

The term "biological sample" or "sample obtained from a subject" refers to any biological material obtained via suitable methods known to the person skilled in the art from a subject, e.g., a prostate cancer patient. The term "prostate cancer subject" refers to a person having or suspected of having prostate cancer.

The biological sample used may be collected in a clinically acceptable manner, e.g., in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "prostate cancer" refers to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 3.0. In another embodiment the term relates to cancer showing PSA levels above 2.0. The term "PSA level" refers to the concentration of PSA in the blood in ng/ml.

The term "non-progressive prostate cancer state" means that a sample of an individual does not show parameter values indicating "biochemical recurrence" and/or "clinical recurrence" and/or "metastases" and/or "castration-resistant disease" and/or "prostate cancer or disease specific death".

The term "progressive prostate cancer state" means that a sample of an individual shows parameter values indicating "biochemical recurrence" and/or "clinical recurrence" and/or "metastases" and/or "castration-resistant disease" and/or "prostate cancer or disease specific death".

The term "biochemical recurrence" generally refers to recurrent biological values of increased PSA indicating the presence of prostate cancer cells in a sample. However, it is also possible to use other markers that can be used in the detection of the presence or that rise suspicion of such presence.

The term "clinical recurrence" refers to the presence of clinical signs indicating the presence of tumour cells as measured, for example using in vivo imaging.

The term "metastases" refers to the presence of metastatic disease in organs other than the prostate.

The term "castration-resistant disease" refers to the presence of hormone-insensitive prostate cancer; i.e., a cancer in the prostate that does not any longer respond to androgen deprivation therapy (ADT).

The term "prostate cancer specific death or disease specific death" refers to death of a patient from his prostate cancer.

It is preferred that the one or more PDE4D7 correlated genes comprise three or more, preferably, six or more, most preferably, all of the PDE4D7 correlated genes.

It is preferred that the determining of the prediction of the radiotherapy response comprises combining the gene expression profiles for two or more, for example, 2, 3, 4, 5, 6, 7 or all, of the PDE4D7 correlated genes with a regression function that had been derived from a population of prostate cancer subjects.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t) = H_0(t) \cdot \exp(w_1 \cdot V_1 + w_2 \cdot V_2 + w_3 \cdot V_3 + ...)$, where $V_1$, $V_2$, $V_3$ ... are predictor variables and $H_0(t)$ is the baseline hazard while H(t) is the hazard at any time t. The hazard ratio (or the risk to reach the event) is represented by $Ln[H(t)/ H_0(t)] = w_1 \cdot V_1 + w_2 \cdot V_2 + w_3 \cdot V_3 + ...$, where the coefficients or weights $w_1$, $w_2$, $w_3$ ... are estimated by the Cox regression analysis and can be interpreted in a similar manner as for logistic regression analysis.

In one particular realization, the prediction of the radiotherapy response is determined as follows:

$$\text{PDE4D7_CORR_model:} \quad (w_1 \cdot \text{ABCC5}) + (w_2 \cdot \text{CUX2}) + (w_3 \cdot \text{KIAA1549}) + (w_4 \cdot \text{PDE4D}) + (w_5 \cdot \text{RAP1GAP2}) + (w_6 \cdot \text{SLC39A11}) + (w_7 \cdot \text{TDRD1}) + (w_8 \cdot \text{VWA2}) \tag{2}$$

where $w_1$ to $w_8$ are weights and ABCC5, CUX2, KIAA1549, PDE4D, RAP1GAP2, SLC39A11, TDRD1, and VWA2 are the expression levels of the genes.

In one example, $w_1$ may be about -0.1 to 0.9, such as 0.418, $w_2$ may be about - 0.2 to 0.8, such as 0.35, $w_3$ may be about -0.1 to 0.9, such as 0.43774, $w_4$ may be about -1.3 to -0.3, such as -0.7596, $w_5$ may be about -1.2 to -0.2, such as -0.7243, $w_6$ may be about 0.0 to 0.1, such as 0.5361, $w_7$ may be about -0.2 to 0.8, such as 0.2686, and $w_8$ may be about -0.8 to 0.2, such as -0.3123.

The prediction of the radiotherapy response may also be classified or categorized into one of at least two risk groups, based on the value of the prediction of the radiotherapy response. For example, there may be two risk groups, or three risk groups, or four risk groups, or more than four predefined risk groups. Each risk group covers a respective range of (non-overlapping) values of the prediction of the radiotherapy response. For example, a risk group may indicate a probability of occurrence of a specific clinical event from 0 to <0.1 or from 0.1 to <0.25 or from 0.25 to <0.5 or from 0.5 to 1.0 or the like.

It is further preferred that the determining of the prediction of the radiotherapy response is further based on one or more clinical parameters obtained from the subject.

As mentioned above, various measures based on clinical parameters have been investigated. By further basing the prediction of the radiotherapy response on such clinical parameter(s), it can be possible to further improve the prediction.

It is preferred that the clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

It is further preferred that the determining of the prediction of the radiotherapy response comprises combining the gene expression profile(s) for the one or more PDE4D7 correlated genes and the one or more clinical parameters obtained from the subject with a regression function that had been derived from a population of prostate cancer subjects.

It is further preferred that the gene expression profiles for the one or more PDE4D7 correlated genes and the one or more clinical parameters obtained from the subject are combined with a regression function that had been derived from a population of prostate cancer subjects.

In one particular realization, the prediction of the radiotherapy response is determined as follows:

$$(w_9 \cdot \text{PDE4D7_CORR_model}) + (w_{10} \cdot \text{pGGG}) \tag{3}$$

where $w_9$ and $w_{10}$ are weights, PDE4D7_CORR_model is the above-described regression model based on the expression profiles for the two or more, for example, 2, 3, 4, 6, 7 or all, of the PDE4D7 correlated genes, and pGGG is the pathological Gleason grade group. In one example, $w_9$ may be about 0.1 to 1.1, such as 0.6117, and $w_{10}$ may be about 0.1 to 1.1, such as 0.6127.

It is preferred that the biological sample is obtained from the subject before the start of the radiotherapy. The gene expression profile(s) may be determined in the form of mRNA or protein in tissue of prostate cancer. Alternatively, if the genes are present in a soluble form, the gene expression profile(s) may be determined in blood.

It is further preferred that the radiotherapy is radical radiotherapy or salvage radiotherapy.

It is preferred that the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy. The degree to which the prediction is negative may determine the degree to which the recommended therapy deviates from the standard form of radiotherapy.

In a further aspect of the present invention, an apparatus for predicting a response of a prostate cancer subject to radiotherapy is presented, comprising:

- an input adapted to receive data indicative of a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said gene expression profile(s) being determined in a biological sample obtained from the subject,
- a processor adapted to determine the prediction of the radiotherapy response based on the gene expression profile(s) for the one or more PDE4D7 correlated genes, and
- optionally, a providing unit adapted to provide the prediction or a therapy recommendation based on the prediction to a medical caregiver or the subject.

In a further aspect of the present invention, a computer program product is presented comprising instructions which, when the program is executed by a computer, cause the computer to carry out a method comprising:

- receiving data indicative of a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said gene expression profile(s) being determined in a biological sample obtained from a prostate cancer subject,
- determining a prediction of a response of a prostate cancer subject to therapy based on the gene expression profile(s) for the one or more PDE4D7 correlated genes, and
- optionally, providing the prediction or a therapy recommendation based on the prediction to a medical caregiver or the subject.

In a further aspect of the present invention, a diagnostic kit is presented, comprising:

- at least one primer and/or probe for determining the gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a biological sample obtained from the subject, and
- optionally, an apparatus as defined in claim 10 or a computer program product as defined in claim 11.

In a further aspect of the present invention, a use of the kit as defined in claim 12 is presented.
It is preferred that the use as defined in claim 13 is in a method of predicting a response of a prostate cancer subject to radiotherapy.
In a further aspect of the present invention, a method is presented, comprising:

- receiving a biological sample obtained from a prostate cancer subject,
- using the kit as defined in claim 12 to determine a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in the biological sample obtained from the subject.

In a further aspect of the present invention, a use of a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a method of predicting a response of a prostate cancer subject to radiotherapy is presented, comprising:

- determining the prediction of the radiotherapy response based on the gene expression profile(s) for the one or more PDE4D7 correlated genes, and
- optionally, providing the prediction or a therapy recommendation based on the prediction to a medical caregiver or the subject.

It shall be understood that the method of claim 1, the apparatus of claim 10, the computer program product of claim 11, the diagnostic kit of claim 12, the use of the diagnostic kit of claim 13, the method of claim 15, and the use of a gene expression profile(s) of claim 16 have similar and/or identical preferred embodiments, in particular, as defined in the dependent claims.
It shall be understood that a preferred embodiment of the present invention can also be any combination of the dependent claims or above embodiments with the respective independent claim.
These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a flowchart of an embodiment of a method of predicting a response of a prostate cancer subject to radiotherapy,
Fig. 2 shows a ROC curve analysis of three predictive models,
Fig. 3 shows a ROC curve analysis of three predictive models,
Fig. 4 shows a Kaplan-Meier curve of the PDE4D7_CORR_model in a 186 patient cohort (training set used to develop the PDE4D7_CORR_model) with all patients undergoing SRT (salvage radiation treatment) after post-surgical BCR (biochemical recurrence),
Fig. 5 shows a Kaplan-Meier curve of the PDE4D7_CORR&pGGG_model in a 186 patient cohort (training set used to develop the PDE4D7_CORR&pGGG_model) with all patients undergoing SRT (salvage radiation treatment) after post-surgical BCR (biochemical recurrence),
Fig. 6 shows a Kaplan-Meier curve of the PDE4D7_CORR_model in a 151 patient cohort (testing set used to validate the PDE4D7_CORR_model as developed on the 186 patient training set) with all patients undergoing SRT (salvage radiation treatment) after post-surgical BCR (biochemical recurrence),
Fig. 7 shows a Kaplan-Meier curve of the PDE4D7_CORR&pGGG_model in a 151 patient cohort (testing set used to validate the PDE4D7_CORR&pGGG model as developed on the 186 patient training set) with all patients undergoing SRT (salvage radiation treatment) after post-surgical BCR (biochemical recurrence),
Fig. 8 shows a Kaplan-Meier curve of the PDE4D7_CORR_model in a 151 patient cohort (testing set used to validate the PDE4D7_CORR_model as developed on the 186 patient training set) with all patients undergoing SRT (salvage radiation treatment) after post-surgical BCR (biochemical recurrence), and
Fig. 9 shows a Kaplan-Meier curve of the PDE4D7_CORR&pGGG_model in a 151 patient cohort (testing set used to validate the PDE4D7_CORR&pGGG model as developed on the 186 patient training set) with all patients undergoing SRT (salvage radiation treatment) after post-surgical BCR (biochemical recurrence).

DETAILED DESCRIPTION OF EMBODIMENTS

**Overview Of Radiotherapy Response Prediction**

Fig. 1 shows schematically and exemplarily a flowchart of an embodiment of a method of predicting a response of a prostate cancer subject to radiotherapy. The method begins at step S100.

At step S102, a biological sample is obtained from each of a first set of patients (subjects) diagnosed with prostate cancer. Preferably, monitoring prostate cancer has been performed for these prostate cancer patients over a period of time, such as at least one year, or at least two years, or about five years, after obtaining the biological sample.

At step S104, a gene expression profile for each of two or more, for example, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the two or more genes which can be normalized using value(s) for each of a set of reference genes, such as B2M, HPRT1, POLR2A, and/or PUM1. In one realization, the gene expression level for each of the two or more genes is normalized with respect to one or more reference genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these reference genes.

At step S106, a regression function for assigning a prediction of the radiotherapy response is determined based on the gene expression profiles for the two or more genes, ABCC5, CUX2, KIAA1549, PDE4D, RAP1GAP2, SLC39A11, TDRD1, and/or VWA2, obtained for at least some of the biological samples obtained for the first set of patients and respective results obtained from the monitoring. In one particular realization, the regression function is determined as specified in Eq. (2) above.

At step S108, a biological sample is obtained from a patient (subject or individual). The patient can be a new patient or one of the first set.

At step S110, a gene expression profile is obtained for each of the two or more, for example, 2, 3, 4, 5, 6, 7 or all, PDE4D7 correlated genes, e.g., by performing PCR on the biological sample. In one realization, the gene expression level for each of the two or more genes is normalized with respect to one or more reference genes selected from the group consisting of ACTB, ALAS1, B2M, HPRT1, POLR2A, PUM1, RPLP0, TBP, TUBA1B, and/or YWHAZ, e.g., at least one, or at least two, or at least three, or, preferably, all of these reference genes. This is substantially the same as in step S104.

At step S112, a prediction of the radiotherapy response based on the gene expression profiles for the two or more genes is determined for the patient using the regression function. This will be described in more detail later in the description.

At S114, a therapy recommendation may be provided, e.g., to the patient or his or her guardian, to a doctor, or to another healthcare worker, based on the prediction. To this end, the prediction may be categorized into one of a predefined set of risk groups, based on the value of the prediction. In one particular realization, the prediction of the radiotherapy response may be negative or positive for the effectiveness of the radiotherapy. If the prediction is negative, the recommended therapy may comprise one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

The method ends at S116.

In one embodiment, the gene expression profiles at steps S104 and S110 are determined by detecting mRNA expression using two or more primers and/or probes and/or two or more sets thereof.

In one embodiment, steps S104 and S110 further comprise obtaining clinical parameters from the first set of patients and the patient, respectively. The clinical parameters may comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score. The regression function for assigning the prediction of the radiotherapy response that is determined in step S106 is then further based on the one or more clinical parameters obtained from at least some of the first set of patients. In step S112, the prediction of the radiotherapy response is then further based on the one or more clinical parameters, e.g., the pathological Gleason grade group (pGGG), obtained from the patient and is determined for the patient using the regression function.

Based on the significant correlation with outcome after RT, we expect that the identified molecules will provide predictive value with regard to the effectiveness of radical RT and/or SRT.

RESULTS

**TPM gene expression values**

For each gene a TPM (transcript per million) expression value was calculated based on the following steps:

1. Divide the read counts derived from the RNAseq fastq raw data after mapping and alignment to the human genome by the length of each gene in kilobases. This results in reads per kilobase (RPK).
2. Sum up all RPK values in a samples and divide this number by 1,000,000. This results in a 'per million' scaling factor.
3. Divide the RPK values by the "per million" scaling factor. This results in a TPM expression value for each gene.

The TPM value per gene was used to calculate the reference normalized gene expression of each gene.

**Normalized gene expression values**

For the Cox regression modeling all gene TPM (transcript per million) based expression values from the RNAseq data were log2 normalized by the following transformation:

$$\mathrm{TPM_log2} = \log2(\mathrm{TPM}+1) \qquad (4)$$

In a second step of normalization of the TPM_log2 expression values were normalized against the mean average of four reference genes (mean(ref_genes)) as follows:

$$\mathrm{TPM_log2_norm} = \log2(\mathrm{TPM}+1) - \log2(\mathrm{mean}(\mathrm{ref_genes})) \qquad (5)$$

The following reference genes were considered (TABLE 2):

TABLE 2: Reference genes.

| Gene Symbol | Ensembl_ID |
|---|---|
| ALAS1 | ENSG00000023330 |
| ACTB | ENSG00000075624 |
| RPLP0 | ENSG00000089157 |
| TBP | ENSG00000112592 |
| TUBA1B | ENSG00000123416 |
| PUM1 | ENSG00000134644 |
| YWHAZ | ENSG00000164924 |
| HPRT1 | ENSG00000165704 |
| B2M | ENSG00000166710 |
| POLR2A | ENSG00000181222 |

For these reference genes, we selected the following four B2M, HPRT1, POLR2A, and PUM1 in order to calculate the:

$$\text{log2(mean(ref_genes))} = \text{AVERAGE((log2(B2M+1), log2(HPRT1+1), log2(POLR2A+1), log2(PUM1+1))} \quad (6)$$

where the input data for the reference genes is their RNAseq measured gene expression in TPM (transcript per million), and AVERAGE is the mathematical mean.

For the multivariate analysis of the genes of interest we used the reference gene normalized log2(TPM) value of each gene as input.

**Cox Regression Analysis**

We then set out to test whether the combination of these eight genes will exhibit more prognostic value. With Cox regression we modelled the expression levels of the eight genes to prostate cancer specific death after post-surgical salvage RT either with (PDE4D7_CORR&pGGG_model) or without (PDE4D7_CORR_model) the presence of the variable pathological Gleason grade group (pGGG) in a cohort of 571 prostate cancer patients. We tested the two models in ROC curve analysis as well as in Kaplan-Meier survival analysis.

The Cox regression function was derived as follows:

PDE4D7_CORR_model:

$$(w_1 \cdot \text{ABCC5}) + (w_2 \cdot \text{CUX2}) + (w_3 \cdot \text{KIAA1549}) + (w_4 \cdot \text{PDE4D}) + (w_5 \cdot \text{RAP1GAP2}) + (w_6 \cdot \text{SLC39A11}) + (w_7 \cdot \text{TDRD1}) + (w_8 \cdot \text{VWA2})$$

PDE4D7_CORR&pGGG_model:

$$(w_9 \cdot \text{PDE4D7_CORR_model}) + (w_{10} \cdot \text{pGGG})$$

The details for the weights $w_1$ to $w_{10}$ are shown in the following TABLE 3.

TABLE 3: Variables and weights for the two Cox regression models, i.e., the PDE4D7 correlation model (PDE4D7_CORR_model) and the PDE4D7 correlation & pGGG combination model (PDE4D7_CORR&pGGG_model); NA - not available.

| **Variable** | **Weights** | | |
|---|---|---|---|
| **Model** | | **PDE4D7_CORR_model** | **PDE4D7_CORR &pGGG_ model** |
| **ABCC5** | $w_1$ | 0.418 | NA |
| **CUX2** | $w_2$ | 0.35 | NA |
| **KIAA1549** | $w_3$ | 0.43774 | NA |
| **PDE4D** | $w_4$ | -0.7596 | NA |
| **RAP1GAP2** | $w_5$ | -0.7243 | NA |
| **SLC39A11** | $w_6$ | 0.5361 | NA |
| **TDRD1** | $w_7$ | 0.2686 | NA |
| **VWA2** | $w_8$ | -0.3123 | NA |
| **PDE4D7_CORR_model** | $w_9$ | NA | 0.6117 |
| **pGGG** | $w_{10}$ | NA | 0.6127 |

**ROC Curve Analysis**

Next, we tested the Cox regression model as outlined above for their power to predict 5-year prostate cancer specific death (PCa Death) after start of salvage radiation therapy (SRT) due to post-surgical disease recurrence. The performance of the model was compared to the EAU-BCR risk groups (see Tilki D. et al., "External validation of the European Association of Urology Biochemical Recurrence Risk groups to predict metastasis and mortality after radical prostatectomy in a European cohort", Eur Urol, Vol. 75, No. 6, pages 896-900, 2019) and to the pathological Gleason grade group (pGGG).

Fig. 2 shows a ROC curve analysis of three predictive models. The PDE4D7_CORR_model (AUC=0.85) is the Cox regression model based on the eight genes that correlate with PDE4D7. The PDE4D7_CORR&pGGG_model (AUC=0.9) is the Cox regression model based on the eight genes that correlate with PDE4D7 and the pathology Gleason grade group (pGGG) information. The EAU_BCR_Risk (AUC=0.79) is the EAU-BCR risk group (European Association of Urology Biochemical Recurrence Risk groups).

Fig. 3 shows a ROC curve analysis of three predictive models. The PDE4D7_CORR_model (AUC=0.86) is the Cox regression model based on the eight genes that correlate with PDE4D7. The PDE4D7_CORR&pGGG_model (AUC=0.91) is the Cox regression model based on the eight genes that correlate with PDE4D7 and the pathology Gleason grade group (pGGG) information. The pGGG (AUC=0.82) is the pathological Gleason grade group.

**Kaplan-Meier Survival Analysis**

For Kaplan-Meier survival curve analysis, the Cox functions of the risk models (PDE4D7_CORR_model and PDE4D7_CORR&pGGG_model) were categorized into two sub-cohorts based on a cut-off. The threshold for group separation into low risk and high risk was based on the mean output value of the PDE4D7_CORR_model and of the PDE4D7_CORR&pGGG_model, respectively, as calculated by use of the Cox regression model per patient in the entire cohort.

The patient classes represent an increasing risk to experience the tested clinical endpoints of prostate cancer specific death (PCa Death) after the start of salvage radiation therapy (SRT) due to post-surgical disease recurrence (Figs. 4 to 7) and death after the start of salvage radiation therapy (SRT) due to post-surgical disease recurrence (Figs. 8 and 9) for the two created risk models (PDE4D7_CORR_model; PDE4D7_CORR&pGGG_model).

Fig. 4 shows a Kaplan-Meier curve of the PDE4D7_CORR_model in a 186 patient cohort (training set used to develop the PDE4D7_CORR_model) with all patients undergoing SRT (salvage radiation treatment) after post-surgical BCR (biochemical recurrence). The clinical endpoint that was tested was the prostate cancer specific death (PCa Death) after the start of salvage radiation therapy (SRT) due to post-surgical disease recurrence (logrank $p<0.0001$; HR=9.4; 95% CI=4.1-21.2). The following supplementary lists indicate the number of patients at risk for the PDE4D7_CORR_model classes analyzed, i.e., the patients at risk at any time interval +20 months after surgery are shown: Low risk: 106, 105, 99, 93, 80, 60, 55, 30, 6, 3, 0; High risk: 80, 74, 66, 51, 37, 24, 18, 8, 1, 1, 0.

Fig. 5 shows a Kaplan-Meier curve of the PDE4D7_CORR&pGGG_model in a 186 patient cohort (training set used to develop the PDE4D7_CORR&pGGG_model) with all patients undergoing SRT (salvage radiation treatment) after post-surgical BCR (biochemical recurrence). The clinical endpoint that was tested was the prostate cancer specific death (PCa Death) after the start of salvage radiation therapy (SRT) due to post-surgical disease recurrence (logrank p<0.0001; HR=12.1; 95% CI=4.9-30.2). The following supplementary lists indicate the number of patients at risk for the PDE4D7_CORR&pGGG_model classes analyzed, i.e., the patients at risk at any time interval +20 months after surgery are shown: Low risk: 123, 123, 117, 109, 94, 72, 65, 34, 6, 3, 0; High risk: 63, 56, 48, 35, 23, 12, 8, 4, 1, 1, 0.

Fig. 6 shows a Kaplan-Meier curve of the PDE4D7_CORR_model in a 151 patient cohort (testing set used to validate the PDE4D7_CORR_model as developed on the 186 patient training set) with all patients undergoing SRT (salvage radiation treatment) after post-surgical BCR (biochemical recurrence). The clinical endpoint that was tested was the prostate cancer specific death (PCa Death) after the start of salvage radiation therapy (SRT) due to post-surgical disease recurrence (logrank p=0.002; HR=5.2; 95% CI=1.9-14.3). The following supplementary lists indicate the number of patients at risk for the PDE4D7_CORR_model classes analyzed, i.e., the patients at risk at any time interval +20 months after surgery are shown: Low risk: 81, 81, 80, 80, 69, 51, 35, 23, 14, 9, 6, 3, 1, 0; High risk: 70, 68, 65, 61, 51, 39, 31, 20, 12, 9, 6, 5, 2, 0.

Fig. 7 shows a Kaplan-Meier curve of the PDE4D7_CORR&pGGG_model in a 151 patient cohort (testing set used to validate the PDE4D7_CORR&pGGG model as developed on the 186 patient training set) with all patients undergoing SRT (salvage radiation treatment) after post-surgical BCR (biochemical recurrence). The clinical endpoint that was tested was the prostate cancer specific death (PCa Death) after the start of salvage radiation therapy (SRT) due to post-surgical disease recurrence (logrank p=0.02; HR=3.5; 95% CI=1.3-9.8). The following supplementary lists indicate the number of patients at risk for the PDE4D7_CORR&pGGG_model classes analyzed, i.e., the patients at risk at any time interval +20 months after surgery are shown: Low risk: 84, 83, 83, 82, 70, 54, 37, 27, 17, 14, 9, 8, 3, 0; High risk: 67, 66, 62, 59, 50, 36, 29, 16, 9, 4, 3, 0, 0, 0.

Fig. 8 shows a Kaplan-Meier curve of the PDE4D7_CORR_model in a 151 patient cohort (testing set used to validate the PDE4D7_CORR_model as developed on the 186 patient training set) with all patients undergoing SRT (salvage radiation treatment) after post-surgical BCR (biochemical recurrence). The clinical endpoint that was tested was the death after the start of salvage radiation therapy (SRT) due to post-surgical disease recurrence (logrank p=0.002; HR=3.0; 95% CI=1.5-5.8). The following supplementary lists indicate the number of patients at risk for the PDE4D7_CORR_model classes analyzed, i.e., the patients at risk at any time interval +20 months after surgery are shown: Low risk: 81, 81, 80, 80, 69, 51, 35, 23, 14, 9, 6, 3, 1, 0; High risk: 70, 68, 65, 61, 51, 39, 31, 20, 12, 9, 6, 5, 2, 0.

Fig. 9 shows a Kaplan-Meier curve of the PDE4D7_CORR&pGGG_model in a 151 patient cohort (testing set used to validate the PDE4D7_CORR&pGGG model as developed on the 186 patient training set) with all patients undergoing SRT (salvage radiation treatment) after post-surgical BCR (biochemical recurrence). The clinical endpoint that was tested was the death after the start of salvage radiation therapy (SRT) due to post-surgical disease recurrence (logrank p=0.0008; HR=3.4; 95% CI=1.7-7.0). The following supplementary lists indicate the number of patients at risk for the PDE4D7_CORR&pGGG_model classes analyzed, i.e., the patients at risk at any time interval +20 months after surgery are shown: Low risk: 84, 83, 83, 82, 70, 54, 37, 27, 17, 14, 9, 8, 3, 0; High risk: 67, 66, 62, 59, 50, 36, 29, 16, 9, 4, 3, 0, 0, 0.

The Kaplan-Meier survival curve analysis as shown in Figs. 4 to 9 demonstrates the presence of different patient risk groups. The risk group of a patient is determined by the probability to suffer from the respective clinical endpoint (death, prostate cancer specific death) as calculated by the risk model PDE4D7_CORR_model or PDE4D7_CORR&pGGG_model. Depending on the predicted risk of a patient (i.e., depending on in which risk group the patient may belong) to die from prostate cancer different types of interventions might be indicated. In the lower risk groups standard of care (SOC), which is SRT potentially combined with SADT (salvage androgen deprivation therapy), delivers acceptable long-term oncological control. For the patient group demonstrating a higher risk to eventually suffer from prostate cancer death dose escalation of the applied RT and/or combination with chemotherapy might provide improved longitudinal survival outcomes. Alternative options for treatment escalation are early combination of SRT (considering higher dose regimens), SADT, and chemotherapy or alternative therapies like immunotherapies (e.g., Sipuleucil-T) or other experimental therapies.

**Discussion**

The effectiveness of both radical RT and SRT for localized prostate cancer is limited, resulting in disease progression and ultimately death of patients, especially for those at high risk of recurrence. The prediction of the therapy outcome is very complicated as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the response to radical RT and to SRT, in order to increase the success rate of these therapies.

We have identified molecules of which expression shows a significant relation to mortality after radical RT and SRT and therefore are expected to improve the prediction of the effectiveness of these treatments. An improved prediction of effectiveness of RT for each patient be it in the radical or the salvage setting, will improve therapy selection and potentially survival. This can be achieved by 1) optimizing RT for those patients where RT is predicted to be effective (e.g. by dose escalation or a different starting time) and 2) guiding patients where RT is predicted not to be effective to an alternative, potentially more effective form of treatment. Further, this would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The invention relates to a method of predicting a response of a prostate cancer subject to radiotherapy, comprising determining or receiving the result of a determination of a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said gene expression profile(s) being determined in a biological sample obtained from the subject, determining the prediction of the radiotherapy response based on the gene expression profile(s) for the one or more PDE4D7 correlated genes, and optionally, providing the prediction or a therapy recommendation based on the prediction to a medical caregiver or the subject. Since the status of the immune system and of the immune microenvironment have an impact on therapy effectiveness, the ability to identify markers predictive for this effect might help to be better able to predict overall RT response. The identified genes were found to exhibit a significant correlation with outcome after RT, wherefore we expect that they will provide predictive value with regard to the effectiveness of radical RT and/or SRT.

**The attached Sequence Listing, entitled 2020PF00491_Sequence Listing_ST25 is incorporated herein by reference, in its entirety.**

**Claims**

1. A method of predicting a response of a prostate cancer subject to radiotherapy, comprising:

   - determining or receiving the result of a determination of a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said gene expression profile(s) being determined in a biological sample obtained from the subject,
   - determining the prediction of the radiotherapy response based on the gene expression profile(s) for the one or more PDE4D7 correlated genes, and
   - optionally, providing the prediction or a therapy recommendation based on the prediction to a medical caregiver or the subject.

2. The method as defined in claim 1, wherein the one or more PDE4D7 correlated genes comprise three or more, preferably, six or more, most preferably, all of the PDE4D7 correlated genes.

3. The method as defined in claim 1 or 2, wherein the determining of the prediction of the therapy response comprises combining the gene expression profiles for two or more, for example, 2, 3, 4, 5, 6, 7 or all, of the PDE4D7 correlated genes with a regression function that had been derived from a population of prostate cancer subjects.

4. The method as defined in any of claims 1 to 3, wherein the determining of the prediction of the radiotherapy response is further based on one or more clinical parameters obtained from the subject.

5. The method as defined in claim 4, wherein the clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

6. The method as defined in claim 4 or 5, wherein the determining of the prediction of the radiotherapy response comprises combining the gene expression profile(s) for the one or more PDE4D7 correlated genes and the one or more clinical parameters obtained from the subject with a regression function that had been derived from a population of prostate cancer subjects.

7. The method as defined in any of claims 1 to 6, wherein the biological sample is obtained from the subject before the start of the radiotherapy.

**8.** The method as defined in any of claims 1 to 7, wherein the radiotherapy is radical radiotherapy or salvage radiotherapy.

**9.** The method as defined in any of claims 1 to 8, wherein the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

**10.** An apparatus for predicting a response of a prostate cancer subject to radiotherapy, comprising:

- an input adapted to receive data indicative of a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said gene expression profile(s) being determined in a biological sample obtained from the subject,
- a processor adapted to determine the prediction of the radiotherapy response based on the gene expression profile(s) for the one or more PDE4D7 correlated genes, and
- optionally, a providing unit adapted to provide the prediction or a therapy recommendation based on the prediction to a medical caregiver or the subject.

**11.** A computer program product comprising instructions which, when the program is executed by a computer, cause the computer to carry out a method comprising:

- receiving data indicative of a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, said gene expression profile(s) being determined in a biological sample obtained from a prostate cancer subject,
- determining a prediction of a response of a prostate cancer subject to therapy based on the gene expression profile(s) for the one or more PDE4D7 correlated genes, and
- optionally, providing the prediction or a therapy recommendation based on the prediction to a medical caregiver or the subject.

**12.** A diagnostic kit, comprising:

- at least one primer and/or probe for determining the gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a biological sample obtained from the subject, and
- optionally, an apparatus as defined in claim 10 or a computer program product as defined in claim 11.

**13.** Use of the kit as defined in claim 12.

**14.** The use as defined in claim 13 in a method of predicting a response of a prostate cancer subject to radiotherapy.

**15.** A method, comprising:

- receiving a biological sample obtained from a prostate cancer subject,
- using the kit as defined in claim 12 to determine a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in the biological sample obtained from the subject.

**16.** Use of a gene expression profile for each of one or more, for example, 1, 2, 3, 4, 5, 6, 7 or all, PDE4D7 correlated genes selected from the group consisting of: ABCC5, CUX2, KIAA1549, PDE4D, RAP1GAP2, SLC39A11, TDRD1, and VWA2, in a method of predicting a response of a prostate cancer subject to radiotherapy, comprising:

- determining the prediction of the radiotherapy response based on the gene expression profile(s) for the one or more PDE4D7 correlated genes, and
- optionally, providing the prediction or a therapy recommendation based on the prediction to a medical caregiver or the subject.

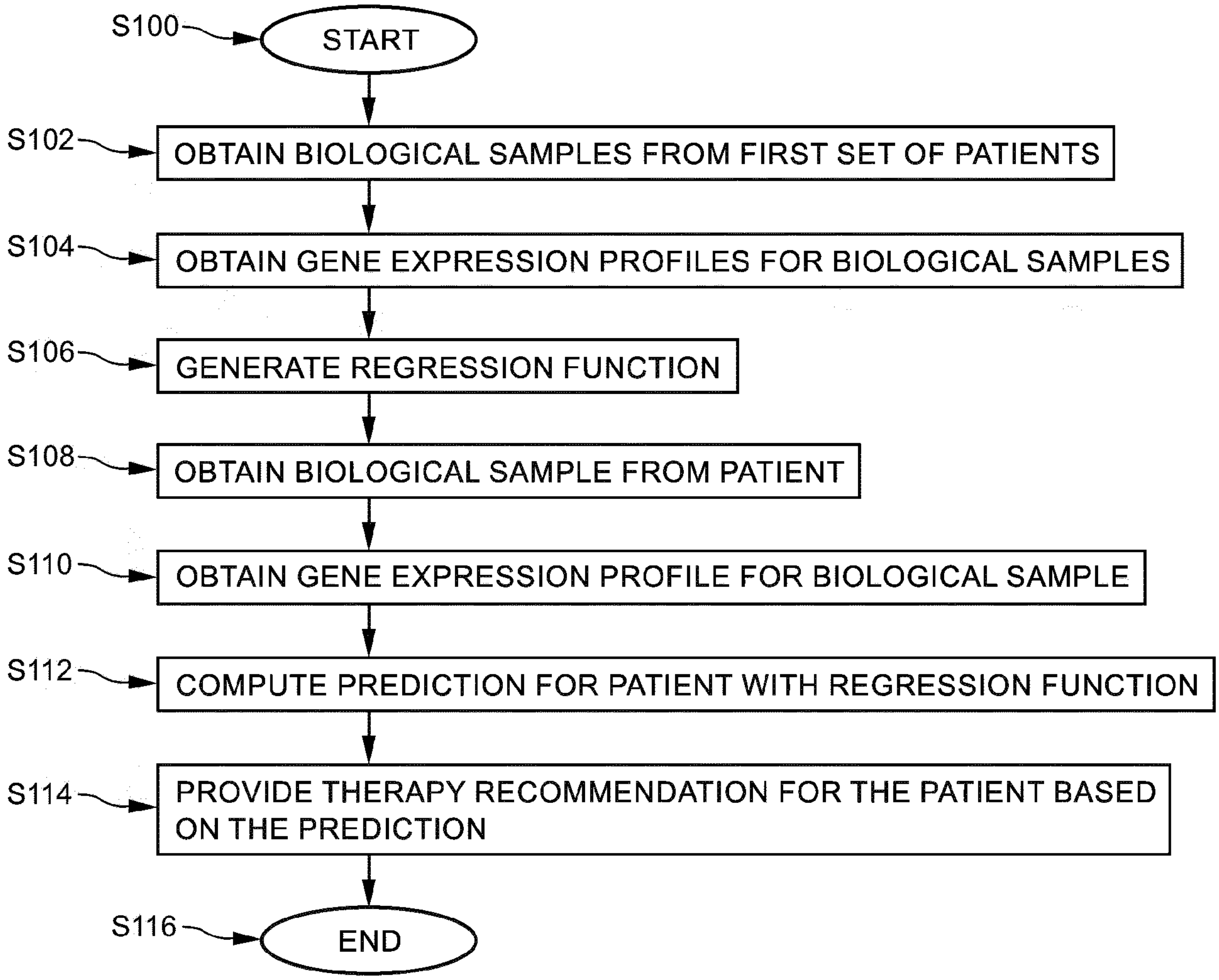
S100
START
S102
OBTAIN BIOLOGICAL SAMPLES FROM FIRST SET OF PATIENTS
S104
OBTAIN GENE EXPRESSION PROFILES FOR BIOLOGICAL SAMPLES
S106
GENERATE REGRESSION FUNCTION
S108
OBTAIN BIOLOGICAL SAMPLE FROM PATIENT
S110
OBTAIN GENE EXPRESSION PROFILE FOR BIOLOGICAL SAMPLE
S112
COMPUTE PREDICTION FOR PATIENT WITH REGRESSION FUNCTION
S114
PROVIDE THERAPY RECOMMENDATION FOR THE PATIENT BASED ON THE PREDICTION
S116
END

FIG. 1

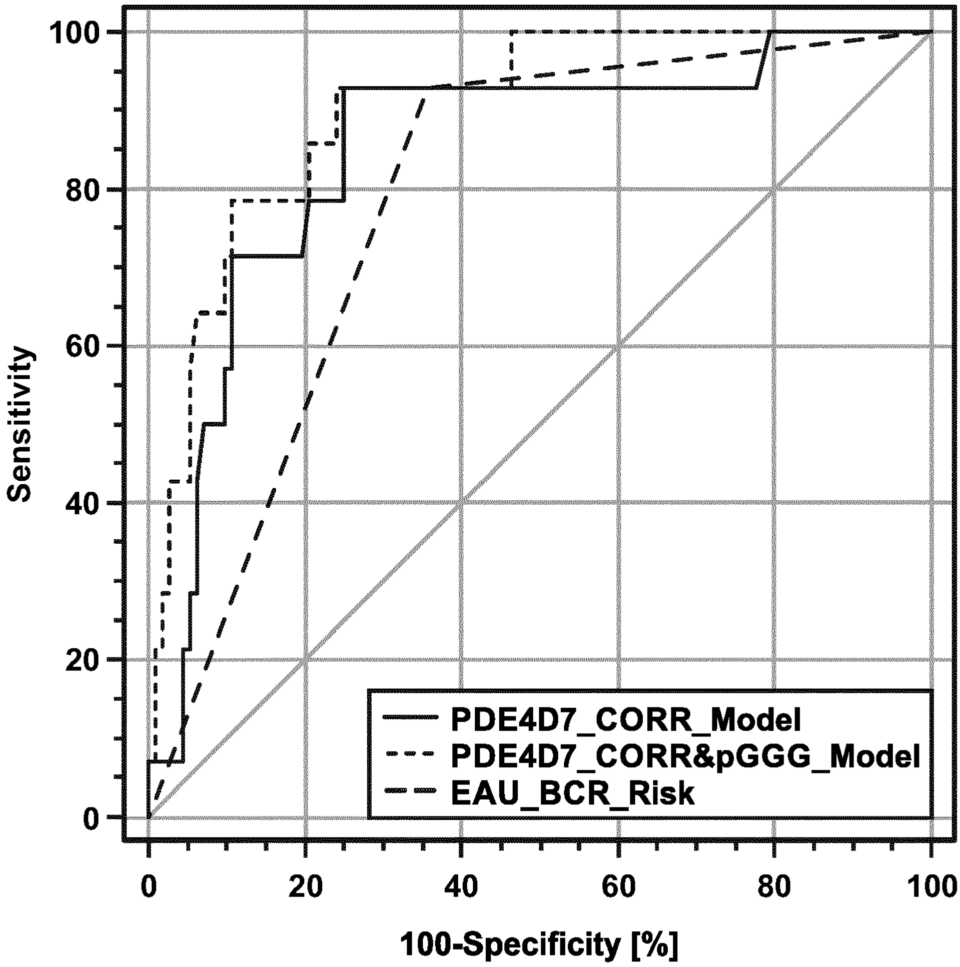
100
80
60
40
20
0
Sensitivity
0
20
40
60
80
100
100-Specificity [%]
PDE4D7_CORR_Model
PDE4D7_CORR&pGGG_Model
EAU_BCR_Risk

FIG. 2

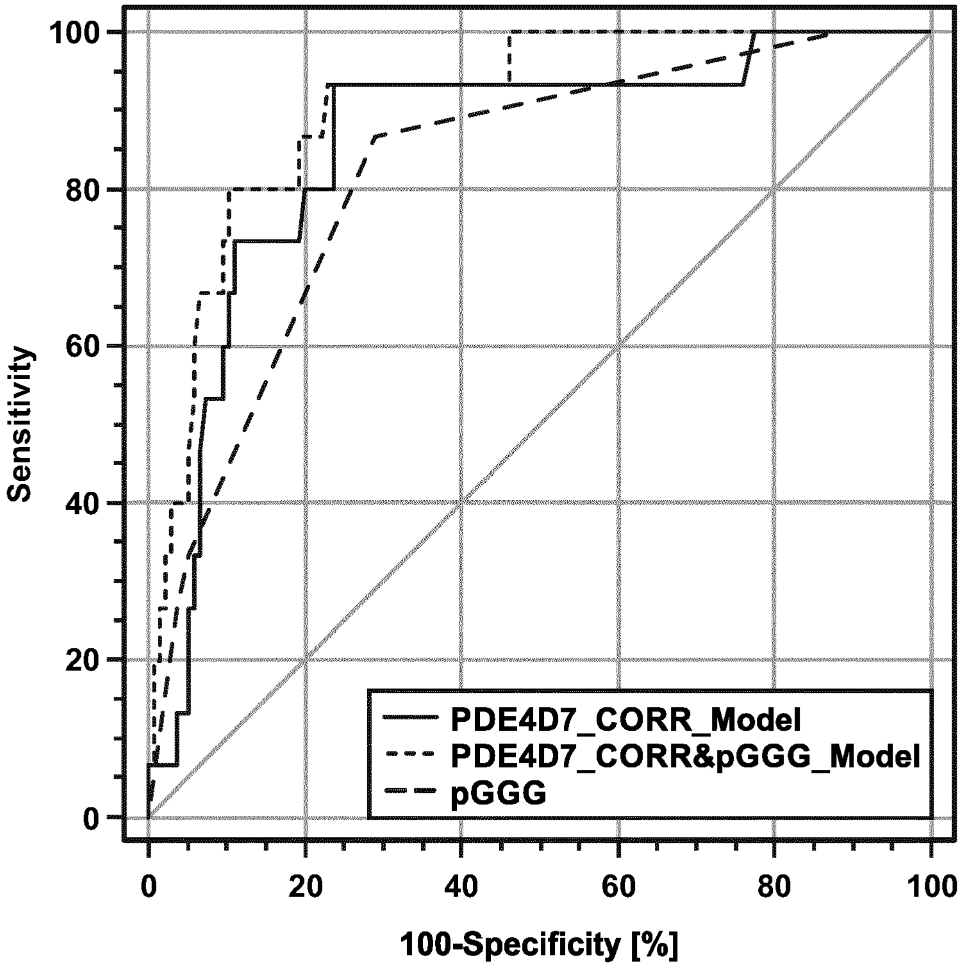
100
80
60
40
20
0
Sensitivity
0
20
40
60
80
100
100-Specificity [%]
PDE4D7_CORR_Model
PDE4D7_CORR&pGGG_Model
pGGG

FIG. 3

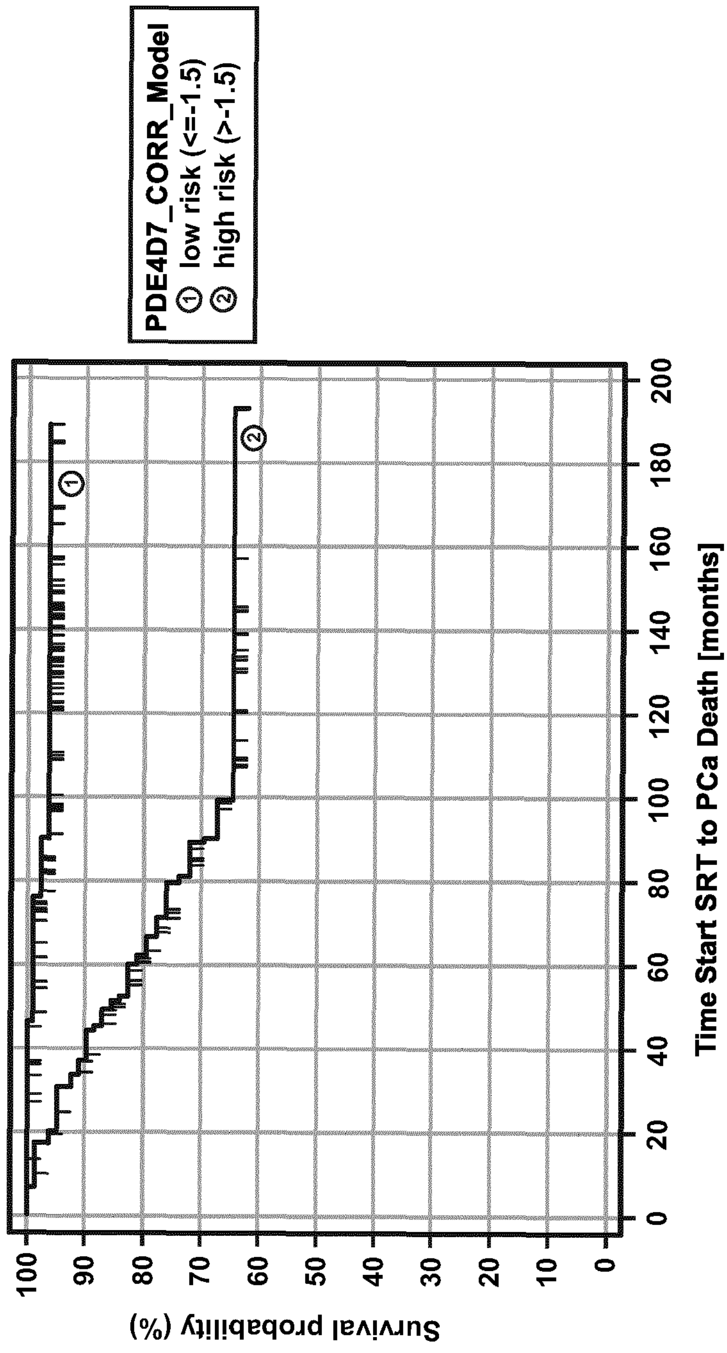
PDE4D7_CORR_Model
① low risk (<=-1.5)
② high risk (>-1.5)
Survival probability (%)
100
90
80
70
60
50
40
30
20
10
0
0
20
40
60
80
100
120
140
160
180
200
Time Start SRT to PCa Death [months]

FIG. 4

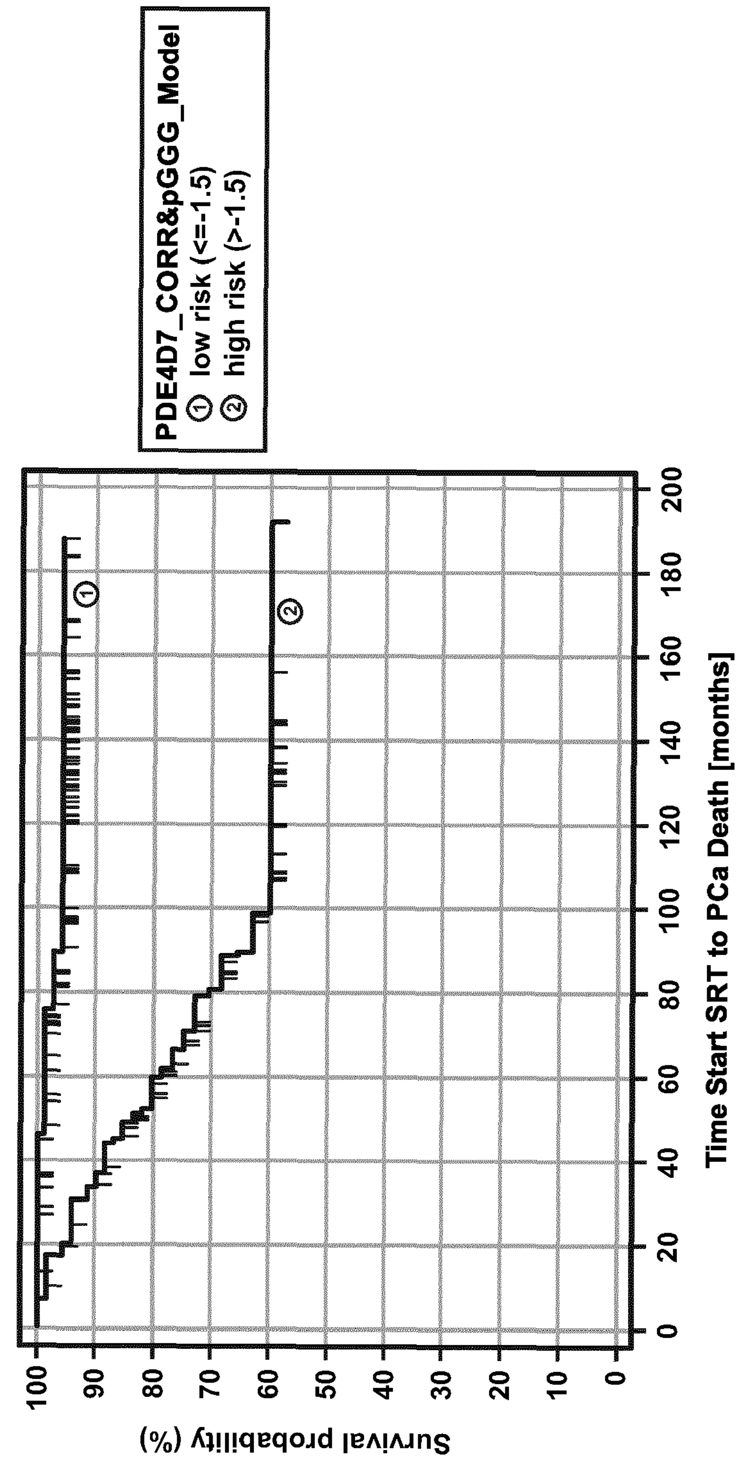
PDE4D7_CORR&pGGG_Model
① low risk (<=-1.5)
② high risk (>-1.5)
Survival probability (%)
100
90
80
70
60
50
40
30
20
10
0
0
20
40
60
80
100
120
140
160
180
200
Time Start SRT to PCa Death [months]
①
②

FIG. 5

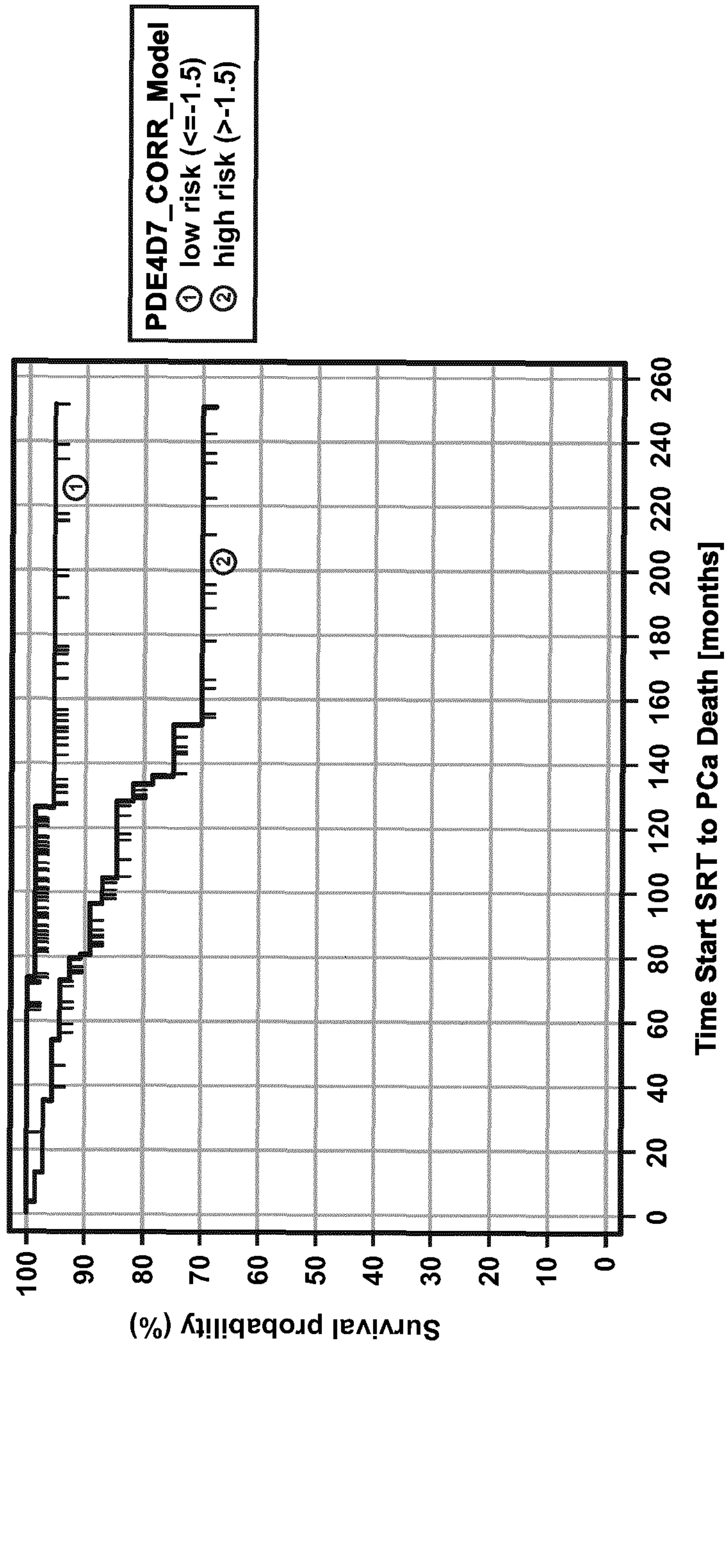
PDE4D7_CORR_Model
① low risk (<=-1.5)
② high risk (>-1.5)
Survival probability (%)
100
90
80
70
60
50
40
30
20
10
0
0
20
40
60
80
100
120
140
160
180
200
220
240
260
Time Start SRT to PCa Death [months]
①
②

FIG. 6

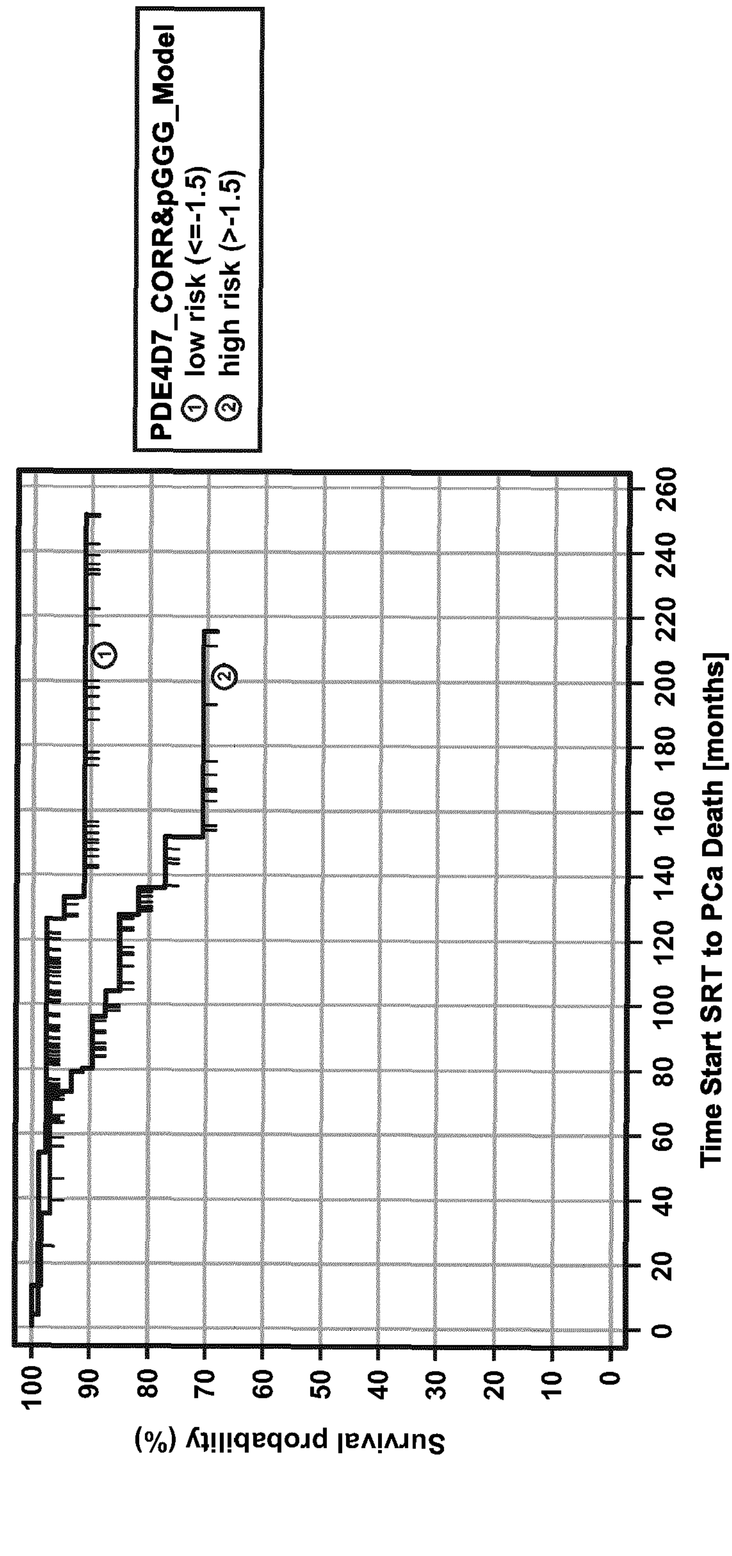
PDE4D7_CORR&pGGG_Model
① low risk (<=-1.5)
② high risk (>-1.5)
Survival probability (%)
100
90
80
70
60
50
40
30
20
10
0
0
20
40
60
80
100
120
140
160
180
200
220
240
260
Time Start SRT to PCa Death [months]
①
②

FIG. 7

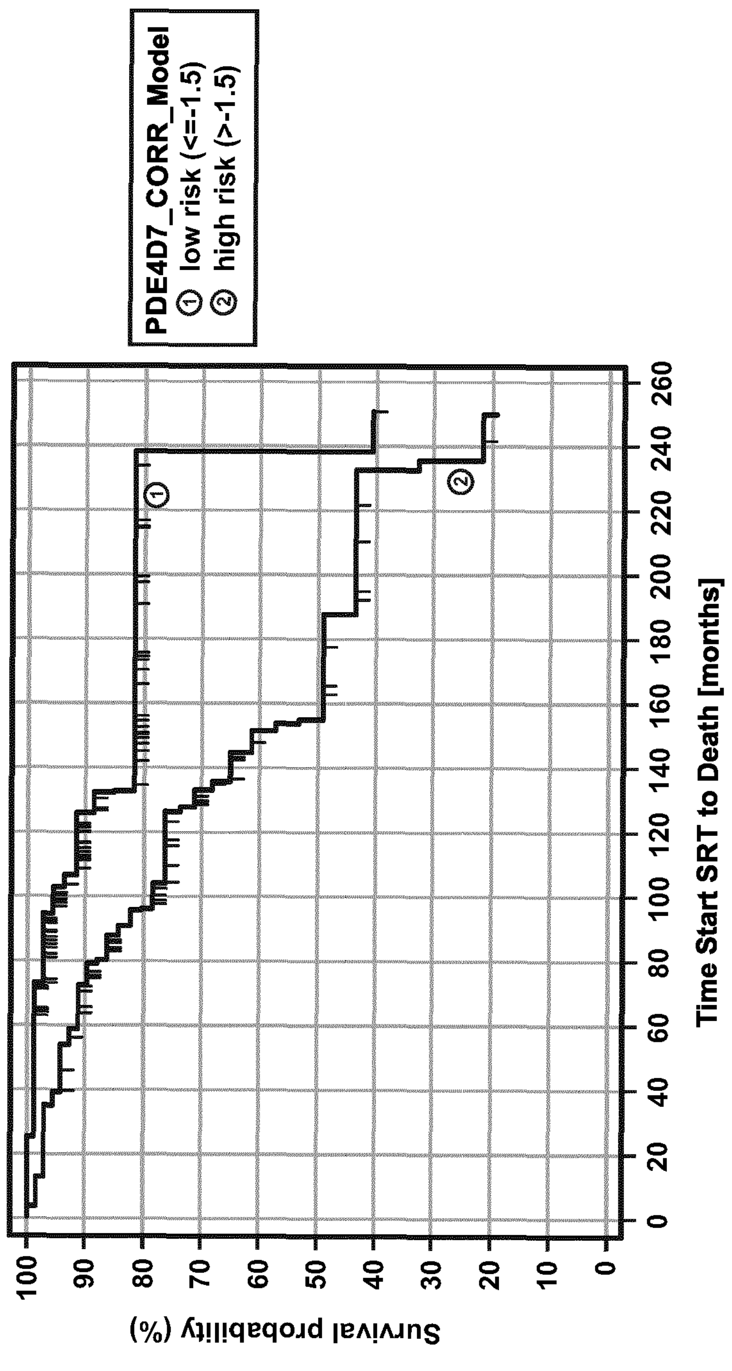
PDE4D7_CORR_Model
① low risk (<=-1.5)
② high risk (>-1.5)
Survival probability (%)
100
90
80
70
60
50
40
30
20
10
0
Time Start SRT to Death [months]
0
20
40
60
80
100
120
140
160
180
200
220
240
260
①
②

FIG. 8

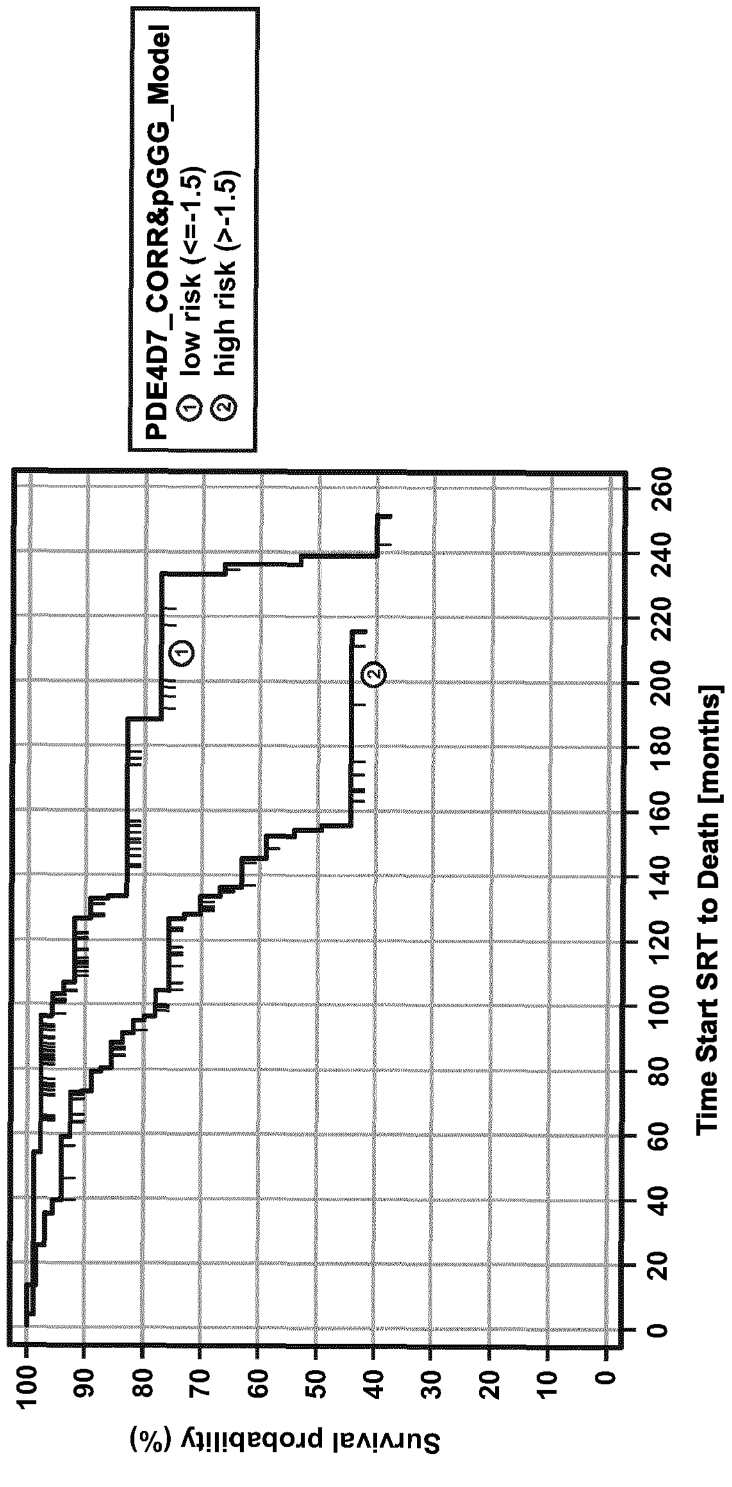
PDE4D7_CORR&pGGG_Model
① low risk (<=-1.5)
② high risk (>-1.5)
Survival probability (%)
100
90
80
70
60
50
40
30
20
10
0
0
20
40
60
80
100
120
140
160
180
200
220
240
260
Time Start SRT to Death [months]
①
②

FIG. 9

Europäisches Patentamt
European Patent Office
Office européen des brevets

# EUROPEAN SEARCH REPORT

Application Number
EP 20 19 3628

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "Affymetrix GeneChip Human Genome U133 Array Set HGU133A", NCBI, GEO, Platform GPL96, 11 March 2002 (2002-03-11), pages 1-511, XP055546924, Retrieved from the Internet: URL:https://www.ncbi.nlm.nih.gov/geo/query/acc.cgi?acc=GPL96 [retrieved on 2019-01-24] * the whole document * | 12,13,15 | INV. C12Q1 |
| X,D | HALL WILLIAM A ET AL: "Biomarkers of Outcome in Patients With Localized Prostate Cancer Treated With Radiotherapy", SEMINARS IN RADIATION ONCOLOGY, ELSEVIER, AMSTERDAM, NL, vol. 27, no. 1, 16 September 2016 (2016-09-16), pages 11-20, XP029847419, ISSN: 1053-4296, DOI: 10.1016/J.SEMRADONC.2016.09.001 * the whole document * | 1-11,14 | |
| A | WO 2019/122037 A1 (KONINKLIJKE PHILIPS NV [NL]) 27 June 2019 (2019-06-27) | 1-16 | TECHNICAL FIELDS SEARCHED (IPC)<br>C12Q |
| A | WO 2015/087088 A2 (ALMAC DIAGNOSTICS LTD [GB]) 18 June 2015 (2015-06-18) | 1-16 | |
| A | WO 2012/158533 A1 (ROSENSTEIN BARRY S [US]; OSTRER HARRY [US] ET AL.) 22 November 2012 (2012-11-22) | 1-16 | |

The present search report has been drawn up for all claims

2

| Place of search | Date of completion of the search | Examiner |
|---|---|---|
| The Hague | 19 February 2021 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 19 3628

This annex lists the patent family members relating to the patent documents cited in the above-mentioned European search report. The members are as contained in the European Patent Office EDP file on
The European Patent Office is in no way liable for these particulars which are merely given for the purpose of information.

19-02-2021

| Patent document cited in search report | Publication date | Patent family member(s) | Publication date |
|---|---|---|---|
| WO 2019122037 A1 | 27-06-2019 | CN 111742061 A | 02-10-2020 |
| | | EP 3502280 A1 | 26-06-2019 |
| | | EP 3728640 A1 | 28-10-2020 |
| | | US 2020308654 A1 | 01-10-2020 |
| | | WO 2019122037 A1 | 27-06-2019 |
| WO 2015087088 A2 | 18-06-2015 | AU 2014363173 A1 | 07-07-2016 |
| | | BR 112016013182 A2 | 26-09-2017 |
| | | CA 2932553 A1 | 18-06-2015 |
| | | CN 105940117 A | 14-09-2016 |
| | | EP 3080293 A2 | 19-10-2016 |
| | | JP 2017507320 A | 16-03-2017 |
| | | KR 20160098351 A | 18-08-2016 |
| | | SG 11201604508P A | 28-07-2016 |
| | | US 2016312294 A1 | 27-10-2016 |
| | | WO 2015087088 A2 | 18-06-2015 |
| WO 2012158533 A1 | 22-11-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BRAY F. et al.** Global cancer statistics 2018: GLOBOCAN estimates of incidence and mortality worldwide for 36 cancers in 185 countries. *CA Cancer J Clin,* 2018, vol. 68 (6), 394-424
- Cancer Facts & Figures 2010. ACS (American Cancer Society), 2010
- **GRIMM P. et al.** Comparative analysis of prostate-specific antigen free survival outcomes for patients with low, intermediate and high risk prostate cancer treatment by radical therapy. Results from the Prostate Cancer Results Study Group. *BJU Int,* 2012, 22-29
- **DAL PRA A. et al.** Contemporary role of postoperative radiotherapy for prostate cancer. *Transl Androl Urol,* 2018, vol. 7 (3), 399-413
- **HERRERA F.G. ; BERTHOLD D.R.** Radiation therapy after radical prostatectomy: Implications for clinicians. *Front Oncol,* 2016, vol. 6 (117
- **PISANSKY T.M. et al.** Salvage radiation therapy dose response for biochemical failure of prostate cancer after prostatectomy - A multi-institutional observational study. *Int J Radiat Oncol Biol Phys,* 2016, vol. 96 (5), 1046-1053
- **RESNICK M.J. et al.** Long-term functional outcomes after treatment for localized prostate cancer. *N Engl J Med,* 2013, vol. 368 (5), 436-445
- **HEGARTY S.E. et al.** Radiation therapy after radical prostatectomy for prostate cancer: Evaluation of complications and influence of radiation timing on outcomes in a large, population-based cohort. *PLoS One,* 2015, vol. 10 (2
- **PARAVATI A.J. et al.** Variation in the cost of radiation therapy among medicare patients with cancer. *J Oncol Pract,* 2015, vol. 11 (5), 403-409
- **HALL W.A. et al.** Biomarkers of outcome in patients with localized prostate cancer treated with radiotherapy. *Semin Radiat Oncol,* 2016, vol. 27, 11-20
- **RAYMOND E. et al.** An appraisal of analytical tools used in predicting clinical outcomes following radiation therapy treatment of men with prostate cancer: A systematic review. *Radiat Oncol,* 2017, vol. 12 (1), 56
- **HENDERSON D.J. et al.** The cAMP phosphodiesterase-4D7 (PDE4D7) is downregulated in androgen-independent prostate cancer cells and mediates proliferation by compartmentalizing cAMP at the plasma membrane of VCaP prostate cancer cells. *Br J Cancer,* 2014, vol. 110 (5), 1278-1287
- **ALVES DE INDA M. et al.** Validation of Cyclic Adenosine Monophosphate Phosphodiesterase-4D7 for its Independent Contribution to Risk Stratification in a Prostate Cancer Patient Cohort with Longitudinal Biological Outcomes. *Eur Urol Focus,* 2018, vol. 4 (3), 376-384
- **BÖTTCHER R. et al.** Human phosphodiesterase 4D7 (PDE4D7) expression is increased in TMPRSS2-ERG-positive primary prostate cancer and independently adds to a reduced risk of post-surgical disease progression. *Br J Cancer,* 2015, vol. 113 (10), 1502-1511
- **BÖTTCHER R. et al.** Human PDE4D isoform composition is deregulated in primary prostate cancer and indicative for disease progression and development of distant metastases. *Oncotarget,* 2016, vol. 7 (43), 70669-70684
- **VAN STRIJP D. et al.** The Prognostic PDE4D7 Score in a Diagnostic Biopsy Prostate Cancer Patient Cohort with Longitudinal Biological Outcomes. *Prostate Cancer,* 2018, 5821616
- **TILKI D. et al.** External validation of the European Association of Urology Biochemical Recurrence Risk groups to predict metastasis and mortality after radical prostatectomy in a European cohort. *Eur Urol,* 2019, vol. 75 (6), 896-900